(19) Europäisches Patentamt
European Patent Office
Office européen des brevets

(11) EP 2 585 600 B1

(12) # EUROPEAN PATENT SPECIFICATION

(45) Date of publication and mention of the grant of the patent:
**03.02.2016 Bulletin 2016/05**

(21) Application number: **11799019.2**

(22) Date of filing: **24.06.2011**

(51) Int Cl.:
*C12N 15/53* (2006.01) *C12N 9/02* (2006.01)
*C12N 1/19* (2006.01) *A01H 5/00* (2006.01)
*A01H 5/10* (2006.01) *C12P 7/64* (2006.01)
*C12N 15/82* (2006.01)

(86) International application number:
**PCT/US2011/041897**

(87) International publication number:
**WO 2011/163632 (29.12.2011 Gazette 2011/52)**

(54) **LOWERING SATURATED FATTY ACID CONTENT OF PLANT SEEDS**

VERRINGERUNG DES GEHALTS AN GESÄTTIGTEN FETTSÄUREN VON PFLANZENSAMEN

DIMINUTION DE LA TENEUR EN ACIDE GRAS SATURÉ DE GRAINES DE PLANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2010 US 358314 P**

(43) Date of publication of application:
**01.05.2013 Bulletin 2013/18**

(60) Divisional application:
**14191879.7 / 2 862 930**
**14191880.5 / 2 862 931**

(73) Proprietor: **Dow AgroSciences LLC**
**Indianapolis, IN 46268 (US)**

(72) Inventors:
- **MERLO, Ann Owens**
  **Carmel**
  **IN 46032 (US)**
- **GACHOTTE, Daniel, J**
  **Indianapolis**
  **IN 46260 (US)**
- **THOMPSON, Mark, A.**
  **Zionsville**
  **IN 46077 (US)**
- **WALSH, Terence, A.**
  **Zionsville**
  **IN 46077 (US)**
- **BEVAN, Scott**
  **Indianapolis**
  **IN 46280 (US)**

(74) Representative: **f & e patent**
**Fleischer, Engels & Partner mbB, Patentanwälte**
**Braunsberger Feld 29**
**51429 Bergisch Gladbach (DE)**

(56) References cited:
**WO-A1-2007/131720** **WO-A2-99/50430**
**WO-A2-02/090493** **WO-A2-2006/076423**
**US-A1- 2010 092 640**

- **ROSENFIELD CLAIRE-LISE ET AL: "Structural and functional conservation and divergence among acyl-CoA desaturases of two noctuid species, the corn earworm, Helicoverpa zea, and the cabbage looper, Trichoplusia ni", 1 September 2001 (2001-09-01), INSECT BIOCHEMISTRY AND MOLECULAR BIOLOGY, ELSEVIER SCIENCE LTD, GB, PAGE(S) 949 - 964, XP002341750, ISSN: 0965-1748 * figure 2 * * paragraph [2.1.] * * paragraph [2.6.] * -& DATABASE Genbank [Online] 31 August 2001 (2001-08-31), XP002716083, Database accession no. AF272343 -& DATABASE UniProt [Online] 18 May 2010 (2010-05-18), XP002716082, Database accession no. Q9NB25**
- **DATABASE NCBI 17 May 2010 'Magnaporthe oryzae 70-15 conserved hypothetical protein (MGG_01790) partial mRNA' Database accession no. XM_363864**
- **RALPH A. DEAN ET AL.: 'The genome sequence of the rice blast fungus Magnaporthe grisea' NATURE vol. 434, 21 April 2005, pages 980 - 986**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## TECHNICAL FIELD

Some embodiments generally relate to a certain delta-9 desaturase enzymes, nucleic acids encoding this enzymes, and methods of expressing the same in a plant cell. Some embodiments relate to utilizing the activity of a certain delta-9 desaturase enzymes to decrease the percent composition of saturated fatty acids in plant materials (*e.g.*, seed) and increasing the percent composition of ω-7 fatty acids. Also disclosed herein are plants and plant materials produced by methods in particular embodiments.

## BACKGROUND

Vegetable-derived oils have gradually replaced animal-derived oils and fats as the major source of dietary fat intake. However, saturated fat intake in most industrialized nations has remained at about 15% to 20% of total caloric consumption. In efforts to promote healthier lifestyles, the United States Department of Agriculture (USDA) has recently recommended that saturated fats make up less than 10% of daily caloric intake. To facilitate consumer awareness, current labeling guidelines issued by the USDA now require total saturated fatty acid levels be less than 1.0 g per 14 g serving to receive the "low-sat" label and less than 0.5 g per 14 g serving to receive the "no-sat" label. This means that the saturated fatty acid content of plant oils needs to be less than 7% and 3.5% to receive the "low-sat" or "no-sat" label, respectively. Since issuance of these guidelines, there has been a surge in consumer demand for "low-sat" and "no-sat" oils. To date, this demand has been met principally with canola oil, and to a much lesser degree with sunflower and safflower oils.

While unsaturated fats (monounsaturated and polyunsaturated) are beneficial (especially when consumed in moderation), saturated and trans fats are not. Saturated fat and trans fat raise undesirable LDL cholesterol levels in the blood. Dietary cholesterol also raises LDL cholesterol and may contribute to heart disease even without raising LDL. Therefore, it is advisable to choose foods low in saturated fat, trans fat, and cholesterol as part of a healthful diet.

The characteristics of oils, whether of plant or animal origin, are determined predominately by the number of carbon and hydrogen atoms in the oil molecule, as well as the number and position of double bonds comprised in the fatty acid chain. Most oils derived from plants are composed of varying amounts of palmitic (16:0), stearic (18:0), oleic (18:1), linoleic (18:2) and linolenic (18:3) fatty acids. Conventionally, palmitic and stearic acids are designated as "saturated," because their carbon chains are saturated with hydrogen atoms, and hence have no double bonds; they contain the maximal number of hydrogen atoms possible. However, oleic, linoleic, and linolenic acids are 18-carbon fatty acid chains having one, two, and three double bonds, respectively, therein. Oleic acid is typically considered a monounsaturated fatty acid, whereas linoleic and linolenic are considered to be polyunsaturated fatty acids. The U.S.D.A. definition of "no sat" oil products as those having less than 3.5% fatty acid content is calculated as the combined saturated fatty acid content by weight (as compared to the total amount of fatty acids).

Canola oil has the lowest level of saturated fatty acids of all vegetable oils. "Canola" refers to rapeseed (*Brassica*) which has an erucic acid (C22:1) content of at most 2% by weight, based on the total fatty acid content of a seed (preferably at most 0.5% by weight, and most preferably essentially 0% by weight), and which produces, after crushing, an air-dried meal containing less than 30 μmol/g of defatted (oil-free) meal. These types of rapeseed are distinguished by their edibility in comparison to more traditional varieties of the species.

It is postulated that, in oilseeds, fatty acid synthesis occurs primarily in the plastid. The major product of fatty acid synthesis is palmitate (16:0), which appears to be efficiently elongated to stearate (18:0). While still in the plastid, the saturated fatty acids may then be desaturated by an enzyme known as acyl-ACP delta-9 desaturase, to introduce one or more carbon-carbon double bonds. Specifically, stearate may be rapidly desaturated by a plastidial delta-9 desaturase enzyme to yield oleate (18:1). In fact, palmitate may also be desaturated to palmitoleate (16:1) by the plastidial delta-9 desaturase, but this fatty acid appears in only trace quantities (0-0.2%) in most vegetable oils. Thus, the major products of fatty acid synthesis in the plastid are palmitate, stearate, and oleate. In most oils, oleate is the major fatty acid synthesized, as the saturated fatty acids are present in much lower proportions.

Newly-synthesized fatty acids are exported from the plastid to the cytoplasm. Subsequent desaturation of plant fatty acids in the cytoplasm appears to be limited to oleate, which may be desaturated to linoleate (18:2) and linolenate (18:3) by microsomal desaturases acting on oleoyl or lineoleoyl substrates esterified to phosphatidyl choline (PC). In addition, depending on the plant, oleate may be further modified by elongation (to 20:1, 22:1, and/or 24:1), or by the addition of functional groups. These fatty acids, along with the saturated fatty acids, palmitate and stearate, are then assembled into triglycerides in endoreticular membranes.

The plant acyl-ACP delta-9 desaturase enzyme is soluble. It is located in the plastid stroma, and uses newly-synthesized fatty acids esterified to ACP, predominantly stearyl-ACP, as substrates. This is in contrast to the other delta-9 desaturase enzymes, which are located in the endoplasmic reticular membrane (ER, or microsomal), use fatty acids esterified to Co-A as substrates, and desaturate both the saturated fatty acids, palmitate and stearate. U.S. Patents 5,723,595 and 6,706,950 relate to a plant desaturase.

The yeast delta-9 desaturase gene has been isolated from *Saccharomyces cerevisiae,* cloned, and sequenced. Stukey et al. (1989) J. Biol. Chem. 264:16537-44; Stukey et al. (1990) J. Biol. Chem. 265:20144-9. This yeast gene has been introduced into tobacco leaf tissue (Polashcok et al. (1991) FASEB J. 5:A1157; Polashok et al. (1992) Plant Physiol. 100:894-901), and was apparently expressed in this tissue. Further, this yeast gene was expressed in tomato. *See* Wang et al. (1996) J. Agric. Food Chem. 44:3399-402; and Wang et al. (2001) Phytochemistry 58:227-32. While some increases in certain unsaturated fatty acids, and some decreases in certain saturated fatty acids, were reported for both tobacco and tomato using this yeast delta-9 desaturase gene, tobacco and tomato are clearly not oil crops. This yeast gene was also introduced into *Brassica napus.* U.S. Patent 5,777,201.

A different fungal acyl-CoA delta-9 desaturase from *Aspergillus nidulans* has been introduced into canola, thereby achieving reduced saturated fatty acid levels in seed oil. U.S. Patent Application Publication US 2008/0260933 A1. The *A. nidulans* acyl-CoA delta-9 desaturase provided greater depletion of stearate (61-90%) than the more abundant palmitate fatty acids (36-49%) in the seed oil.

## DISCLOSURE OF THE INVENTION

Disclosed herein is an isolated nucleic acid molecule encoding a delta-9 desaturase enzyme comprising an amino acid sequence being at least 80% identical to SEQ ID NO:12, wherein the nucleic acid molecule comprises a nucleotide sequence at least 60% identical to a sequence selected from the group consisting of SEQ ID NO:3, SEQ ID NO:4, SEQ ID NO:9, and SEQ ID NO:15.

Also disclosed are methods of expressing at least one of the aforementioned nucleic acids and/or polypeptides in a plant cell. Particular embodiments take advantage of a delta-9 desaturase enzyme's activity, such that the percent composition of saturated fatty acids may be decreased in a plant, plant material (*e.g.*, seed), and/or plant part comprising the plant cell, and/or a plant commodity product produced from any of the foregoing. In certain embodiments, $\omega$-7 fatty acids may concomitantly be increased in the plant, plant material, plant part, and/or plant commodity product.

Some embodiments include a method for decreasing the amount of saturated fatty acids in a plant, plant material, plant part, and/or plant commodity product, the method comprising transforming a plant cell with a nucleic acid molecule encoding a delta-9 desaturase polypeptide of the invention, such that the amount of saturated fatty acids in the cell is decreased. Some embodiments include a method for creating a genetically engineered plant that comprises decreased amounts of saturated fatty acids in the plant compared to a wild-type plant of the same species. Such a method may comprise transforming a plant material (or plant cell) with a nucleic acid molecule encoding a delta-9 desaturase polypeptide of the invention, and culturing the transformed plant material (or plant cell) to obtain a plant. In particular examples, a plant cell and/or plant material from an *Arabidopsis* sp. may be transformed with a nucleic acid molecule encoding a delta-9 desaturase polypeptide of the invention.

The foregoing and other features will become more apparent from the following detailed description of several embodiments, which proceeds with reference to the accompanying figures.

## BRIEF DESCRIPTION OF THE DRAWINGS

**FIG. 1** includes a schematic phylogenetic analysis of various fungal desaturase protein sequences. The complete protein sequences of the depicted desaturases were aligned using ClustalX and displayed using MEGA.

**FIGs. 2(a-d)** include an alignment of fungal delta-9 desaturase gene sequences. Capital font represents conserved nucleotides in this alignment. Shaded font represents identical nucleotides in this alignment.

**FIGs. 3(a-b)** include an alignment of fungal delta-9 desaturase polypeptides.

**FIGs. 4-18** include plasmid maps of exemplary plasmids comprising fungal delta-9 desaturase polypeptide-encoding nucleotide sequences that may be useful in some embodiments. **FIG. 4** specifically includes plasmid maps of exemplary plasmids comprising LnD9DS-2-encoding (**FIG. 4a;** pDAB110110) and HzD9DS-encoding (**FIG. 4b;** pDAB110112) nucleotide sequences that further comprise the PvPhas 5' UTR and PvPhas 3' UTR.

**FIG. 19** includes data showing the total saturated fatty acid content (%FAMEs) of exemplary $T_2$ *Arabidopsis* seed from plants transformed with certain exemplary fungal delta-9 desaturase gene sequences.

**FIG. 20** includes data showing the palmitic acid (C16:0) content (%FAMEs) of exemplary $T_2$ *Arabidopsis* seed from plants transformed with certain exemplary fungal delta-9 desaturase gene sequences.

**FIG. 21** includes data showing the stearic acid (C18:0) content (%FAMEs) of exemplary $T_2$ *Arabidopsis* seed from plants transformed with certain exemplary fungal delta-9 desaturase gene sequences.

**FIG. 22** includes data showing the palmitoleic acid (C16:1) content (%FAMEs) of exemplary $T_2$ *Arabidopsis* seed

from plants transformed with certain exemplary fungal delta-9 desaturase gene sequences.

**FIG. 23** includes a graphical representation of the accumulation of HzD9DS and LnD9DS-2 mRNA transcripts (relative to AnD9DS transcripts) in developing seeds from canola plants transformed with pDAB7319 (AnD9DS v3 and LnD9DS-2 v2) or pDAB7324 (AnD9DS v3 and HzD9DS v2). The qRT-PCR ΔΔCt of each gene was determined relative to the actin transcript level, and the amount of transcript for HzD9DS and LnD9DS-2 then normalized to the level of AnD9DS transcript in each sample.

**SEQUENCE LISTING**

The nucleic acid sequences listed in the accompanying sequence listing are shown using standard letter abbreviations for nucleotide bases, as defined in 37 C.F.R. § 1.822. Only one strand of each nucleic acid sequence is shown, but the complementary strand is understood to be included by any reference to the displayed strand. In the accompanying sequence listing:

SEQ ID NO: 1 shows a forward primer used to PCR amplify a fragment of a *Magnaporthe grisea* acyl-CoA delta-9 desaturase gene (referred to in some places as MgD9DS).

SEQ ID NO:2 shows a reverse primer used to PCR amplify a fragment of a *M. grisea* acyl-CoA delta-9 desaturase gene (referred to in some places as MgD9DS).

SEQ ID NO:3 shows an exemplary fragment of a *M. grisea* acyl-CoA delta-9 desaturase gene (referred to in some places as MgD9DS) that was amplified by PCR.

SEQ ID NO:4 shows an exemplary intronless MgD9DS clone.

SEQ ID NO:5 shows an exemplary nucleic acid sequence encoding a first *Leptosphaeria nodorum* acyl-CoA delta-9 desaturase, referred to in some places as LnD9DS-1.

SEQ ID NOs:6 and 7 show primer sequences that may be useful in some embodiments..

SEQ ID NO:8 shows an exemplary nucleic acid sequence encoding a second exemplary *L. nodorum* acyl-CoA delta-9 desaturase, referred to in some places as LnD9DS-2.

SEQ ID NO:9 shows a coding region from an exemplary native delta-9 desaturase gene from *M. grisea* (labeled as MgD9DS v1).

SEQ ID NO:10 shows a coding region from an exemplary native delta-9 desaturase gene from *Helicoverpa zea* (labeled as HzD9DS v1).

SEQ ID NO:11 shows a coding region from an exemplary native delta-9 desaturase (LnD9DS-2 v1) gene from *L. nodorum.*

SEQ ID NO:12 shows the amino acid sequence of an exemplary native delta-9 desaturase from *M. grisea* (MgD9DS).

SEQ ID NO:13 shows the amino acid sequence of an exemplary native delta-9 desaturase from *H. zea* (HzD9DS).

SEQ ID NO:14 shows the amino acid sequence of an exemplary native delta-9 desaturase from *L. nodorum* (LnD9DS-2).

SEQ ID NO:15 shows the sequence of an exemplary canola-optimized delta-9 desaturase gene from *M. grisea* (MgD9DS v2).

SEQ ID NO:16 shows the sequence of an exemplary canola-optimized delta-9 desaturase gene from *H. zea* (HzD9DS v2).

SEQ ID NO:17 shows the sequence of an exemplary canola-optimized delta-9 desaturase gene from *L. nodorum* (LnD9DS-2 v2).

SEQ ID NOs:18-39 show the sequence of primers and probes that may be useful in some embodiments.

SEQ ID NOs:40-43 show exemplary alternative Kozak sequences that may be used to increase expression in some embodiments.

SEQ ID NO:44 shows the sequence of a further exemplary canola-optimized delta-9 desaturase gene from *L. nodorum* (LnD9DS-2 v3).

SEQ ID NO:45 shows the sequence of a further exemplary canola-optimized delta-9 desaturase gene from *H. zea* (HzD9DS v3).

SEQ ID NO:46 shows the amino acid sequence of a Myc tag.

SEQ ID NO:47 shows the amino acid sequence of a HA tag.

SEQ ID NO:48 shows an exemplary nucleic acid sequence encoding an *Aspergillus nidulans* delta-9 desaturase, referred to in some places as AnD9DS v2.

SEQ ID NO:49 shows a second exemplary nucleic acid sequence encoding an *A. nidulans* delta-9 desaturase, referred to in some places as AnD9DS v3.

SEQ ID NO:50 shows the amino acid sequence encoded by nucleic acids as exemplified by SEQ ID NOs:48-49 (AnD9DS).

SEQ ID NO:51 shows the amino acid sequence of another exemplary AnD9DS desaturase.

SEQ ID NO:52 shows the amino acid sequence of an exemplary native delta-9 desaturase (ScOLE1) from *Saccharomyces cerevisiae.*

SEQ ID NOs:53-66 show plasmids that may be useful in some embodiments.

SEQ ID NOs:67-71 include several nucleic acid regulatory control elements that may be useful in some embodiments.

SEQ ID NO:72 shows the N-terminal 68 residues (1-68) of an exemplary AnD9DS desaturase.

SEQ ID NO:73 shows the C-terminal 175 residues (281-455) of an exemplary AnD9DS desaturase.

SEQ ID NO:74 shows a map of plasmid pDAB110110.

SEQ ID NO:75 shows a map of plasmid pDAB110112.

SEQ ID NO:76 shows an exemplary nucleic acid sequence encoding an exemplary *M. grisea* acyl-CoA delta-9 desaturase, referred to in some places as MgD9DS.

SEQ ID NO:77 shows an amino acid sequence comprised within the exemplary native delta-9 desaturase from *L. nodorum* of SEQ ID NO:14.

SEQ ID NO:78 shows an amino acid sequence comprised within the exemplary native delta-9 desaturase from *H. zea* of SEQ ID NO:13.

## MODE(S) FOR CARRYING OUT THE INVENTION

### *I. Overview of several embodiments*

We previously introduced a fungal acyl-CoA delta-9 desaturase from *Aspergillus nidulans* into canola, thereby achieving reduced saturated fatty acid levels in seed oil. U.S. Patent Application Publication US 2008/0260933 A1. The *A. nidulans* delta-9 desaturase provided greater depletion of stearate (61-90%) than the more abundant palmitate fatty acids (36-49%) in the seed oil. Therefore, co-introduction of a delta-9 desaturase that acts preferentially on palmitate saturates will achieve further reductions in total saturates by complementing the stearate-preferring activity of the *A. nidulans* delta-9 desaturase. In some embodiments of the present invention, fungal delta-9 desaturase polypeptides having a range of substrate specificities are disclosed. Particular embodiments include a palmitate-preferring delta-9 desaturase (*e.g.*, a native fungal enzyme as disclosed herein, or a functional equivalent thereof; and a synthetic polypeptide designed to have a preference for a palmitic acid substrate).

Disclosed herein are nucleic acid molecules encoding a delta-9 desaturase polypeptide comprising a nucleotide sequence being at least 60% identical to a sequence selected from the group consisting of SEQ ID NO:3, SEQ ID NO:4, SEQ ID NO:5, SEQ ID NO:8, SEQ ID NO:9, SEQ ID NO:10, SEQ ID NO:11, SEQ ID NO:15, SEQ ID NO:16, SEQ ID NO:17, SEQ ID NO:44, SEQ ID NO:45, SEQ ID NO:48, and SEQ ID NO:49. In some embodiments, the nucleic acid molecule may further comprises a gene regulatory element operably linked to the delta-9 desaturase polypeptide-encoding sequence. In particular embodiments, a gene regulatory element may be a phaseolin promoter, a phaseolin 5' untranslated region, a phaseolin 3' untranslated region, an *Agrobacterium tumefaciens* ORF1 3' untranslated region, a Cassava vein Mosaic Virus promoter, a *Nicotiana tabacum* RB7 Matrix Attachment Region, a T-strand border sequence, a LfKCS3 promoter, and FAE 1 promoter.

Also disclosed are delta-9 desaturase polypeptides comprising an amino acid sequence being at least 80% identical to a sequence selected from the group consisting of SEQ ID NO:12, SEQ ID NO:13, SEQ ID NO:14, SEQ ID NO:50, SEQ ID NO:52, SEQ ID NO:72, and SEQ ID NO:73, as well as nucleic acid molecules encoding such delta-9 desaturase polypeptides.

In some embodiments, nucleic acid molecules and delta-9 desaturase polypeptides may be expressed in a plant material, cell, tissue, or whole plant, to decrease the amount of saturated fatty acids in the plant material, cells, tissues, or whole plants, relative to the amount observed in a wild-type plant of the same species. Alternative embodiments of the invention include methods for decreasing the amount of saturated fatty acids in the plant material, cell, tissue, or whole plant. Such methods may comprise transforming a plant material, cell, tissue, or whole plant with at least one of the aforementioned nucleic acid molecules, such that the amount of saturated fatty acids in the plant material, cell, tissue, or whole plant is decreased. Particular embodiments include methods for preferentially decreasing palmitic and/or stearic fatty acids in a plant material, cell, tissue, or whole plant.

Methods disclosed herein may be performed, for example, on plants, or plant materials derived from plants (*e.g.*, plants of the genus *Arabidopsis,* or canola). A particular embodiment is drawn to methods for creating or regenerating a genetically engineered plant comprising decreased amounts of saturated fatty acids in the plant compared to a wild-type plant of the same species, the method comprising transforming a plant cell or material with at least one of the aforementioned nucleic acid molecules; and culturing the transformed plant material to obtain a plant. Plants, plant materials, plant cells, and seeds obtained by any of the aforementioned methods are also disclosed.

### II. Abbreviations

| | |
|---|---|
| x:yΔ$^z$ | fatty acid containing x carbons and y double bonds in position z counting from the carboxyl end |
| ACP | acyl carrier protein |
| CoA | coenzyme A |
| FA | fatty acids |
| FAM | fluorescein |
| FAS | fatty acid synthase |
| FAME | fatty acid methyl ester |
| KASII | β-ketoacyl-ACP synthase II |
| MUFA | monounsaturated fatty acid |
| WT | wild type |

### III. Terms

Fatty acid: As used herein, the term "fatty acid" refers to long chain aliphatic acids (alkanoic acids) of varying chain lengths, for example, from about C12 to C22, although both longer and shorter chain-length acids are known. The structure of a fatty acid is represented by the notation, x:yΔ$^z$, where "x" is the total number of carbon (C) atoms in the particular fatty acid, and "y" is the number of double bonds in the carbon chain in the position "z," as counted from the carboxyl end of the acid.

Metabolic pathway: The term, "metabolic pathway," refers to a series of chemical reactions occurring within a cell, catalyzed by enzymes, to achieve either the formation of a metabolic product, or the initiation of another metabolic pathway. A metabolic pathway may involve several or many steps, and may compete with a different metabolic pathway for specific reaction substrates. Similarly, the product of one metabolic pathway may be a substrate for yet another metabolic pathway.

Metabolic engineering: For the purposes of the present invention, "metabolic engineering" refers to the rational design of strategies to alter one or more metabolic pathways in a cell, such that the step-by-step modification of an initial substance into a product having the exact chemical structure desired is achieved within the overall scheme of the total metabolic pathways operative in the cell.

Desaturase: As used herein, the term "desaturase" refers to a polypeptide that can desaturate *(i.e.,* introduce a double bond) in one or more fatty acids to produce a fatty acid or precursor of interest. A plant-soluble fatty acid desaturase enzyme may introduce a double bond regiospecifically into a saturated acyl-ACP substrate. Acyl-CoA desaturases introduce a double bond regiospecifically into a saturated fatty acyl-CoA substrate. The reaction involves activation of molecular oxygen by a two-electron reduced diiron center coordinated by a four-helix bundle that forms the core of the desaturase architecture. Of particular interest in some embodiments are acyl-CoA delta-9 desaturases.

The delta-9-18:0$^1$-ACP desaturase is required by all plants for the maintenance of membrane fluidity. While this enzyme primarily desaturates stearoyl-ACP, it is also active to a minor extent with palmitoyl-ACP.

Variant desaturase: As used herein, the term "variant desaturase" encompasses those desaturases that exhibit specific activity profiles consistent with a role in producing unusual fatty acids. A variant desaturase may be isolated from an organism, engineered *via* a directed evolution program, or engineered as a synthetic desaturase incorporating conserved amino acids from one or more characterized desaturase.

Progeny plant: For the purposes of the present invention, "progeny plant," refers to any plant, or plant material obtained therefrom, that may be obtained by plant breeding methods. Plant breeding methods are well-known in the art, and include natural breeding, artificial breeding, selective breeding involving DNA molecular marker analysis, transgenics, and commercial breeding.

Plant material: As used herein, the term "plant material" refers to any cell or tissue obtained from a plant.

Nucleic acid molecule: A polymeric form of nucleotides, which can include both sense and anti-sense strands of RNA, cDNA, genomic DNA, and synthetic forms and mixed polymers of the above. A nucleotide refers to a ribonucleotide, deoxynucleotide, or a modified form of either type of nucleotide. A "nucleic acid molecule" as used herein is synonymous with "nucleic acid" and "polynucleotide." The term includes single- and double-stranded forms of DNA. A nucleic acid molecule can include either or both naturally occurring and modified nucleotides linked together by naturally occurring and/or non-naturally occurring nucleotide linkages.

Nucleic acid molecules can be modified chemically or biochemically, or can contain non-natural or derivatized nucleotide bases, as will be readily appreciated by those of ordinary skill in the art. Such modification include, for example, labels, methylation, substitution of one or more of the naturally occurring nucleotides with an analog, internucleotide modifications, such as uncharged linkages (for example, methyl phosphonates, phosphotriesters, phosphoramidates, carbamates, etc.), charged linkages (for example, phosphorothioates, phosphorodithioates, etc.), pendent moieties (for example, peptides), intercalators (for example, acridine, psoralen, etc.), chelators, alkylators, and modified linkages (for example, alpha anomeric nucleic acids, etc.). The term "nucleic acid molecule" also includes any topological conformation, including single-stranded, double-stranded, partially duplexed, triplexed, hairpinned, circular and padlocked conformations.

Operably linked: A first nucleic acid sequence is operably linked with a second nucleic acid sequence when the first nucleic acid sequence is in a functional relationship with the second nucleic acid sequence. For instance, a promoter is operably linked to a coding sequence if the promoter affects the transcription or expression of the coding sequence. When recombinantly produced, operably linked nucleic acid sequences are generally contiguous and, where necessary to join two protein-coding regions, in the same reading frame. However, nucleic acids need not be contiguous to be operably linked.

Regulatory element: As used herein, the term "regulatory element" refers to a nucleic acid molecule having gene regulatory activity; *i.e.*, one that has the ability to affect the transcription or translation of an operably-linked transcribable nucleic acid molecule. Regulatory elements such as promoters, leaders, introns, and transcription termination regions are non-coding nucleic acid molecules having gene regulatory activity which play an integral part in the overall expression of genes in living cells. Isolated regulatory elements that function in plants are therefore useful for modifying plant phenotypes through the techniques of molecular engineering. By "regulatory element," it is intended a series of nucleotides that determines if, when, and at what level a particular gene is expressed. The regulatory DNA sequences specifically interact with regulatory proteins or other proteins.

As used herein, the term "gene regulatory activity" refers to a nucleic acid molecule capable of affecting transcription or translation of an operably linked nucleic acid molecule. An isolated nucleic acid molecule having gene regulatory activity may provide temporal or spatial expression or modulate levels and rates of expression of the operably linked nucleic acid molecule. An isolated nucleic acid molecule having gene regulatory activity may comprise a promoter, intron, leader, or 3' transcriptional termination region.

Promoters: As used herein, the term "promoter" refers to a nucleic acid molecule that is involved in recognition and binding of RNA polymerase II or other proteins such as transcription factors (trans-acting protein factors that regulate transcription) to initiate transcription of an operably linked gene. Promoters may themselves contain sub-elements such as cis-elements or enhancer domains that effect the transcription of operably linked genes. A "plant promoter" is a native or non-native promoter that is functional in plant cells. A plant promoter can be used as a 5' regulatory element for modulating expression of an operably linked gene or genes. Plant promoters may be defined by their temporal, spatial, or developmental expression pattern. The nucleic acid molecules described herein may comprise nucleic acid sequences comprising promoters.

Sequence identity: The term "sequence identity" or "identity," as used herein in the context of two nucleic acid or polypeptide sequences, may refer to the residues in the two sequences that are the same when aligned for maximum correspondence over a specified comparison window.

When percentage of sequence identity is used in reference to proteins, it is recognized that residue positions which are not identical often differ by conservative amino acid substitutions, where amino acid residues are substituted for other amino acid residues with similar chemical properties (*e.g.*, charge, hydrophobicity, or steric effects), and therefore do not change the functional properties of the molecule.

Therefore, when sequences differ by conservative substitutions, the percent sequence identity may be adjusted upwards to correct for the conservative nature of the substitution at the site of the non-identical residue. Sequences that differ by such conservative substitutions are said to have "sequence similarity" or "similarity." Techniques for making this adjustment are well known to those of ordinary skill in the art. Typically, such techniques involve scoring a conservative substitution as a partial, rather than a full, mismatch, thereby increasing the percentage sequence identity. For example, where an identical amino acid is given a score between 0 and 1, and a non-conservative substitution is given a score of 0, a conservative substitution is given a score between 0 and 1. The scoring of conservative substitutions may be calculated, for example, as implemented in the program PC/GENE (Intelligenetics, Mountain View, CA).

As used herein, the term "percentage of sequence identity" may refer to the value determined by comparing two optimally aligned sequences over a comparison window, wherein the portion of the sequence in the comparison window may comprise additions or deletions *(i.e.,* gaps) as compared to the reference sequence (which does not comprise additions or deletions) for optimal alignment of the two sequences. The percentage is calculated by determining the number of positions at which the identical nucleotide or amino acid residue occurs in both sequences to yield the number of matched positions, dividing the number of matched positions by the total number of positions in the comparison window, and multiplying the result by 100 to yield the percentage of sequence identity.

Analogous position in an amino acid sequence: Nucleic acid and amino acid sequences may be aligned by the methods described in the following paragraphs. When aligned, a position in one sequence is in "an analogous position" with a position in the aligned sequence if the positions are identical within the consensus sequence.

Methods for aligning sequences for comparison are well-known in the art. Various programs and alignment algorithms are described in: Smith and Waterman, Adv. Appl. math. 2:482, 1981; Needleman and Wunsch, J. Mol. Biol. 48:443, 1970; Pearson and Lipman, Proc. Natl. Acad. Sci. USA 85:2444, 1988; Higgins and Sharp, Gene 73:237-44, 1988; Higgins and Sharp, CABIOS 5:151-3, 1989; Corpet et al., Nucleic Acids Research 16:10881-10890, 1988; Huang, et al., Computer Applications in the Biosciences 8:155-65, 1992; Pearson et al., Methods in Molecular Biology 24:307-31, 1994; Tatiana et al., FEMS Microbiol. Lett., 174:247-50, 1990. Altschul et al., J. Mol. Biol. 215:403-10, 1990 (detailed consideration of sequence-alignment methods and homology calculations).

The National Center for Biotechnology Information (NCBI) Basic Local Alignment Search Tool (BLAST) is available on the Internet (at blast.ncbi.nlm.nih.gov/Blast.cgi), for use in connection with sequence-analysis programs, for example, blastp and blastn. A description of how to determine sequence identity using this program is available on the Internet through NCBI at blast.ncbi.nlm.nih.govBlast.cgi?CMD=Web&PAGE_TYPE=BlastDocs.

For comparisons of amino acid sequences, the "Blast 2 sequences" function of the BLAST program (bl2seq) is employed using the default parameters. Specific parameters may be adjusted within the discretion of one of skill in the art, to for example, provide a penalty for a mismatch or reward for a match.

Transformed: As used herein, the term "transformed" refers to a cell, tissue, organ, or organism into which has been introduced a foreign nucleic acid molecule, such as a construct. The introduced nucleic acid molecule may be integrated into the genomic DNA of the recipient cell, tissue, organ, or organism such that the introduced polynucleotide molecule is inherited by subsequent progeny. A "transgenic" or "transformed" cell or organism also includes progeny of the cell or organism and progeny produced from a breeding program employing such a transgenic plant as a parent in, for example, a cross and exhibiting an altered phenotype resulting from the presence of a foreign nucleic acid molecule.

### *IV. Metabolic engineering approaches to decreasing saturated fatty acids in a host cell, tissue, or organism*

A. Overview

The present the invention includes introducing a_delta-9 desaturases with specific acyl-CoA preferences (for example, for palmitic or stearic acid) in plant seeds. The specific acyl-CoA preference of the delta-9 desaturase enables targeting of certain specific saturated fatty acid pools (*e.g.*, palmitate for conversion to monounsaturated products). The acyl-CoA delta-9 desaturases was selected for lowering the saturated fatty acid content in plants as they are not normally produced in plant systems to any appreciable extent.

B. Polypeptides

Polypeptides according to some embodiments of the present invention comprise an amino acid sequence showing increasing percentage identities when aligned with a sequence consisting of SEQ ID NO:12. Specific amino acid sequences within these and other embodiments may comprise sequences having, for example, at least about 70%, about 75%, about 80%, 81%, 82%, 83%, 84%, 85%, 86%, 87%, 88%, 89%, 90%, 91%, 92%, 93%, 94%, 95% 96%, 97%, 98%, 99%, or 100% identity with the aforementioned sequences. In many embodiments, the amino acid sequence having the aforementioned sequence identity when aligned with the aforementioned sequences encode a peptide with enzymatic delta-9-18:0-ACP desaturase activity, or part of a such a peptide.

C. Nucleic acids

Some embodiments include nucleic acid molecules encoding a polypeptide described above. For example, nucleic acid sequences in some embodiments show increasing percentage identities when aligned with a sequence selected from the group consisting of SEQ ID NO:3, SEQ ID NO:4, SEQ ID NO:9, SEQ ID NO:15. Specific nucleic acid sequences within these and other embodiments may comprise sequences having, for example, at least about 60%, about 65%, about 70%, about 75%, about 80%, 81%, 82%, 83%, 84%, 85%, 86%, 87%, 88%, 89%, 90%, 91%, 92%, 93%, 94%, 95% 96%, 97%, 98%, 99%, or 100% identity with a sequence selected from the group consisting of SEQ ID NO:3, SEQ ID NO:4, SEQ ID NO:9, SEQ ID NO:10, SEQ ID NO:11, SEQ ID NO:15. It is understood by those of ordinary skill in the art that nucleic acid molecules may be modified without substantially changing the amino acid sequence of an encoded polypeptide, for example, by introducing permissible nucleotide substitutions according to codon degeneracy.

In some embodiments, nucleic acid molecules of the present invention comprise a gene regulatory element (*e.g.*, a promoter). Promoters may be selected on the basis of the cell type into which the vector construct will be inserted. Promoters which function in bacteria, yeast, and plants are well-known in the art. The promoters may also be selected on the basis of their regulatory features. Examples of such features include enhancement of transcriptional activity, inducibility, tissue-specificity, and developmental stage-specificity. In plants, promoters that are inducible, of viral or synthetic origin, constitutively active, temporally regulated, and spatially regulated have been described. *See, e.g.*, Poszkowski et al. (1989) EMBO J. 3:2719; Odell et al. (1985) Nature 313:810; and Chau et al. (1989) Science 244:174-81).

Useful inducible promoters include, for example, promoters induced by salicylic acid or polyacrylic acids induced by application of safeners (substituted benzenesulfonamide herbicides), heat-shock promoters, a nitrate-inducible promoter derived from the spinach nitrate reductase transcribable nucleic acid molecule sequence, hormone-inducible promoters, and light-inducible promoters associated with the small subunit of RuBP carboxylase and LHCP families.

Examples of useful tissue-specific, developmentally-regulated promoters include the β-conglycinin 7Sα promoter and seed-specific promoters. Plant functional promoters useful for preferential expression in seed plastid include those from proteins involved in fatty acid biosynthesis in oilseeds and from plant storage proteins. Examples of such promoters include the 5' regulatory regions from such transcribable nucleic acid molecule sequences as phaseolin, napin, zein, soybean trypsin inhibitor, ACP, stearoyl-ACP desaturase, and oleosin. Another exemplary tissue-specific promoter is the lectin promoter, which is specific for seed tissue.

Other useful promoters include the nopaline synthase, mannopine synthase, and octopine synthase promoters, which are carried on tumor-inducing plasmids of *Agrobacterium tumefaciens*; the cauliflower mosaic virus (CaMV) 19S and 35S promoters; the enhanced CaMV 35S promoter; the Figwort Mosaic Virus 35S promoter; the light-inducible promoter from the small subunit of ribulose-1,5-bisphosphate carboxylase (ssRUBISCO); the EIF-4A promoter from tobacco (Mandel et al. (1995) Plant Mol. Biol. 29:995-1004); corn sucrose synthetase; corn alcohol dehydrogenase I; corn light harvesting compolex; corn heat shock protein; the chitinase promoter from Arabidopsis; the LTP (Lipid Transfer Protein) promoters; petunia chalcone isomerase; bean glycine rich protein 1; potato patatin; the ubiquitin promoter; and the actin promoter. Useful promoters are preferably seed-selective, tissue selective, or inducible. Seed-specific regulation is discussed in, for example, EP 0 255 378.

To obtain higher expression of a heterologous gene(s), it may be preferred to reengineer the gene(s) so that it is more efficiently expressed in the expression host cell (*e.g.*, a plant cell, for example, canola, rice, tobacco, maize, cotton, and soybean). Therefore, an optional additional step in the design of a gene encoding a delta-9 desaturase for plant expression (*i.e.*, in addition to the provision of one or more gene regulatory elements) is reengineering of a heterologous gene protein coding region for optimal expression. Particular embodiments include redesigned genes that have been optimized to increase the expression level (*i.e.* produce more protein) in a transgenic canola plant cell or *Arabidopsis* plant cell than in a canola plant cell or *Arabidopsis* plant cell transformed with the naturally-occurring heterologous gene sequence.

Due to the plasticity afforded by the redundancy/degeneracy of the genetic code (*i.e.*, some amino acids are specified by more than one codon), evolution of the genomes in different organisms or classes of organisms has resulted in differential usage of synonymous codons. This "codon bias" is reflected in the mean base composition of protein coding regions. For example, organisms having genomes with relatively low G+C contents utilize more codons having A or T in the third position of synonymous codons, whereas those having higher G+C contents utilize more codons having G or C in the third position. Further, it is thought that the presence of "minor" codons within an mRNA may reduce the absolute translation rate of that mRNA, especially when the relative abundance of the charged tRNA corresponding to the minor codon is low. An extension of this reasoning is that the diminution of translation rate by individual minor codons would be at least additive for multiple minor codons. Therefore, mRNAs having high relative contents of minor codons in a particular expression host would have correspondingly low translation rates. This rate may be reflected by correspondingly low levels of the encoded protein.

In engineering optimized genes encoding a delta-9 desaturase for expression in canola or *Arabidopsis* (or other plants, such as rice, tobacco, maize, cotton or soybean), it is helpful if the codon bias of the prospective host plant(s) has been determined. Multiple publicly-available DNA sequence databases exist wherein one may find information about the codon distribution of plant genomes or the protein coding regions of various plant genes.

The codon bias is the statistical distribution of codons that the expression host (*e.g.*, a plant such as canola or *Arabidopsis*) uses for coding the amino acids of its proteins. The codon bias can be calculated as the frequency at which a single codon is used relative to the codons for all amino acids. Alternatively, the codon bias may be calculated as the frequency at which a single codon is used to encode a particular amino acid, relative to all the other codons for that amino acid (synonomous codons).

In designing optimized coding regions for plant expression of delta-9 desaturase genes, the primary ("first choice") codons preferred by the plant should be determined, as well as the second, third, fourth *etc.* choices of preferred codons when multiple choices exist. A new DNA sequence can then be designed which encodes the amino sequence of the delta-9 desaturase gene, wherein the new DNA sequence differs from the native DNA sequence (encoding the desaturase) by the substitution of expression host-preferred (first preferred, second preferred, third preferred, or fourth preferred, *etc.*) codons to specify the amino acid at each position within the amino acid sequence. The new sequence is then analyzed for restriction enzyme sites that might have been created by the modifications. The identified putative restriction sites are further modified by replacing these codons with a next-preferred codon to remove the restriction site. Other sites in the sequence which may affect transcription or translation of heterologous sequence are exon:intron junctions (5' or 3'), poly-A addition signals, and/or RNA polymerase termination signals. The sequence may be further analyzed and modified to reduce the frequency of TA or CG doublets. In addition to these doublets, sequence blocks that have more than about six G or C nucleotides that are the same may also adversely affect transcription or translation of the sequence. Therefore, these blocks are advantageously modified by replacing the codons of first or second choice, *etc.* with the next-preferred codon of choice.

The method described above enables one skilled in the art to modify gene(s) that are foreign to a particular plant so that the genes are optimally expressed in plants. The method is further illustrated in PCT application WO 97/13402. Thus, optimized synthetic genes that are functionally equivalent to desaturases/genes of some embodiments may be used to transform hosts, including plants. Additional guidance regarding the production of synthetic genes can be found in, for example, U.S. Patent 5,380,831.

Once a plant-optimized DNA sequence has been designed on paper or *in silico*, actual DNA molecules can be synthesized in the laboratory to correspond in sequence precisely to the designed sequence. Such synthetic DNA molecules may be cloned and otherwise manipulated exactly as if they were derived from natural or native sources.

D. Methods for genetic transformation of plant material

Some embodiments are directed to a method of producing a transformed cell that comprises one or more nucleic acid molecule(s) comprising a nucleic acid sequence at least 60% identical to a sequence selected from the group consisting of SEQ ID NO:3, SEQ ID NO:4, SEQ ID NO:9. Such nucleic acid molecules may also comprise, for example, non-coding regulatory elements, such as promoters. Other sequences may also be introduced into the cell along with the non-coding regulatory elements and transcribable nucleic acid molecule sequences. These other sequences may include 3' transcriptional terminators, 3' poly-adenylation signals, other untranslated sequences, transit or targeting sequences, selectable markers, enhancers, and operators.

A method of transformation generally comprises the steps of selecting a suitable host cell, transforming the host cell with a recombinant vector, and obtaining the transformed host cell. Technology for introduction of DNA into cells is well-known to those of skill in the art. These methods can generally be classified into five categories: (1) chemical methods (Graham and Van der Eb (1973) Virology 54(2):536-9; Zatloukal et al. (1992) Ann. N.Y. Acad. Sci. 660:136-53); (2) physical methods such as microinjection (Capechi (1980) Cell 22(2):479-88), electroporation (Wong and Neumann (1982) Biochim. Biophys. Res. Commun. 107(2):584-7; Fromm et al. (1985) Proc. Natl. Acad. Sci. USA 82(17):5824-8; U.S. Patent 5,384,253), and particle acceleration (Johnston and Tang (1994) Methods Cell Biol. 43(A):353-65; Fynan et al. (1993) Proc. Natl. Acad. Sci. USA 90(24):11478-82; (3) viral vectors (Clapp (1993) Clin. Perinatol. 20(1):155-68; Lu et al. (1993) J. Exp. Med. 178(6):2089-96; Eglitis and Anderson (1988) Biotechniques 6(7):608-14); (4) receptor-mediated mechanisms (Curiel et al. (1992) Hum. Gen. Ther. 3(2):147-54; Wagner et al. (1992) Proc. Natl. Acad. Sci. USA 89(13):6099-103); and (5) bacterial-mediated mechanisms, such as with *Agrobacterium.* Alternatively, nucleic acids may be directly introduced into pollen by directly injecting a plant's reproductive organs. Zhou et al. (1983) Methods in Enzymology 101:433; Hess (1987) Intern. Rev. Cytol. 107:367; Luo et al. (1988) Plant Mol. Biol. Reporter 6:165; Pena et al. (1987) Nature 325:274. Other transformation methods include, for example, protoplast transformation as illustrated in U.S. Patent 5,508,184. Nucleic acid molecules may also be injected into immature embryos. Neuhaus et al. (1987) Theor. Appl. Genet. 75:30.

The most commonly used methods for transformation of plant cells are: the *Agrobacterium*-mediated DNA transfer process (Fraley et al. (1983) Proc. Natl. Acad. Sci. USA 80:4803) (as illustrated in U.S. Patent 5,824,877; U.S. Patent 5,591,616; U.S. Patent 5,981,840; and U.S. Patent 6,384,301) and the biolistics or microprojectile bombardment-mediated process *(i.e.,* the gene gun) (such as described in U.S. Patent 5,550,318; U.S. Patent 5,538,880; U.S. Patent 6,160,208; U.S. Patent 6,399,861; and U.S. Patent 6,403,865). Typically, nuclear transformation is desired, but where it is desirable to specifically transform plastids, such as chloroplasts or amyloplasts, plant plastids may be transformed utilizing a microprojectile-mediated delivery of the desired nucleic acid molecule in certain plant species, such as for example, *Arabidopsis,* tobacco, potato, and *Brassica* species.

*Agrobacterium*-mediated transformation is achieved through the use of a genetically engineered soil bacterium belonging to the genus *Agrobacterium.* Several *Agrobacterium* species mediate the transfer of a specific DNA known as "T-DNA," which can be genetically engineered to carry any desired piece of DNA into many plant species. The major events marking the process of T-DNA mediated pathogensis are: induction of virulence genes, and processing and transfer of T-DNA. This process is the subject of many reviews. *See, e.g.*, Ream (1989) Ann. Rev. Phytopathol. 27:583-618; Howard and Citovsky (1990) Bioassays 12:103-8; Kado (1991) Crit. Rev. Plant Sci. 10:1-32; Zambryski (1992) Annual Rev. Plant Physiol. Plant Mol. Biol. 43:465-90; Gelvin (1993) in Transgenic Plants, Kung and Wu eds., Academic Press, San Diego, CA, pp. 49-87; Binns and Howitz (1994) In Bacterical Pathogenesis of Plants and Animals, Dang, ed., Berlin: Springer Verlag., pp. 119-38; Hooykaas and Beijersbergen (1994) Ann. Rev. Phytopathol. 32:157-79; Lessl and Lanka (1994) Cell 77:321-4; and Zupan and Zambryski (1995) Annual Rev. Phytopathol. 27:583-618.

To select or score for transformed plant cells regardless of transformation methodology, the DNA introduced into the cell may contain a gene that functions in a regenerable plant tissue to produce a compound that confers upon the plant tissue resistance to an otherwise toxic compound. Genes of interest for use as a selectable, screenable, or scorable marker include, but are not limited to, β-glucuronidase (GUS), green fluorescent protein (GFP), luciferase, and antibiotic or herbicide tolerance genes. Examples of antibiotic resistance genes include genes conferring resistance to the penicillins, kanamycin (and neomycin, G418, bleomycin); methotrexate (and trimethoprim); chloramphenicol; and tetracycline. For example, glyphosate resistance may be conferred by a herbicide resistance gene. Della-Cioppa et al. (1987) Bio/Technology 5:579-84. Other selection devices can also be implemented, including for example and without limitation, tolerance to phosphinothricin, bialaphos, and positive selection mechanisms (Joersbro et al. (1998) Mol. Breed. 4:111-7), and are considered within the scope of embodiments of the present invention.

The transformed cells, identified by selection or screening and cultured in an appropriate medium that supports regeneration, may then be allowed to mature into plants.

The presently disclosed methods may be used with any transformable plant cell or tissue. Transformable cells and tissues, as used herein, includes but is not limited to those cells or tissues that are capable of further propagation to give rise to a plant. Those of skill in the art recognize that a number of plant cells or tissues are transformable in which after insertion of exogenous DNA and appropriate culture conditions the plant cells or tissues can form into a differentiated plant. Tissue suitable for these purposes can include but is not limited to immature embryos, scutellar tissue, suspension cell cultures, immature inflorescence, shoot meristem, nodal explants, callus tissue, hypocotyl tissue, cotyledons, roots, and leaves.

The regeneration, development, and cultivation of plants from transformed plant protoplast or explants are known in the art. Weissbach and Weissbach (1988) Methods for Plant Molecular Biology, (Eds.) Academic Press, Inc., San Diego, CA; Horsch et al. (1985) Science 227:1229-31. This regeneration and growth process typically includes the steps of selecting transformed cells and culturing those cells through the usual stages of embryonic development through the rooted plantlet stage. Transgenic embryos and seeds are similarly regenerated. In this method, transformants are generally cultured in the presence of a selective media which selects for the successfully transformed cells and induces the regeneration of plant shoots. Fraley et al. (1993) Proc. Natl. Acad. Sci. USA 80:4803. These shoots are typically obtained within two to four months. The resulting transgenic rooted shoots are thereafter planted in an appropriate plant growth medium such as soil. Cells that survive the exposure to a selective agent, or cells that have been scored positive in a screening assay, may be cultured in media that supports regeneration of plants. The shoots may then be transferred to an appropriate root-inducing medium containing the selective agent and an antibiotic to prevent bacterial growth. Many of the shoots will develop roots. These are then transplanted to soil or other media to allow the continued development of roots. The method, as outlined above, will generally vary depending on the particular plant strain employed, and particulars of the methodology are therefore within the discretion of one of skill in the art.

The regenerated transgenic plants may be self-pollinated to provide homozygous transgenic plants. Alternatively, pollen obtained from the regenerated transgenic plants may be crossed with non-transgenic plants, preferably inbred lines of agronomically important species. Conversely, pollen from non-transgenic plants may be used to pollinate the regenerated transgenic plants.

The transgenic plant may pass along the transformed nucleic acid sequence to its progeny. The transgenic plant is preferably homozygous for the transformed nucleic acid sequence and transmits that sequence to all of its offspring upon, and as a result of, sexual reproduction. Progeny may be grown from seeds produced by the transgenic plant. These additional plants may then be self-pollinated to generate a true breeding line of plants.

The progeny from these plants may be evaluated, among other things, for gene expression. The gene expression may be detected by several common methods such as western blotting, northern blotting, immunoprecipitation, and ELISA (Enzyme-Linked ImmunoSorbent Assay). The transformed plants may also be analyzed for the presence of the introduced DNA and the expression level and/or fatty acid profile conferred by the nucleic acid molecules and amino acid molecules of the present invention. Those of skill in the art are aware of the numerous methods available for the analysis of transformed plants. For example, methods for plant analysis include, but are not limited to, Southern blots or northern blots, PCR-based approaches, biochemical assays, phenotypic screening methods, field evaluations, and immunodiagnostic assays.

Methods for specifically transforming dicots are well-known to those skilled in the art. Transformation and plant regeneration using these methods have been described for a number of crops including, but not limited to, members of the genus *Arabidopsis,* cotton (*Gossypium hirsutum*), soybean (*Glycine max*), peanut (*Arachis hypogaea*), and members of the genus *Brassica.* Methods for transforming dicots, primarily by use of *Agrobacterium tumefaciens,* and obtaining transgenic plants have been published for cotton (U.S. Patent 5,004,863; U.S. Patent 5,159,135; U.S. Patent 5,518,908); soybean (U.S. Patent 5,569,834; U.S. Patent 5,416,011; McCabe et al. (1988) Biotechnology 6:923; Christou et al. (1988) Plant Physiol. 87:671-4); *Brassica* (U.S. Patent 5,463,174); peanut (Cheng et al. (1996) Plant Cell Rep. 15:653-7; McKently et al. (1995) Plant Cell Rep. 14:699-703); papaya; and pea (Grant et al. (1995) Plant Cell Rep. 15:254-8).

Methods for transforming monocots are also well-known in the art. Transformation and plant regeneration using these methods have been described for a number of crops including, but not limited to, barley (*Hordeum vulgarae*); maize (*Zea mays*); oats (*Avena sativa*); orchard grass (*Dactylis glomerata*); rice (*Oryza sativa,* including indica and japonica varieties); sorghum (*Sorghum bicolor*); sugar cane (*Saccharum sp*); tall fescue (*Festuca arundinacea*); turfgrass species (*e.g., Agrostis stolonifera*, *Poa pratensis*, *Stenotaphrum secundatum*); wheat (*Triticum aestivum*); and alfalfa (*Medicago sativa*). It is apparent to those of skill in the art that a number of transformation methodologies can be used and modified for production of stable transgenic plants for any number of target crops of interest.

Any plant may be chosen for use in the presently disclosed methods. Preferred plants for modification according to the present invention include, for example and without limitation, oilseed plants, *Arabidopsis thaliana,* borage *(Borago* spp.), canola (*Brassica* spp.), castor *(Ricinus communis),* cocoa bean *(Theobroma cacao)*, corn *(Zea mays),* cotton *(Gossypium* spp), *Crambe* spp., *Cuphea* spp., flax *(Linum* spp.), *Lesquerella* and *Limnanthes* spp., Linola, nasturtium *(Tropaeolum* spp.), *Oenothera* spp., olive *(Olea* spp.), palm *(Elaeis* spp.), peanut *(Arachis* spp.), rapeseed, safflower *(Carthamus* spp.), soybean *(Glycine* and *Soja* spp.), sunflower *(Helianthus* spp.), tobacco *(Nicotiana* spp.), *Vernonia* spp., wheat *(Triticum* spp.), barley *(Hordeum* spp.), rice (Oryza spp.), oat *(Avena* spp.) sorghum *(Sorghum* spp.), and rye *(Secale* spp.) or other members of the *Gramineae.*

It is apparent to those of skill in the art that a number of transformation methodologies can be used and modified for production of stable transgenic plants from any number of target crops of interest.

E. Transgenic seeds

In some embodiments, a transgenic seed may comprise a delta-9 desaturase polypeptide comprising an amino acid sequence being at least 80% identical to a sequence selected from the group consisting of SEQ ID NO:12, SEQ ID NO:13, SEQ ID NO:14, SEQ ID NO:50, SEQ ID NO:51, SEQ ID NO:52, SEQ ID NO:72, and SEQ ID NO:73. In these and other embodiments, the transgenic seed may comprise a nucleic acid sequence being at least 60% identical to a sequence selected from the group consisting of SEQ ID NO:3, SEQ ID NO:4, SEQ ID NO:5, SEQ ID NO:8, SEQ ID NO:9, SEQ ID NO:10, SEQ ID NO:11, SEQ ID NO:15, SEQ ID NO:16, SEQ ID NO:17, SEQ ID NO:44, SEQ ID NO:45, SEQ ID NO:48, and SEQ ID NO:49. In certain embodiments, a transgenic seed may exhibit decreased levels of saturated fatty acids (for example, palmitic fatty acids and/or stearic fatty acids). The seeds may be harvested from a fertile transgenic plant, and may be used to grow progeny generations of transformed plants, including hybrid plant lines comprising at least one nucleic acid sequence as set forth above, and optionally at least one additional gene or nucleic acid construct of interest.

The following examples are provided to illustrate certain particular features and/or embodiments. These examples should not be construed to limit the invention to the particular features or embodiments described.

## EXAMPLES

### Example I: Cloning of acyl-CoA delta-9 desaturases and functional characterization in *ole1*-deficient yeast

<u>*Cloning of Magnaporthe grisea* acyl-CoA delta-9 desaturases</u>

The *Magnaporthe grisea* acyl-CoA delta-9 desaturase gene (MgD9DS) was isolated from genomic DNA using primers based on a published NCBI/Broad Institute sequence originally annotated as a "hypothetical protein," and having 55.4% identity at the nucleotide level to the *S. cerevisiae* acyl-CoA delta-9 desaturase (*i.e., OLE1*). Forward and reverse primers, each 41 base pairs in length, were designed. The forward primer, MgΔ9F (SEQ ID NO:1), included an *EcoRI* site at the 5' end. The reverse primer, Mg9ΔR (SEQ ID NO:2), contained stop codons in each of three reading frames and a terminal *XhoI* site.

The MgD9DS gene was PCR amplified using the Takara EZ Taq™ PCR kit (Takara Bio Inc., Otsu, Shiga, Japan) following the manufacturer's protocol. The amplification conditions were 94 °C for 1 minute, followed by 30 cycles of 94 °C for 30 sec, 60 °C for 60 seconds, and an extension at 72 °C for 90 seconds. A final extension step was performed at 72 °C for 10 minutes. The expected 1,425 base pair PCR product was excised from an agarose gel and purified using Montage spin columns per manufacturer's recommendations (Millipore, Billerica, MA). The purified fragment was cloned into the pCR®2.1 TOPO® cloning vector (Invitrogen, Carlsbad, CA). The TOPO reaction was transformed into chemically competent Top 10 *E. coli* cells per supplier conditions. Bacterial colonies containing the putative clone were isolated. Mini-plasmid preps were preformed with a Macherey-Nagel Nucleospin DNA isolation kit (Machery-Nagel, Neumann-Neander-Strasse, Düren, Germany), and DNA was digested with *EcoRI* and *XhoI* restriction enzymes. Positive clones containing the expected 1,425 bp MgD9DS gene fragment were identified. The nucleotide sequence was obtained *via* sequencing reactions. The sequence of the PCR amplified fragment is listed as SEQ ID NO:3.

Sequence analysis revealed a small (90 bp) intron located in the 5' end of the MgD9DS gene. The intron was removed using Splice Overlap Extension PCR. The resulting PCR amplicon was gel purified, cloned into the pCR®2.1 TOPO® cloning vector, and transformed into Top 10 *E. coli* cells. Several clones were identified *via* analysis of restriction enzyme digests of purified DNA from single transformant colonies. These clones were sequenced to confirm the presence of an intronless MgD9DS clone. The resulting sequence is listed as SEQ ID NO:4.

The MgD9DS genes, with and without the intron, were each subcloned as an *EcoRI*/*XhoI* fragment into a yeast expression vector. This yeast expression vector contains an *Aspergillus nidulans* delta-9 desaturase (AnD9DS) gene flanked by the *S. cerevisiae* delta-9 desaturase promoter and delta-9 desaturase 3'UTR/terminator. The *Aspergillus nidulans* delta-9 desaturase gene was excised on an *EcoRI*/*XhoI* fragment, which was replaced with either the MgD9DS gene-containing fragment or the intronless MgD9DS gene-containing fragment. Two clones containing the MgD9DS gene (one with an intron, and one without an intron) were advanced for *S. cerevisiae* transformation.

A delta-9 desaturase deficient *S. cerevisiae* strain (OFY093), which is maintained on Yeast Peptone Dextrose (YPD) media with Tween® 80, was transformed using the Alkali-Cation Yeast Transformation Kit (Qbiogene, Montreal, Canada). Complemented strains were identified by growth on media that did not contain Tween® 80 (monounsaturated fatty acid supplement) or uracil (Dropout Base with Agar with SC-URA). Complemented strains were single colony purified on selective media three times. Complemented strains were further verified by PCR amplification of the delta-9 desaturase gene, and sequencing of the PCR product. In addition, strains containing the MgD9DS clone were reverted to fatty acid and uracil dependence by passing the strain at least three times on YPD + Tween 80® media, then patching strains to DOBA SC-URA minus Tween® 80 media.

Expression of the intron-containing MgD9DS coding sequence was unsuccessful, indicating that the intron was not spliced by the yeast machinery. The substrate specificity of the yeast strain containing the intronless MgD9DS coding sequence was further characterized by FAME analysis.

Cloning of *Leptosphaeria nodorum* acyl-CoA delta-9 desaturases

Two *Leptosphaeria nodorum* EST sequences (1,246 and 429 base pairs, respectively) were identified from a collection of *L. nodorum* ESTs by using a BlastN search as sharing high levels of sequence identity (54.0% and 54.2% respectively) with the *S. cerevisiae* acyl-CoA delta-9 desaturase (*OLE1*). When aligned, these sequences were 64.6% identical to one another, suggesting the presence of two distinct *Leptosphaeria nodorum* acyl-CoA delta-9 desaturases. An LnD9DS-1 gene (SEQ ID NO:5) was isolated by screening a *L. nodorum* cDNA library with the 1,246 bp gene probe. The sequence of this gene was obtained, and the coding sequence was isolated. The entire sequence of an LnD9DS-2 gene was isolated by first BLAST searching the published Broad Institute *Leptosphaeria nodorum* genome sequence with the 429 bp EST sequence. This search identified Supercontig Ln 1.4 as containing a gene with 100% homology to the 429 bp fragment, which gene was annotated as encoding a "hypothetical protein." Next, the LnD9DS-2 gene was cloned from a *Leptosphaeria nodorum* cDNA library using PCR primers based on the Ln1.4 supercontig sequence. The primer sequences used were Lnd9FAD2F (SEQ ID NO:6) and Lnd9FAD2R (SEQ ID NO:7). The forward primer was designed with a 5' *BamHI* site, and the reverse primer contained stop codons in three reading frames and a terminal *NcoI* site.

An aliquot of the *Leptosphaeria nodorum* cDNA library was diluted 1/10 to provide 400 ng of template DNA for the PCR reaction. PCR amplification was performed using a Takara EZ Taq™ PCR kit following the recommended amplification conditions of 94 °C for 1 minute, followed by 30 cycles of 94 °C for 30 seconds, 60 °C for 60 seconds, and extension at 72 °C for 90 seconds. A final extension step was performed at 72 °C for 10 minutes. The expected 1,370 base pair product was excised from an agarose gel, and purified using Montage spin columns per the manufacturer's recommendations. The purified fragment was cloned into the pCR®2.1 TOPO® cloning vector. The ligation reaction was transformed into chemically competent Top 10 *E. coli* cells according to the manufacturer's recommended protocol. Colonies containing a putative clone were isolated. Mini plasmid preps were preformed with Macherey-Nagel Nucleospin columns, and DNA was digested with *BamHI* and *NcoI* restriction enzymes. Putative LnD9DS-2 clones were identified and sequenced.

Upon sequencing, a clone of LnD9DS-2 (SEQ ID NO:8) was confirmed by comparison with the "hypothetical protein" sequence. A conservative change in the sequence of LnD9DS-2 was identified. The codon <u>T</u>GC (cysteine) was changed to <u>A</u>GC (serine) by substitution of an adenine for a thymidine at base position 271, which codon is translated to amino acid 89 of the published sequence. This is a conservative change, and the cysteine is not found to be a highly conserved amino acid among multiple filamentous fungi, so no correction was attempted.

The LnD9DS-1 and LnD9DS-2 genes of SEQ ID NOs:5 and 8, respectively, were cloned into a yeast expression vector. Clones containing either of the LnD9DS-1 and LnD9DS-2 coding sequences were confirmed by restriction enzyme analysis and DNA sequencing.

A delta-9 desaturase deficient *S. cerevisiae* strain (OFY093), which is maintained on YPD media with Tween® 80, was transformed using the Alkali-Cation Yeast Transformation Kit from Qbiogene. Complemented strains were identified by growth on media that did not contain Tween® 80 (monounsaturated fatty acid supplement) or uracil (DOBA sc-ura). The complemented strains were single colony purified on selective media three times. Complemented strains were further verified by PCR amplification of the delta-9 desaturase gene and sequencing of the PCR product. In addition, strains containing a LnD9DS-2 clone were reverted to fatty acid and uracil dependence by passing each strain at least three times on YPD + Tween® 80 media, then patching strains to DOBA SC-URA minus Tween® 80 media. The substrate specificities of the yeast strains containing either the LnD9DS-1 or LnD9DS-2 coding sequence were further characterized by FAME analysis.

Cloning and Transformation of Delta-9 Desaturase Deficient *S. cerevisiae* with HzD9DS Gene

A plant-optimized synthetic gene encoding the *Helicoverpa zea* acyl-CoA delta-9 desaturase (HzD9DS) (identified as HzPGDS2 in Rosenfield et al. (2001) Insect Biochem. Mol. Biol. 31(10):949-64) was excised from DASPICO89 (described below) on a *BamHI/XhoI* fragment and gel purified using Montage spin columns. This fragment was ligated into corresponding restriction enzyme sites of a yeast expression vector described previously and transformed into *E. coli* strain DH5α using standard molecular biology techniques and supplier protocols (Invitrogen, Carlsbad, CA).

Following restriction analysis and DNA sequencing, a clone containing the HzD9DS gene was selected for transformation into the delta-9 desaturase deficient *S. cerevisiae* strain, OFY093. The OFY093 strain, which is maintained on YPD media with Tween® 80, was transformed using the Alkali-Cation Yeast Transformation Kit from Qbiogene. Complemented strains were identified by growth on media that did not contain Tween® 80 (fatty acid supplement) and uracil (DOBA SC-URA). Putative complemented strains were single colony purified on selective media three times. Complemented strains were further verified by: i) extraction of plasmid DNA, using the Qbiogene Yeast plasmid purification kit, followed by PCR amplification using HzD9DS gene-specific primers; ii) sequencing of the HzD9DS gene-specific PCR product; and iii) reversion of the strain to fatty acid and URA-3 dependence by passing the strain at least three times on YPD + Tween® 80 media, then patching strains to DOBA SC-URA minus Tween® 80 media. The substrate specificity of one verified complemented HzD9DS yeast strain was further characterized by FAME analysis.

Analysis of LnD9DS-1, LnD9DS-2, MgD9DS, and HzD9DS expressed in OLE1-deficient yeast strain

As set forth, *supra*, three exemplary acyl-CoA delta-9 desaturase (D9DS) genes were cloned from the plant pathogenic fungi, *Magnaporthe grisea* (MgD9DS) and *Leptosphaeria nodorum* (LnD9DS-1 and LnD9DS-2). These genes and their encoded proteins have not been previously characterized. Acyl-CoA delta-9 desaturases catalyze the formation of a cis double bond between carbon atoms 9 and 10 of saturated 14-, 16-, and 18-carbon fatty acyl thioesters of Coenzyme A, resulting in production of myristoleic (14:1), palmitoleic (16:1), or oleic acid (18:1), respectively. Effects related to organism-specific biology are eliminated by expressing the different fungal acyl-CoA delta-9 desaturase genes in the same biological context. Expression of the fungal acyl-CoA delta-9 desaturase genes was therefore driven using the endogenous *ole1* gene promoter within a palmitoyl-stearoyl CoA desaturase (OLE1)-deficient OFY093 yeast strain. Thus, observed differences in fatty acid substrate specificity in this system are attributable to the specific fungal delta-9 desaturase expressed in the complemented *S. cerevisiae* strain.

The substrate specificities of the MgD9DS, LnD9DS-1 and LnD9DS-2 CoA desaturases expressed in the complemented OYF093 strains were characterized and compared to OFY093 complemented with the AnD9DS (*sdeA*) described in WO/1999/050430. A yeast expression construct containing the AnD9DS gene, expression of which is driven by the *ole1* gene promoter, was transformed into the *S. cerevisiae* OFY093 strain and expressed using the protocol described above.

The complemented *S. cerevisiae* strains were grown in minimal media with no fatty acid supplementation at 30 °C for 24 hours. Quantitative FAME analysis was performed on washed and lyophilized cell pellets. The results of this analysis are shown in **Table 1.** LnD9DS-2 promotes formation of C14:1 and C16:1, whereas LnD9DS-1 and MgD9DS have a preference for C18:0, as indicated by the ratio of C16:1/18: fatty acids in the yeast fatty acid compositional analyses.

**Table 1:** Comparison of fatty acid composition of ole1-deficient yeast expressing four different fungal desaturases

| Desaturase | C14:0 | C14:1 | C16:0 | C16:1 | C18:0 | C18:1 | C16:1/ 16:0 | C18:1/ 18:0 | C16:1/ 18:1 |
|---|---|---|---|---|---|---|---|---|---|
| LnD9DS-1 | 1.5 | 0.0 | 36.5 | 8.7 | 1.8 | 51.5 | 0.2 | 28.2 | 0.17 |
| LnD9DS-2 | 1.0 | 0.1 | 26.6 | 38.1 | 6.3 | 27.9 | 1.4 | 4.4 | 1.37 |
| AnD9DS | 0.5 | 0.0 | 26.3 | 7.8 | 2.0 | 63.4 | 0.3 | 31.7 | 0.12 |
| MgD9DS | 0.5 | 0.0 | 22.7 | 9.1 | 1.8 | 65.9 | 0.4 | 37.0 | 0.14 |
| wild type yeast | 0.6 | 0.0 | 9.6 | 38.6 | 6.9 | 44.3 | | | |
| old1-null + Tween® 80 | 2.6 | 0.4 | 38.0 | 10.9 | 7.8 | 40.4 | | | |

(continued)

| Desaturase | C14:0 | C14:1 | C16:0 | C16:1 | C18:0 | C18:1 | C16:1/ 16:0 | C18:1/ 18:0 | C16:1/ 18:1 |
|---|---|---|---|---|---|---|---|---|---|
| Empty vector + Tween® 80 | 2.2 | 0.3 | 40.3 | 8.7 | 8.7 | 39.8 | | | |

The novel desaturases were further compared to the native *S. cerevisiae* stearoyl-CoA delta-9 desaturase (ole1) transferred into the same recombinant expression environment. A yeast expression vector containing the nucleotide sequence from *S. cerevisiae* described in WO/2000/011012 was constructed. The yeast expression construct containing the native *S. cerevisiae* stearoyl-CoA delta-9 desaturase was transformed into the *S. cerevisiae* OFY093 strain and expressed using the protocol described above. Another non-fungal acyl-CoA delta-9 desaturase from the insect species, *Helicoverpa zea* (HzD9DS), was also evaluated in these experiments.

Complemented *S. cerevisiae* strains containing one of the MgD9DS, LnD9DS-2 and HzD9DS genes were grown in Drop Out Broth SC-URA. A control strain, pDAB467EV-1 (pDAB467B/N transformed into OFY093 by previously described Yeast Transformation methodology), was grown in DOB SC-URA + Tween® 80, and the parent delta-9 desaturase-deficient *S. cerevisiae* strain, OFY093, was grown in DOB scAA + Tween® 80. Cultures were inoculated with a loop of cells from a fresh streak plate of the same media containing 1.5% agar. Strains were grown at 30 °C for 24 hours. Cultures were spun at 6,000 rpm for 10 minutes. Pellets were washed in water, spun again at 6,000 rpm for 10 minutes, and then frozen at -20 °C until FAME analysis was performed. Three sets of expression cultures were analyzed.

Freeze-dried yeast pellets were saponified in methanol containing 10% (w/v) NaOH. Nonsaponifiable lipid contaminants (sterols) were removed with hexane. The methanol fraction was acidified by addition of $H_2SO_4$, and the protonated fatty acids were extracted with hexane. The isolated hexane fraction was dried down, and fatty acids were methylated with 0.5 N MeOHCl at 80 °C for 30 minutes. The resulting FAMEs were extracted with hexane containing undecanoate methyl ester as an internal standard. The FAME extracts were analyzed with a HP6890 Gas Chromatograph-Flame Ionization Detector (Santa Clara, CA) equipped with a capillary column BPX 70 (15m x 0.25mm x 0.25μm) from SGE (Austin,TX). FAMEs were separated in a temperature gradient using helium as the carrier gas. Each FAME species was identified by retention time, and quantified by the injection of a FAME rapeseed oil reference mix from Matreya, LLC (Pleasant Gap, PA), as the calibration standard.

**Table 2** shows the fatty acid composition (as %FAMEs) of ole1-deficient OFY093 yeast cells expressing various exemplary acyl Co-A delta-9 desaturases. All strains grew well and were fully-complemented by the introduced desaturases without any requirement for exogenous MUFAs (monounsaturated fatty acids).

**Table 2:** Fatty acid composition (as % Total FAMEs) of ole1-deficient yeast strain OFY093 expressing acyl Co-A delta-9 desaturases. (Standard Deviation is in parentheses).

| Desaturase | n | C14:0 | C14:1 | C16:0 | C16:1 | C18:0 | C18:1 |
|---|---|---|---|---|---|---|---|
| LnD9DS-2 | 7 | 1.4 (0.7) | 1.4 (1.0) | 26.6 (4.5) | 38.8 (2.8) | 6.0 (1.3) | 25.4 (4.4) |
| HzD9DS | 6 | 2.6 (1.3) | 0.9 (0.5) | 34.7 (6.8) | 37.5 (4.2) | 6.0 (1.1) | 18.4 (4.1) |
| ole1 | 6 | 1.1 (0.4) | 0.6 (0.4) | 14.4 (2.6) | 49.2 (1.6) | 5.6 (1.1) | 24.0 (1.1) |
| AnD9DS | 8 | 0.5 (0.3) | 0.2 (0.2) | 23.5 (2.2) | 9.3 (3.0) | 2.1 (0.5) | 64.6 (3.2) |
| MgD9DS | 2 | 0.9 (0.0) | 0.1 (0.0) | 21.2 (0.2) | 12.1 (0.1) | 1.6 (0.1) | 64.2 (0.3) |

These data show that the fatty acid composition of the complemented yeast strains varies according to the introduced gene. LnD9DS-2 produces relatively high amounts of C16:1, as does HzD9DS and *ole1*, whereas AnD9DS and MgD9DS produce relatively high amounts of C18:1.

The differential level of conversion based upon chain length can be further shown by calculating the proportion of MUFA relative to the total fatty acids for each fatty acid chain length; C14, C16, or C18. These data show the relatively high conversion to C16:1 for LnD9DS-2 and HzD9DS, and to C18:1 for AnD9DS and MgD9DS. **Table 3.** The bottom four rows represent control samples complemented with added tergitol, unsaturated fatty acids, or Tween®. Samples with different letters are significantly different, as determined *via* the Tukey-Kramer Test performed in the JMP statistical software suite (SAS Institute Inc., Cary, NC).

**Table 3:** Proportion of MUFA of total fatty acids for each chain length (Cxx:1/(Cxx:0 + Cxx:1).

| Desaturase | C14 | C16* | C18 |
|---|---|---|---|
| LnD9DS-2 | 0.49 | 0.60 (b) | 0.81 (b) |
| HzD9DS | 0.30 | 0.52 (b) | 0.75 (c) |
| ole1 | 0.34 | 0.79 (a) | 0.81 (b) |
| AnD9DS | 0.25 | 0.28 (c) | 0.97 (a) |
| MgD9DS | 0.07 | 0.36 (c) | 0.98 (a) |
| None + tergitol | 0.06 | 0 | 0 |
| None + tergitol + ricinoleic | 0.07 | 0 | 0.01 |
| None + tergitol + linoleic | 0 | 0 | 0.04 |
| None + tween | 0.65 | 0.23 | 0.87 |
| * C16 MUFA includes cis-vaccenic acid (C18:1 Δ11), as it is derived from elongation of palmitoleic acid (C16:1 Δ9). | | | |

Phylogeny of fungal Acyl-CoA Desaturases

Phylogenetic analysis of multiple fungal acyl-CoA delta-9 desaturase amino acid sequences suggests that LnD9DS-2 is distinct from the 18:0-preferring delta-9 desaturases. Thus, we hypothesized that characterization of other fungal delta-9 desaturases closely associated with either the 18:0-preferring delta-9 desaturases, or with LnD9DS-2, may identify desaturases with a range of 18:0 or 16:0 activities. Our hypothesis predicts that a fungal delta-9 desaturase that is more closely associated with LnD9DS-2 will have increased 16:0 activity.

A search of the public DNA sequence databases (Broad Institute, NCBI, etc.) did not identify any gene sequences specifically annotated as delta-9 desaturases in *Magnaporthe grisea* or *Leptosphaeria nodorum.* Pfam analysis of the Broad Institute sequences that were identified within this disclosure indicates that these proteins contain cytochrome B5 and desaturase motifs that are also found in other fungal acyl-CoA delta-9 desaturases. However, the proteins had not been previously identified as acyl-CoA delta-9 desaturases. We have demonstrated this function of these proteins by complementation in yeast, reversal studies, and DNA sequence analysis.

The relationships of several fungal desaturase gene sequences were analyzed phylogenetically using the neighbor-joining method *via* the MEGA software package. Tamura et al. (2007) Mol. Biol. and Evolution 24:1596-9. **FIG. 1** illustrates this phylogenetic analysis of the fungal desaturase sequences. These sequences were recovered by BlastN searches of the NCBI sequence database using the AnD9DS (sdeA) amino acid sequence. LnD9DS-1 and MgD9DS share higher levels of sequence identity with one another, as compared to LnD9DS-2. Additionally, a ClustalW alignment of LnD9DS-1, LnD9DS-2, and MgD9DS shows the divergence of LnD9DS-2 from LnD9DS-1 and MgD9DS. **FIG. 2**. The nucleotide sequences of LnD9DS-1 and MgD9DS share a higher number of base pairs in common.

**Table 4** and **FIG. 3** further illustrate the phylogenetic relationship of newly-identified proteins, LnD9DS-1, LnD9DS-2, and MgD9DS, as well as AnD9DS and the yeast desaturase, ScOLE1. The LnD9DS-1, MgD9DS, and AnD9DS (sdeA) amino acids sequences share a greater percentage of identity with one another as compared to LnD9DS-2. The conservation of amino acid identity allows us to predict that the substrate specificity for 18:0 acyl-CoA is dependent upon the conserved sequence shared between LnD9DS-1, MgD9DS, and AnD9DS (sdeA). In comparison, the acyl-CoA substrate specificity of LnD9DS-2 is preferential for 16:0 as a result of its divergent amino acid sequence.

**Table 4:** Amino acid identity of various fungal desaturase sequences aligned using ClustalW.

| | LnD9DS-1 | LnD9DS-2 | MgD9DS | Yeast OLE1 |
|---|---|---|---|---|
| **AnD9DS (sdeA)** | 81% | 61% | 75% | 49% |
| **LnD9DS-1** | -- | 61% | 75% | 47% |
| **LnD9DS-2** | 61% | -- | 62% | 49% |
| **MgD9DS** | 75% | 62% | -- | 49% |

**Example 2: Design and synthesis of optimized delta-9 desaturase genes from *Magnaporthe grisea, Helicoverpa zea,* and *Leptosphaeria nodorum***

To obtain higher expression of fungal delta-9 desaturase genes in canola, we engineered these genes so that they are more efficiently expressed in transgenic canola cells containing the heterologous gene. Extensive analysis of the DNA sequence of the native *Magnaporthe grisea, Helicoverpa zea* and *Leptosphaeria nodorum* delta-9 desaturase coding regions disclosed herein as SEQ ID NO:9, SEQ ID NO:10 and SEQ ID NO:11, respectively, revealed the presence of several sequence motifs that are thought to be detrimental to optimal plant expression, as well as a non-optimal codon composition for such optimal plant expression. In order to design optimized genes encoding a delta-9 desaturase protein, we generated DNA sequences *in silico* that are more "plant-like" (and specifically, more "canola-like") in nature, in which the sequence modifications do not hinder translation or create mRNA instability.

To engineer plant-optimized genes encoding a delta-9 desaturase, DNA sequences were designed to encode the amino acid sequences of the protein desaturases, utilizing a redundant genetic code established from a codon bias table compiled from the protein coding sequences for the particular host plants (*i.e.*, canola). Preferred codon usages for canola are shown in **Table 5.** Columns C and G of **Table 5** present the distributions (in % of usage for all codons for that amino acid) of synonymous codons for each amino acid, as found in the coding regions of *Brassica napus.* It is evident that some synonymous codons for some amino acids are found only rarely in plant genes (*e.g.*, CGG in canola). A codon was considered to be rarely used if it is represented at about 10% or less of the time to encode the relevant amino acid in genes of either plant type (indicated by "DNU" in Columns D and H of **Table 5**). To balance the distribution of the remaining codon choices for an amino acid, a Weighted Average representation for each codon was calculated, using the formula:

$$\text{Weighted Average \% of C1} = 1/(\%C1 + \%C2 + \%C3 + \textit{etc.}) \times \%C1 \times 100,$$

where C1 is the codon in question and %C2, %C3, *etc.* represent the averages of the % values for *Brassica napus* of remaining synonymous codons (average % values for the relevant codons are taken from Columns C and G) of **Table 5.**

The Weighted Average % value for each codon is given in Columns D and H of **Table 5.**

**Table 5:** Synonomous codon representation in coding regions of canola (*B. napus*) genes (Columns C and G). Values for a balanced-biased codon representation set for a plant-optimized synthetic gene design are in Columns D and H.

| A | B | C | D | E | F | G | H |
|---|---|---|---|---|---|---|---|
| **Amino Acid** | **Codon** | **Canola %** | **Weighted Average** | **Amino Acid** | **Codon** | **Canola %** | **Weighted Average** |
| **ALA (A)** | **GCA** | 23.3 | 23.3 | **LEU (L)** | **CTA** | 10.1 | DNU |
| | **GCC** | 21.2 | 21.2 | | **CTC** | 22.8 | 28.5 |
| | **GCG** | 14.2 | 14.2 | | **CTG** | 11.6 | 14.6 |
| | **GCT** | 41.3 | 41.3 | | **CTT** | 25.2 | 31.6 |
| **ARG (R)** | **AGA** | 31.8 | 43.8 | | **TTA** | 10.1 | DNU |
| | **AGG** | 22.1 | 30.5 | | **TTG** | 20.2 | 25.3 |
| | **CGA** | 9.9 | DNU | **LYS (K)** | **AAA** | 44.6 | 44.6 |
| | **CGC** | 8.9 | DNU | | **AAG** | 55.4 | 55.4 |
| | **CGG** | 8.6 | DNU | **MET (M)** | **ATG** | 100.0 | 100.0 |
| | **CGT** | 18.6 | 25.7 | **PHE (F)** | **TTC** | 58.6 | 58.6 |
| **ASN (N)** | **AAC** | 62.6 | 62.6 | | **TTT** | 41.4 | 41.4 |
| | **AAT** | 37.4 | 37.4 | **PRO (P)** | **CCA** | 29.6 | 29.6 |
| **ASP (D)** | **GAC** | 42.5 | 42.5 | | **CCC** | 14.6 | 14.6 |
| | **GAT** | 57.5 | 57.5 | | **CCG** | 18.4 | 18.4 |
| **CYS (C)** | **TGC** | 49.2 | 49.2 | | **CCT** | 37.3 | 37.3 |
| | **TGT** | 50.8 | 50.8 | **SER (S)** | **AGC** | 16.0 | 17.9 |
| **END** | **TAA** | 38.5 | DNU | | **AGT** | 14.1 | 15.8 |
| | **TAG** | 22.1 | DNU | | **TCA** | 18.2 | 20.4 |
| | **TGA** | 39.4 | 100.0 | | **TCC** | 16.7 | 18.7 |
| **GLN (Q)** | **CAA** | 50.0 | 50.0 | | **TCG** | 10.7 | DNU |
| | **CAG** | 50.0 | 50.0 | | **TCT** | 24.3 | 27.2 |
| **GLU (E)** | **GAA** | 43.6 | 43.6 | **THR (T)** | **ACA** | 26.3 | 26.3 |
| | **GAG** | 56.4 | 56.4 | | **ACC** | 26.9 | 26.9 |
| **GLY (G)** | **GGA** | 36.4 | 36.4 | | **ACG** | 16.9 | 16.9 |
| | **GGC** | 16.2 | 16.2 | | **ACT** | 30.0 | 30.0 |
| | **GGG** | 15.2 | 15.2 | **TRP (W)** | **TGG** | 100.0 | 100.0 |
| | **GGT** | 32.1 | 32.1 | **TYR (Y)** | **TAC** | 59.4 | 59.4 |
| **HIS (H)** | **CAC** | 49.6 | 49.6 | | **TAT** | 40.6 | 40.6 |
| | **CAT** | 50.4 | 50.4 | **VAL (V)** | **GTA** | 10.8 | DNU |
| **ILE (I)** | **ATA** | 21.1 | 21.1 | | **GTC** | 24.1 | 27.0 |
| | **ATC** | 42.7 | 42.7 | | **GTG** | 28.3 | 31.7 |
| | **ATT** | 36.2 | 36.2 | | **GTT** | 36.8 | 41.3 |

**NA = Not Applicable
***DNU = Do Not Use

New DNA sequences which encode essentially the amino acid sequence of the *Magnaporthe grisea, Helicov-*

*erpa zea,* and *Leptosphaeria nodorum* delta-9 desaturases of SEQ ID NO:12, SEQ ID NO:13 and SEQ ID NO:14, respectively, were designed for optimal expression in canola using a first and second choice codon distribution of frequently used codons found in canola genes. The new DNA sequences differ from the native DNA sequences encoding the delta-9 desaturase proteins by the substitution of plant-preferred *(i.e.,* first preferred, second preferred, third preferred, or fourth preferred) codons to specify an appropriate amino acid at each position within the protein amino acid sequence.

Design of the plant-optimized DNA sequences were initiated by reverse-translation of the protein sequences of SEQ ID NO:12, SEQ ID NO:13 and SEQ ID NO:14, using a canola codon bias table constructed from **Table 5** Columns D and H. The initial sequences were then modified by compensating codon changes (while retaining overall weighted average codon representation) to remove restriction enzyme recognition sites, remove highly stable intrastrand secondary structures, and remove other sequences that might be detrimental to cloning manipulations or expression of the engineered gene in plants. The DNA sequences were then re-analyzed for restriction enzyme recognition sites that might have been created by the modifications. The identified sites were then further modified by replacing the relevant codons with first, second, third, or fourth choice preferred codons. The modified sequences were further analyzed and further modified to reduce the frequency of TA and CG doublets, and to increase the frequency of TG and CT doublets. In addition to these doublets, sequence blocks that have more than about six consecutive residues of [G+C] or [A+T] were modified by replacing the codons of first or second choice, *etc.* with other preferred codons of choice. Rarely used codons were not included to a substantial extent in the gene design, and were used only when necessary to accommodate a different design criterion than codon composition *per se* (*e.g.*, addition or deletion of restriction enzyme recognition sites). Exemplary synthetic canola-optimized desaturase DNA sequences designed by this process are listed in SEQ ID NO:15, SEQ ID NO:16, and SEQ ID NO:17.

The resulting DNA sequences, as represented by SEQ ID NOs: 15-17, have a higher degree of codon diversity and a desirable base composition. Furthermore, these sequences contain strategically placed restriction enzyme recognition sites, and lack sequences that might interfere with transcription of the gene, or translation of the product mRNA. **Tables 6-8** present a comparison of the codon compositions of the coding regions for the delta-9 desaturase proteins as found in the native gene, and in the plant-optimized versions, and compare both to the codon composition recommendations for a plant-optimized sequence as calculated from **Table 5** Columns D and H.

**Table 6:** Codon compositions of coding regions for a MgD9DS protein. The native *M. grisa* desaturase coding region is compared to a Plant-Optimized version.

| Amino Acid | Codon | Native Gene # | Native Gene % | Plnt Opt Gene# # | Plnt Opt Gene % | Plnt Opt Recm'd | Amino Acid | Codon | Native Gene # | Native Gene % | Plnt Opt Gene # | Plnt Opt Gene % | Plnt Opt Recm'd |
|---|---|---|---|---|---|---|---|---|---|---|---|---|---|
| **ALA (A)** | **GCA** | 4 | 10.5 | 10 | 26.3 | 23.3 | **LEU (L)** | **CTA** | 0 | 0.0 | 0 | 0.0 | 0.0 |
| | **GCC** | 18 | 47.4 | 8 | 21.1 | 21.2 | | **CTC** | 11 | 28.9 | 11 | 28.9 | 28.5 |
| | **GCG** | 3 | 7.9 | 4 | 10.5 | 14.2 | | **CTG** | 11 | 28.9 | 5 | 13.2 | 14.6 |
| | **GCT** | 13 | 34.2 | 16 | 42.1 | 41.3 | | **CTT** | 12 | 31.6 | 12 | 31.6 | 31.6 |
| **ARG (R)** | **AGA** | 2 | 9.5 | 10 | 47.6 | 43.8 | | **TTA** | 0 | 0.0 | 0 | 0.0 | 0.0 |
| | **AGG** | 1 | 4.8 | 6 | 28.6 | 30.5 | | **TTG** | 4 | 10.5 | 10 | 26.3 | 25.3 |
| | **CGA** | 2 | 9.5 | 0 | 0.0 | 0.0 | **LYS (K)** | **AAA** | 1 | 3.4 | 13 | 44.8 | 44.6 |
| | **CGC** | 12 | 57.1 | 0 | 0.0 | 0.0 | | **AAG** | 28 | 96.6 | 16 | 55.2 | 55.4 |
| | **CGG** | 0 | 0.0 | 0 | 0.0 | 0.0 | **MET (M)** | **ATG** | 7 | 100 | 7 | 100 | 100.0 |
| | **CGT** | 4 | 19.0 | 5 | 23.8 | 25.7 | **PHE (F)** | **TTC** | 17 | 89.5 | 11 | 57.9 | 58.6 |
| **ASN (N)** | **AAC** | 23 | 100.0 | 14 | 60.9 | 62.6 | | **TIT** | 2 | 10.5 | 8 | 42.1 | 41.4 |
| | **AAT** | 0 | 0.0 | 9 | 39.1 | 37.4 | **PRO (P)** | **CCA** | 0 | 0.0 | 6 | 28.6 | 29.6 |
| **ASP (D)** | **GAC** | 17 | 68.0 | 11 | 44.0 | 42.5 | | **CCC** | 9 | 42.9 | 3 | 14.3 | 14.6 |
| | **GAT** | 8 | 32.0 | 14 | 56.0 | 57.5 | | **CCG** | 5 | 23.8 | 4 | 19.0 | 18.4 |
| **CYS (C)** | **TGC** | 2 | 66.7 | 1 | 33.3 | 49.2 | | **CCT** | 7 | 33.3 | 8 | 38.1 | 37.3 |
| | **TGT** | 1 | 33.3 | 2 | 66.7 | 50.8 | **SER (S)** | **AGC** | 3 | 10.7 | 5 | 17.9 | 17.9 |
| **END** | **TAA** | 0 | 0.0 | 0 | 0.0 | 0.0 | | **AGT** | 0 | 0.0 | 4 | 14.3 | 15.8 |
| | **TAG** | 0 | 0.0 | 0 | 0.0 | 0.0 | | **TCA** | 5 | 17.9 | 7 | 25.0 | 20.4 |
| | **TGA** | 1 | 100.0 | 1 | 100.0 | 100.0 | | **TCC** | 9 | 32.1 | 4 | 14.3 | 18.7 |

(continued)

| Amino Acid | Codon | Native Gene # | Native Gene % | PInt Opt Gene# # | PInt Opt Gene % | PInt Opt Recm'd | Amino Acid | Codon | Native Gene # | Native Gene % | PInt Opt Gene # | PInt Opt Gene % | PInt Opt Recm'd |
|---|---|---|---|---|---|---|---|---|---|---|---|---|---|
| GLN (Q) | CAA | 2 | 9.5 | 11 | 52.4 | 50.0 | | TCG | 9 | 32.1 | 0 | 0.0 | 0.0 |
| | CAG | 19 | 90.5 | 10 | 47.6 | 50.0 | | TCT | 2 | 7.1 | 8 | 28.6 | 27.2 |
| GLU (E) 16 | GAA | 1 | 6.7 | 7 | 46.7 | 43.6 | THR (T) | ACA | 4 | 16.7 | 6 | 25.0 | 26.3 |
| | GAG | 14 | 93.3 | 8 | 53.3 | 56.4 | | ACC | 15 | 62.5 | 7 | 29.2 | 26.9 |
| GLY (G) | GGA | 8 | 19.5 | 15 | 36.6 | 36.4 | | ACG | 1 | 4.2 | 4 | 16.7 | 16.9 |
| | GGC | 13 | 31.7 | 7 | 17.1 | 16.2 | | ACT | 4 | 16.7 | 7 | 29.2 | 30.0 |
| | GGG | 1 | 2.4 | 6 | 14.6 | 15.2 | TRP (W) | TGG | 21 | 100 | 21 | 100 | 100.0 |
| | GGT | 19 | 46.3 | 13 | 31.7 | 32.1 | TYR (Y) | TAC | 16 | 94.1 | 10 | 58.8 | 59.4 |
| HIS (H) | CAC | 19 | 95.0 | 10 | 50.0 | 49.6 | | TAT | 1 | 5.9 | 7 | 41.2 | 40.6 |
| | CAT | 1 | 5.0 | 10 | 50.0 | 50.4 | VAL(V) | GTA | 1 | 2.5 | 0 | 0.0 | 0.0 |
| ILE (I) | ATA | 1 | 4.2 | 5 | 20.8 | 21.1 | | GTC | 21 | 52.5 | 11 | 27.5 | 27.0 |
| | ATC | 15 | 62.5 | 10 | 41.7 | 42.7 | | GTG | 4 | 10.0 | 13 | 32.5 | 31.7 |
| | ATT | 8 | 33.3 | 9 | 37.5 | 36.2 | | GTT | 14 | 35.0 | 16 | 40.0 | 41.3 |
| | Totals | 232 | | 232 | | | | Totals | 244 | | 244 | | |

**Table 7:** Codon compositions of coding regions for a HzD9DS protein. The native *H. zea* desaturase coding region is compared to a Plant-Optimized version.

| Amino Acid | Codon | Native Gene # | Native Gene % | Plnt Opt Gene # | Plnt Opt Gene % | Plnt Opt Recm'd | Amino Acid | Codon | Native Gene # | Native Gene % | Plnt Opt Gene # | Plnt Opt Gene % | Plnt Opt Recm'd |
|---|---|---|---|---|---|---|---|---|---|---|---|---|---|
| **ALA (A)** | **GCA** | 4 | 11.4 | 9 | 25.7 | 23.3 | **LEU (L)** | **CTA** | 2 | 5.9 | 0 | 0.0 | 0.0 |
| | **GCC** | 7 | 20.0 | 7 | 20.0 | 21.2 | | **CTC** | 8 | 23.5 | 10 | 29.4 | 28.5 |
| | **GCG** | 8 | 22.9 | 4 | 11.4 | 14.2 | | **CTG** | 14 | 41.2 | 6 | 17.6 | 14.6 |
| | **GCT** | 16 | 45.7 | 15 | 42.9 | 41.3 | | **CTT** | 6 | 17.6 | 10 | 29.4 | 31.6 |
| **ARG (R)** | **AGA** | 1 | 7.7 | 6 | 46.2 | 43.8 | | **TTA** | 2 | 5.9 | 0 | 0.0 | 0.0 |
| | **AGG** | 5 | 38.5 | 4 | 30.8 | 30.5 | | **TTG** | 2 | 5.9 | 8 | 23.5 | 25.3 |
| | **CGA** | 2 | 15.4 | 0 | 0.0 | 0.0 | **LYS(K)** | **AAA** | 11 | 44.0 | 10 | 40.0 | 44.6 |
| | **CGC** | 5 | 38.5 | 0 | 0.0 | 0.0 | | **AAG** | 14 | 56.0 | 15 | 60.0 | 55.4 |
| | **CGG** | 0 | 0.0 | 0 | 0.0 | 0.0 | **MET (M)** | **ATG** | 8 | 100 | 8 | 100 | 100.0 |
| | **CGT** | 0 | 0.0 | 3 | 23.1 | 25.7 | **PHE(F)** | **TTC** | 20 | 83.3 | 14 | 58.3 | 58.6 |
| **ASN (N)** | **AAC** | 13 | 72.2 | 11 | 61.1 | 62.6 | | **TTT** | 4 | 16.7 | 10 | 41.7 | 41.4 |
| | **AAT** | 5 | 27.8 | 7 | 38.9 | 37.4 | **PRO (P)** | **CCA** | 1 | 6.3 | 5 | 31.3 | 29.6 |
| **ASP (D)** | **GAC** | 16 | 64.0 | 12 | 48.0 | 42.5 | | **CCC** | 5 | 31.3 | 3 | 18.8 | 14.6 |
| | **GAT** | 9 | 36.0 | 13 | 52.0 | 57.5 | | **CCG** | 2 | 12.5 | 2 | 12.5 | 18.4 |
| **CYS (C)** | **TGC** | 1 | 100.0 | 0 | 0.0 | 49.2 | | **CCT** | 8 | 50.0 | 6 | 37.5 | 37.3 |
| | **TGT** | 0 | 0.0 | 1 | 100.0 | 50.8 | **SER (S)** | **AGC** | 2 | 12.5 | 3 | 18.8 | 17.9 |
| **END** | **TAA** | 1 | 100.0 | 0 | 0.0 | 0.0 | | **AGT** | 1 | 6.3 | 3 | 18.8 | 15.8 |
| | **TAG** | 0 | 0.0 | 0 | 0.0 | 0.0 | | **TCA** | 1 | 6.3 | 3 | 18.8 | 20.4 |
| | **TGA** | 0 | 0.0 | 1 | 100.0 | 100.0 | | **TCC** | 6 | 37.5 | 3 | 18.8 | 18.7 |
| **GLN (Q)** | **CAA** | 2 | 33.3 | 3 | 50.0 | 50.0 | | **TCG** | 3 | 18.8 | 0 | 0.0 | 0.0 |

(continued)

| Amino Acid | Codon | Native Gene # | Native Gene % | PInt Opt Gene # | PInt Opt Gene % | PInt Opt Recm'd | Amino Acid | Codon | Native Gene # | Native Gene % | PInt Opt Gene # | PInt Opt Gene % | PInt Opt Recm'd |
|---|---|---|---|---|---|---|---|---|---|---|---|---|---|
| | CAG | 4 | 66.7 | 3 | 50.0 | 50.0 | | TCT | 3 | 18.8 | 4 | 25.0 | 27.2 |
| GLU (E) | GAA | 7 | 63.6 | 5 | 45.5 | 43.6 | THR (T) | ACA | 3 | 16.7 | 5 | 27.6 | 26.3 |
| 16 | GAG | 4 | 36.4 | 6 | 54.5 | 56.4 | | ACC | 7 | 38.9 | 5 | 27.8 | 26.9 |
| GLY (G) | GGA | 8 | 40.0 | 9 | 45.0 | 36.4 | | ACG | 4 | 22.2 | 3 | 16.7 | 16.9 |
| | GGC | 6 | 30.0 | 4 | 20.0 | 16.2 | | ACT | 4 | 22.2 | 5 | 27.8 | 30.0 |
| | GGG | 2 | 10.0 | 3 | 15.0 | 15.2 | TRP (W) | TGG | 14 | 100 | 14 | 100 | 100.0 |
| | GGT | 4 | 20.0 | 4 | 20.0 | 32.1 | TYR (Y) | TAC | 12 | 80.0 | 9 | 60.0 | 59.4 |
| HIS (H) | CAC | 11 | 73.3 | 8 | 53.3 | 49.6 | | TAT | 3 | 20.0 | 6 | 40.0 | 40.6 |
| | CAT | 4 | 26.7 | 7 | 46.7 | 50.4 | VAL (V) | GTA | 0 | 0.0 | 0 | 0.0 | 0.0 |
| ILE (I) | ATA | 3 | 15.0 | 4 | 20.0 | 21.1 | | GTC | 5 | 26.3 | 5 | 26.3 | 27.0 |
| | ATC | 10 | 50.0 | 9 | 45.0 | 42.7 | | GTG | 13 | 68.4 | 6 | 31.6 | 31.7 |
| | ATT | 7 | 35.0 | 7 | 35.0 | 36.2 | | GTT | 1 | 5.3 | 8 | 42.1 | 41.3 |
| | Totals | 165 | | 165 | | | | Totals | 189 | | 189 | | |

**Table 8:** Codon compositions of coding regions for a LnD9DS-2 protein. The native *L. nodorum* desaturase coding region is compared to a Plant-Optimized version.

| Amino Acid | Codon | Native Gene # | Native Gene % | PInt Opt Gene # | PInt Opt Gene % | Pint Opt Recm'd | Amino Acid | Codon | Native Gene # | Native Gene % | PInt Opt Gene # | PInt Opt Gene % | PInt Opt Recm'd |
|---|---|---|---|---|---|---|---|---|---|---|---|---|---|
| ALA (A) | GCA | 3 | 9.4 | 7 | 21.9 | 23.3 | LEU (L) | CTA | 7 | 15.6 | 0 | 0.0 | 0.0 |
| | GCC | 9 | 28.1 | 7 | 21.9 | 21.2 | | CTC | 14 | 31.1 | 13 | 28.9 | 28.5 |
| | GCG | 12 | 37.5 | 5 | 15.6 | 14.2 | | CTG | 7 | 15.6 | 7 | 15.6 | 14.6 |
| | GCT | 8 | 25.0 | 13 | 40.6 | 41.3 | 41.3 | CTT | 5 | 11.1 | 14 | 31.1 | 31.6 |
| ARG (R) | AGA | 4 | 13.8 | 13 | 44.8 | 43.8 | | TTA | 3 | 6.7 | 0 | 0.0 | 0.0 |
| | AGG | 3 | 10.3 | 9 | 31.0 | 30.5 | | TTG | 9 | 20.0 | 11 | 24.4 | 25.3 |
| | CGA | 7 | 24.1 | 0 | 0.0 | 0.0 | LYS (K) | AAA | 9 | 45.0 | 9 | 45.0 | 44.6 |
| | CGC | 8 | 27.6 | 0 | 0.0 | 0.0 | | AAG | 11 | 55.0 | 11 | 55.0 | 55.4 |
| | CGG | 5 | 17.2 | 0 | 0.0 | 0.0 | MET (M) | ATG | 9 | 100 | 9 | 100 | 100.0 |
| | CGT | 2 | 6.9 | 7 | 24.1 | 25.7 | PHE (F) | TTC | 16 | 80.0 | 12 | 60.0 | 58.6 |
| ASN N) | AAC | 6 | 50.0 | 8 | 66.7 | 62.6 | | TTT | 4 | 20.0 | 8 | 40.0 | 41.4 |
| | AAT | 6 | 50.0 | 4 | 33.3 | 37.4 | PRO (P) | CCA | 3 | 16.7 | 5 | 27.8 | 29.6 |
| ASP (D) | GAC | 16 | 66.7 | 10 | 41.7 | 42.5 | | CCC | 8 | 44.4 | 3 | 16.7 | 14.6 |
| | GAT | 8 | 33.3 | 14 | 58.3 | 57.5 | | CCG | 2 | 11.1 | 3 | 16.7 | 18.4 |
| CYS (C) | TGC | 4 | 80.0 | 2 | 40.0 | 49.2 | | CCT | 5 | 27.8 | 7 | 38.9 | 37.3 |
| | TGT | 1 | 20.0 | 3 | 60.0 | 50.8 | SER (S) | AGC | 8 | 27.6 | 5 | 17.2 | 17.9 |
| END | TAA | 0 | 0.0 | 0 | 0.0 | 0.0 | | AGT | 6 | 20.7 | 5 | 17.2 | 15.8 |
| | TAG | 1 | 100.0 | 0 | 0.0 | 0.0 | | TCA | 1 | 3.4 | 6 | 20.7 | 20.4 |
| | TGA | 0 | 0.0 | 1 | 100.0 | 100.0 | | TCC | 6 | 20.7 | 5 | 17.2 | 18.7 |
| GLN (Q) | CAA | 10 | 55.6 | 10 | 55.6 | 50.0 | | TCG | 7 | 24.1 | 0 | 0.0 | 0.0 |

(continued)

| Amino Acid | Codon | Native Gene # | Native Gene % | PInt Opt Gene # | PInt Opt Gene % | Pint Opt Recm'd | Amino Acid | Codon | Native Gene # | Native Gene % | PInt Opt Gene # | PInt Opt Gene % | PInt Opt Recm'd |
|---|---|---|---|---|---|---|---|---|---|---|---|---|---|
| | CAG | 8 | 44.4 | 8 | 44.4 | 50.0 | | TCT | 1 | 3.4 | 8 | 27.6 | 27.2 |
| GLU (E) | GAA | 5 | 33.3 | 7 | 46.7 | 43.6 | THR (T) | ACA | 11 | 44.0 | 7 | 28.0 | 26.3 |
| 16 | GAG | 10 | 66.7 | 8 | 53.3 | 56.4 | | ACC | 5 | 20.0 | 7 | 28.0 | 26.9 |
| GLY (G) | GGA | 13 | 34.2 | 14 | 36.8 | 36.4 | | ACG | 7 | 28.0 | 4 | 16.0 | 16.9 |
| | GGC | 16 | 42.1 | 6 | 15.8 | 16.2 | | ACT | 2 | 8.0 | 7 | 28.0 | 30.0 |
| | GGG | 6 | 15.8 | 6 | 15.8 | 15.2 | TRP (W) | TGG | 19 | 100 | 19 | 100 | 100.0 I |
| | GGT | 3 | 7.9 | 12 | 31.6 | 32.1 | TYR(Y) | TAC | 11 | 64.7 | 10 | 58.8 | 59.4 |
| HIS (H) | CAC | 12 | 66.7 | 9 | 50.0 | 49.6 | | TAT | 6 | 35.3 | 7 | 41.2 | 40.6 |
| | CAT | 6 | 33.3 | 9 | 50.0 | 50.4 | VAL (V) | GTA | 6 | 17.6 | 0 | 0.0 | 0.0 |
| ILE (I) | ATA | 4 | 18.2 | 5 | 22.7 | 21.1 | | GTC | 10 | 29.4 | 9 | 26.5 | 27.0 |
| | ATC | 9 | 40.9 | 10 | 45.5 | 42.7 | | GTG | 12 | 35.3 | 11 | 32.4 | 31.7 |
| | ATT | 9 | 40.9 | 7 | 31.8 | 36.2 | | GTT | 6 | 17.6 | 14 | 41.2 | 41.3 |
| | Totals | 214 | | 214 | | | | Totals | 236 | | 236 | | |

Syntheses of DNA fragments comprising SEQ ID NO:15, SEQ ID NO:16, and SEQ ID NO:17 were performed by commercial suppliers (PicoScript, Houston, TX and Blue Heron Biotechnology, Bothell, WA). These canola-optimized sequences were labeled as version 2 (v2). The synthetic DNA fragments were then cloned into expression vectors, and transformed into *Agrobacterium* and canola as described in the Examples below.

**Example 3: Plasmid construction**

The following plasmids were constructed using standard molecular biology techniques. Polynucleotide fragments containing plant transcription units (comprised of a promoter linked to a gene of interest, terminated by a 3'UTR), or "PTUs," were constructed and combined with additional plant transcription units within the T-strand region of a binary vector.

Description of pDAB7318: pDAB7318 (**FIG. 6**; SEQ ID NO:58) was constructed using standard molecular biology techniques. This plasmid contains two desaturase PTU sequences. The first desaturase PTU contains the *Phaseolus vulgaris* phaseolin promoter (PvPhas promoter v2 (SEQ ID NO:67); Genbank: J01263), *Phaseolus vulgaris* 5' untranslated region (PvPhas 5' UTR (SEQ ID NO:68); Genbank: J01263), AnD9DS v3 gene (SEQ ID NO:49), *Phaseolus vulgaris* 3' untranslated region (PvPhas 3' UTR v1 (SEQ ID NO:69); Genbank: J01263) and *Phaseolus vulgaris* matrix attachment region (PvPhas 3' MAR v2 (SEQ ID NO:70); Genbank: J01263). The second desaturase PTU contains the PvPhas promoter v2, PvPhas 5' UTR, LnD9DS-2 v2 (SEQ ID NO:17), and *Agrobacterium tumefaciens* ORF23 3' untranslated region (AtuORF23 3' UTR (SEQ ID NO:71); Huang et al. (1990) J. Bacteriol. 172:1814-22).

The elements in the desaturase PTUs are connected by additional short intervening sequences. The two desaturase PTU sequences are flanked by Invitrogen's Gateway® Recombination sites, which are used to facilitate the transfer of these PTU expression cassettes into the *Agrobacterium* transformation plasmid. Additionally, the plasmid contains an origin of replication, and a kanamycin selectable marker.

Description of pDAB7319: pDAB7319 (**FIG. 7**; SEQ ID NO:60) was constructed *via* Gateway® recombination between pDAB7318 and pDAB7309 (**FIG. 5**; SEQ ID NO:53). This plasmid contains the two desaturase PTU sequences set forth in the preceding "Description of pDAB7318." These PTUs were orientated in a head-to-tail orientation within the T-strand DNA border regions of the plant transformation binary vector, pDAB7309. This binary vector contains the phosphinothricin acetyl transferase PTU, which consists of the Cassava vein Mosaic Virus Promoter (CsVMV promoter v2; Verdaguer et al. (1996) Plant Mol. Biol. 31:1129-39); phosphinothricin acetyl transferase (PAT v5; Wohlleben et al. (1988) Gene 70:25-37); and *Agrobacterium tumefaciens* ORF1 3' untranslated region (AtuORF1 3'UTR v4; Huang *et al.* (1990), *supra*), in addition to other regulatory elements such as the *Nicotiana tabacum* RB7 Matrix Attachment Region (RB7 MARv2; Genbank: U67919), Overdrive (Toro et al. (1988) Proc. Natl. Acad. Sci. U.S.A. 85(22):8558-62), and T-strand border sequences (T-DNA Border A and T-DNA Border B; Gardner et al. (1986) Science 231:725-7, and PCT International Patent Publication No. WO2001/025459A1). Plasmids containing the PTUs described above were isolated and confirmed *via* restriction enzyme digestion and DNA sequencing.

Description of pDAB7320: pDAB7320 (**FIG. 8**; SEQ ID NO:55) was constructed using standard molecular biology techniques. This plasmid contains one desaturase PTU sequence. The desaturase PTU contains the PvPhas promoter v2, PvPhas 5' UTR, LnD9DS-2 v2 (SEQ ID NO:17), and the AtuORF23 3' UTR. The elements in the desaturase PTUs are connected by additional short intervening sequences. The desaturase PTU sequence also is flanked by Invitrogen's Gateway® Recombination sites to facilitate its transfer into an *Agrobacterium* transformation plasmid. Additionally, the plasmid contains an origin of replication and kanamycin selectable marker.

Description of pDAB7321: pDAB7321 (**FIG. 9**; SEQ ID NO:61) was constructed *via* Gateway® recombination between pDAB7320 and pDAB7309. This plasmid contains the desaturase PTU sequence set forth in the preceding "Description of pDAB7319." This PTU was orientated in a head-to-tail orientation within the T-strand DNA border regions of the plant transformation binary vector, pDAB7309. This binary vector contains the phosphinothricin acetyl transferase PTU: CsVMV promoter v2; PAT v5; and AtuORF1 3'UTR v4, in addition to other regulatory elements such as Overdrive and T-strand border sequences (T-DNA Border A and T-DNA Border B). Plasmids containing the PTU described above were isolated and confirmed *via* restriction enzyme digestion and DNA sequencing.

Description of pDAB7323: pDAB7323 (**FIG. 10**; SEQ ID NO:56) was constructed using standard molecular biology techniques. This plasmid contains two desaturase PTU sequences. The first desaturase PTU contains the PvPhas promoter v2, PvPhas 5' UTR, AnD9DS v3 (SEQ ID NO:47), PvPhas 3' UTR, and PvPhas 3' MAR v2. The second desaturase PTU contains the PvPhas promoter v2, PvPhas 5' UTR, HzD9DS v2 (SEQ ID NO:16), and AtuORF23 3' UTR. The elements in the desaturase PTUs are connected by additional short intervening sequences. The two desaturase PTU sequences are flanked by Invitrogen's Gateway® Recombination sites to facilitate their transfer into an *Agrobacterium* transformation plasmid. Additionally, the plasmid contains an origin of replication and kanamycin selectable marker.

Description of pDAB7324: pDAB7324 (**FIG. 11**; SEQ ID NO:62) was constructed *via* Gateway® recombination between pDAB7323 and pDAB7309. This plasmid contains the two desaturase PTU sequences set forth in the preceding "Description of pDAB7323." These PTUs were orientated in a head-to-tail orientation within the T-strand DNA border regions of the plant transformation binary vector, pDAB7309. This binary vector contains the phosphinothricin acetyl transferase PTU: CsVMV promoter v2; PAT v5; and AtuORF1 3'UTR v4, in addition to other regulatory elements such as Overdrive and T-stand border sequences (T-DNA Border A and T-DNA Border B). Plasmids containing the PTUs described above were isolated and confirmed *via* restriction enzyme digestion and DNA sequencing.

Description of pDAB7325: pDAB7325 (**FIG. 12**; SEQ ID NO:57) was constructed using standard molecular biology techniques. This plasmid contains one desaturase PTU sequence. This desaturase PTU contains the PvPhas promoter v2, PvPhas 5' UTR, HzD9DS v2 (SEQ ID NO:16), and AtuORF23 3' UTR. The elements in the desaturase PTU are connected by additional short intervening sequences, and the desaturase PTU sequence is flanked by Invitrogen's Gateway® Recombination sites to facilitate its transfer into an *Agrobacterium* transformation plasmid. Additionally, the plasmid contains an origin of replication and kanamycin selectable marker.

Description of pDAB7326: pDAB7326 (**FIG. 13**; SEQ ID NO:63) was constructed *via* Gateway® recombination between pDAB7325 and pDAB7309. This plasmid contains the desaturase PTU sequence set forth in the preceding "Description of pDAB7325." The PTU was orientated in a head-to-tail orientation within the T-strand DNA border regions of the plant transformation binary vector, pDAB7309. This binary vector contains the phosphinothricin acetyl transferase PTU: CsVMV promoter v2; PAT v5; and AtuORF1 3'UTR v4, in addition to other regulatory elements such as Overdrive and T-stand border sequences (T-DNA Border A and T-DNA Border B). Plasmids containing the PTU described above were isolated and confirmed *via* restriction enzyme digestion and DNA sequencing.

Description of pDAB7327: pDAB7327 (**FIG. 14**; SEQ ID NO:58) was constructed using standard molecular biology techniques. This plasmid contains one desaturase PTU sequence. The desaturase PTU contains the PvPhas promoter v2, PvPhas 5' UTR, AnD9DS v3 gene (SEQ ID NO:49), and AtuORF23 3' UTR. The elements in the desaturase PTU are connected by additional short intervening sequences. The desaturase PTU sequence also is flanked by Invitrogen's Gateway® Recombination sites to facilitate its transfer into an *Agrobacterium* transformation plasmid. Additionally, the plasmid contains an origin of replication and kanamycin selectable marker.

Description of pDAB7328: pDAB7328 (**FIG. 15**; SEQ ID NO:64) was constructed *via* Gateway® recombination between pDAB7327 and pDAB7309. This plasmid contains the desaturase PTU sequence set forth in the preceding "Description of pDAB7327." This PTU was orientated in a head-to-tail orientation within the T-strand DNA border regions of the plant transformation binary vector, pDAB7309. This binary vector contains the phosphinothricin acetyl transferase PTU: CsVMV promoter v2; PAT v5; and AtuORF1 3'UTR v4, in addition to other regulatory elements such as Overdrive and T-stand border sequences (T-DNA Border A and T-DNA Border B). Plasmids containing the PTU described above were isolated and confirmed *via* restriction enzyme digestion and DNA sequencing.

Description of pDAB7329: pDAB7329 (**FIG. 16**; SEQ ID NO:59) was constructed using standard molecular biology techniques. This plasmid contains one desaturase PTU sequence, which contains the PvPhas promoter v2, PvPhas 5' UTR, MgD9DS v2 (SEQ ID NO:15), and AtuORF23 3' UTR. The elements in this desaturase PTU are connected by additional short intervening sequences. The desaturase PTU sequence is flanked by Invitrogen's Gateway® Recombination sites to facilitate its transfer into an *Agrobacterium* transformation plasmid. Additionally, the plasmid contains an origin of replication and kanamycin selectable marker.

Description of pDAB7330: pDAB7330 (**FIG. 17**; SEQ ID NO:65) was constructed *via* Gateway® recombination between pDAB7329 and pDAB7309. This plasmid contains the desaturase PTU sequence set forth in the preceding "Description of pDAB7325." This PTU was orientated in a head-to-tail orientation within the T-strand DNA border regions of the plant transformation binary vector, pDAB7309. This binary vector contains the phosphinothricin acetyl transferase PTU: CsVMV promoter v2; PAT v5; and AtuORF1 3'UTR v4, in addition to other regulatory elements such as Overdrive and T-stand border sequences (T-DNA Border A and T-DNA Border B). Plasmids containing the PTU described above were isolated and confirmed *via* restriction enzyme digestion and DNA sequencing.

Description of pDAB7331: In addition to the foregoing, a control plasmid that did not contain a desaturase PTU was constructed (SEQ ID NO:66). **FIG**. **18**. This construct only contained the phosphinothricin acetyl transferase PTU, in addition to the other regulatory elements described in pDAB7309.

### Example 4: *Agrobacterium* Transformation

Electro-competent *Agrobacterium tumefaciens* cells (**Table 9**) were prepared using a protocol from Weigel and Glazebrook (2002) "How to Transform Arabidopsis," Ch. 5, in Arabidopsis, Cold Spring Harbor Laboratory Press, Cold Spring Harbor, NY. 50 μL of competent *Agrobacterium* cells were thawed on ice, and were transformed using 300 to 400 ng of binary vector plasmid DNA. The cell mix was electroporated in the presence of the DNA, using pre-chilled electroporation cuvettes (0.2 cm), and a Bio-Rad Gene Pulser® electroporator (Hercules, CA) under the following conditions: Voltage: 2.5 kV, Pulse length: 5 msec, capacitance output 25 μF, resistance 200 Ω. After electroporation, 1 mL of YEP broth (Yeast Extract (10 g/L), Peptone (10 g/L), and NaCl (5 g/L)) was added to each cuvette, and the cell-YEP suspension was transferred to a 15 mL culture tube. The cells were incubated at 28 °C with gentle agitation for 4 hours, after which the culture was plated on YEP + agar with the appropriate selection according to **Table 9**. The plates were incubated for 2-4 days at 28 °C, and colonies were selected and streaked onto fresh YEP + agar plates with antibiotic selection and incubated at 28 °C for 1-3 days. Colonies were verified as *Agrobacterium* using the Ketolactose test, and Ketolactose positive colonies were further isolated using two passages of single colony isolation. A final patch plate was made of colonies after single colony isolation was completed.

**Table 9.** *Agrobacterium* strains and antibiotic selection.

| Strain | Genomic Selection | Ti Helper Selection | Binary Vector Selection |
|---|---|---|---|
| **Z707S** | Streptomycin | Kanamycin | Spectinomycin |
| **DA2569** | Erythromycin | Kanamycin | Spectinomycin |
| **EHA105** | Streptomycin | None Available | Spectinomycin |
| **DA2552** | Erythromycin | None | Spectinomycin |

*Agrobacterium* Colony Validation: Restriction digestion analysis was used to verify the presence of the intact plasmid by using vector specific restriction digest enzymes. Macherey-Nagel NucleoBond® Plasmid DNA kits were used according to the manufacturer's recommended protocol to purify the plasmid DNA from selected transformed *Agrobacterium* colonies. Plasmid DNA from the binary vector used in the *Agrobacterium* transformation was included as a control. Four separate digest reactions were run using 0.75-1 μg of DNA. The reaction was allowed to run for 1-2 hrs, and was then analyzed by agarose gel electrophoresis and ethidium bromide staining. Colonies were selected for which the digests for all enzymes were identical to the plasmid control and matched the expected band sizes.

*A. tumefaciens* strain LBA404 (Invitrogen Carlsbad, California) was used for *Arabidopsis* transformation, and *A. tumefaciens* strain Z707S (Hepburn et al. (1985) J. Gen. Microbiol. 131:2961-9) was used for canola transformation.

**Example 5: *Agrobacterium*-mediated Transformation of *Arabidopsis thaliana***

*Arabidopsis* Transformation: *Arabidopsis* was transformed using a floral dip method based on the method of Clough and Bent (1998) Plant J. 16:735-743. A selected *Agrobacterium* colony was used to inoculate one or more 30 mL pre-cultures of YEP broth containing appropriate antibiotics for selection. The culture(s) were incubated overnight at 28 °C with constant agitation at 220 rpm. Each pre-culture was used to inoculate two 500 mL cultures of YEP broth containing antibiotics for selection, and the cultures were incubated overnight at 28 °C with constant agitation. The cells were then plated at approx. 8700 g for 10 minutes at room temperature, and the resulting supernatant was discarded. The cell pellet was gently resuspended in 500 mL infiltration media containing: 1/2x Murashige and Skoog salts/Gamborg's B5 vitamins, 10% (w/v) sucrose, 0.044 μM benzylamino purine (10 μL/liter of 1 mg/mL stock in DMSO), and 300 μL/liter Silwet® L-77. Plants approximately 1 month old were dipped into the media for 15 seconds, with care taken to submerge the newest inflorescence. The plants were then laid down on their sides, and covered (transparent or opaque) for 24 hours, then washed with water, and placed upright. The plants were grown at 22 °C, with a 16-hour light/8-hour dark photoperiod. Approximately 4 weeks after dipping, seeds were harvested from the plants.

*Arabidopsis thaliana* Growth Conditions: Freshly harvested seed was dried for 7 days at room temperature in the presence of a desiccant. After drying, seed was suspended in a 0.1% Agarose (Sigma Chemical Co., St. Louis, MO) solution. The suspended seed was stored at 4 °C for 2 days to complete dormancy requirements and ensure synchronous seed germination (stratification). Sunshine Mix LP5 (Sun Gro Horticulture Inc., Bellevue, WA) was covered with fine vermiculite and sub-irrigated with Hoaglan's solution until wet. The soil mix was drained for 24 hours. Stratified seed was sown onto the vermiculite and covered with humidity domes (KORD Products, Bramalea, Ontario, Canada) for 7 days. Seeds were germinated, and plants were grown in a Conviron controller (models CMP4030 and CMP3244, Controlled Environments Limited, Winnipeg, Manitoba, Canada) under long day conditions (16-hours light/8-hours dark) at a light intensity of 120-150 $\mu mol/m^2 sec$ under constant temperature (22 °C) and humidity (40-50%). Plants were initially watered with Hoaglan's solution, and subsequently with deionized water to keep the soil moist but not wet. Plants nearing seed harvest (1-2 weeks before harvest) were dried out.

Selection of $T_1$ Transformed Plants: $T_1$ seed was sown on 10.5" x 21" germination trays (T.O. Plastics Inc., Clearwater, MN) as described above and grown under the conditions outlined. The domes were removed 5-6 days post-sowing. 5 days post-sowing, and again 10 days post-sowing, seedlings were sprayed with a 0.20% solution of glufosinate herbicide (Liberty) in a spray volume of 10 mL/tray (703 L/ha) using a DeVilbiss compressed air spray tip to deliver an effective rate of 280 g/ha glufosinate per application. 10 mL of the glufosinate herbicide solution was pipetted into a 20 mL scintillation vial for each tray to be sprayed. The spray was delivered using a horizontal and vertical application pattern. After each spray, a spray label with the herbicide name, application rate, and application date was added to each selection tray. 4 to 7 days after the second spray, herbicide-resistant plants were identified and transplanted into pots prepared with Sunshine mix LP5. Transplanted plants were placed in a greenhouse with the above mentioned growth conditions. Six to eight weeks after transplanting, the seed from each plant was harvested and stored separately with a unique identification number.

**Example 7: *Agrobacterium*-mediated transformation of canola**

*Agrobacterium* Preparation: *Agrobacterium* strains containing either pDAB7319, pDAB7321, pDAB7324, pDAB7326, pDAB7328, pDAB7330 or pDAB7331 were used to streak YEP (Bacto Peptone (20.0 g/L) and Yeast Extract (10.0 g/L)) plates containing streptomycin (100 mg/mL) and spectinomycin (50 mg/mL), and incubated for 2 days at 28 °C. A loop of the 2-day streak plate was inoculated into 150 mL modified YEP liquid with streptomycin (100 mg/mL) and spectinomycin (50 mg/mL) into sterile 500 mL baffled flask(s) and shaken at 200 rpm at 28 °C. The cultures were resuspended in M-medium (LS salts; 3% glucose; modified B5 vitamins; 1 μM kinetin; 1 μM 2,4-D; pH 5.8), and diluted to the appropriate density (50 Klett Units), prior to transformation of canola hypocotyls.

Canola Transformation:

*Seed germination:* Canola seeds (variety Nexera 710) were surface-sterilized in 10% Clorox for 10 minutes, and rinsed in steel strainers three times with sterile distilled water. Seeds were planted for germination on ½ MS Canola medium (1/2 MS, 2% sucrose, 0.8% Agar) contained in Phytatrays (25 seeds per Phytatray). The trays were placed in an environmental growth chamber (Percival Scientific, Inc., Perry, IA) with a growth regime set at 25 °C and a photoperiod of 16-hours light/8-hours dark, and germinated for 5 days.

*Pre-treatment*: On day 5, ~3 mm hypocotyl segments were aseptically excised, discarding the root and shoot sections (drying of hypocotyls was prevented by placing them into 10 mL of sterile milliQ water during the excision process). Hypocotyl segments were placed horizontally on sterile filter paper on callus induction medium, MSK1D1 (MS; 1 mg/L Kinetin; 1 mg/L 2,4-D; 3% sucrose; 0.7% Phytagar) for 3 days pre-treatment in an environmental growth chamber with a growth regime set at 22-23 °C and a photoperiod of 16-hours light/8-hours dark.

*Co-cultivation with Agrobacterium:* The day before *Agrobacterium* treatment, flasks of YEP medium containing the appropriate antibiotics were inoculated. Hypocotyl segments were transferred from filter paper to empty 100 x 25 mm petri dishes containing 10 mL of liquid M medium to prevent the hypocotyl segments from drying. A spatula was used at this stage to scoop the segments and transfer. The liquid M medium was removed with a pipette, and 40 mL of *Agrobacterium* suspension was added to the petri dish (500 segments with 40 mL of *Agrobacterium* solution). The segments were treated for 30 minutes with periodic swirling of the petri dish so that the hypocotyls stayed immersed in the *Agrobacterium* solution. At the end of the treatment period, the *Agrobacterium* solution was pipetted into a waste beaker, autoclaved, and discarded (the *Agrobacterium* solution was completely removed to prevent *Agrobacterium* overgrowth). The treated hypocotyls were transferred with forceps back to the original plates containing MSK1D1 with filter paper, with care taken to ensure that the segments did not dry. The hypocotyl segments, along with control segments, were returned to the an environmental growth chamber under reduced light intensity (by covering the plates with aluminum foil), and the treated hypocotyls were co-cultivated with *Agrobacterium* for 3 days.

*Callus induction on selection medium*: After 3 days of co-cultivation, the hypocotyl segments were transferred individually with forceps onto callus induction medium, MSK1D1H1 (MS; 1 mg/L Kinetin; 1 mg/L 2,4-D; 0.5 g/L MES; 5 mg/L $AgNO_3$; 300 mg/L Timentin; 200 mg/L Carbenicillin; 1 mg/L Herbiace; 3% sucrose; 0.7% Phytagar). The hypocotyl segments were anchored on the medium, but were not embedded in the medium.

*Selection and shoot regeneration*: After 7 days on callus induction medium, the callusing hypocotyl segments were transferred to Shoot Regeneration Medium 1 with selection, MSB3Z1H1 (MS; 3 mg/L BAP; 1 mg/L Zeatin; 0.5 g/L MES; 5 mg/L $AgNO_3$; 300 mg/L Timentin; 200 mg/L Carbenicillin; 1 mg/L Herbiace; 3% sucrose; 0.7% Phytagar). After 14 days, the hypocotyls with shoots were transferred to Regeneration Medium 2 with increased selection, MSB3Z1H3 (MS; 3 mg/L BAP; 1 mg/L Zeatin; 0.5 gm/L MES; 5 mg/L $AgNO_3$; 300 mg/L Timentin; 200 mg/L Carbenicillin; 3 mg/L Herbiace®; 3% sucrose; 0.7% Phytagar).

*Shoot elongation*: After 14 days, the segments with shoots were transferred to shoot elongation medium, MSMESH5 (MS; 300 mg/L Timentin; 5 mg/L Herbiace®; 2% sucrose; 0.7% TC Agar). Shoots that were already elongated were isolated and transferred to MSMESH5. After 14 days, the remaining shoots that had not elongated in the first round were placed on MSMESH5, and transferred to fresh selection medium of the same composition. At this stage, all remaining hypocotyl segments were discarded.

Shoots that elongated on MSB3Z1H3 medium after 2 weeks were isolated and transferred to MSMESH5 medium. Remaining shoots that had not elongated in the first round on MSMESH5 were isolated, and transferred to fresh selection medium of the same composition. At this stage all remaining hypocotyl segments were discarded.

*Root induction*: After 14 days, the shoots were transferred to MSMEST medium (MS; 0.5 g/L MES; 300 mg/L Timentin; 2% sucrose; 0.7% TC Agar) for root induction. The shoots that did not root in the first transfer on MSMEST medium were transferred for a second or third cycle on MSMEST medium until rooted plants were obtained. The shoots that did not elongate / root in the first transfer on MSMEST medium were transferred for a second or third cycle on MSMEST medium until rooted plants were obtained. Plants that rooted on MSMESH5 or MSMEST and were PCR-positive were sent for transplanting into soil. After hardening, the To canola plants were further analyzed for events which contained the transgene PTU cassettes. Plants were then transferred to a greenhouse, grown to maturity, and the seed was harvested for additional analysis.

**Example 8: DNA analysis of $T_1$ *Arabidopsis* leaf tissue and $T_0$ canola leaf tissue**

To canola plants and $T_1$ *Arabidopsis* plants were analyzed to identify plants which contained the PTU expression cassettes. Invader® assays were performed to initially screen samples of putatively transformed plants, and identify events which contained a single copy of the *pat* PTU. Events that were identified as single copy events were kept and further analyzed for the presence of the desaturase PTU(s) *via* PCR. Events that were PCR positive for the desaturase expression cassette PTU(s) were further analyzed *via* Southern blot analysis. Southern blot analysis was completed to confirm that the plants contained the gene expression cassette PTUs from the binary vector used to transform the plants. Single copy events containing all of the PTUs were selected for advancement.

DNA Isolation: Total genomic DNA (gDNA) was extracted from lyophilized leaf tissue using Qiagen's DNeasy® 96 Plant Kit (Qiagen, Valencia, CA). This gDNA was then diluted to 10 ng/μL (canola) or 0.7 ng/μL (*Arabidopsis*) for use in PCR and Invader® assays for copy number.

Invader® Analysis: Copy number analysis of the selectable marker, *pat,* was completed using the Invader® assay (Third Wave Technologies, Madison, WI). Genomic DNA was denatured at 95 °C for 10 minutes, chilled on ice, and mixed with a master mix of reagents containing oligonucleotide probes, dye molecules capable of fluorescence resonance energy transfer (FRET), and cleavase enzyme, according to the manufacturer's recommended protocol. The reactions contained probes for the internal reference genes. The *1-deoxyxylulose-5 phosphate reductoisomerase* (*DXR1*) gene was used as an internal reference gene for *Arabidopsis* Invader® assay reactions, and *high mobility group protein* gene (*HMGa)* was used as an internal reference gene for canola Invader® assay reactions. In addition, the plates contained 1 copy, 2 copy, and 4 copy standards, as well as wild-type control samples and blank wells containing no sample. The whole reaction was overlayed with mineral oil before incubation in a thermocycler at 63 °C for 1.5 hrs. The resulting reaction was read on a fluorometric plate reader (Synergy™ 2, BioTek Instruments, Winooski, VT). Readings were collected for both FAM (λ 485-528 nm) and RED (λ 560-620 nm) channels. From these, the fold-over-zero (*i.e.*, background) for each channel was determined for each sample by dividing the sample raw signal by the no template raw signal. From this data, a standard curve was constructed, and the best fit was determined by linear regression analysis. Using the parameters identified from this fit, the apparent *pat* copy number was then determined for each sample.

PCR Analysis: PCR analysis was completed using primers which amplified each plant transcription unit. These primers were located in the promoter (Phaseolin) and the 3' UTR (Phaseolin or ORF23). These same primer sets were used for PCR analysis of both canola and *Arabidopsis.* For PCR analysis of pDAB7319 and pDAB7324 events, primers MAS414 (SEQ ID NO: 18) and MAS415 (SEQ ID NO: 19) were used to amplify the first PTU. This PTU consisted of the Phaseolin promoter, a functional equivalent of an acyl-CoA delta-9 desaturase gene from *Aspergillus nidulans* (AnD9DS v3; SEQ ID NO:49), and the Phaseolin 3'UTR terminator. For PCR amplification of the second PTU in construct pDAB7319, primers MAS415 and MAS413 (SEQ ID NO: 20) were used. This PTU consists of the Phaseolin promoter, a functional equivalent of an acyl-CoA delta-9 desaturase gene from *Leptosphaeria nodorum* (LnD9DS-2 v2; SEQ ID NO:17), and the ORF23 3'UTR. The MAS415 and MAS413 primer pairs were also used to amplify the second PTU of events generated by transformation with pDAB7324 (Phaseolin promoter, *Helicoverpa zea* acyl-CoA delta-9 desaturase gene v2 (HzD9DS v2; SEQ ID NO:16), and ORF23 3'UTR). In addition, MAS415 and MAS413 primer pairs were used to amplify the PTUs in constructs pDAB7321 and pDAB7326.

The PCR reactions were carried out in 25 μL volumes using 20 ng genomic DNA, 5 units Ex Taq (Takara), 1x reaction buffer, 0.2 μM of each dNTP, and 0.8 μM of each primer. The amplification reactions were performed in a DNA Engine Tetrad® 2 thermal cycler (BioRad, Hercules, CA). The following cycling conditions were used for primers MAS413 and MAS415: 3 minutes at 94 °C; followed by 35 cycles of 30 sec at 94 °C, 30 sec at 63 °C, and 3 min at 72 °C; and a final extension of 10 minutes at 72 °C. The cycling conditions used for primers MAS414 and MAS415 were the same with the sole difference that the annealing temperature was reduced from 63 °C to 60 °C. The reaction products were run on a 1% agarose gel, stained with ethidium bromide, and visualized on a Gel-Doc™.

Southern Blot Analysis: Southern blot analysis was used to establish the integration pattern of the canola events. These experiments generated data which demonstrated the integration and integrity of the desaturase transgene within the canola genome. Selected events were characterized as a full-length, simple integration event containing a single copy of the desaturase transgene from the binary vector used for plant transformation.

Detailed Southern blot analysis was conducted using probes specific to the desaturase genes and descriptive restriction enzymes, which cleaved at sites located within the plasmid. These digests produced hybridizing fragments internal to the plasmid, or fragments that spanned the junction of the plasmid with canola genomic DNA (border fragments). The molecular sizes indicated from the Southern hybridization for the combination of the restriction enzymes and the probes were unique for each event. These analyses also showed that the plasmid fragment had been inserted into canola genomic DNA without rearrangements of the T-strand DNA.

For Southern blot analysis, 100 mg of lyophilized canola leaf tissue was extracted using the Plant Mini Kit (Qiagen). Five micrograms (5 $\mu$g) of gDNA per sample was digested simultaneously with *SpeI* and *PacI* restriction endonucleases (New England Biolabs, Ipswich, MA) to obtain fragments containing either the PTUs of interest, and/or the selectable marker (PAT), to determine copy number. The digested DNA was separated on a 0.8% agarose gel.

Briefly, following electrophoretic separation and visualization of the DNA fragments, the gels were depurinated with 0.25N HCl for approximately 20 minutes, and then exposed to a denaturing solution for approximately 30 minutes, followed by a neutralizing solution for at least 30 minutes. Southern transfer was performed overnight onto nylon membranes (Millipore, Billerica, MA) using a wicking system with 10× SSC. After transfer, the membranes were washed with a 2x SSC solution, and the DNA was bound to the membrane by UV crosslinking. This process produced Southern blot membranes ready for hybridization.

Probes were generated and PCR fragments were amplified from plasmid DNA and purified *via* gel extraction using the QIAquick® Gel Extraction kit (Qiagen). The primers used to create the LnD9DS probe were arw008 (SEQ ID NO:21) and arw009 (SEQ ID NO:22). The primers used to create the HzD9 probe were arw010 (SEQ ID NO:23) and arw011 (SEQ ID NO:24). PCR conditions for all three reactions consisted of 35 cycles with an annealing temperature of 63 °C and an extension time of 1 minute. The PCR fragments were labeled with $^{32}P$ using the Prime-It® RmT Random Primer Labeling kit (Stratagene, La Jolla, CA).

The hybridization step was conducted at approximately 65 °C overnight in the hybridization oven. The nylon membrane blots were rinsed, and the blot was exposed on a phosphor image screen overnight, and scanned on a Storm™ 860 Scanner (Molecular Dynamics, Sunnyvale, CA).

### Example 9: Fatty acid composition of seeds from transgenic *Arabidopsis* containing an acyl-CoA delta-9 desaturase

*Arabidopsis* plants were transformed with *Agrobacterium* vectors containing genes for LnD9DS-2 v2 (pDAB7321; SEQ ID NO:61), HzD9DS v2 (pDAB7326; SEQ ID NO:63) or MgD9DS v2 (pDAB7330; SEQ ID NO:65). Plants were also transformed with a vector containing a AnD9DS gene (pDAB7328; SEQ ID NO:64). An empty vector containing only the selectable marker *pat* gene (pDAB7331; SEQ ID NO:66) was used as a negative control. Transformations were also performed using two desaturases in combination, to combine a stearoyl-preferring desaturase (AnD9DS) with a palmitoyl-preferring desaturase, either LnD9DS-2 (pDAB7319; SEQ ID NO:60), or HzD9DS (pDAB7324; SEQ ID NO:62). In all cases, the desaturase genes were driven by the seed-specific PvPhas promoter (U.S. Patent 5,504,200). Bulk $T_2$ seed was harvested from herbicide-resistant $T_1$ plants that were confirmed to contain the *pat* gene by Invader® assay analysis and the desaturase PTU by PCR analysis.

Seed samples were homogenized in heptane-containing triheptadecanoin (Nu-Chek prep, Elysian, MN) as a surrogate using a steel ball and ball mill. Prior to homogenization, a solution of 0.25 M freshly-prepared MeONa (Sigma) in MeOH was added to the sample. The reaction was conducted under mild heat (40 °C) and constant shaking. The reaction was verified by the recovery of the methylated surrogate. Extraction of FAMEs was repeated three times, and all heptane layers were pooled prior to analysis. The completeness of the extraction was verified by checking for the presence of FAMEs in a fourth extraction/derivatization. The resulting FAMEs were analyzed by GC-FID using a capillary column BPX 70 from SGE (15 m x 0.25 mm x 0.25 $\mu$m). Each FAME was identified by retention time, and quantified by the injection of a rapeseed oil reference mix from Matreya, LLC (Pleasant Gap, PA), as a calibration standard.

FAME analysis of $T_2$ seed from the transgenic events showed that expression of each of the desaturases had a significant effect on reducing the total saturated fatty acid content of the seeds, as determined from the mean saturated fatty acid content of each set of events. **Table 10** and **FIG. 19**. In this table and the following tables, the values not connected by the same letter are significantly different, as determined using the Tukey-Kramer HSD test performed in the JMP® statistical software package (SAS Institute Inc., Cary, NC). Combinations of AnD9DS with LnD9DS-2 or HzD9DS yielded the lowest mean total saturated fatty acid content.

**Table 10:** Total saturated fatty acid content of $T_2$ *Arabidopsis* seed

| Gene | Number of T2 samples | | | | Mean Total Saturated FAs |
|---|---|---|---|---|---|
| Control | 204 | A | | | 13.49 |
| WT | 60 | A | | | 13.16 |

(continued)

| Gene | Number of T2 samples | | | | Mean Total Saturated FAs |
|---|---|---|---|---|---|
| MgD9DS v2 | 42 | | B | | 10.26 |
| LnD9DS-2 v2 | 49 | | B | | 10.00 |
| HzD9DS v2 | 70 | | B | | 9.58 |
| AnD9DS v3 | 32 | | | C | 8.73 |
| AnD9DS v3 + HzD9DS v2 | 39 | | | C | 8.23 |
| AnD9DS v3 + LnD9DS-2 v2 | 51 | | | C | 8.09 |

Although the desaturases all lowered the total saturated fatty acid content in *Arabidopsis* seeds, they had different effects on the palmitic and stearic acid fatty acid contents, as predicted from the yeast experiments. **Table 11** and **FIG. 20** show the mean palmitic acid content for each set of events. **Table 12** and **FIG. 21** show the mean stearic acid content of $T_2$ seed for each set of events.

**Table 11:** Palmitic acid content of $T_2$ *Arabidopsis* seed

| Gene | | | | | Mean Palmitic acid |
|---|---|---|---|---|---|
| Control | A | | | | 7.72 |
| WT | A | | | | 7.54 |
| MgD9DS v2 | | B | | | 7.19 |
| AnD9DS v3 | | | C | | 6.02 |
| LnD9DS-2 v2 | | | C | | 5.98 |
| HzD9DS v2 | | | | D | 5.57 |
| AnD9DS v3 + LnD9DS-2 v2 | | | | D | 5.54 |
| AnD9DS v3 + HzD9DS v2 | | | | D | 5.41 |

**Table 12:** Stearic acid content of $T_2$ *Arabidopsis* seed

| Gene | | | | Mean Stearic acid |
|---|---|---|---|---|
| Control | A | | | 2.96 |
| WT | A | | | 2.94 |
| LnD9DS-2 v2 | | B | | 2.09 |
| HzD9DS v2 | | B | | 2.04 |
| MgD9DS v2 | | | C | 1.53 |
| AnD9DS v3 + HzD9DS v2 | | | C | 1.42 |
| AnD9DS v3 | | | C | 1.35 |
| AnD9DS v3 + LnD9DS-2 v2 | | | C | 1.28 |

AnD9DS and MgD9DS had greater effects on the stearic acid content than LnD9DS-2 and HzD9DS. Conversely, LnD9DS-2 and HzD9DS had greater effects on the palmitic content than AnD9DS and MgD9DS. Combinations of the desaturases have the greatest effect on both fatty acids. These results were also observed in the effects of the desaturases on increasing the seed content of palmitoleic acid, which is the primary product of delta-9 desaturation of palmitic acid. **Table 13** and **FIG. 22**.

**Table 13:** Palmitoleic acid content of $T_2$ *Arabidopsis* seed

| Gene | | | | | | Mean Palmitoleic Acid |
|---|---|---|---|---|---|---|
| AnD9DS v3 + HzD9DS v2 | A | | | | | 3.32 |
| AnD9DS v3 + LnD9DS-2 v2 | A | | | | | 2.93 |
| HzD9DS v2 | | B | | | | 2.48 |
| AnD9DS v3 | | B | C | | | 2.10 |
| LnD9DS-2 v2 | | | C | | | 1.91 |
| MgD9DS v2 | | | | D | | 1.40 |
| Control | | | | | E | 0.31 |
| WT | | | | | E | 0.30 |

There was expected variation in the effect of the desaturases on saturated fatty acid content across the events analyzed, due to position and copy number effects. A comparison of the complete fatty acid profile of events with the lowest total saturated fatty acid content (average of the five lowest events) is shown in **Table 14** alongside the profile of seed from wild-type and control-transformed plants.

**Table 14:** Fatty acid profile of $T_2$ transgenic *Arabidopsis* with lowest total saturated fatty acid content. Standard deviations are in parentheses.

| | C14:0 | C16:0 | C16:1 | C18:0 | C18:1 | Vacc.* |
|---|---|---|---|---|---|---|
| WT | 0.08 (0.02) | 7.54 (0.41) | 0.31 (0.05) | 2.94 (0.19) | 14.91 (1.44) | 1.47 (0.10) |
| Control | 0.08 (0.02) | 7.72 (0.05) | 0.32 (0.04) | 2.96 (0.34) | 14.20 (2.04) | 1.46 (0.11) |
| AnD9DS v3 | 0.07 (0.01) | 5.10 (0.38) | 2.92 (0.55) | 0.72 (0.03) | 20.52 (2.12) | 1.72 (0.26) |
| HzD9DS v2 | 0.06 (0.00) | 4.13 (0.23) | 4.11 (0.47) | 1.26 (0.08) | 19.34 (1.01) | 1.94 (0.25) |
| LnD9DS-2 v2 | 0.05 (0.00) | 4.68 (0.30) | 3.49 (0.69) | 1.53 (0.12) | 19.35 (0.81) | 2.05 (0.21) |
| MgD9DS v2 | 0.08 (0.02) | 6.64 (0.26) | 1.60 (0.54) | 1.05 (0.20) | 18.01 (1.86) | 1.60 (0.16) |
| AnD9DS v3 + LnD9DS-2 v2 | 0.06 (0.00) | 4.41 (0.17) | 3.71 (0.35) | 0.97 (0.33) | 19.60 (0.88) | 2.03 (0.21) |
| AnD9DS v3 + HzD9DS v2 | 0.08 (0.02) | 4.86 (0.35) | 4.09 (0.65) | 1.01 (0.22) | 18.10 (2.40) | 2.03 (0.31) |

* Vacc. = cis-vaccenic acid (18:1 n-7)

| | C18:2 | C18:3 | C20:0 | C20:1 | C20:2 | C22:0 | C22:1 | C24:0 |
|---|---|---|---|---|---|---|---|---|
| WT | 28.72 (0.97) | 17.85 (0.81) | 2.06 (0.16) | 20.11 (0.90) | 1.78 (0.15) | 0.34 (0.10) | 1.68 (0.19) | 0.21 (0.10) |
| Control | 29.28 (1.29) | 18.07 (1.35) | 2.08 (0.16) | 19.62 (1.23) | 1.85 (0.17) | 0.39 (0.13) | 1.70 (0.04) | 0.27 (0.14) |
| AnD9DS v3 | 29.64 (1.34) | 17.59 (1.28) | 0.44 (0.04) | 18.26 (0.83) | 1.42 (0.15) | 0.24 (0.16) | 1.26 (0.09) | 0.10 (0.05) |
| HzD9DS v2 | 29.31 (0.94) | 17.26 (0.39) | 0.81 (0.06) | 18.39 (0.66) | 1.47 (0.10) | 0.18 (0.05) | 1.50 (0.04) | 0.23 (0.03) |
| LnD9DS-2 v2 | 27.72 (0.18) | 17.46 (0.55) | 1.00 (0.11) | 19.33 (0.46) | 1.45 (0.11) | 0.32 (0.14) | 1.48 (0.10) | 0.10 (0.09) |
| MgD9DS v2 | 29.76 (1.10) | 17.98 (0.84) | 0.63 (0.63) | 19.19 (0.86) | 1.60 (0.09) | 0.26 (0.20) | 1.44 (0.09) | 0.16 (0.03) |
| AnD9DS v3 + LnD9DS-2 v2 | 29.17 (0.31) | 18.84 (0.41) | 0.59 (0.27) | 17.65 (0.23) | 1.40 (0.04) | 0.39 (0.03) | 1.13 (0.06) | 0.03 (0.02) |
| AnD9DS v3 + HzD9DS v2 | 29.28 (1.78) | 18.83 (1.69) | 0.65 (0.21) | 17.88 (1.90) | 1.55 (0.20) | 0.20 (0.12) | 1.33 (0.24) | 0.11 (0.08) |

In addition to reducing the content of the saturated palmitic and stearic fatty acids, and increasing the monounsaturated fatty acid content (palmitoleic and oleic), the presence of the desaturases also lowered the amount of arachidic acid (C20:0) in the seeds. This is presumably because this fatty acid is derived from elongation of stearic and palmitic acids. There appeared to be no direct desaturation of C20:0 by the introduced desaturases, as there is no concomitant rise in eicosenoic acid (C20:1) as C20:1Δ9.

**Example 10: Delta-9 desaturase antibody preparation**

Diagnostic tools such as antibodies are desirable to characterize transgenic delta-9 desaturase protein expression in plants. Because acyl-CoA delta-9 desaturases are membrane-bound proteins, routine over-expression in *Escherichia coli* is difficult. However, antibodies were successfully generated by over-expression of a C-terminal fragment of each delta-9 desaturase protein that does not include any of the transmembrane domains of the protein.

Polymerase Chain Reactions: PCR primers were designed to amplify an equivalent C-terminal fragment for each desaturase. The 3' primer was designed to encode a protein fragment with a C-terminal 6x His tag. *NdeI* and *BamHI* restriction sites were incorporated into the 5' and 3' primers, respectively, to facilitate cloning. The primer sequences are given below in **Table 15**. The expected amplification products were 659 bp for LnD9DS-2, 683 bp for MgD9DS, and 335 bp for HzD9DS. PCR reactions were carried out using the Takara Ex Taq™ PCR kit (Clontech, Mountain View, CA) using supplier conditions. The total PCR reaction volume was 50 μL. Each reaction contained 200 ng of plasmid DNA and 50 pmol of each primer. The DNA was denatured at 94 °C for 1 min, followed by 30 cycles of 94 °C for 30 sec, 60 °C for 1 minute, and 72 °C for 30 sec. A final extension was carried out at 72 °C for 10 minutes. Each PCR product was run across two wells on a sterile 0.75% agarose gel, and DNA was gel purified using Montage spin columns and eluted in 15 μL TE buffer.

**Table 15:** Sequences of the oligonucleotide primers used in the PCR amplifications of C- terminal fragments from LnD9DS-2, MgD9DS, and HzD9DS.

| Primer | Sequence | Purpose |
|---|---|---|
| AntiLnD9DS2F | SEQ ID NO:25<br>CATATGTTCGACGACAGACGCACGCCTCGAGAC | Forward |
| AntiLnD9DS2Rh | SEQ ID NO:26<br>GGATCCGCAGCCACAGCCCCCTCAACCAACCTCTC | Reverse primer for LnD9DS2 C-terminal |
| AntiMgD9DSF | SEQ ID NO:27<br>CATATGTTCGACGATCGCAACTCGCCGCGTGATCAC | Forward primer for MgD9DS C-terminal |
| AntiMgD9DSRh | SEQ ID NO:28<br>GGATCCGCGGCCTGAGCACCCGGAACAGGCTG | Reverse primer for MgD9DS C-terminal |
| AntiHzD9DSF | SEQ ID NO:29<br>CATATGTATGACAAGTCCATCAAGCCTTCC | Forward primer for HzD9DS C-terminal |
| AntiHzD9DSRh | SEQ ID NO:30<br>GGATCCTCGTCTTTAGGGTTGATCCTAATGGCTGC | Reverse primer for HzD9DS C-terminal |

TOPO cloning: The purified C-terminal fragments were TA cloned into TOPO® pCR®2.1 vectors (Invitrogen, Carlsbad, CA), and transformed into Top 10 *E. coli* cells following the manufacturer's protocol (Invitrogen). Transformations were selected, and plasmid DNA was purified using NucleoSpin® columns (Macherey-Nagel GmbH & Co, Duren, Germany). Three microliters (3 μL) of DNA was digested with *NdeI* and *BamHI* in a total volume of 20 μL for 90 minutes at 37 °C, and run on a 0.8% agarose gel. In each case, a gene-specific fragment (plus a 3.9 kb TOPO® vector band) was visible. Three positive clones were chosen for each cloned gene and sequenced to confirm that the amplified PCR fragment was free of errors. Each of the MgD9DS clones contained a silent point mutation at base pair 45, indicating either a single nucleotide polymorphism between the published sequence and the PCR template, or a silent PCR error. Since the mutation was silent, no correction was necessary, and one clone was chosen for subcloning.

Preparation of the delta-9 desaturase C-terminal fragment expression plasmids: The PCR-amplified delta-9 desaturase fragments were digested with *NdeI* and *BamHI* restriction enzymes and ligated into corresponding restriction sites within the pET30b(+) expression vector. The cloning step resulted in the addition of 15 C-terminal amino acids, constituting a C-terminal 6x His tag to facilitate full-length protein purification. These additional amino acids were not expected to affect protein expression. Positive clones were obtained and confirmed *via* restriction enzyme digestion and sequencing reactions.

Expression of delta-9 desaturase C-terminal peptide fragments in *E. coli*: The delta-9 desaturase/pET30b(+) expression plasmids were transformed into BL21(DE3) *E. coli* cells according to the manufacturer's recommended protocol (Novagen, Madison, WI). Cells were plated on LA plates containing kanamycin (50 μg/mL) and glucose (1.25 M). The plates were incubated overnight at 37 °C. A full loop of cells was scraped from the plates, and inoculated into 500 mL flasks containing 250 mL LB and kanamycin (50 μg/mL) with isopropyl-P-D-thiogalactoside (0.75 mM) inducer. Three induction conditions were tested. Cultures were induced at different temperatures, and harvested at different times as follows: overnight (~18 hrs) at 28 °C; overnight at 16 °C; or 4 hours at 37 °C. Cells were harvested by centrifugation in 250 mL bottles at 6,000 rpm for 15 minutes, and then frozen at -20 °C.

Protein purification of delta-9 desaturase C-terminal peptide fragments: Cell pellets from 250 mL cultures were thawed and resuspended in 50 mL cold Phosphate Buffered Saline (PBS) containing 10% glycerol and 0.5 mL of Protease Inhibitor Cocktail (Sigma, St. Louis, MO) using a hand-held homogenizer. The cells were disrupted on ice for approximately 10 minutes using a Branson Model 450 Sonifier (Danbury, CT). Inclusion bodies were pelleted by centrifugation at 10,000 x g for 15 minutes, and extracted 2-3 times with PBS containing 0.5% Triton X-100 until the protein concentration of the supernatant reached baseline, as measured by a Bradford protein assay. The recovered inclusion bodies were solubilized in a PBS solution containing 6 M Urea and 5 mM DTT at room temperature with stirring for about 1 hour. Solubilized proteins were separated from insoluble materials by centrifugation at 30,000 x g for 15 minutes, and the retained supernatant was applied onto a 5 mL Ni-affinity column (GE Healthcare, HiTrap Chelating, Piscataway, NJ). The histidine tags of the C-terminal delta-9 desaturase peptides bound to the metal resin, and each fragment was eluted with a 50-200 mM imidazole gradient using an Akta® Explorer 100 (GE Healthcare, Piscataway, NJ). Fractions (3 mL each) were collected, and eluted peaks were analyzed by SDS-PAGE. Fractions containing C-terminal delta-9 desaturase peptide were pooled and concentrated using an Amicon® Ultra 10,000 MWCO filter device (Millipore, Billerica, MA) to less than 5 mL volume. The protein sample was then injected onto a Hi Load™ XK16/60 Superdex™ 200 size exclusion column (GE Healthcare, Piscataway, NJ), and equilibrated with 6 M Urea in 20 mM Tris-HCl, 150 mM NaCl, and 1 mM DTT. The peak fractions (4 mL each) containing pure C-terminal delta-9 desaturase peptide were saved (after validation by SDS-PAGE analysis and other biochemical characterization) and used for antibody production. Peptides with the expected sizes of 27 kDa for LnD9DS-2 peptide, 15 kDa for HzD9DS peptide, and 28 kDa for MgD9DS peptide were produced. The induction conditions produced sufficient protein for visualization by Coomassie blue staining of SDS-PAGE gels.

Polyclonal Antibody Production: A contract service (Strategic BioSolutions, Newark, DE) produced rabbit antibodies against each of the three C-terminal delta-9 desaturase peptides. Following their standard procedures, high titer (validated by using ELISA) antisera for each of the three protein fragments was obtained. Each purified C-terminal delta-9 desaturase peptide was diluted with 20 mM Tris-HCl, 150 mM NaCl, 1 mM DTT buffer, and with a final concentration of 2-3 M urea, to keep the protein in solution. Approximately 10 mg of protein was sent to Strategic BioSolutions for generation of a polyclonal antibody. Two rabbits were chosen for each immunogen, and standard protocols (70 days immunization) were used. A new adjuvant called TiterMax® Gold was purchased for preparation of the emulsion. ELISA titration during immunization and at the end of protocol was also performed to ensure the success of antibody production. The antisera were delivered in two separate time points; one from the standard 2 month procedure, and the other from exsanguination.

To isolate total IgG from the rabbit sera, approximately 20-30 mL of high-titer antisera were applied to a 5 mL alkali-tolerant Protein A column (GE Healthcare, HiTrap™ MabSelect SuRe™, cat#11-0034-94). Following a standard wash with PBS buffer, bound IgG was eluted from the resin by short exposure to 0.1 M sodium citrate, 0.3 M NaCl, pH 3.3, and immediately neutralized by adding 1/10 volume of 2 M Tris-HCl, pH 9 buffer to each fraction. The affinity column was sanitized by treating with 0.5 N NaOH following standard cleaning-in-place (CIP) procedure to avoid cross contamination of the IgG. Final recovered IgG from each sample was dialyzed against 50 volumes of PBS at 4 °C overnight, and protein concentration was determined by Bradford assay using BSA standard (Pierce, prod# 23208). One mL aliquots were transferred to individual tubes and stored at -80 °C.

These antibodies are diagnostic tools that were used to measure desaturase protein expression in transgenic plant material. The antibodies were used to develop correlations between low saturated fatty acid oil phenotype changes and the level of expression of the delta-9 desaturase proteins.

### Example 11: Levels of acyl-CoA delta-9 desaturase proteins in $T_2$ *Arabidopsis* seed

Delta-9 desaturase polypeptides were detected in mature transgenic seed samples by Western blot. Seed was prepared for analysis by cracking dry seeds with stainless steel beads in a Kleco™ Bead Beater (Garcia Machine, Visalia, CA). Extraction buffer was added (50 mM Tris, 10 mM EDTA, 2% SDS), and sample tubes were rocked gently for 30 minutes. Samples were centrifuged for 15 minutes at 3,000 rcf. Then, the supernatant was collected and used for analysis. The amount of total soluble protein in the seed extract was determined by Lowry assay (BioRad, Hercules, CA). Samples were normalized to 1.55 mg/mL total soluble protein and prepared in LDS sample buffer (Invitrogen, Carlsbad, CA) with 40 mM DTT, for a normalized load of 20 μg total soluble protein per lane. Samples were electrophoresed in 4-12% Bis-Tris gels (Invitrogen), and transferred to nitrocellulose membranes. Blots were blocked in blocking buffer, and probed with antibodies against four different delta-9 desaturase polypeptides (AnD9DS, LnD9DS-2, HzD9DS, and MgD9DS) (*see* Example 10).

In all cases, polyclonal antibody was developed in rabbits against a His-tag purified C-terminal peptide fragment of the individual desaturases as described above. The purified C-terminal fragments were used as reference antigens for quantitation of the Western blots. An anti-rabbit fluorescent labeled secondary antibody (Goat Anti-Rabbit AF 633; Invitrogen) was used for detection. Blots were visualized on a Typhoon™ Trio Plus fluorescence imager (GE Healthcare).

Standard curves were generated with quadratic curve fitting, and linear regression was used to quantify expression.

SDS-PAGE Western blots of extracts from mature $T_2$ seed from *Arabidopsis* events showed bands at the appropriate size when probed with specific antisera. These bands were quantified against specific reference antigens. Quantitative Western blotting of *Arabidopsis* $T_2$ seed extracts with appropriate antiserum indicated that an average of 63 ng LnD9DS-2/mg total protein (tp) (max. 228 ng/mg tp) was detected in mature seeds, and for HzD9DS, an average of 34 ng/mg tp (max. 100 ng/mg tp) was detected. For MgD9DS, an average of 58 ng/mg tp (max. 1179 ng/mg tp) was detected in $T_2$ seed. For the AnD9DS events, an average of 625 ng/mg tp (max 1.5 μg/mg tp) was detected in mature $T_2$ seeds. Thus, there was 10-18-fold less of the palmitoyl-preferring desaturases, LnD9DS-2 and HzD9DS, expressed in the transgenic seed, relative to AnD9DS. Higher levels of expression of these desaturases would therefore drive further reductions in saturates, especially palmitic acid.

**Example 12: Expression of delta-9 desaturase genes in canola**

A series of transgenic canola events were obtained from transformations performed with pDAB7321 (SEQ ID NO:61) and pDAB7326 (SEQ ID NO:63) (containing LnD9DS-2 and HzD9DS genes, respectively, driven by the seed-specific PvPhas promoter). Thirty nine pDAB7321 events containing the LnD9DS-2 gene were identified by PCR analysis of genomic DNA, and were grown in the greenhouse to produce $T_1$ seed. Similarly, 80 pDAB7326 events were identified that contained the HzD9DS gene, and produced $T_1$ seed. Canola was also transformed with pDAB7319 (SEQ ID NO:60) or pDAB7324 (SEQ ID NO:62), which contain an AnD9DS gene coupled with the LnD9DS-2 or HzD9DS genes, all driven by the PvPhas promoter. 44 and 76 events were recovered, respectively, that were confirmed to contain both desaturase genes by PCR analysis, and were grown in the greenhouse to produce $T_1$ seed.

FAME analysis of $T_1$ seed samples from events transformed with pDAB7321 (LnD9DS-2 v2) or pDAB7326 (HzD9DS v2) did not show significant reduction in saturated fatty acid levels relative to untransformed canola plants or plants transformed with an empty vector control. Western blots of the $T_1$ seed did not show detectable levels of the delta-9 desaturase proteins. In addition, no detectable protein for LnD9DS-2or HzD9DS was detected in $T_1$ seed from plants transformed with pDAB7319 (AnD9DS v3 and LnD9DS-2 v2) or pDAB7324 (AnD9DS v3 and HzD9DS v2), whereas the AnD9DS protein could be readily detected. In these events, a reduction of saturated fatty acids was observed relative to control plants, but this was attributable to expression of AnD9DS.

To evaluate the relative mRNA levels of the delta-9 desaturase genes, total RNA was extracted from developing canola seed from events transformed with double desaturase constructs (pDAB7319 and pDAB7324) and analyzed by quantitative real-time PCR. Seeds were harvested on dry ice at 20, 25, 29, 32, 39, or 41 days after pollination from several canola plants and stored at -80 °C. Total RNA was prepared from 50 mg of pooled frozen seeds using a Plant RNeasy® RNA extraction kit (Qiagen) according to the manufacturer's recommended protocol. Extracted RNA was used as a template for cDNA synthesis using the SuperScript® III First Strand Synthesis Supermix for qRT-PCR (Invitrogen) according to the manufacturer's recommended protocol.

RT-PCR assays were designed against the desaturase targets using the Roche Assay Design Center (Roche Diagnostics, Indianapolis, IN). Primers used in the assay are described in **Table 16**. Target assays utilized FAM-labeled UPL probes (Roche Diagnostics). These assays were executed in duplex reactions with a Texas-Red-labeled canola *actin* reference assay synthesized by Integrated DNA Technologies.

**Table 16:** q-RT-PCR assay details

| Target | Forward primer | Reverse Primer | Probe |
|---|---|---|---|
| AnD9Ds | SEQ ID NO:31<br>GGACTTCTCTACTCTCACCTTGGA | SEQ ID NO:32<br>TCCGATCCTCTTTGGGTTCT | UPL #9 |
| HzD9Ds | SEQ ID NO:33<br>GACCCACACAATGCAACG | SEQ ID NO:34<br>CCTAACAAGAAGCCAGCCAAT | UPL #143 |
| LnD9Ds | SEQ ID NO:35<br>GTTCTGACTGCGTTGGTCAC | SEQ ID NO:36<br>CGGAAACTCATGGTGGAAGT | UPL #7 |
| Actin | SEQ ID NO:37<br>CTACTGGTATTGTGCTCGACT | SEQ ID NO:38<br>CTCTCTCGGTGAGAATCTTCAT | SEQ ID NO:39<br>CACGCTATCCTCCGTCTCGATC |

| Target | Label |
|---|---|

| | |
|---|---|
| AnD9Ds | FAM |
| HzD9Ds | FAM |
| LnD9Ds | FAM |
| Actin | Tx-Red |

RT-PCR reactions were run on a LightCycler® 480II real-time PCR thermal cycler (Roche). Data for target UPL assays was collected using a 533 nm emission filter and a 483 nm excitation signal. Data for the *actin* reference assay was collected using a 610 nm filter and a 558 nm excitation signal. Cycle time values and target to reference ratios were calculated automatically using the LC480II software's "Advanced Relative Quantification" analysis workflow. Relative accumulation of desaturase transcript levels within each sample was calculated using the standard ΔΔCt method (Roche).

For each canola seed sample from pDAB7319 (AnD9DS v3 and LnD9DS-2 v2) and pDAB7324 (AnD9DS v3 and HzD9DS v2), transcript accumulation of HzD9DS or LnD9DS-2 transgenes was significantly lower than the transcript of AnD9DS in the same events. The observed differences in transcript accumulation varied between 3- and 20-fold less. **FIG. 23**. Thus, insufficient expression of HzD9DS and LnD9DS-2 may account for the lack of detection of the polypeptide and absence of phenotype attributable to these genes.

**Example 13: Expression of the delta-9 desaturase PTUs by alternative promoters**

The use of additional transcriptional regulatory regions to express gene(s) encoding LnD9DS-2, HzD9DS, and MgD9DS proteins can further increase the content of these delta-9 desaturases within canola. Identification and use of transcriptional regulatory regions which express earlier in development, and for longer periods of time, can increase the levels of heterologous delta-9 desaturases within canola seed by promoting robust seed-specific transcription of a heterologous gene at earlier stages of seed development. Examples of such transcriptional regulatory regions include, but are not limited to, the LfKCS3 promoter (U.S. Patent 7,253,337) and FAE 1 promoter (U.S. Patent 6,784,342). These promoters are used singularly, or in combination, to drive the expression of LnD9DS-2, HzD9DS, and MgD9DS expression cassettes, for example, through operable linkage with genes such as those previously described in plasmids, pDAB7319; pDAB7321; pDAB7324; pDAB7326; pDAB7328; and pDAB7330. Methods to replace transcriptional regulatory regions within a plasmid are well-known within the art. As such, a polynucleotide fragment comprising the PvPhas promoter is removed from pDAB7319, pDAB7321, pDAB7324, pDAB7326, pDAB7328, or pDAB7330 (or the preceding plasmids used to build pDAB7319, pDAB7321, pDAB7324, pDAB7326, pDAB7328, or pDAB7330), and replaced with either a LfKCS3 or FAE 1 promoter region. The newly-constructed plasmids are used to stably transform canola plants, according to the procedures set forth in the previous examples. Transgenic canola plants are isolated and molecularly characterized. The resulting delta-9 desaturase accumulation is determined, and canola plants which robustly express delta-9 desaturase are identified.

Further modifications to the transcriptional regulatory regions for increased expression of a delta-9 desaturase include replacing the existing Kozak sequence with any of the sequences described in **Table 17**. The engineering of alternative Kozak sequences upstream of the start site of a delta-9 desaturase is completed using standard molecular biology techniques. Synthetic polynucleotide fragments are synthesized and cloned upstream of a delta-9 desaturase coding sequence using techniques known within the art. The context of the start codon has a strong effect on the level of expression of a transgene. Modifying the Kozak sequence to one listed in **Table 17** increases the levels of expression of the heterologous delta-9 desaturase.

**Table 17**: Kozak sequences which are incorporated upstream of a heterologous delta-9 desaturase gene to increase expression.

| **Kozak Sequence** | **SEQ ID NO:** | **Sequence** |
|---|---|---|
| Kozak #1 | SEQ ID NO:40 | GGATCCAACA**ATG** |
| Kozak #2 | SEQ ID NO:41 | ACAACCAAAA**ATG** |
| Kozak #3 | SEQ ID NO:42 | ACAACCAACCTACC**ATGG** |
| Kozak #4 | SEQ ID NO:43 | ACAACCAAAAA**ATG** |

**Example 14: Design And synthesis of delta-9 desaturase genes from *Helicoverpa zea* and *Leptosphaeria nodorum***

To obtain higher levels of expression of heterologous genes in plants, the codon optimization strategy described in Example 2 was modified, and the heterologous gene protein coding regions for HzD9DS and LnD9DS-2 were reengineered using a new design protocol.

Codon selection was made using a table which had calculated the codon bias of the prospective host plant, which in this case was canola. In designing coding regions for plant expression of delta-9 desaturase genes, the primary ("first choice") codons preferred by the plant were determined, and used at about 95% of the time. "Second choice" codons were used sparingly, at a frequency of about 5%. Accordingly, a new DNA sequence was designed which encodes the amino sequence of each delta-9 desaturase, wherein the new DNA sequence differed from the native delta-9 desaturase gene by the substitution of plant first preferred and second preferred codons to specify an appropriate amino acid at each position within the amino acid sequence. The new sequence was then analyzed for restriction enzyme sites that might have been created by the modifications. The identified restriction enzyme sites were then removed by replacing the codons with first or second choice preferred codons. Other sites in the sequence which could affect transcription or translation of the gene of interest, specifically highly stable stem loop structures, were also removed.

The selections of preferred codon choices (first and second choices) from the genetic code of canola were determined from a codon bias table compiled from the protein coding sequences for canola. In **Tables 18** and **19**, Columns labeled as "Native Gene %" present the distributions (in % of usage for all codons for that amino acid) of synonymous codons for each amino acid, as found in the coding regions of *Brassica napus* (canola). New DNA sequences which encode essentially the amino acid sequence of the *M. grisea*, *H. zea* and *L. nodorum* delta-9 desaturases were designed for optimal expression in canola using the preferred codon distribution of first and second choice codons found in canola genes. Design of the plant-optimized DNA sequences were initiated by reverse-translation of the protein sequences of SEQ ID NO:12 (*M. grisea*), SEQ ID NO:13 (*H. zea*), and SEQ ID NO:14 (*L. nodorum*) using the canola codon bias table constructed. Columns labeled as "Plnt Opt Gene %" indicate the preferred codons and the frequency with which they were incorporated into the delta-9 desaturase gene design. SEQ ID NO:44 and SEQ ID NO:45 set forth the nucleotide sequences of the new canola-optimized LnD9DS-2 and HzD9DS desaturases, respectively. These new canola-optimized sequences were labeled as LnD9DS-2 v3 and HzD9DS v3.

**Table 18.** Codon compositions of coding regions for the HzD9DS protein. The native *H. zea* desaturase coding region is compared to a Plant-Optimized version.

| Amino Acid | Codon | Native Gene # | Native Gene % | Plnt Opt Gene # | Plnt Opt Gene % | Plnt Opt Recm'd | Amino Acid | Codon | Native Gene # | Native Gene % | Plnt Opt Gene # | Plnt Opt Gene % | Plnt Opt Recm'd |
|---|---|---|---|---|---|---|---|---|---|---|---|---|---|
| ALA (A) | GCA | 4 | 11.4 | 1 | 2.9 | 0.0 | LEU (L) | CTA | 2 | 5.9 | 0 | 0.0 | 0.0 |
| | GCC | 7 | 20.0 | 0 | 0.0 | 0.0 | | CTC | 8 | 23.5 | 0 | 0.0 | 0.0 |
| | GCG | 8 | 22.9 | 0 | 0.0 | 0.0 | | CTG | 14 | 41.2 | 0 | 0.0 | 0.0 |
| | GCT | 16 | 45.7 | 34 | 97.1 | 100.0 | | CTT | 6 | 17.6 | 34 | 100.0 | 100.0 |
| ARG (R) | AGA | 1 | 7.7 | 0 | 0.0 | 0.0 | | TTA | 2 | 5.9 | 0 | 0.0 | 0.0 |
| | AGG | 5 | 38.5 | 13 | 100.0 | 100.0 | | TTG | 2 | 5.9 | 0 | 0.0 | 0.0 |
| | CGA | 2 | 15.4 | 0 | 0.0 | 0.0 | LYS (K) | AAA | 11 | 44.0 | 0 | 0.0 | 0.0 |
| | CGC | 5 | 38.5 | 0 | 0.0 | 0.0 | | AAG | 14 | 56.0 | 25 | 100.0 | 100.0 |
| | CGG | 0 | 0.0 | 0 | 0.0 | 0.0 | MET (M) | ATG | 8 | 100 | 8 | 100 | 100.0 |
| | CGT | 0 | 0.0 | 0 | 0.0 | 0.0 | PHE (F) | TTC | 20 | 83.3 | 24 | 100.0 | 100.0 |
| ASN (N) | AAC | 13 | 72.2 | 18 | 100.0 | 100.0 | | TTT | 4 | 16.7 | 0 | 0.0 | 0.0 |
| | AAT | 5 | 27.8 | 0 | 0.0 | 0.0 | PRO (P) | CCA | 1 | 6.3 | 16 | 100.0 | 100.0 |
| ASP (D) | GAC | 16 | 64.0 | 2 | 8.0 | 0.0 | | CCC | 5 | 31.3 | 0 | 0.0 | 0.0 |
| | GAT | 9 | 36.0 | 23 | 92.0 | 100.0 | | CCG | 2 | 12.5 | 0 | 0.0 | 0.0 |
| CYS (C) | TGC | 1 | 100.0 | 1 | 100.0 | 100.0 | | CCT | 8 | 50.0 | 0 | 0.0 | 0.0 |
| | TGT | 0 | 0.0 | 0 | 0.0 | 0.0 | SER (S) | AGC | 2 | 12.5 | 0 | 0.0 | 0.0 |
| END | TAA | 1 | 100.0 | 0 | 0.0 | 0.0 | | AGT | 1 | 6.3 | 0 | 0.0 | 0.0 |
| | TAG | 0 | 0.0 | 0 | 0.0 | 0.0 | | TCA | 1 | 6.3 | 1 | 6.3 | 0.0 |
| | TGA | 0 | 0.0 | 1 | 100.0 | 100.0 | | TCC | 6 | 37.5 | 0 | 0.0 | 0.0 |
| GLN (Q) | CAA | 2 | 33.3 | 6 | 100.0 | 100.0 | | TCG | 3 | 18.8 | 0 | 0.0 | 0.0 |

(continued)

| Amino Acid | Codon | Native Gene # | Native Gene % | Plnt Opt Gene # | Plnt Opt Gene % | Plnt Opt Recm'd | Amino Acid | Codon | Native Gene # | Native Gene % | Plnt Opt Gene # | Plnt Opt Gene % | Plnt Opt Recm'd |
|---|---|---|---|---|---|---|---|---|---|---|---|---|---|
| | **CAG** | 4 | 66.7 | 0 | 0.0 | 0.0 | | **TCT** | 3 | 18.8 | 15 | 93.8 | 100.0 |
| **GLU (E)** | **GAA** | 7 | 63.6 | 0 | 0.0 | 0.0 | **THR (T)** | **ACA** | 3 | 16.7 | 0 | 0.0 | 0.0 |
| **16** | **GAG** | 4 | 36.4 | 11 | 100.0 | 100.0 | | **ACC** | 7 | 38.9 | 18 | 100.0 | 100.0 |
| **GLY (G)** | **GGA** | 8 | 40.0 | 20 | 100.0 | 100.0 | | **ACG** | 4 | 22.2 | 0 | 0.0 | 0.0 |
| | **GGC** | 6 | 30.0 | 0 | 0.0 | 0.0 | | **ACT** | 4 | 22.2 | 0 | 0.0 | 0.0 |
| | **GGG** | 2 | 10.0 | 0 | 0.0 | 0.0 | **ITRP (W)** | **TGG** | 14 | 100 | 14 | 100 | 0.0 |
| | **GGT** | 4 | 20.0 | 0 | 0.0 | 0.0 | **TYR(Y)** | **TAC** | 12 | 80.0 | 15 | 100.0 | 100.0 |
| **HIS (H)** | **CAC** | 11 | 73.3 | 15 | 100.0 | 100.0 | | **TAT** | 3 | 20.0 | 0 | 0.0 | 0.0 |
| | **CAT** | 4 | 26.7 | 0 | 0.0 | 0.0 | **VAL (V)** | **GTA** | 0 | 0.0 | 0 | 0.0 | 0.0 |
| **ILE (I)** | **ATA** | 3 | 15.0 | 1 | 5.0 | 0.0 | | **GTC** | 5 | 26.3 | 0 | 0.0 | 0.0 |
| | **ATC** | 10 | 50.0 | 19 | 95.0 | 100.0 | | **GTG** | 13 | 68.4 | 0 | 0.0 | 0.0 |
| | **ATT** | 7 | 35.0 | 0 | 0.0 | 0.0 | | **GTT** | 1 | 5.3 | 19 | 100.0 | 100.0 |
| | **Totals** | 165 | | 165 | | | | **Totals** | 189 | | 189 | | |

**Table 19.** Codon compositions of coding regions for the LnD9DS-2 protein. The native *L. nodorum* desaturase coding region is compared to a Plant-Optimized version.

| Amino Acid | Codon | Native Gene # | Native Gene % | PInt Opt Gene # | PInt Opt Gene % | PInt Opt Recm'd | Amino Acid | Codon | Native Gene # | Native Gene % | PInt Opt Gene # | PInt Opt Gene % | PInt Opt Recm'd |
|---|---|---|---|---|---|---|---|---|---|---|---|---|---|
| ALA (A) | GCA | 3 | 9.4 | 0 | 0.0 | 0.0 | LEU (L) | CTA | 7 | 15.6 | 0 | 0.0 | 0.0 |
| | GCC | 9 | 28.1 | 0 | 0.0 | 0.0 | | CTC | 14 | 31.1 | 0 | 0.0 | 0.0 |
| | GCG | 12 | 37.5 | 0 | 0.0 | 0.0 | | CTG | 7 | 15.6 | 0 | 0.0 | 0.0 |
| | GCT | 8 | 25.0 | 32 | 100.0 | 100.0 | | CTT | 5 | 11.1 | 45 | 100.0 | 100.0 |
| ARG (R) | AGA | 4 | 13.8 | 1 | 3.4 | 0.0 | | TTA | 3 | 6.7 | 0 | 0.0 | 0.0 |
| | AGG | 3 | 10.3 | 28 | 96.6 | 100.0 | | TTG | 9 | 20.0 | 0 | 0.0 | 0.0 |
| | CGA | 7 | 24.1 | 0 | 0.0 | 0.0 | LYS(K) | AAA | 9 | 45.0 | 0 | 0.0 | 0.0 |
| | CGC | 8 | 27.6 | 0 | 0.0 | 0.0 | | AAG | 11 | 55.0 | 20 | 100.0 | 100.0 |
| | CGG | 5 | 17.2 | 0 | 0.0 | 0.0 | MET (M) | ATG | 9 | 100 | 9 | 100 | 100.0 |
| | CGT | 2 | 6.9 | 0 | 0.0 | 0.0 | PHE (F) | TTC | 16 | 80.0 | 20 | 100.0 | 100.0 |
| ASN (N) | AAC | 6 | 50.0 | 12 | 100.0 | 100.0 | | TTT | 4 | 20.0 | 0 | 0.0 | 0.0 |
| | AAT | 6 | 50.0 | 0 | 0.0 | 0.0 | PRO (P) | CCA | 3 | 16.7 | 18 | 100.0 | 100.0 |
| ASP (D) | GAC | 16 | 66.7 | 2 | 8.3 | 0.0 | | CCC | 8 | 44.4 | 0 | 0.0 | 0.0 |
| | GAT | 8 | 33.3 | 22 | 91.7 | 100.0 | | CCG | 2 | 11.1 | 0 | 0.0 | 0.0 |
| CYS (C) | TGC | 4 | 80.0 | 5 | 100.0 | 100.0 | | CCT | 5 | 27.8 | 0 | 0.0 | 0.0 |
| | TGT | 1 | 20.0 | 0 | 0.0 | 0.0 | SER (S) | AGC | 8 | 27.6 | 0 | 0.0 | 0.0 |
| END | TAA | 0 | 0.0 | 0 | 0.0 | 0.0 | | AGT | 6 | 20.7 | 0 | 0.0 | 0.0 |
| | TAG | 1 | 100.0 | 0 | 0.0 | 0.0 | | TCA | 1 | 3.4 | 1 | 3.4 | 0.0 |
| | TGA | 0 | 0.0 | 1 | 100.0 | 100.0 | | TCC | 6 | 20.7 | 0 | 0.0 | 0.0 |
| GLN (Q) | CAA | 10 | 55.6 | 18 | 100.0 | 100.0 | | TCG | 7 | 24.1 | 0 | 0.0 | 0.0 |

(continued)

| Amino Acid | Codon | Native Gene # | Native Gene % | PInt Opt Gene # | PInt Opt Gene % | PInt Opt Recm'd | Amino Acid | Codon | Native Gene # | Native Gene % | PInt Opt Gene # | PInt Opt Gene % | PInt Opt Recm'd |
|---|---|---|---|---|---|---|---|---|---|---|---|---|---|
| | **CAG** | 8 | 44.4 | 0 | 0.0 | 0.0 | | **TCT** | 1 | 3.4 | 28 | 96.6 | 100.0 |
| **GLU (E)** | **GAA** | 5 | 33.3 | 1 | 6.7 | 0.0 | **THR (T)** | **ACA** | 11 | 44.0 | 0 | 0.0 | 0.0 |
| **16** | **GAG** | 10 | 66.7 | 14 | 93.3 | 100.0 | | **ACC** | 5 | 20.0 | 25 | 100.0 | 100.0 |
| **GLY (G)** | **GGA** | 13 | 34.2 | 38 | 100.0 | 100.0 | | **ACG** | 7 | 28.0 | 0 | 0.0 | 0.0 |
| | **GGC** | 16 | 42.1 | 0 | 0.0 | 0.0 | | **ACT** | 2 | 8.0 | 0 | 0.0 | 0.0 |
| | **GGG** | 6 | 15.8 | 0 | 0.0 | 0.0 | **ITRP (W)** | **TGG** | 19 | 100 | 19 | 100 | 0.0 |
| | **GGT** | 3 | 7.9 | 0 | 0.0 | 0.0 | **TYR (Y)** | **TAC** | 11 | 64.7 | 17 | 100.0 | 100.0 |
| **HIS (H)** | **CAC** | 12 | 66.7 | 18 | 100.0 | 100.0 | | **TAT** | 6 | 35.3 | 0 | 0.0 | 0.0 |
| | **CAT** | 6 | 33.3 | 0 | 0.0 | 0.0 | **VAL (V)** | **GTA** | 6 | 17.6 | 0 | 0.0 | 0.0 |
| **ILE (I)** | **ATA** | 4 | 18.2 | 1 | 4.5 | 0.0 | | **GTC** | 10 | 29.4 | 0 | 0.0 | 0.0 |
| | **ATC** | 9 | 40.9 | 21 | 95.5 | 100.0 | | **GTG** | 12 | 35.3 | 0 | 0.0 | 0.0 |
| | **ATT** | 9 | 40.9 | 0 | 0.0 | 0.0 | | **GTT** | 6 | 17.6 | 34 | 100.0 | 100.0 |
| | **Totals** | 214 | | 214 | | | | **Totals** | 236 | | 236 | | |

Syntheses of DNA fragments comprising SEQ ID NO:44 and SEQ ID NO:45 were performed by PicoScript and Blue Heron Biotechnology. The synthetic DNA was then cloned into expression vectors and transformed into canola substantially as described in the foregoing examples.

**Example 15: Modification of N- and C-termini to increase the accumulation of acyl-CoA desaturase polypeptides in plants**

The accumulation and stability of membrane-bound proteins in the endoplasmic reticulum (ER) can be influenced by amino acid sequence motifs and modifications at their N- and C-termini. Ravid and Hochstrasser (2008) Nat. Rev. Mol. Cell. Biol. 9:679-90. In particular, N- and C-terminal motifs and modifications have been shown to modulate the accumulation and stability of lipid desaturases in fungi and plants, as well as animals. McCartney et al. (2004) Plant J. 37:156-73; Mziaut et al. (2000) Proc. Natl. Acad. Sci. U.S.A. 97:8883-8.

The addition of either a Myc or the hemagglutin (HA) epitope tag to the N-terminal of FAD2 or FAD3 significantly increases the steady state level of these enzymes within yeast. O'Quin et al. (2009) Appl Microbiol Biotechnol 83:117-25. Accordingly, the addition of these, or similar epitopes, to the N-terminus of a delta-9 desaturase of the present invention is utilized to increase the expression of the polypeptide in a plant. A polynucleotide linker that encodes a Myc tag (SEQ ID NO:46) or a HA tag (SEQ ID NO:47) is cloned within the 5' end of a delta-9 desaturase (*e.g.*, HzD9DS, MgD9DS, AnD9DS, LnD9DS-1, and LnD9DS-2) coding sequence as a contiguous open reading frame. The resulting coding sequence is cloned within a plant expression plasmid using the cloning strategy described in Example 3. The newly-constructed plasmid is used to stably transform an *Arabidopsis* and/or canola plant cell, material, or tissue. Transgenic plants are regenerated from the transformed plant cell, material, or tissue. Transgenic plants are isolated and molecularly characterized. The resulting delta-9 desaturase accumulation in seeds of transgenic plants is determined, and plants which robustly express the delta-9 desaturase polypeptide are identified.

Evidence from expression of AnD9DS in *Arabidopsis* and canola (Examples 11 and 12) indicates a significantly higher level of expression of this particular desaturase enzyme, relative to HzD9DS and LnD9DS-2. Thus, all or parts of the N- and C-termini lying outside the core desaturase domain (containing the transmembrane segments and conserved catalytic histidine residues) of AnD9DS may be used to replace equivalent residues in the lower-expressing desaturases and increase expression thereof. Accordingly, all or part of N-terminal residues 1-68 and C-terminal residues 281-455 of AnD9DS (SEQ ID NO:72 and SEQ ID NO:73, respectively) are used to replace all or part of the 68 N-terminal residues (1-68) and 168 C-terminal residues (281-449) of LnD9DS-2 (SEQ ID NO:14) and/or the 76 N-terminal residues (1-76) and 60 C-terminal residues (293-353) of HzD9DS (SEQ ID NO:13). The resulting coding sequence is cloned within a plant expression plasmid using the cloning strategy described in Example 3. The newly-constructed plasmid is used to stably transform an *Arabidopsis* and/or canola plant cell, material, or tissue. Transgenic plants are regenerated from the transformed plant cell, material, or tissue. Transgenic plants are isolated and molecularly characterized. The resulting delta-9 desaturase accumulation in seeds of transgenic plants is determined, and plants which robustly express the modified HzD9DS or modified LnD9DS-2 polypeptide are identified.

**Example 16: Modifications to enhance mRNA expression of acyl-CoA desaturase within plants**

It is known within the art that the expression of mRNA can be enhanced by the incorporation of genetic elements that stabilize and increase mRNA accumulation. The incorporation of 5' and 3' untranslated regions (*e.g.*, Tobacco Osmotin 5' and 3' UTR sequences (Liu et al. (2003) Nat. Biotechnol. 21:1222-8), and the Tobacco Mosaic Virus Ω sequence (Gallie et al. (1987) Nucleic Acids Res. 15:8693-711)) or introns (Koziel et al. (1996) Plant Mol. Biol. 32:393-405), within close proximity of a HzD9Ds or LnD9DS-2 coding sequence, is used to increase the levels of expression of the transgene when compared to the expression of the same coding sequence lacking the aforementioned genetic elements. The addition one or more of these genetic elements within a desaturases PTU is performed according to methods well-known in the art. Polynucleotide fragments comprising the 5' untranslated region, 3' untranslated region, and/or intron are added to a plant expression plasmid (*e.g.*, pDAB7319, pDAB7321, pDAB7324, pDAB7326, pDAB7328, pDAB7330, or the preceding plasmids used to build pDAB7319, pDAB7321, pDAB7324, pDAB7326, pDAB7328, or pDAB7330) *via* standard cloning methods. The newly-constructed plasmid is used to stably transform an *Arabidopsis* and/or canola plant cell, material, or tissue. Transgenic plants are regenerated from the transformed plant cell, material, or tissue. Transgenic plants are isolated and molecularly characterized. The resulting delta-9 desaturase accumulation in seeds of transgenic plants is determined, and plants which robustly express the HzD9DS or LnD9DS-2 polypeptide are identified.

Furthermore, it is know in the art that yeast desaturase genes such as *OLE1* are highly-regulated. The deletion of sequences that encode transmembrane regions and that are a part of the cytochrome b5 domain reduce the stability of the *OLE1* transcript. Vemula et al. (2003) J. Biol. Chem. 278(46):45269-79. The presence of these sequences within *OLE1* act as mRNA stabilizing sequences. Accordingly, incorporation of the *OLE1* sequences that encode the transmembrane region and cytochrome b5 domain into a LnD9DS-2 or HzD9DS coding sequence is utilized to increase stability of the mRNA transcript of the coding sequence, thereby resulting in higher levels of expression and a subsequent increase of LnD9DS-2 or HzD9DS polypeptide. A chimeric LnD9DS-2 or HzD9DS coding sequence that includes the *OLE1* transmembrane region and cytochrome b5 domain sequences is constructed using methods known in the art. The coding sequence produced thereby is incorporated into a plant expression plasmid (*e.g*., as described in the foregoing examples), and used to generate transgenic plants *via Agrobacterium*-mediated plant transformation. Transgenic plants are isolated and characterized. The resulting delta-9 desaturase accumulation is determined, and plants which robustly express the delta-9 desaturase are identified.

**Example 17**

**Use of an alternative 3' untranslated region terminator for stable expression of a delta-9 desaturase in a plant**

Due to a limited number of available 3' UTR-terminators, the *Agrobacterium* ORF 23 3' UTR-terminator (AtuORF23 3' UTR) is typically used to terminate transcription. It was recently shown that other 3' UTR-terminators are more effective in terminating transcriptional read-through in *Arabidopsis thaliana.* Accordingly, the *Phaseolus vulgaris* Phaseolin 3'UTR-teminator (SEQ ID NO:69) is used in combination with the *Phaseolus vulgaris* Phaseolin promoter to reduce transcriptional read-through of upstream genes, thereby reducing transcriptional interference.

The *Phaseolus vulgaris* Phaseolin 3'UTR-teminator (PvPhas 3'UTR v1) was incorporated within an LnD9DS-2 v2 expression cassette, and within an HzD9DS v2 expression cassette, which were previously described in plasmid pDAB7321 and pDAB7326. According to methods well-known to those of skill in the art, a polynucleotide fragment comprising the PvPhas 3'UTR v1 was placed downstream of a LnD9DS-2 v2 gene to create binary plasmid, pDAB110110 (**FIG. 4a;** SEQ ID NO:74). A polynucleotide fragment comprising the PvPhas 3'UTR v1 was also placed downstream of a HzD9DS v2 gene to create binary plasmid pDAB110112 (**FIG. 4b;** SEQ ID NO:75).

The resulting binary plasmids were confirmed *via* restriction enzyme digestion and sequencing. The newly-constructed plasmids are each used to stably transform an *Arabidopsis* and/or canola plant cell, material, or tissue. Transgenic plants are regenerated from the transformed plant cell, material, or tissue. Transgenic plants are isolated and molecularly characterized. The resulting delta-9 desaturase accumulation in seeds of the transgenic plants is determined, and plants which robustly express the HzD9DS or LnD9DS-2 polypeptide are identified.

## Claims

1. An isolated nucleic acid molecule encoding a delta-9 desaturase enzyme comprising an amino acid sequence being at least 80% identical to SEQ ID NO:12, wherein the nucleic acid molecule comprises a nucleotide sequence at least 60% identical to a sequence selected from the group consisting of SEQ ID NO:3, SEQ ID NO:4, SEQ ID NO:9, and SEQ ID NO:15.

2. An isolated nucleic acid molecule encoding a delta-9 denaturase enzyme comprising an amino acid sequence being at least 80% identical to SEQ ID NO:12, wherein the nucleic acid molecule further comprises a gene regulatory element selected from the group consisting of the *Saccharomyces cerevisiae* delta-9 desaturase promoter, the delta-9 desaturase 3'UTR/terminator, the *ole1* gene promoter, the phaseolin promoter, the *Phaseolus vulgaris* phaseolin 5' untranslated region, the *Phaseolus vulgaris* phaseolin 3' untranslated region, the *Phaseolus vulgaris* phaseolin matrix attachment region, the *Agrobacterium tumefaciens* ORF23 3' untranslated region, the Cassava vein Mosaic Virus Promoter, the *Agrobacterium tumefaciens* ORF1 3' untranslated region, the *Nicotiana tabacum* RB7 Matrix Attachment Region, Overdrive, T-stand border sequences, the LfKCS3 promoter, FAE 1 promoter, SEQ ID NO:40 SEQ ID NO:41, SEQ ID NO:42, SEQ ID NO:43, a Myc tag, and a hemagglutinin tag.

**3.** A method for decreasing the amount of saturated fatty acids in a cell, the method comprising:

transforming a plant cell with the nucleic acid molecule of claim 1, such that the amount of saturated fatty acids in the cell is decreased.

**4.** The method according to claim 3, comprising further transforming the plant cell with at least one nucleic acid molecule encoding a delta-9 desaturase enzyme comprising an amino acid sequence being at least 80% identical to SEQ ID NO:12.

**5.** The method according to claim 4, wherein further transforming the plant cell introduces into the plant cell a further means for decreasing levels of 16:0-ACP in the plant cell.

**6.** The method according to claim 5, wherein the means for decreasing levels of 16:0-ACP in the plant cell is an extraplastidial desaturase, preferably the desaturase is MgD9DS desaturase.

**7.** An oil seed plant comprising the nucleic acid sequence of claim 1.

**8.** A plant seed comprising the nucleic acid molecule of claim 1, which expresses an extraplastidial desaturase comprising an amino acid sequence that is at least identical to SEQ ID NO:12.

**9.** The seed of claim 8, wherein the seed is a seed of a transgenic *Brassica napus* line, the seed having a decreased levels of 16:0, relative to an isogenic version of the transgenic *Brassica napus* line.

**10.** A method for creating a genetically engineered plant comprising decreased amounts of saturated fatty acids in the plant compared to the wild type plant, the method comprising:

transforming plant material with a nucleic acid molecule encoding a delta-9 desaturase enzyme comprising an amino acid sequence that is at least 80% identical to SEQ ID NO:12; and
culturing the transformed plant material to obtain a plant, wherein the seed of the plant expresses the delta-9 desaturase enzyme.

**11.** The method of claim 3 or 10, wherein the plant cell or the plant is selected from a genus selected from the group consisting of *Arabidopsis, Borago,* Canola, *Ricinus, Theobroma, Zea, Gossypium, Crambe, Cuphea, Linum, Lesquerella, Limnanthes,* Linola, *Tropaeolum, Oenothera, Olea, Elaeis, Arachis,* rapeseed, *Carthamus, Glycine, Soja, Helianthus, Nicotiana, Vernonia, Triticum, Hordeum, Oryza, Avena, Sorghum, Secale,* and the other members of the *Gramineae.*

**12.** A plant comprising the nucleic acid molecule of claim 1, obtained by the method of claim 10.

**13.** A plant material comprising the nucleic acid molecule of claim 1, obtained from the plant of claim 12, preferably the plant material is a seed.

**14.** The plant seed of claim 8, wherein the extraplastidial desaturase comprises the amino acid sequence of SEQ ID NO:12.

**Patentansprüche**

**1.** Ein isoliertes Nukleinsäuremolekül, welches ein delta-9-Desaturase-Enzym kodiert, umfassend eine Aminosäuresequenz, die mindestens 80% identisch mit SEQ ID NO:12 ist, wobei das Nukleinsäuremolekül eine Nukleotidsequenz umfasst, die mindestens 60% identisch mit einer Sequenz ist, ausgewählt aus der Gruppe bestehend aus SEQ ID NO:3, SEQ ID NO:4, SEQ ID NO:9 und SEQ ID NO: 15.

2. Ein isoliertes Nukleinsäuremolekül, welches ein delta-9-Desaturase-Enzym kodiert, umfassend eine Aminosäuresequenz, die mindestens 80% identisch mit SEQ ID NO:12 ist, wobei das Nukleinsäuremolekül ferner ein genregulatorisches Element, ausgewählt aus der Gruppe bestehend aus dem *Saccharomyces cerevisiae* delta-9-Desaturase-Promotor, dem delta-9-Desaturase 3'UTR/Terminator, dem *ole1* Gen-Promotor, dem Phaseolin-Promotor, der *Phaseolus vulgaris* Phaseolin 5' untranslatierten Region, der *Phaseolus vulgaris* Phaseolin 3' untranslatierten Region, der *Phaseolus vulgaris* Phaseolin Matrix-Anheftungsregion, der *Agrobacterium tumefaciens* ORF23 3' untranslatierten Region, dem Cassava Vein Mosaik Virus-Promotor, der *Agrobacterium tumefaciens* ORF1 3' untranslatierten Region, der *Nicotiana tabacum* RB7 Matrix-Anheftungsregion, Overdrive, T-stand Grenzsequenzen, dem LfKCS3-Promotor, dem FAE 1-Promotor, SEQ ID NO:40, SEQ ID NO:41, SEQ ID NO:42, SEQ ID NO:43, einem Myc-Tag und einem Hämagglutinin-Tag umfasst.

3. Ein Verfahren zur Verringerung der Menge an gesättigten Fettsäuren in einer Zelle, wobei das Verfahren folgendes umfasst:

   das Transformieren einer Pflanzenzelle mit dem Nukleinsäuremolekül gemäß Anspruch 1, so dass die Menge an gesättigten Fettsäuren in einer Zelle verringert wird.

4. Das Verfahren nach Anspruch 3, weiterhin umfassend das Transformieren der Pflanzenzelle mit mindestens einem Nukleinsäuremolekül, welches ein delta-9-Desaturase-Enzym kodiert, welches eine Aminosäuresequenz umfasst, die mindestens 80% identisch mit SEQ ID NO:12 ist.

5. Das Verfahren nach Anspruch 4, wobei das weitere Transformieren der Pflanzenzelle in die Pflanze ein weiteres Mittel zur Verringerung der 16:0-ACP-Level in der Pflanzenzelle einbringt.

6. Das Verfahren nach Anspruch 5, wobei das Mittel zur Verringerung der 16:0-ACP-Level in der Pflanzenzelle eine extraplastidale Desaturase ist, wobei vorzugsweise die Desaturase die MgD9DS-Desaturase ist.

7. Eine Ölsaat-Pflanze, umfassend die Nukleinsäuresequenz gemäß Anspruch 1.

8. Eine Pflanzensaat, umfassend das Nukleinsäuremolekül gemäß Anspruch 1, welches eine extraplastidale Desaturase exprimiert, umfassend eine Aminosäuresequenz, die wenigstens mit SEQ ID NO:12 identisch ist.

9. Die Saat nach Anspruch 8, wobei die Saat eine Saat einer transgenen *Brassica napus* Linie ist, wobei die Saat verringerte 16:0-Level im Verhältnis zu einer isogenen Version der transgenen *Brassica napus* Linie aufweist.

10. Ein Verfahren zur Erzeugung einer gentechnisch veränderten Pflanze, die geringere Mengen an gesättigten Fettsäuren in der Pflanze im Verhältnis zu der Wildtyp-Pflanze enthält, wobei das Verfahren umfasst:

    das Transformieren von Pflanzenmaterial mit einem Nukleinsäuremolekül, welches ein delta-9-Desaturase-Enzym, umfassend eine Aminosäuresequenz, die mindestens 80% identisch mit SEQ ID NO:12 ist, umfasst; und die Kultivierung des transformierten Pflanzenmaterials, um eine Pflanze zu erhalten, wobei die Saat der Pflanze das delta-9-Desaturase-Enzym exprimiert.

11. Das Verfahren nach Anspruch 3 oder 10, wobei die Pflanzenzelle oder die Pflanze aus dem Genus, ausgewählt aus der Gruppe bestehend aus *Arabidopsis, Borago,* Canola, *Ricinus, Theobroma,* Zea, *Gossypium, Crambe, Cuphea, Linum, Lesquerella, Limnanthes,* Linola, *Tropaeolum, Oenothera, Olea, Elaeis, Arachis,* Raps, *Carthamus, Glycine, Soja, Helianthus, Nicotiana, Vernonia, Triticum, Hordeum, Oryza, Avena, Sorghum, Secale,* und den weiteren Mitgliedern der *Gramineae,* ausgewählt ist.

12. Eine Pflanze, umfassend das Nukleinsäuremolekül gemäß Anspruch 1, erhalten durch das Verfahren gemäß Anspruch 10.

13. Ein Pflanzenmaterial, umfassend das Nukleinsäuremolekül gemäß Anspruch 1, erhalten von der Pflanze gemäß Anspruch 12, wobei das Pflanzenmaterial vorzugsweise eine Saat ist.

14. Die Pflanzensaat gemäß Anspruch 8, wobei die extraplastidale Desaturase die Aminosäuresequenz SEQ ID NO:12 umfasst.

## Revendications

1. Molécule isolée d'acide nucléique qui code une enzyme delta-9 désaturase comprenant une séquence d'acides aminés qui est identique, à un degré d'au moins 80 %, à la Séquence N° 12, laquelle molécule d'acide nucléique comprend une séquence de nucléotides identique, à un degré d'au moins 60 %, à une séquence choisie dans l'ensemble formé par la Séquence N° 3, la Séquence N° 4, la Séquence N° 9 et la Séquence N° 15.

2. Molécule isolée d'acide nucléique qui code une enzyme delta-9 désaturase comprenant une séquence d'acides aminés qui est identique, à un degré d'au moins 80 %, à la Séquence N° 12, laquelle molécule d'acide nucléique comporte en outre un élément régulateur de gène choisi dans l'ensemble constitué par les suivants : promoteur du gène de delta-9 désaturase de *Saccharomyces cerevisiae,* région 3 ☐ non traduite et terminateur de gène de delta-9 désaturase, promoteur de gène *ole*1, promoteur de gène de phaséoline, région 5 ☐ non traduite du gène de phaséoline de *Phaseolus vulgaris,* région 3 ☐ non traduite du gène de phaséoline de *Phaseolus vulgaris,* région d'attachement à la matrice du gène de phaséoline de *Phaseolus vulgaris,* région 3 ☐ non traduite du cadre ouvert de lecture 23 *d'Agrobacterium tumefaciens,* promoteur du virus de la mosaïque des nervures du manioc, région 3 ☐ non traduite du cadre ouvert de lecture 1 d'*Agrobacterium tumefaciens,* région d'attachement à la matrice RB7 de *Nicotiana tabacum,* séquences overdrive et bordure de brin T, promoteur du gène LfKCS3, promoteur du gène FAE 1, Séquence N° 40, Séquence N° 41, Séquence N° 42, Séquence N° 43, étiquette Myc, et étiquette hémagglutinine.

3. Procédé visant à réduire la quantité d'acides gras saturés dans une cellule, lequel procédé comporte le fait de transformer une cellule végétale avec une molécule d'acide nucléique conforme à la revendication 1, de telle sorte que la quantité d'acides gras saturés dans la cellule est réduite.

4. Procédé conforme à la revendication 3, qui comporte le fait de transformer davantage la cellule végétale avec au moins une molécule d'acide nucléique qui code une enzyme delta-9 désaturase comprenant une séquence d'acides aminés qui est identique, à un degré d'au moins 80 %, à la Séquence N° 12.

5. Procédé conforme à la revendication 4, dans lequel transformer davantage la cellule végétale introduit dans la cellule végétale un moyen supplémentaire de réduire les niveaux de protéine 16:0-ACP dans la cellule végétale.

6. Procédé conforme à la revendication 5, dans lequel le moyen de réduire les niveaux de protéine 16:0-ACP dans la cellule végétale est une désaturase extraplastidiale, cette désaturase étant de préférence la désaturase MgD9DS.

7. Végétal à graines oléagineuses, comprenant une séquence d'acide nucléique conforme à la revendication 1.

8. Graine de végétal comprenant une molécule d'acide nucléique conforme à la revendication 1, dans laquelle est exprimée une désaturase extraplastidiale comprenant une séquence d'acides aminés qui est au moins identique à la Séquence N° 12.

9. Graine conforme à la revendication 8, laquelle graine est une graine d'une lignée transgénique de *Brassica Napus* et présente un niveau réduit d'acide gras 16:0, par rapport à une version isogénique de la lignée transgénique de *Brassica Napus.*

10. Procédé de création d'un végétal génétiquement modifié, contenant de moindres quantités d'acides gras saturés que le végétal de type sauvage, lequel procédé comporte les opérations suivantes :

    - transformer un matériel végétal avec une molécule d'acide nucléique qui code une enzyme delta-9 désaturase comprenant une séquence d'acides aminés qui est identique, à un degré d'au moins 80 %, à la Séquence N° 12,
    - et cultiver le matériel végétal transformé pour obtenir un végétal dans les graines duquel est exprimée l'enzyme delta-9 désaturase.

11. Procédé conforme à la revendication 3 ou 10, dans lequel la cellule végétale ou le végétal est choisi(e) dans un genre choisi dans l'ensemble constitué par les suivants : *Arabidopsis, Borago*, Canola, *Ricinus, Theobroma, Zea, Gossypium, Crambe, Cuphea, Linum, Lesquerella, Limnanthes*, Linola, *Tropaeolum, Oenothera, Olea, Elaeis, Ara-*

*chis*, colza, *Carthamus, Glycine, Soja, Helianthus, Nicotiana, Vernonia*, *Triticum, Hordeum, Oryza, Avena, Sorghum, Secale,* et les autres membres de la famille des Graminées.

**12.** Végétal comprenant une molécule d'acide nucléique conforme à la revendication 1, obtenu selon un procédé conforme à la revendication 10.

**13.** Matériel végétal comprenant une molécule d'acide nucléique conforme à la revendication 1, obtenu à partir d'un végétal conforme à la revendication 12, lequel matériel végétal est de préférence une graine.

**14.** Graine de végétal conforme à la revendication 8, dans laquelle la désaturase extraplastidiale comprend la séquence d'acides aminés de la Séquence N° 12.

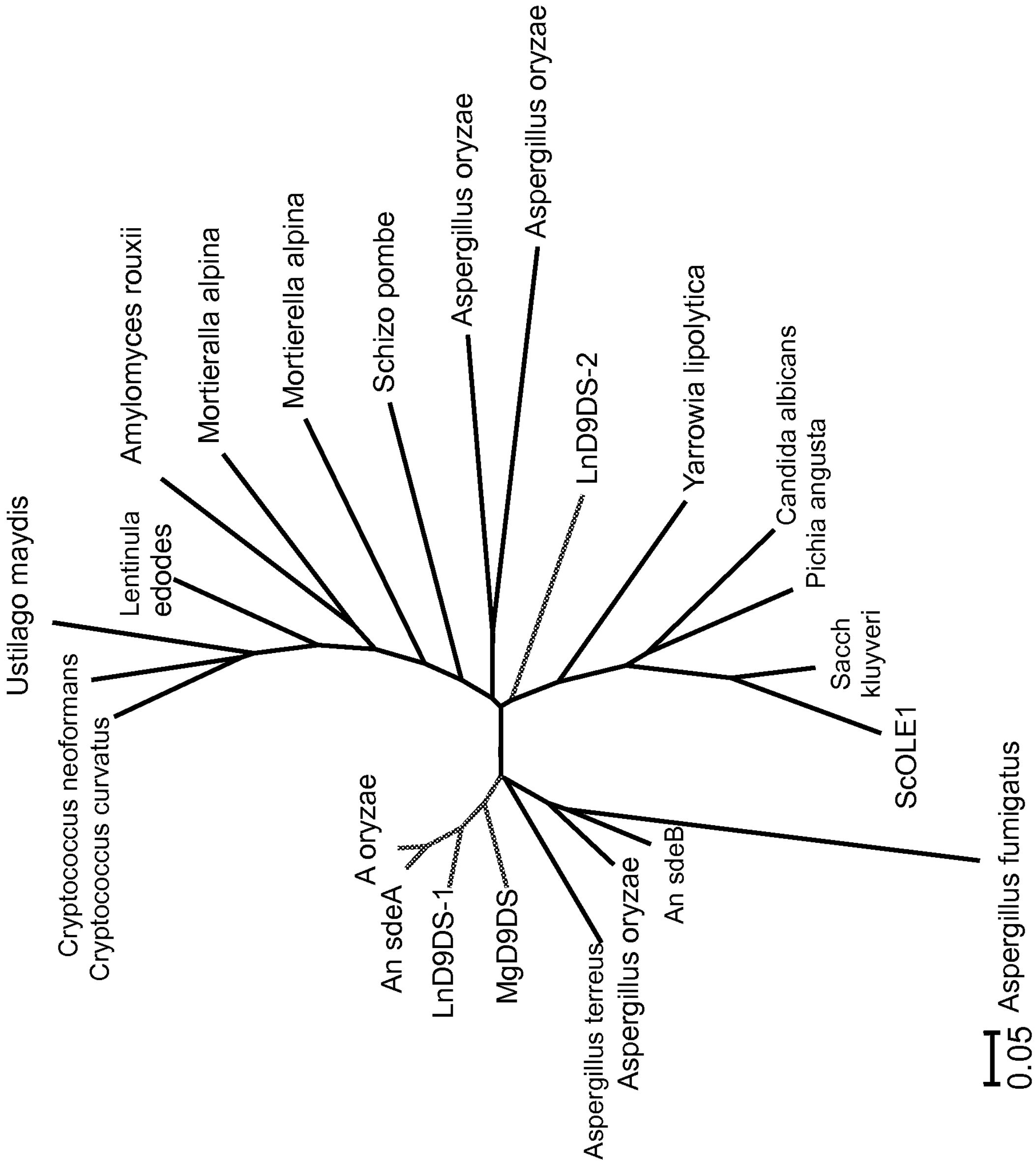
Ustilago maydis
Cryptococcus neoformans
Cryptococcus curvatus
Lentinula edodes
Amylomyces rouxii
Mortieralla alpina
Mortierella alpina
Schizo pombe
Aspergillus oryzae
Aspergillus oryzae
LnD9DS-2
Yarrowia lipolytica
Candida albicans
Pichia angusta
Sacch kluyveri
ScOLE1
Aspergillus fumigatus
An sdeB
Aspergillus oryzae
Aspergillus terreus
MgD9DS
LnD9DS-1
An sdeA
A oryzae
0.05

Fig. 1

```
LnD9DS-1  (SEQ ID NO:5)  (1)  cccgattcattaatgcagctggcacgacaggtttcc
LnD9DS-2  (SEQ ID NO:8)  (1)  ------------------------------------
MgD9DS   (SEQ ID NO:76)  (1)  ------------------------------------

 (37) cgactggaaagcgggcagtgagcgcaacgcaattaatgtgagttagctcactcatta
  (1) ---------------------------------------------------------
  (1) ---------------------------------------------------------

 (94) caggctttacactttatgcttccggctcgtatgttgtgtggaattgtgagggcaccc
  (1) ---------------------------------------------------------
  (1) ---------------------------------------------------------

(151) cggataacaatttcacacaggaaacagctatgaccatgattacgccaagctcgaaat
  (1) ---------------------------------------------------------
  (1) ---------------------------------------------------------

(208) taaccctcactaaagggaacaaaagctggagctccaccgcggtggcggccgctctag
  (1) ---------------------------------------------------------
  (1) ---------------------------------------------------------

(265) aactagtggatcccccgggctgcaggaattcggcacgagtatgccttcccaccAggc
  (1) ---------------------------------------------------------
  (1) ----------------------------------------------------gAatt

(322) tgTtGCTggcatgcaggCCatCGacCCcGAGTTtGtcaaGCAGCCgTCTC--CTaTG
  (1) ---------------------------------------GgAtCCaTggCgGCctTG
  (6) caTgGCTtcgtcatcttCCtcCGtgCCgGAGTTgGc--tGCcGCCtTCcCtGaTggc

(377) GCgAGCAcCTCgGAGCCCAAccGcAACTCCAAG---TACGAtcCtAAgAAGCCGCAC
 (19) GacAGCAttcCaGAGgatAAGgctAcCTC---G---T------CgAAatcGaCtCAt
 (61) aCtAcCgaCTtcaAGCCCAtGaGgAACaCCAAGggcTACGAcgtcAgcAAGCCGCAC

(431) ATTaCAGAcAtgCCcATcACgCggtcaAACTGGTAccAGCAtGTCAACTGGCTCAAC
 (64) ATTcaAtAtcaAgaagTaACttTtcgGAACTGGTATAAGaAgaTaAAtTGGCTCAAC
(118) ATTtCcGAgAcACCtATgACaCTcaaGAACTGGcATAAGCAcGTCAACTGGCTCAAC

(488) gtCAtCTTCATCaTcggCGTGCCtCTCGCTGGCTgCGTCgCCgCCTtCTGGaCCCCT
(121) ACgACgcTggTggTGcTCaTaCC---CGCT--CTTgGaCTCtACCTAacacGCaCCa
(175) ACCACCTTCATCtTGtTtGTGCCcCTgGCTGGtcTCaTaTCCACtTACTGGGtCCCT

(545) CTGCAGTGGAA-GACCGCTGCg----TGGGCTGTCaTCTACTAtTTCTGgACtGGCC
(173) CgcCAcTtacAcGACCtacGCtcatcTGGtCcGTCcTgTACTACTTCTGCACaGctt
(232) CTGCAGTGGAA-GACgGCTGta----TGGGCTGTCgTCTACTACTTCaaCACcGGCC
```

**Fig. 2a**

```
(597)  TCGGtATCACcGCCGGATACCATCGtCTcTGGGCaCACAagTCaTACAACGCCgGTC
(230)  TCGGcATCACaGgCGGATAtCATCGACTaTGGagTCAtcGCagcTACtcCGCtcGTC
(284)  TgGGaATtACtGCCGGtTACCAcCGACTtTGGGCTCACAGCTCgTACAAgGCCtcgC

(654)  TTCCtCTgAGgATCTggCTcGCCGCCGTcGGCGCTGGTGCtGTtGAGGGTTCCATCC
(287)  TaCCGCTacGctTaTtCCTaGCCttCacaGGCGCcGGaGCCaTCcAaGGTagtgctC
(341)  TTCCGCTcAaaATCTaCCTtGCCGCCGTtGGCGCTGGTGCCGTCGAGGGcTCCATCa

(711)  GcTGGTGGAGCcgtGAcCACCGCGCCCACCACCGcTACACCGACACCAAcAAGGACC
(344)  GATGGTGGAGCgcaaATCACCGCGCCCACCACCGATggACCGACACaAtGAAGGACC
(398)  GATGGTGGtcCaacGgTCACCGCGCaCACCACCGATACACCGAtACCgAGAAGGACC

(768)  CCTACagtGTCCGCAAGGGCCTtCTcTACagcCAtcTCGGATGGATGGTcaTGAAGC
(401)  CCTACTCcGTtatgcgcGGCCTatTaTtCTCgCACATCGGATGGATGGTatTGAAca
(455)  CCTACTCaGTCCGCAAGGGtCTcCTgTACTCaCACATgGGATGGATGcTtcTGAAGC

(825)  AGAACCCCAAGcgtAtcGGCCGCACCGACATCACCGACtTGAACGAGGACCCCGTTG
(458)  gcgACCCCAAagtcAAaGGCCGaACaGACgTCAgtGAtCTcgACagcGACCCCGTcG
(512)  AGAACCCCAAGaagcAgGGCCGCACCGACATCACCGACCTGAACGAGGACCCCGTTG

(882)  TCGTCTGGCAGCACAAGAACTACaTCAAGgcCGTcgTcacCATGGgCTTGATCTTtC
(515)  TaGTCTGGCAGCACAAGcACTACggCAAGTGCcTgcTgTTCgccGCgTgGATaTTCC
(569)  TCGTtTGGCAGCACcgcAACTtCcTCAAGTGtGTtaTCTTCATGGCCcTcgTCTTCC

(939)  CCtCtgcCGTcGCCGGtCTCatgTGGGGcGATTGGatGGGtGGCTTCATCTACGCtG
(572)  CCAtgaTCGTaGCCGGCCTCGGaTGGGGaGATTGGTGGGGAGGCcTtgTCTACGCcG
(626)  CCACacTtGTgGCtGGCCTtGGcTGGGGtGAcTacTGGGGAGGtTTCATCTACGgaG

(996)  GTATCCTcCGTaTCTTCTTCGTCCAGCAGGCCACCTTCTGCGTCAACTCGCTTGCtC
(629)  GcATCaTtCGaGcgTgtTTCGTCCAGCAGGCgACaTTtTGCGTgAACTCtCTcGCgC
(683)  GTATtCTgCGTGTCTTCTTCGTCCAGCAGGCCACCTTCTGCGTCAACTCGCTTGCcC

(1053) ACTGGCTCGGTGACCAGCCcTTCGACGACCGCAACTCtCCTCGTGACCACGTCATtA
(686)  AtTGGaTCGGcGAgCAGCCgTTCGACGACaGacgCaCGCCTCGaGACCACGTttTgA
(740)  ACTGGCTCGGTGACCAGCCtTTCGACGAtCGCAACTCGCCgCGTGAtCACGTCATcA

(1110) CcGCtCTtGTCACtCTcGGAGAGGGcTACCACAACTTCCACCACGAGTTCCCcTCCG
(743)  CAGCgtTGGTaACgaTgGGAGAaGGATAtCAtAACTTCCACCACGAaTTCCCaagCG
(797)  CAGCcCTGGTCACcCTtGGAGAGGGATACCACAACTTCCACCACGAGTTCCCtTCgG

(1167) ACTACCGCAACGCcATCGAGTGGcACCAGTACGACCCTACCAAGTGGTCCATCTGGC
(800)  AtTAtCGCAACGCgATCatcTGGTACCAaTACGACCCTACCAAaTGGctCATtTacC
(854)  ACTACCGCAACGCtATtGAGTGGTACCAGTAtGACCCcACCAAGTGGTCaATCTGGa
```

**Fig. 2b**

```
(1224) TgTGGA------GCaAGCTCGGCCTCGCCTcCAACCTCAAGCAGTTCCGCTCCAACG
 (857) TCTtctccctcgGCCcctTCccCCTCGCaTACtcgCTCAAaaccTTCCGgTCCAAtG
 (911) TCTGGA------agCAGCTtGGtCTtGCCcACAACCTgAAGCAGTTCCGCcaaAACG

(1275) AaATcGAGAAGGGtCGtGTCCAGCAGCTcCAGAAGAAGaTtGACCAGAAGCGCGCCA
 (914) AGATTGAaAAaGGgCGgtTgCAaCAaCaaCAaAAagccCTgGACaAGAAGCGCtCag
 (962) AGATTGAGAAGGGaCGcGTCCAGCAGCTgCAGAAGAAGCTcGACCAGAAGCGCGCCA

(1332) AGCTcGAcTGGGGTgTcCCtCTCGACCAGCTgCCTGTCATAgaaTGGGACGACTaTG
 (971) gaCTTGATTGGGGccTaCCCCTCttCCAaCTcCCTGTCATAtcgTGGGACGACTTcc
(1019) AGCTTGATTGGGGTaTtCCCtTgGAgCAGCTtCCcGTtgTtagcTGGGAtGACTTTG

(1389) TcGAGCAG-GCCAAGAA-CgGcCGtGGT-CTcATcGCTGTCGCtGGTGTCgTTCAtG
(1028) aaGcGCgttGCaAAGAgtCCGGCGAGaTgCTGgTTGCTGTCGCaGGTGTgATTCACG
(1076) TtGAGCAG-tCCAAGAA--CGGaaAGGcttgGATTGCaGTtGCcGGTGTCATcCACG

(1443) ACGTTAcCGACTTCATCAAcGAgCACCCcGGTGGCAaGACgCTtATCAAGAGcGgCG
(1085) ACGTcAGCcAgTTtATtgAaGAtCACCCTGGaGGCAGGAgTtTgATtcgGAGtGCgG
(1130) AtGTTgGtGACTTCATCAAgGAcCACCCTGGTGGCAGagCTCTcATCAActcgGCCa

(1500) TTGGCAAGGATGCcACCGCcATGTTCAACGGCGGTGTcTACttCCACTCCAACGgaG
(1142) TgGGCAAaGATGggACaGggATGTTtAAtGGaGGcGTaTAtgAgCACagtAAtGCgG
(1187) TTGGCAAGGAcGCaACCGCaATcTTCAACGGCGGTGTtTACaACCACTCCAACGCcG

(1557) CcCACAACCTcCTtTCtACcATGAGGGTtGGTGTcaTcCGcGGTGGCTGtGAaGTTG
(1199) CgCAtAAtCTGtTgTCGACaATGAGGGTGGGaGTgcTtaGaGGTGGcagGAGGTgG
(1244) CtCACAACCTGCTcTCGACtATGcGtGTGGGTGTttTgCGtGGcGGCTGcGAGGTTG

(1614) AGATCTGGAAGCGCGCtCAGcGTGAgAACAAGGATGTCGGtctGGTC--CTgGAcGA
(1256) AGgTgTGGAAGaa-GCagAGaGTGg-----AtGtTtTaGGgAaGagCGaCaTttTGA
(1301) AGATCTGGAAGCGCGCcCAGtccGAaAACAAGGAcGTCtcaAccGTCG--TTGATtc

(1669) cgCAGGcAACCCaATCaTCaGGGCTGGTaacCAGattACCAAGGTTGCGCAaCCcAT
(1307) gaCAGGTtACgCgggtGgagaGGtTGGTt--gAGGgGgCtgtGGcTGCGtAGCtaAc
(1356) ttCgGGTAACCgcATCGTCcGcGCgGGTgggCAaGcGACCAAGGTcGtcCAGCCtgT

(1726) TCAGaGTGCTagtGCaGCaTagattggatcttcatcttcacgagcgatgtatggcgt
(1362) TgAccaTGg------------------------------------------------
(1413) TCcGgGTGCTcagGCcGCgTga-----------------------------------
```

# Fig. 2c

```
(1783) ttggttgtctctcttccttggcggacagagtaatattcaatttcttagcgatcgtta
(1371) ---------------------------------------------------------
(1435) ---------------------------------------------------------

(1840) gaaagcatcatggttacgatgctcagtcatgttagatggcgtatgtttgtagccttc
(1371) ---------------------------------------------------------
(1435) ---------------------------------------------------------

(1897) ctcgagtgattggstatgaaaagtagcctcacggcctagaccaagaatgaaaacatt
(1371) ---------------------------------------------------------
(1435) ---------------------------------------------------------

(1954) cacgatttcagaaaaaaaaaaaaaaaaaaactcgagggggggcc
(1371) --------------------------------------------
(1435) --------------------------------------------
```

**Fig. 2d**

```
LnD9DS-2 (SEQ ID NO:14)             (1) ----------------------
LnD9DS-1 (SEQ ID NO:49)             (1) ----------------------
AnD9DS (SEQ ID NO:50)               (1) ----------------------
MgD9DS (SEQ ID NO:12)               (1) ----------------------
ScOLE1 AAA34826 (SEQ ID NO:52)      (1) MPTSGTTIELIDDQFPKDDSAS

  (1) ---------------------------------------------------MAALDSI
  (1) ---------------------------MPSHQAVAGMQAIDPEFVKQPSPMASTSEPN
  (1) --------------------------------------------------MSAPTADI
  (1) ---------------------------------MASSSSSVPELAAAFPDGTTDFKPM
 (23) SGIVDEVDLTEANILATGLNKKAPRIVNGFGSLMGSKEMVSVEFDKKGNEKKSNLDRL

  (8) PEDKA----TSSKSTHIQYQEVTFRNWYKKINWLNTTLVVLIPALGLYLTRT--TPLT
 (32) RNSK-----YDPKKPHITDMPITRSNWYQHVNWLNVIFIIGVPLAGCVAAFW--TPLQ
  (9) RARA-----PEAKKVHIADTAINRHNWYKHVNWLNVFLIIGIPLYGCIQAFW--VPLQ
 (26) RNTKG----YDVSKPHISETPMTLKNWHKHVNWLNTTFILFVPLAGLISTYW--VPLQ
 (81) LEKDNQEKEEAKTKIHISEQPWTLNNWHQHLNWLNMVLVCGMPMIGWYFALSGKVPLH

 (60) RPTLIWSVLYYFCTAFGITGGYHRLWSHRSYSARLPLRLFLAFTGAGAIQGSARWWSA
 (83) WKTAAWAVIYYFWTGLGITAGYHRLWAHKSYNAGLPLRIWLAAVGAGAVEGSIRWWSR
 (60) LKTAIWAVIYYFFTGLGITAGYHRLWAHCSYSATLPLRIWLAAVGGGAVEGSIRWWAR
 (78) WKTAVWAVVYYFNTGLGITAGYHRLWAHSSYKASLPLKIYLAAVGAGAVEGSIRWWSN
(139) LNVFLFSVFYYAVGGVSITAGYHRLWSHRSYSAHWPLRLFYAIFGCASVEGSAKWWGH

(118) NHRAHHRWTDTMKDPYSVMRGLLFSHIGWMVLNSDPKVKGRTDVSDLDSDPVVVWQHK
(141) DHRAHHRYTDTNKDPYSVRKGLLYSHLGWMVMKQNPKRIGRTDITDLNEDPVVVWQHK
(118) DHRAHHRYTDTDKDPYSVRKGLLYSHLGWMVMKQNPKRIGRTDISDLNEDPVVVWQHR
(136) GHRAHHRYTDTEKDPYSVRKGLLYSHMGWMLLKQNPKKQGRTDITDLNEDPVVVWQHR
(197) SHRIHHRYTDTLRDPYDARRGLWYSHMGWMLLKPNPKYKARADITDMTDDWTIRFQHR

(176) HYGKCLLFAAWIFPMIVAGLGWGDWWGGLVYAGIIRACFVQQATFCVNSLAHWIGEQP
(199) NYIKAVVTMGLIFPSAVAGLMWGDWMGGFIYAGILRIFFVQQATFCVNSLAHWLGDQP
(176) NYLKVVFTMGLAVPMLVAGLGWGDWLGGFVYAGILRIFFVQQATFCVNSLAHWLGDQP
(194) NFLKCVIFMALVFPTLVAGLGWGDYWGGFIYGGILRVFFVQQATFCVNSLAHWLGDQP
(255) HYILLMLLTAFVIPTLICGYFFNDYMGGLIYAGFIRVFVIQQATFCINSMAHYIGTQP

(234) FDDRRTPRDHVLTALVTMGEGYHNFHHEFPSDYRNAIIWYQYDPTKWLIYLFSLGPFP
(257) FDDRNSPRDHVITALVTLGEGYHNFHHEFPSDYRNAIEWHQYDPTKWSIWLWSK--LG
(234) FDDRNSPRDHVITALVTLGEGYHNFHHEFPSDYRNAIEWHQYDPTKWSIWAWKQ--LG
(252) FDDRNSPRDHVITALVTLGEGYHNFHHEFPSDYRNAIEWYQYDPTKWSIWIWKQ--LG
(313) FDDRRTPRDNWITAIVTFGEGYHNFHHEFPTDYRNAIKWYQYDPTKVIIYLTSL--VG
```

**Fig. 3a**

```
(292) LAYSLKTFRSNEIEKGRLQQQQKALDKKRSGLDWGLPLFQLPVISWDDFQARCKESGE
(313) LASNLKQFRSNEIEKGRVQQLQKKIDQKRAKLDWGVPLDQLPVIEWDDYVEQAK-NGR
(290) LAYDLKKFRANEIEKGRVQQLQKKLDRKRATLDWGTPLDQLPVMEWDDYVEQAK-NGR
(308) LAHNLKQFRQNEIEKGRVQQLQKKLDQKRAKLDWGIPLEQLPVVSWDDFVEQSK-NGK
(369) LAYDLKKFSQNAIEEALIQQEQKKINKKKAKINWGPVLTDLPMWDKQTFLAKSK-ENK

(350) MLVAVAGVIHDVSQFIEDHPGGRSLIRSAVGKDGTGMFNGGVYEHSNAAHNLLSTMRV
(370) GLIAVAGVVHDVTDFINEHPGGKTLIKSGVGKDATAMFNGGVYFHSNGAHNLLSTMRV
(347) GLVAIAGVVHDVTDFIKDHPGGKAMISSGIGKDATAMFNGGVYYHSNAAHNLLSTMRV
(365) AWIAVAGVIHDVGDFIKDHPGGRALINSAIGKDATAIFNGGVYNHSNAAHNLLSTMRV
(426) GLVIISGIVHDVSGYISEHPGGETLIKTALGKDATKAFSGGVYRHSNAAQNVLADMRV

(408) GVLRGGQEVEVWKKQR-------VDVLGKSD----ILRQVTRVERLVEGAVAA
(428) GVIRGGCEVEIWKRAQRENKDVGLVLDDAGNPIIRAGNQITKVAQPIQSASAA
(405) GVIRGGCEVEIWKRAQKEN--VEYVRDGSGQRVIRAGEQPTKIPEPIPTADAA
(423) GVLRGGCEVEIWKRAQSENKDVSTVVDSSGNRIVRAGGQATKVVQPVPGAQAA
(484) AVIK------------------ESKN-S------AIRMASKRGEIYETGKFF
```

**Fig. 3b**

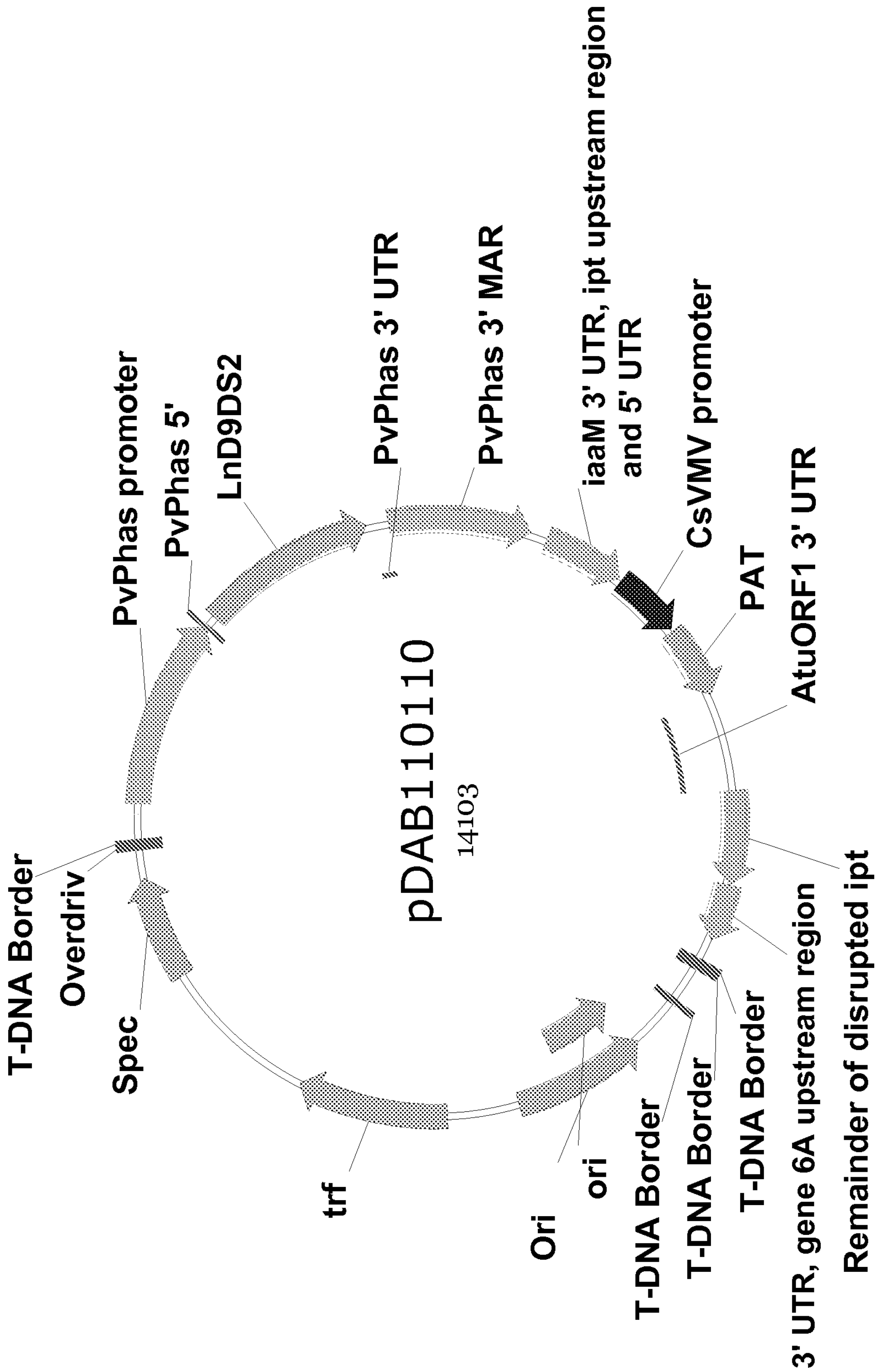
pDAB110110
14103
T-DNA Border
Overdriv
Spec
PvPhas promoter
PvPhas 5'
LnD9DS2
PvPhas 3' UTR
PvPhas 3' MAR
iaaM 3' UTR, ipt upstream region and 5' UTR
CsVMV promoter
PAT
AtuORF1 3' UTR
Remainder of disrupted ipt
ipt 3' UTR, gene 6A upstream region
T-DNA Border
T-DNA Border
T-DNA Border
ori
Ori
trf

Fig. 4a

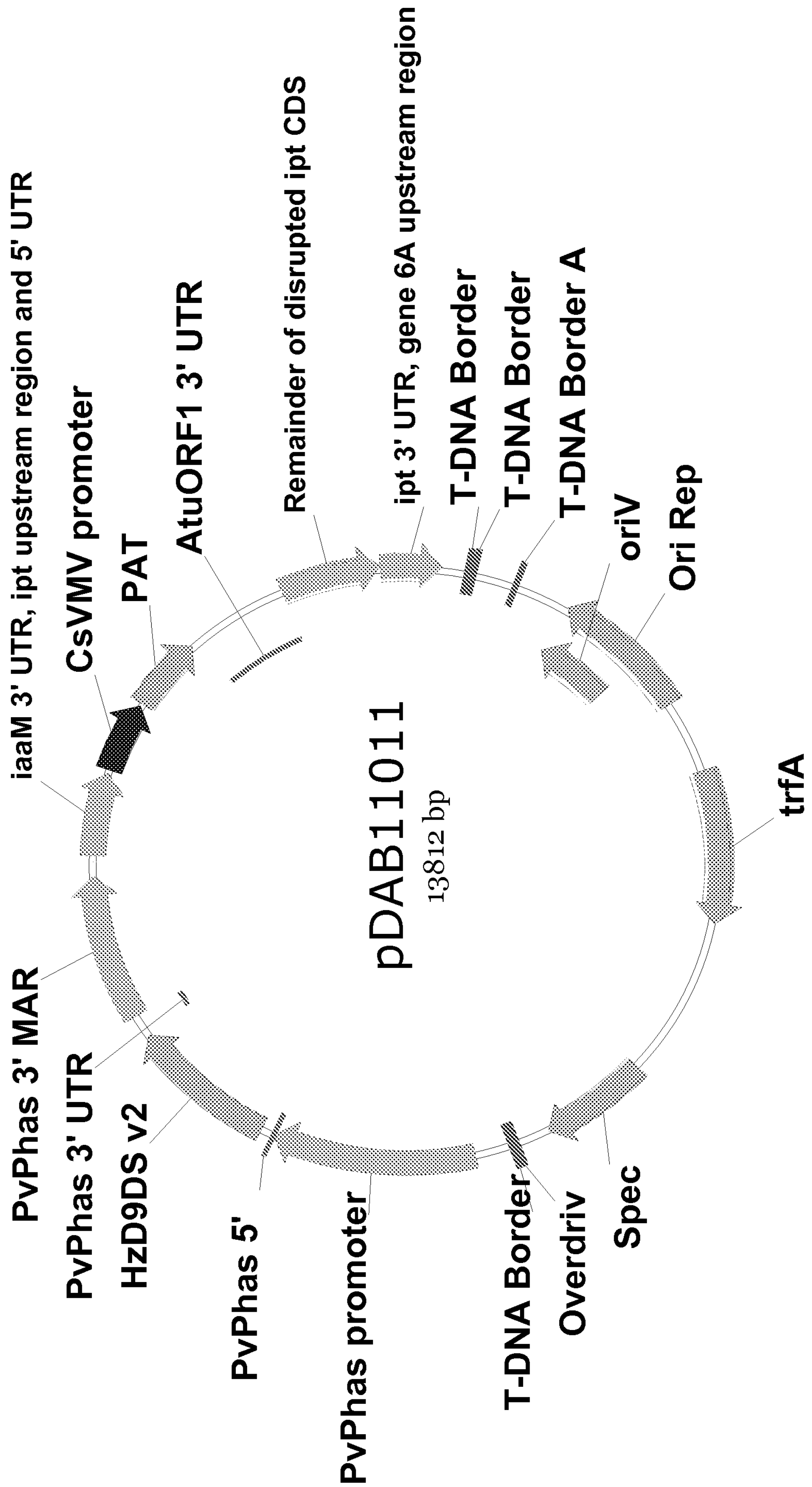
pDAB11011
13812 bp
iaaM 3' UTR, ipt upstream region and 5' UTR
CsVMV promoter
PAT
AtuORF1 3' UTR
Remainder of disrupted ipt CDS
ipt 3' UTR, gene 6A upstream region
T-DNA Border
T-DNA Border
T-DNA Border A
oriV
Ori Rep
trfA
Spec
Overdriv
T-DNA Border
PvPhas promoter
PvPhas 5'
HzD9DS v2
PvPhas 3' UTR
PvPhas 3' MAR

Fig. 4b

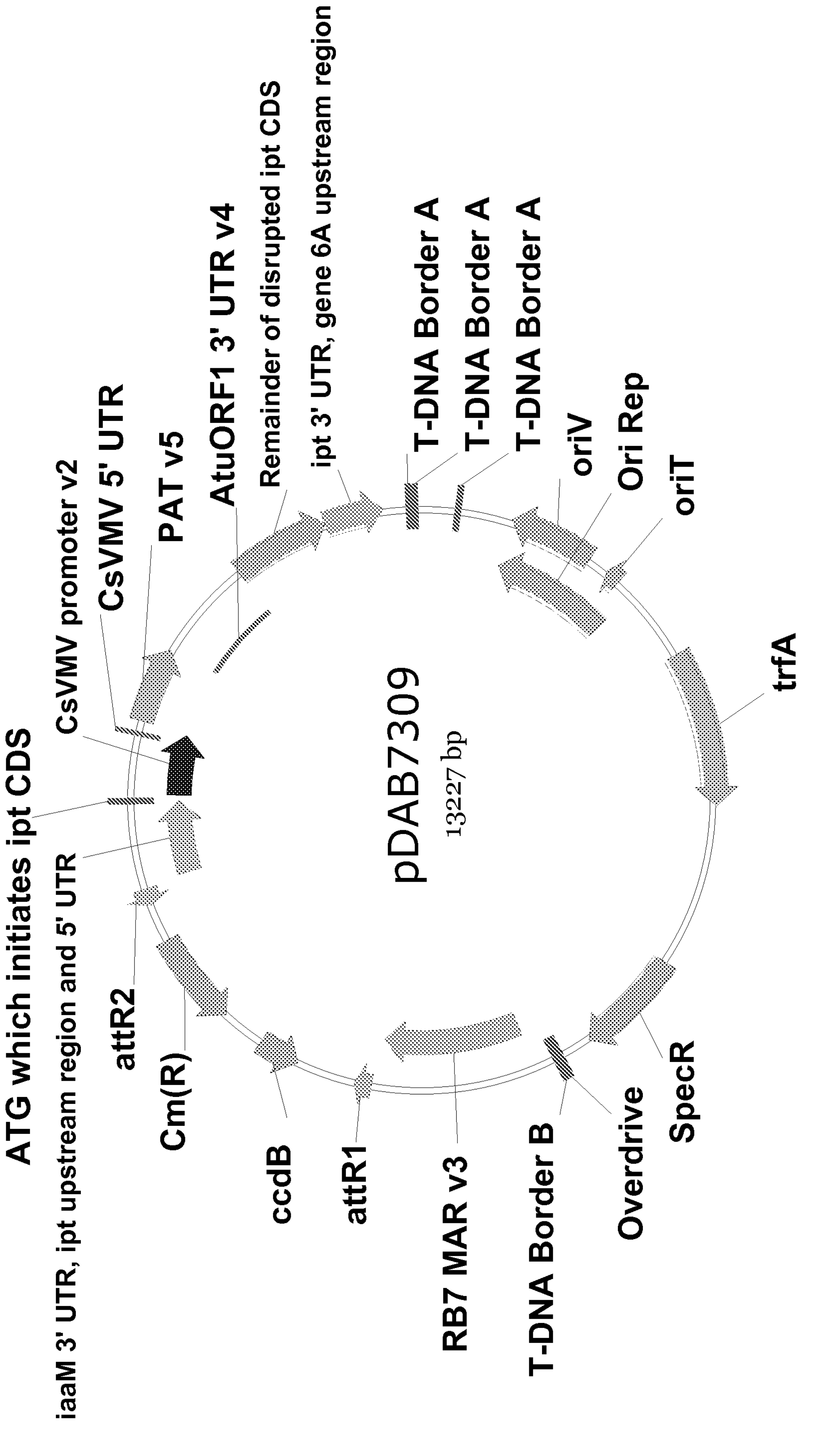
pDAB7309
13227 bp
ATG which initiates ipt CDS
iaaM 3' UTR, ipt upstream region and 5' UTR
CsVMV promoter v2
CsVMV 5' UTR
PAT v5
AtuORF1 3' UTR v4
Remainder of disrupted ipt CDS
ipt 3' UTR, gene 6A upstream region
T-DNA Border A
T-DNA Border A
T-DNA Border A
oriV
Ori Rep
oriT
trfA
SpecR
Overdrive
T-DNA Border B
RB7 MAR v3
attR1
ccdB
Cm(R)
attR2

Fig. 5

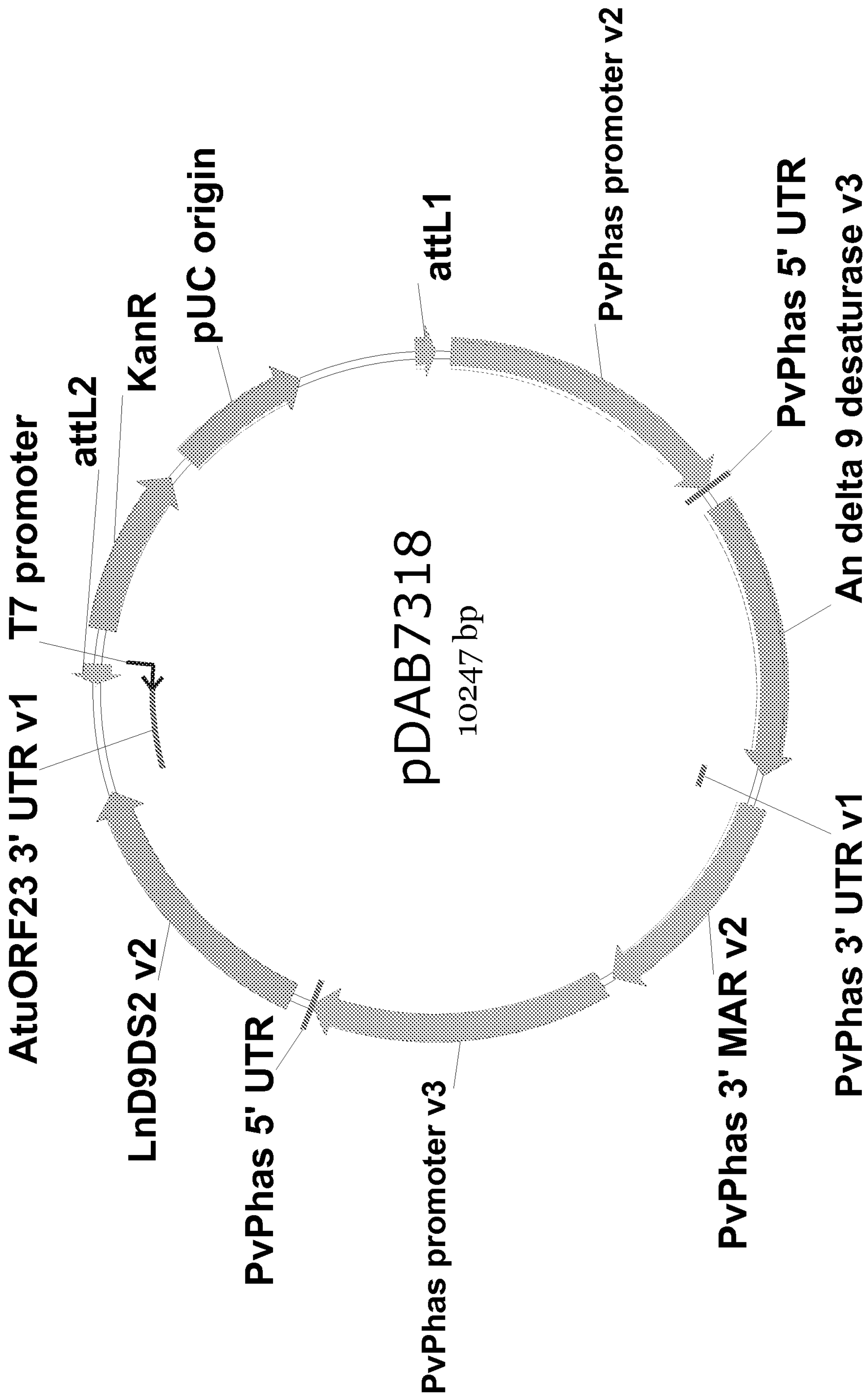
pDAB7318
10247 bp
attL1
PvPhas promoter v2
PvPhas 5' UTR
An delta 9 desaturase v3
PvPhas 3' UTR v1
PvPhas 3' MAR v2
PvPhas promoter v3
PvPhas 5' UTR
LnD9DS2 v2
AtuORF23 3' UTR v1
T7 promoter
attL2
KanR
pUC origin

Fig. 6

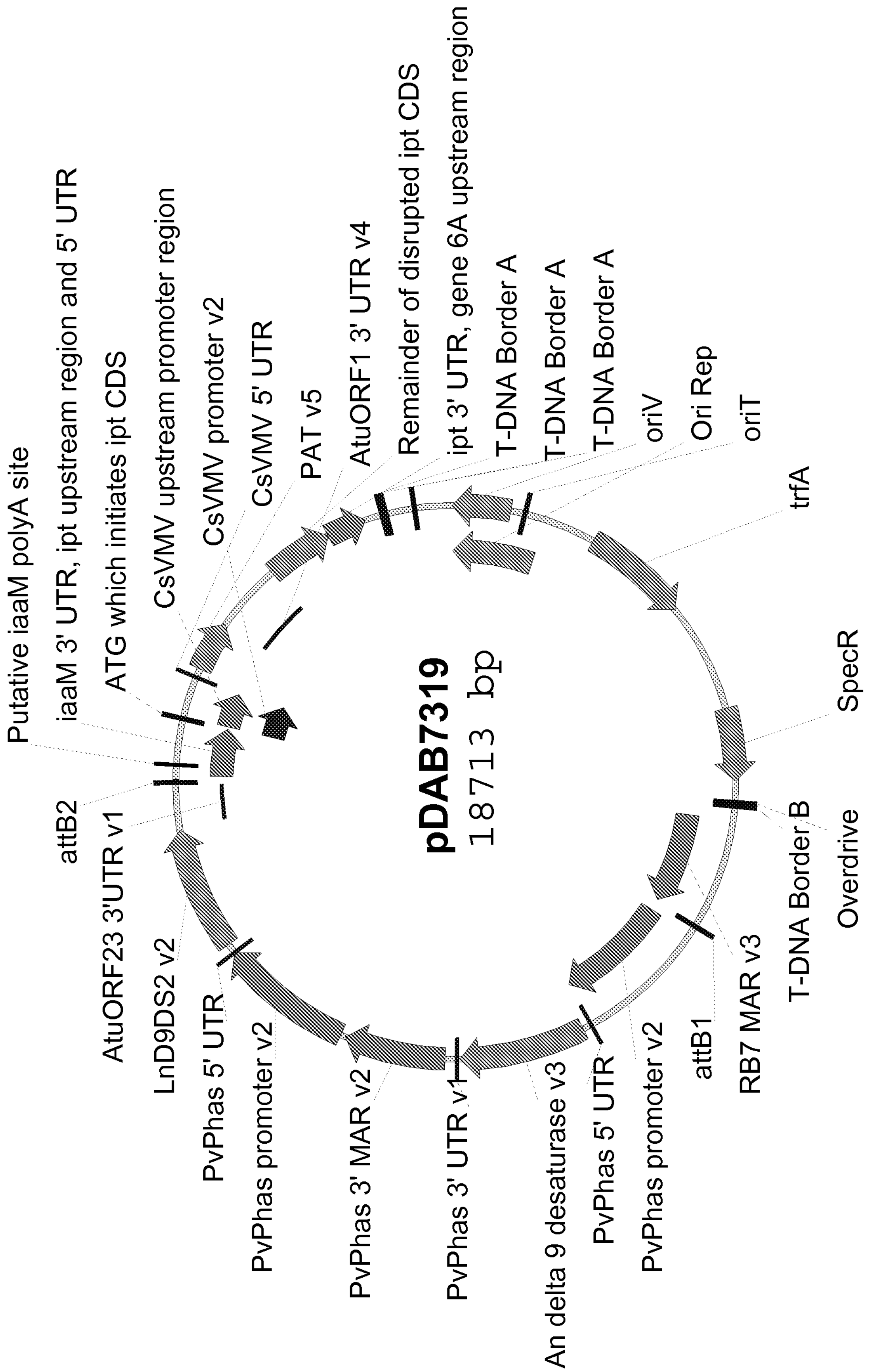
pDAB7319
18713 bp
Putative iaaM polyA site
iaaM 3' UTR, ipt upstream region and 5' UTR
ATG which initiates ipt CDS
CsVMV upstream promoter region
CsVMV promoter v2
CsVMV 5' UTR
PAT v5
AtuORF1 3' UTR v4
Remainder of disrupted ipt CDS
ipt 3' UTR, gene 6A upstream region
T-DNA Border A
T-DNA Border A
T-DNA Border A
oriV
Ori Rep
oriT
trfA
SpecR
T-DNA Border B
Overdrive
RB7 MAR v3
attB1
PvPhas promoter v2
PvPhas 5' UTR
An delta 9 desaturase v3
PvPhas 3' UTR v1
PvPhas 3' MAR v2
PvPhas promoter v2
PvPhas 5' UTR
LnD9DS2 v2
AtuORF23 3'UTR v1
attB2

Fig. 7

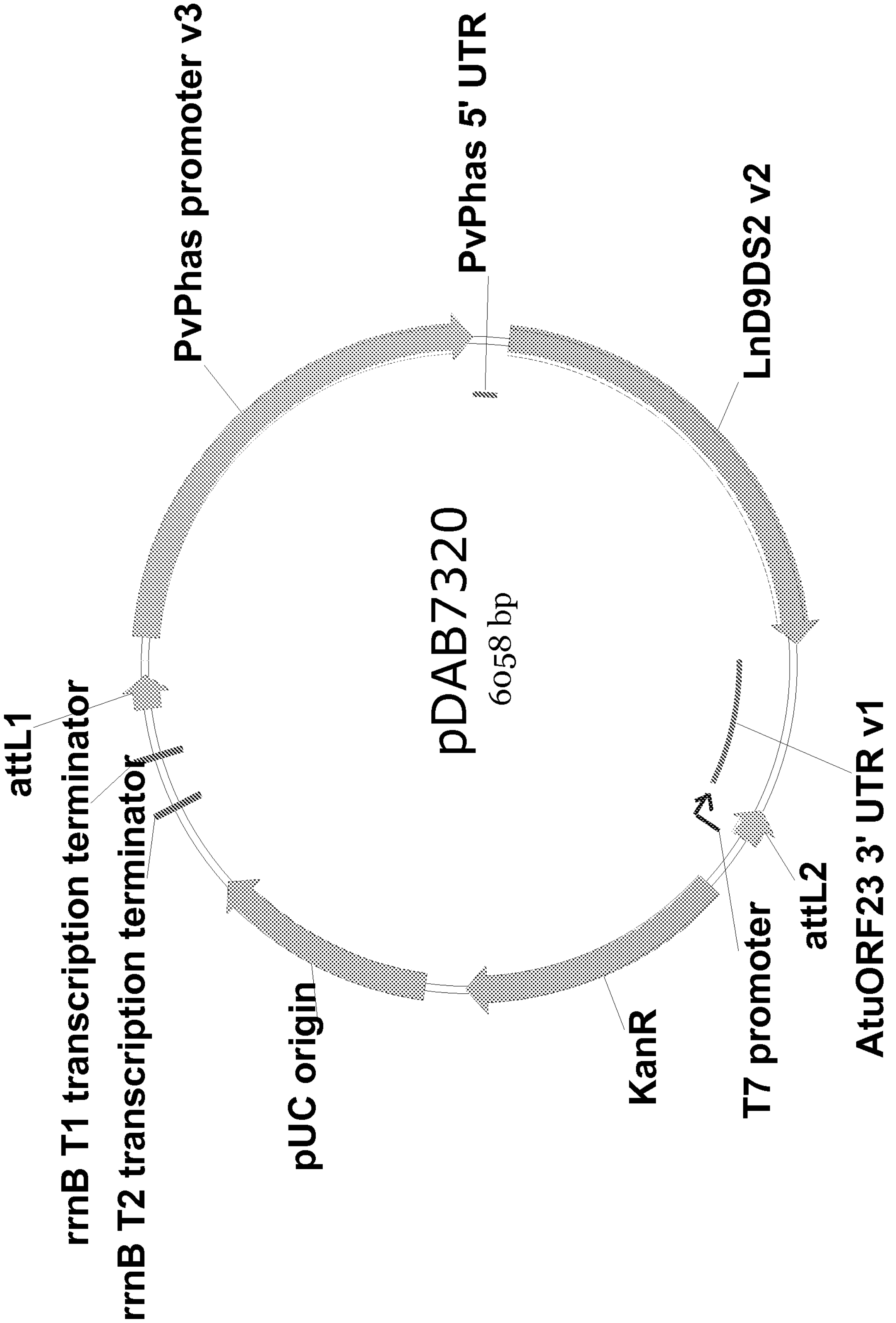
pDAB7320
6058 bp
PvPhas promoter v3
PvPhas 5' UTR
LnD9DS2 v2
AtuORF23 3' UTR v1
attL2
T7 promoter
KanR
pUC origin
rrnB T2 transcription terminator
rrnB T1 transcription terminator
attL1

Fig. 8

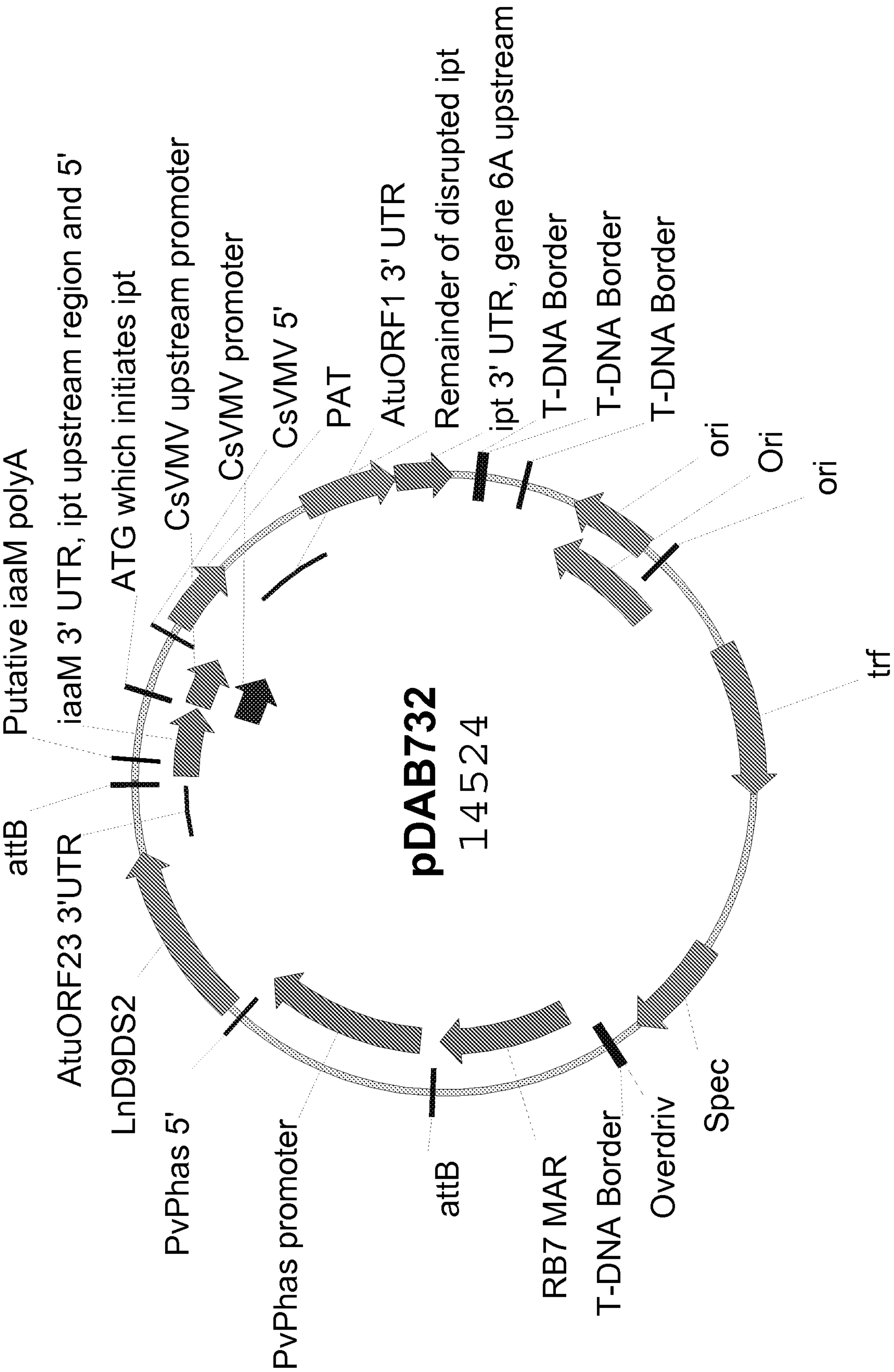
pDAB732
14524
Putative iaaM polyA
iaaM 3' UTR, ipt upstream region and 5'
ATG which initiates ipt
CsVMV upstream promoter
CsVMV promoter
CsVMV 5'
PAT
AtuORF1 3' UTR
Remainder of disrupted ipt
ipt 3' UTR, gene 6A upstream
T-DNA Border
T-DNA Border
T-DNA Border
ori
Ori
ori
trf
Spec
Overdriv
T-DNA Border
RB7 MAR
attB
PvPhas promoter
PvPhas 5'
LnD9DS2
AtuORF23 3'UTR
attB

Fig. 9

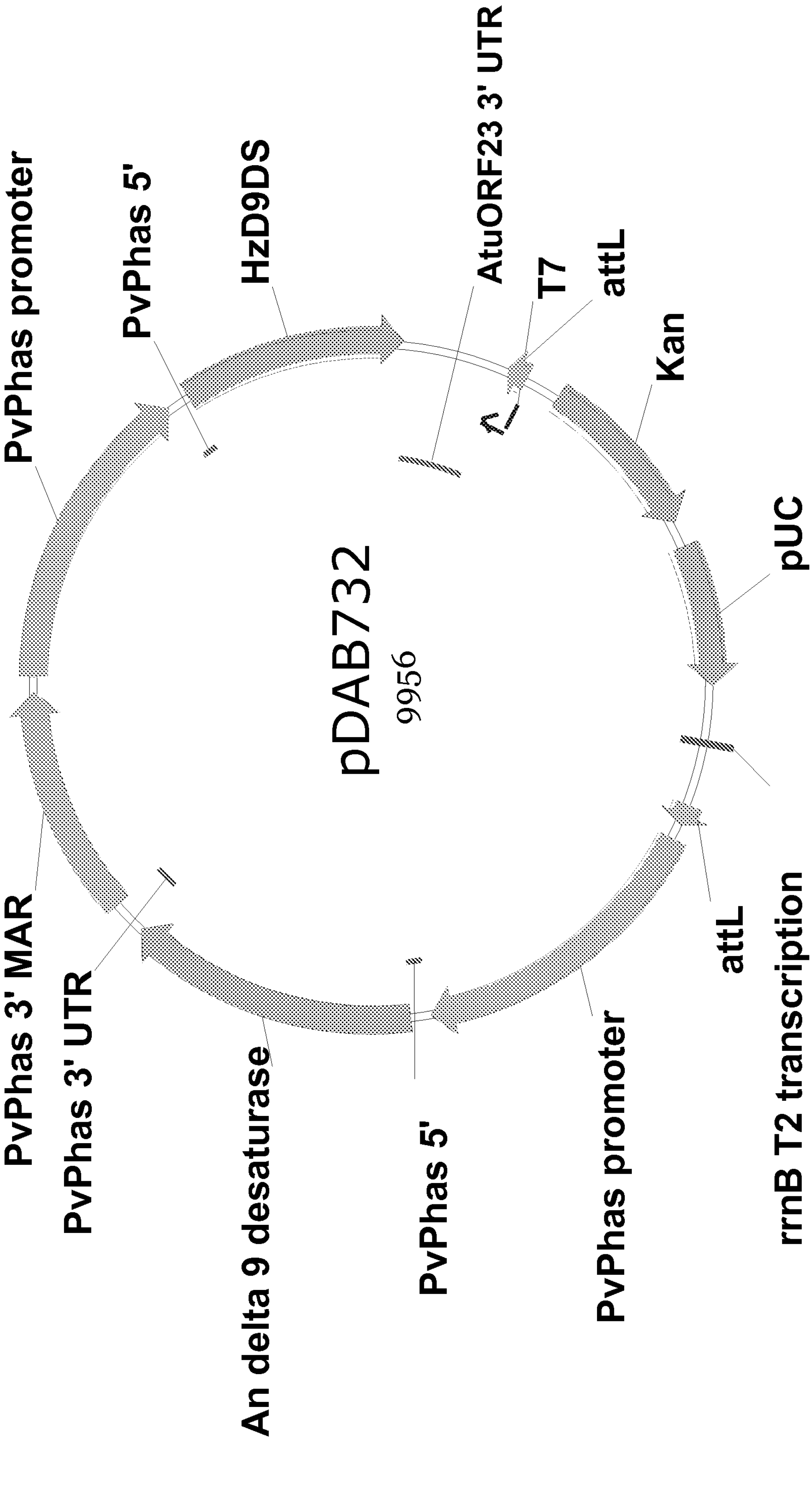
pDAB732
9956
PvPhas promoter
PvPhas 5'
HzD9DS
AtuORF23 3' UTR
T7
attL
Kan
pUC
rrnB T2 transcription
attL
PvPhas promoter
PvPhas 5'
An delta 9 desaturase
PvPhas 3' UTR
PvPhas 3' MAR

Fig. 10

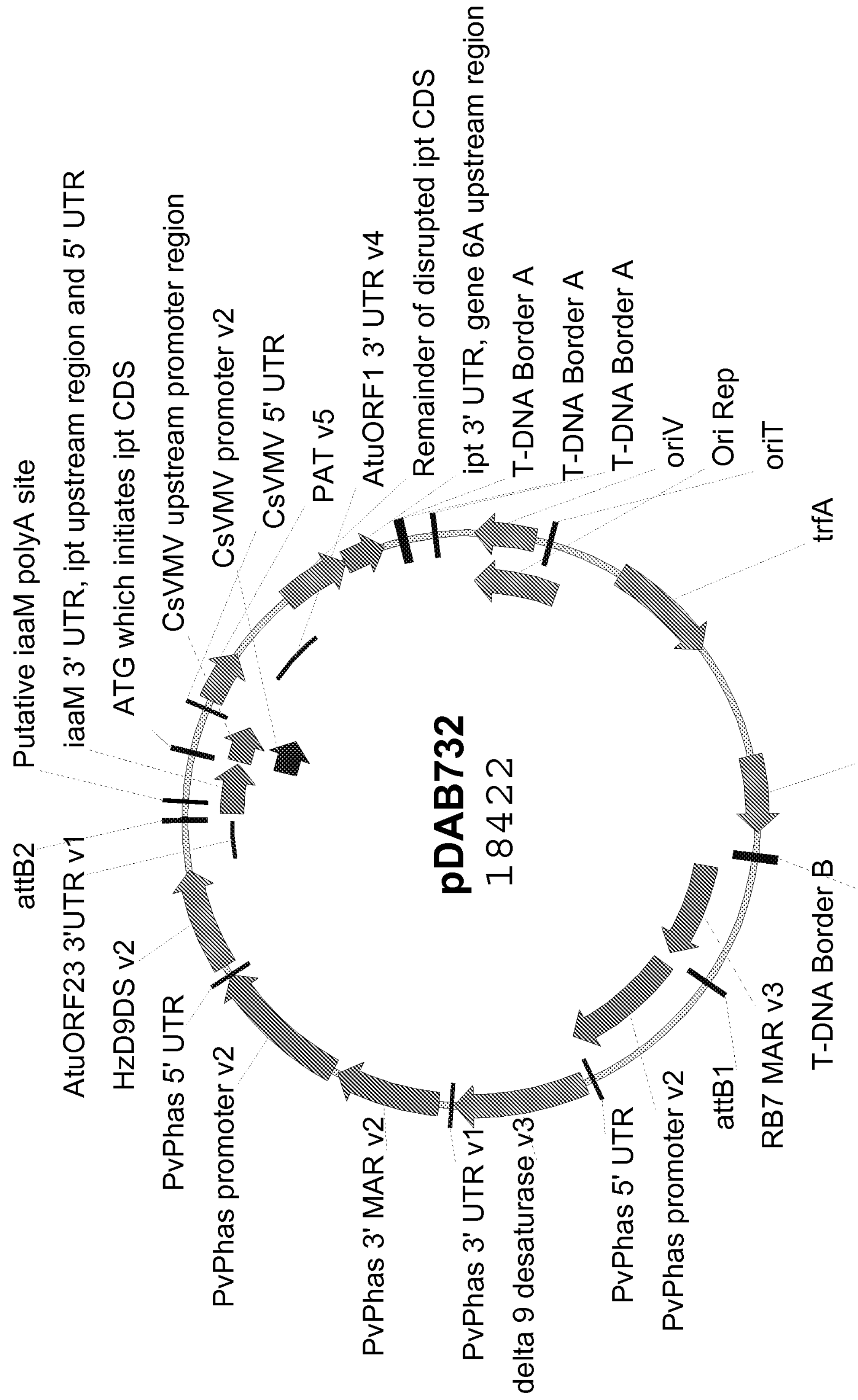
pDAB732
18422
Putative iaaM polyA site
iaaM 3' UTR, ipt upstream region and 5' UTR
ATG which initiates ipt CDS
CsVMV upstream promoter region
CsVMV promoter v2
CsVMV 5' UTR
PAT v5
AtuORF1 3' UTR v4
Remainder of disrupted ipt CDS
ipt 3' UTR, gene 6A upstream region
T-DNA Border A
T-DNA Border A
T-DNA Border A
oriV
Ori Rep
oriT
trfA
SpecR
T-DNA Border B
Overdrive
T-DNA Border B
RB7 MAR v3
attB1
PvPhas promoter v2
PvPhas 5' UTR
An delta 9 desaturase v3
PvPhas 3' UTR v1
PvPhas 3' MAR v2
PvPhas promoter v2
PvPhas 5' UTR
HzD9DS v2
AtuORF23 3'UTR v1
attB2

Fig. 11

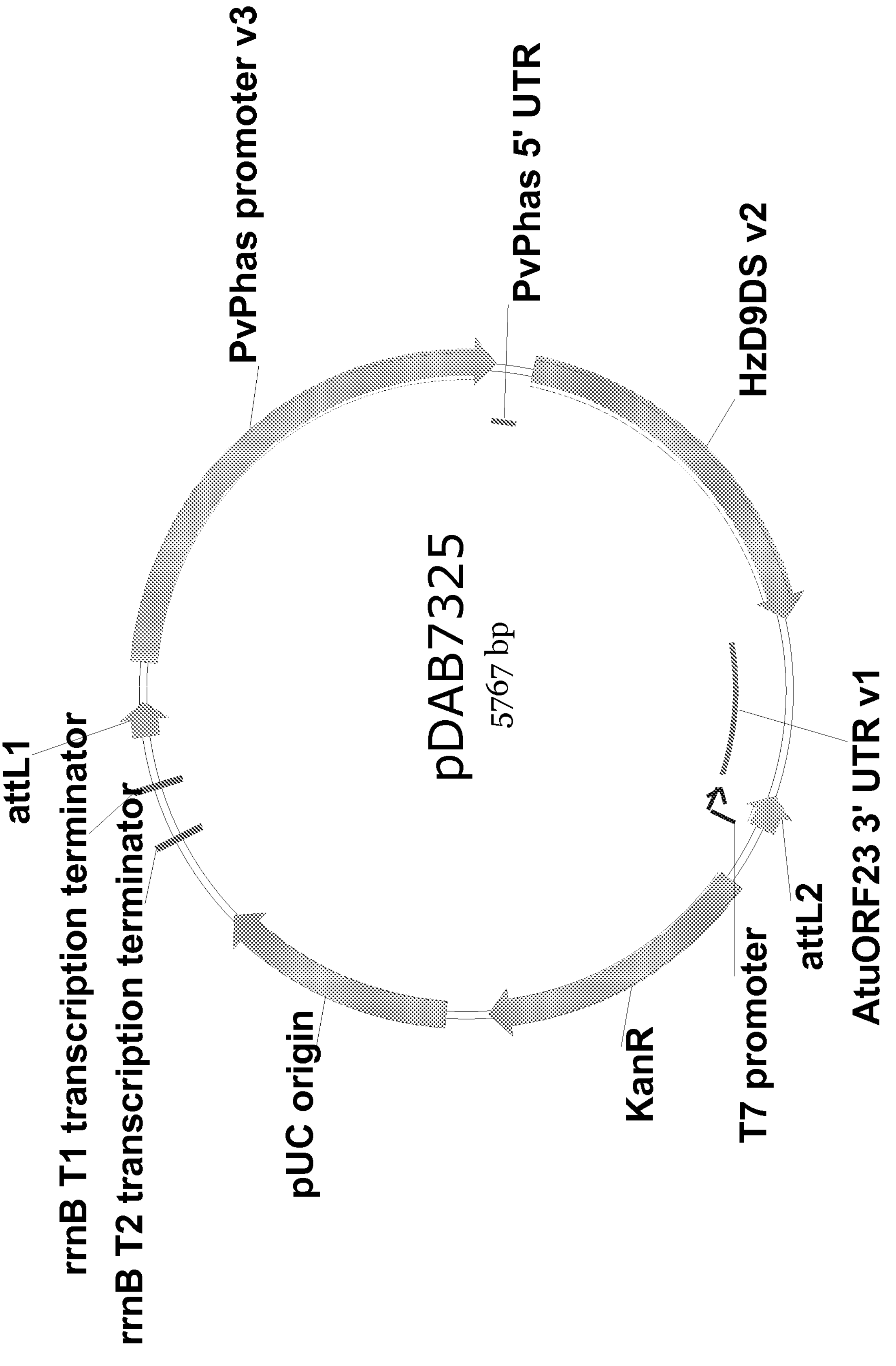
pDAB7325
5767 bp
PvPhas promoter v3
PvPhas 5' UTR
HzD9DS v2
AtuORF23 3' UTR v1
attL2
T7 promoter
KanR
pUC origin
rrnB T2 transcription terminator
rrnB T1 transcription terminator
attL1

Fig. 12

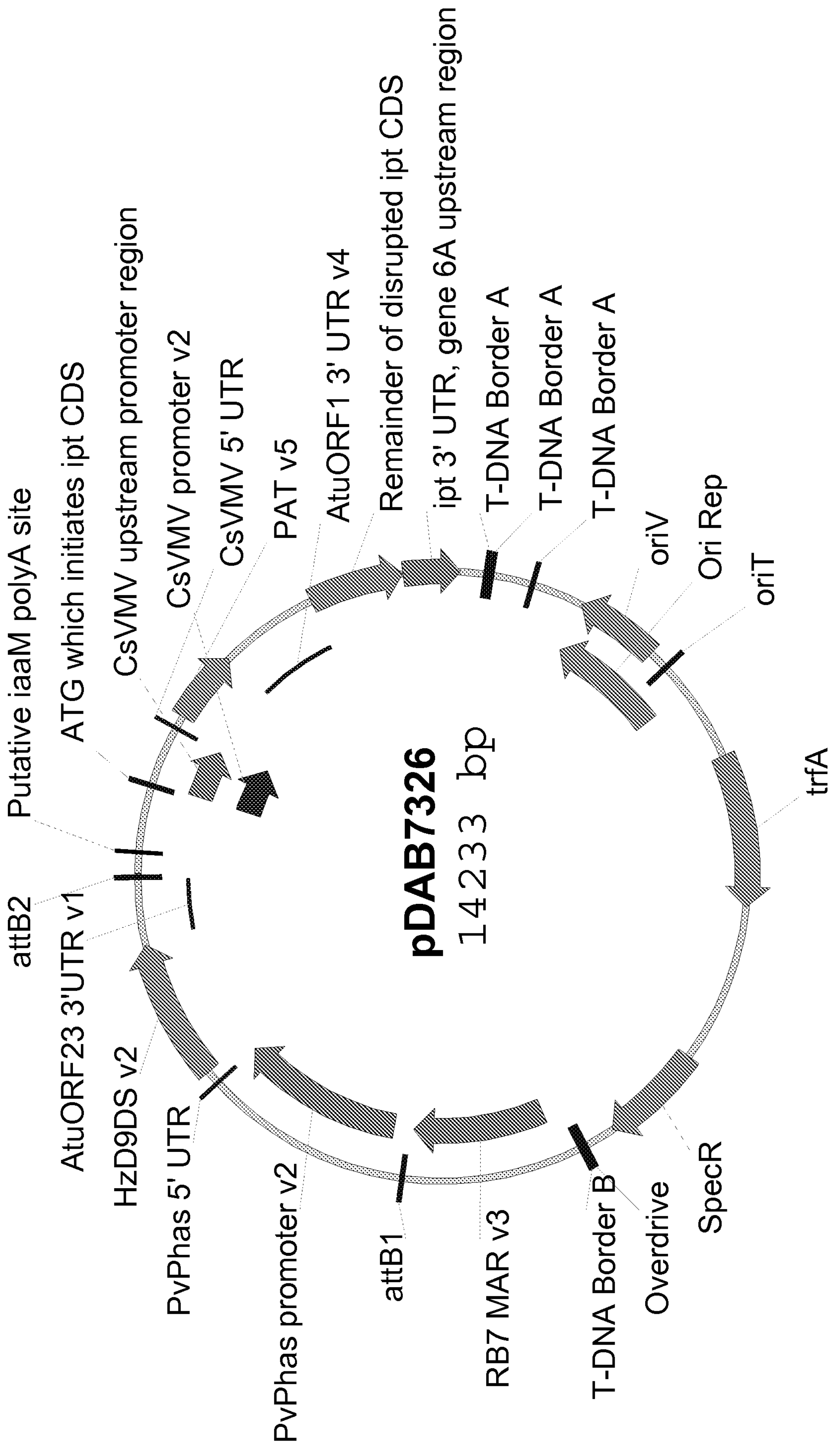
pDAB7326
14233 bp
Putative iaaM polyA site
ATG which initiates ipt CDS
CsVMV upstream promoter region
CsVMV promoter v2
CsVMV 5' UTR
PAT v5
AtuORF1 3' UTR v4
Remainder of disrupted ipt CDS
ipt 3' UTR, gene 6A upstream region
T-DNA Border A
T-DNA Border A
T-DNA Border A
oriV
Ori Rep
oriT
trfA
SpecR
Overdrive
T-DNA Border B
RB7 MAR v3
attB1
PvPhas promoter v2
PvPhas 5' UTR
HzD9DS v2
AtuORF23 3'UTR v1
attB2

**Fig. 13**

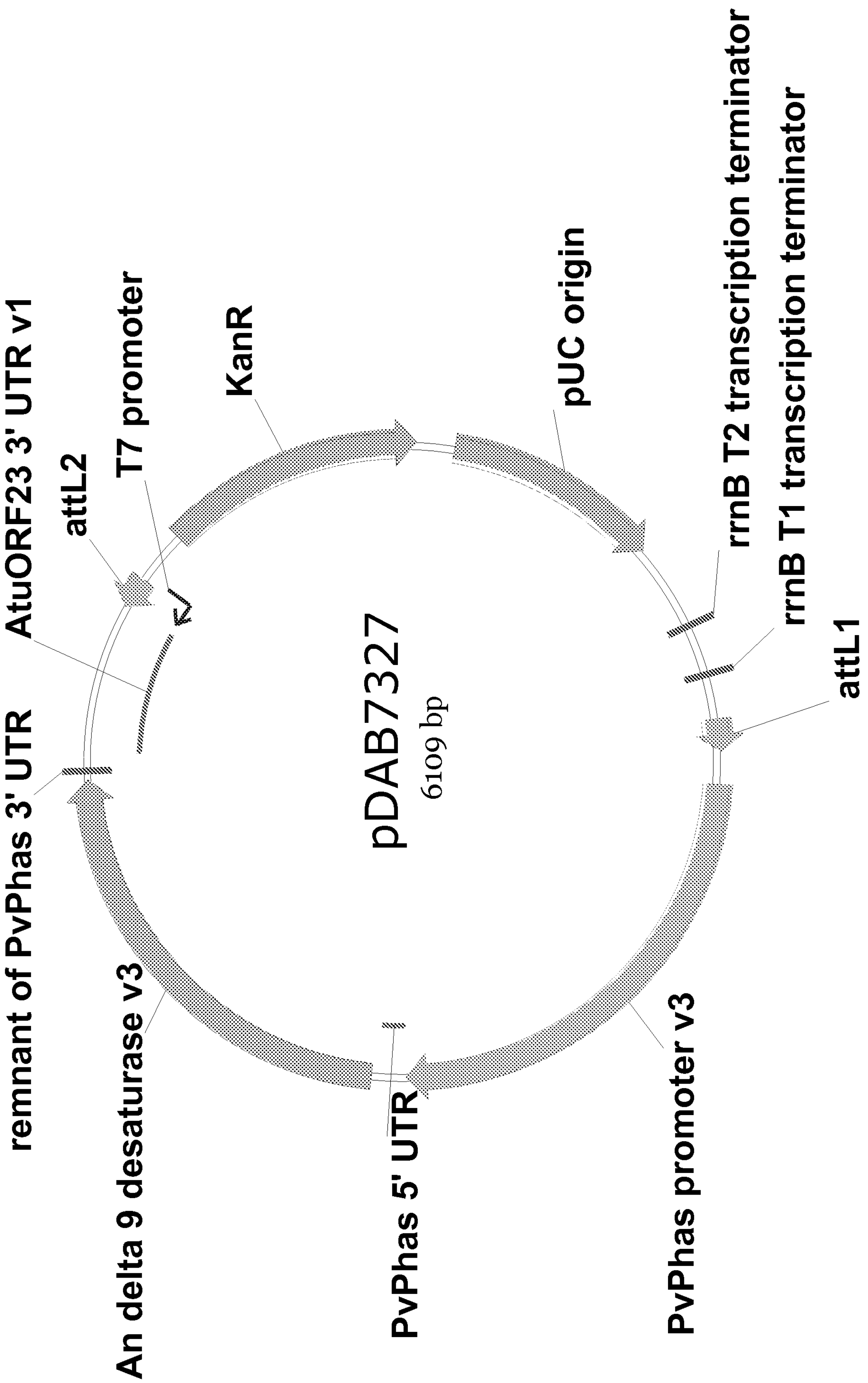
pDAB7327
6109 bp
AtuORF23 3' UTR v1
attL2
T7 promoter
KanR
pUC origin
rrnB T2 transcription terminator
rrnB T1 transcription terminator
attL1
PvPhas promoter v3
PvPhas 5' UTR
An delta 9 desaturase v3
remnant of PvPhas 3' UTR

Fig. 14

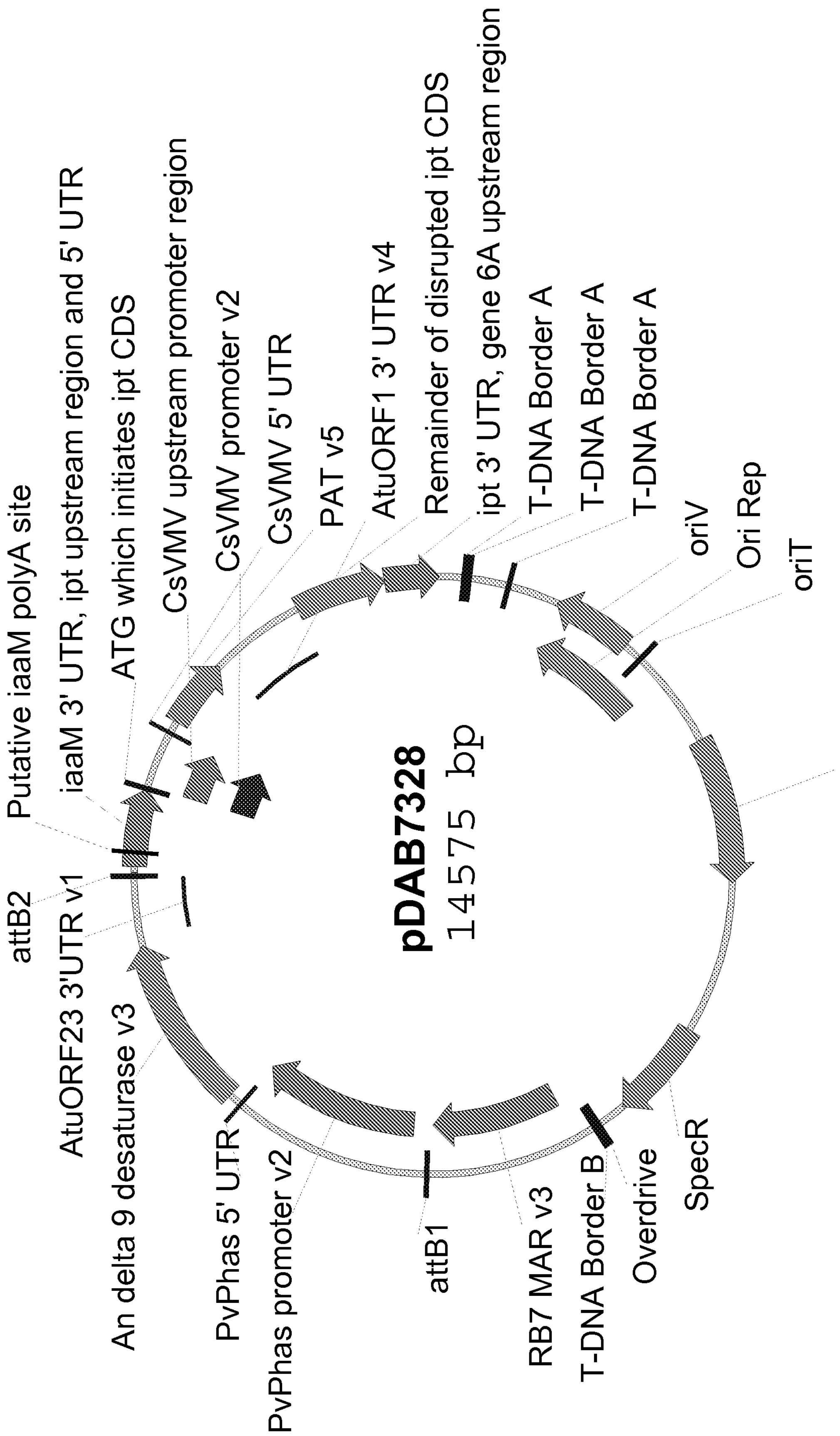
pDAB7328
14575 bp
Putative iaaM polyA site
iaaM 3' UTR, ipt upstream region and 5' UTR
ATG which initiates ipt CDS
CsVMV upstream promoter region
CsVMV promoter v2
CsVMV 5' UTR
PAT v5
AtuORF1 3' UTR v4
Remainder of disrupted ipt CDS
ipt 3' UTR, gene 6A upstream region
T-DNA Border A
T-DNA Border A
T-DNA Border A
oriV
Ori Rep
oriT
trfA
SpecR
Overdrive
T-DNA Border B
RB7 MAR v3
attB1
PvPhas promoter v2
PvPhas 5' UTR
An delta 9 desaturase v3
AtuORF23 3'UTR v1
attB2

Fig. 15

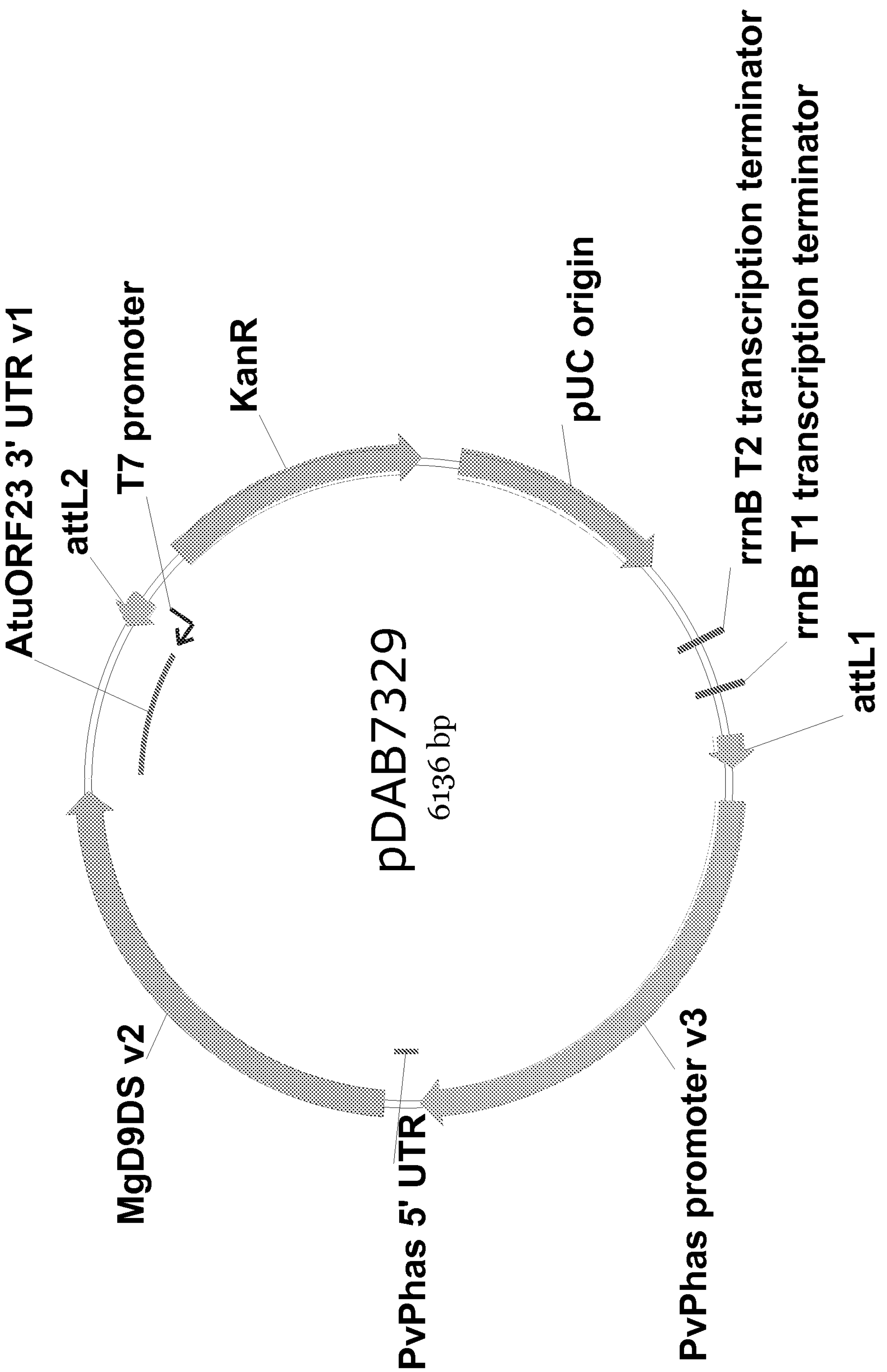
pDAB7329
6136 bp
AtuORF23 3' UTR v1
attL2
T7 promoter
KanR
pUC origin
rrnB T2 transcription terminator
rrnB T1 transcription terminator
attL1
PvPhas promoter v3
PvPhas 5' UTR
MgD9DS v2

Fig. 16

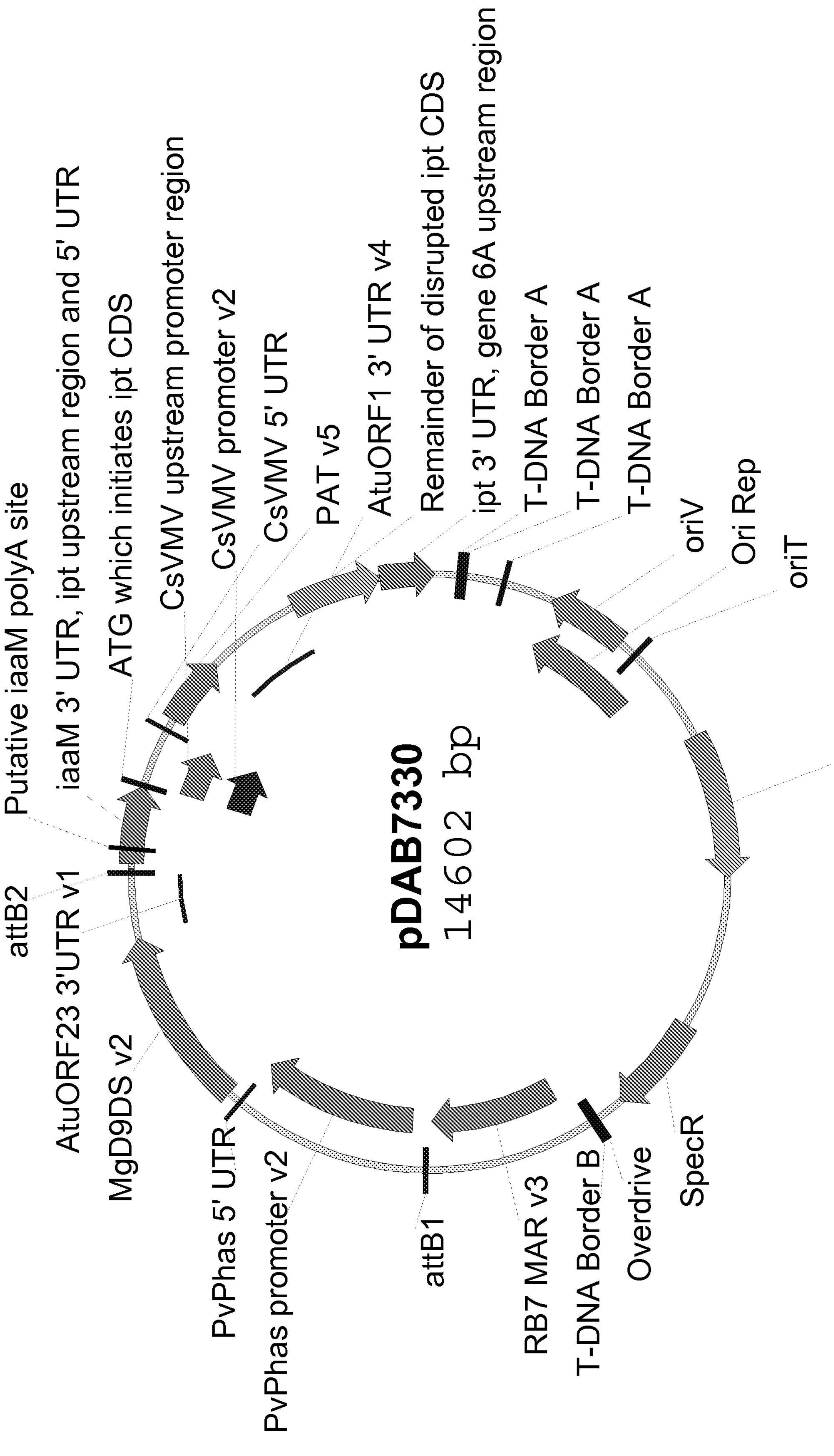
pDAB7330
14602 bp
Putative iaaM polyA site
iaaM 3' UTR, ipt upstream region and 5' UTR
ATG which initiates ipt CDS
CsVMV upstream promoter region
CsVMV promoter v2
CsVMV 5' UTR
PAT v5
AtuORF1 3' UTR v4
Remainder of disrupted ipt CDS
ipt 3' UTR, gene 6A upstream region
T-DNA Border A
T-DNA Border A
T-DNA Border A
oriV
Ori Rep
oriT
trfA
SpecR
Overdrive
T-DNA Border B
RB7 MAR v3
attB1
PvPhas promoter v2
PvPhas 5' UTR
MgD9DS v2
AtuORF23 3'UTR v1
attB2

Fig. 17

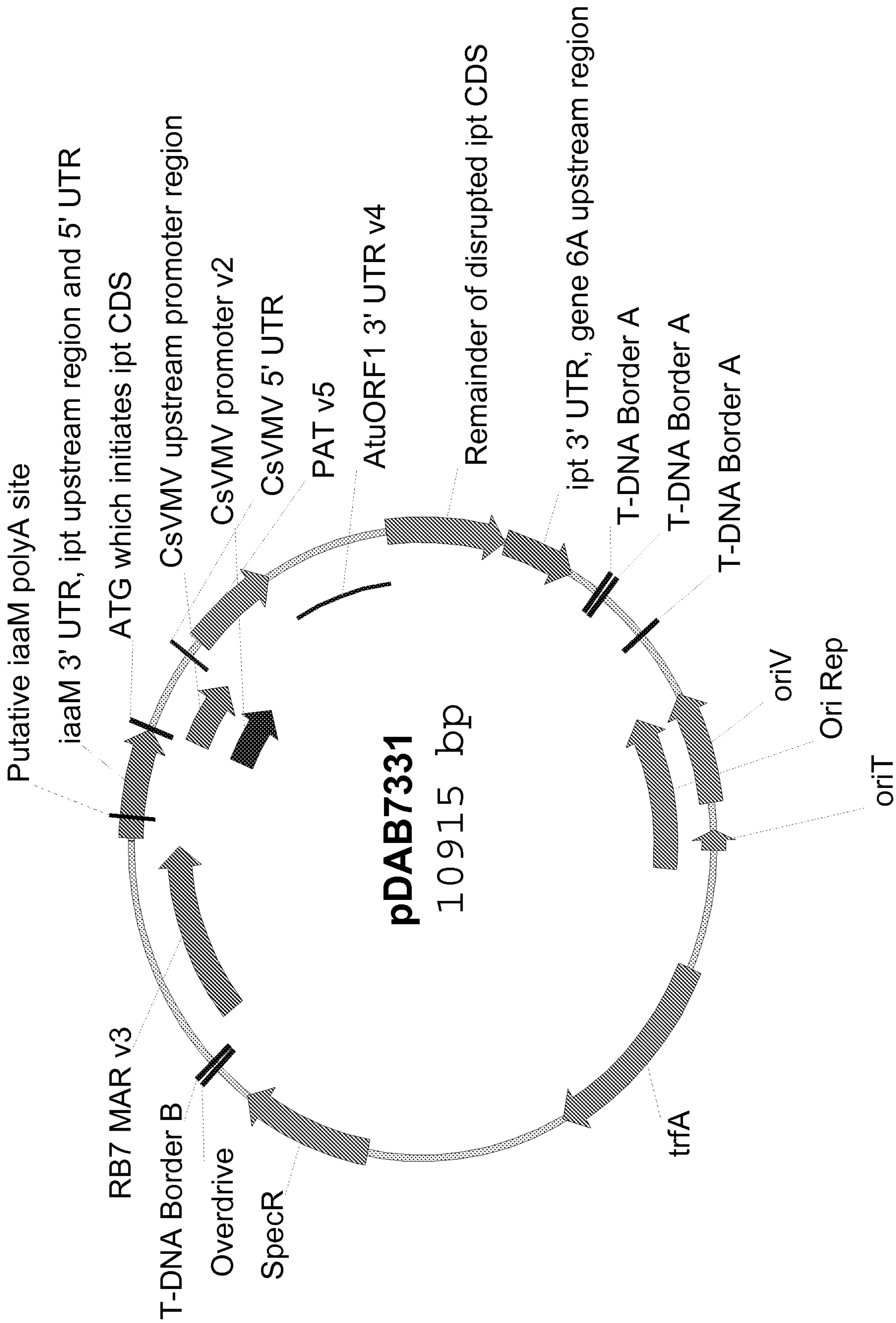
pDAB7331
10915 bp
Putative iaaM polyA site
iaaM 3' UTR, ipt upstream region and 5' UTR
ATG which initiates ipt CDS
CsVMV upstream promoter region
CsVMV promoter v2
CsVMV 5' UTR
PAT v5
AtuORF1 3' UTR v4
Remainder of disrupted ipt CDS
ipt 3' UTR, gene 6A upstream region
T-DNA Border A
T-DNA Border A
T-DNA Border A
oriV
Ori Rep
oriT
trfA
SpecR
Overdrive
T-DNA Border B
RB7 MAR v3

Fig. 18

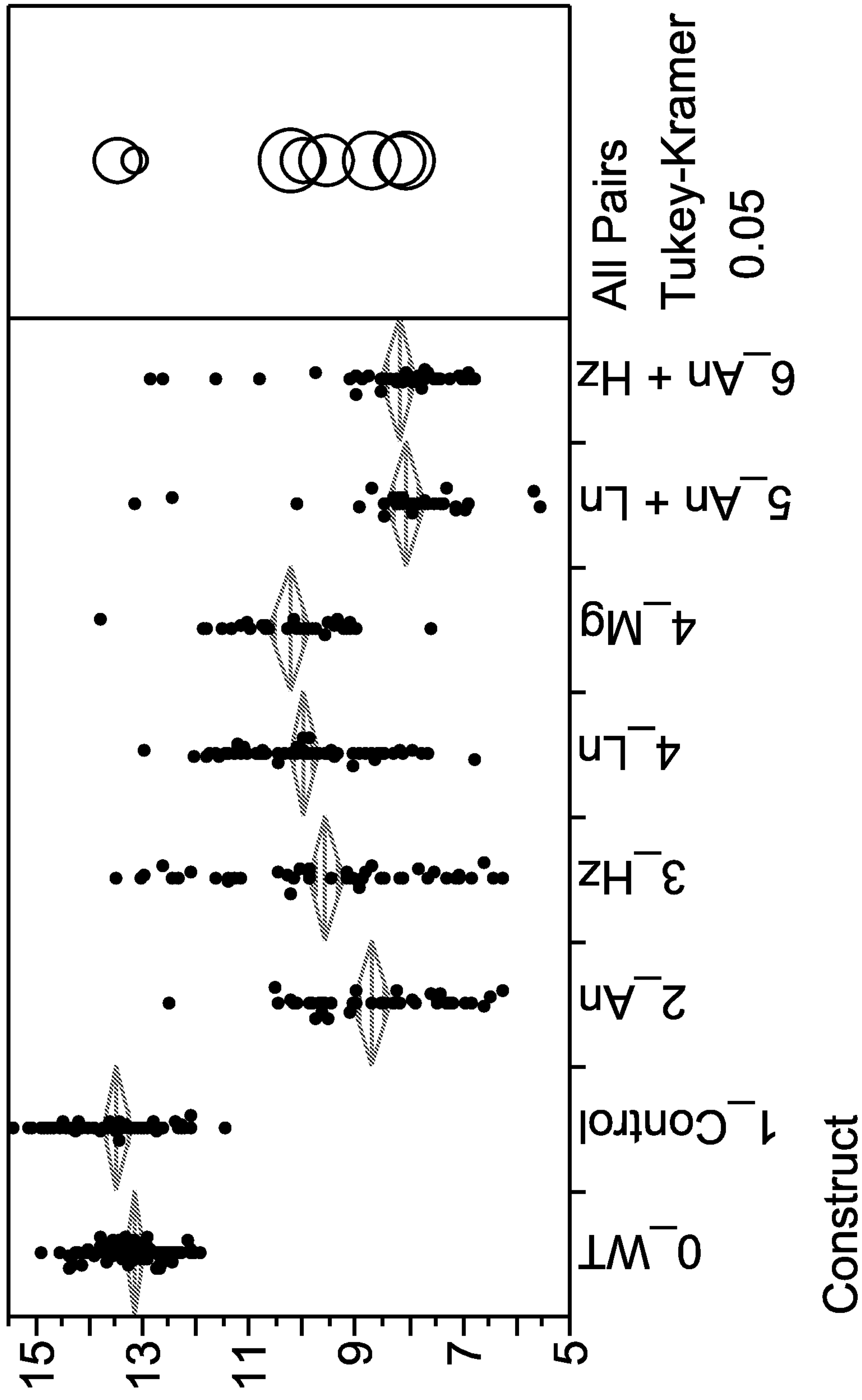
Total Sat (%)
15
13
11
9
7
5
0_WT
1_Control
2_An
3_Hz
4_Ln
4_Mg
5_An + Ln
6_An + Hz
Construct
All Pairs
Tukey-Kramer
0.05

Fig. 19

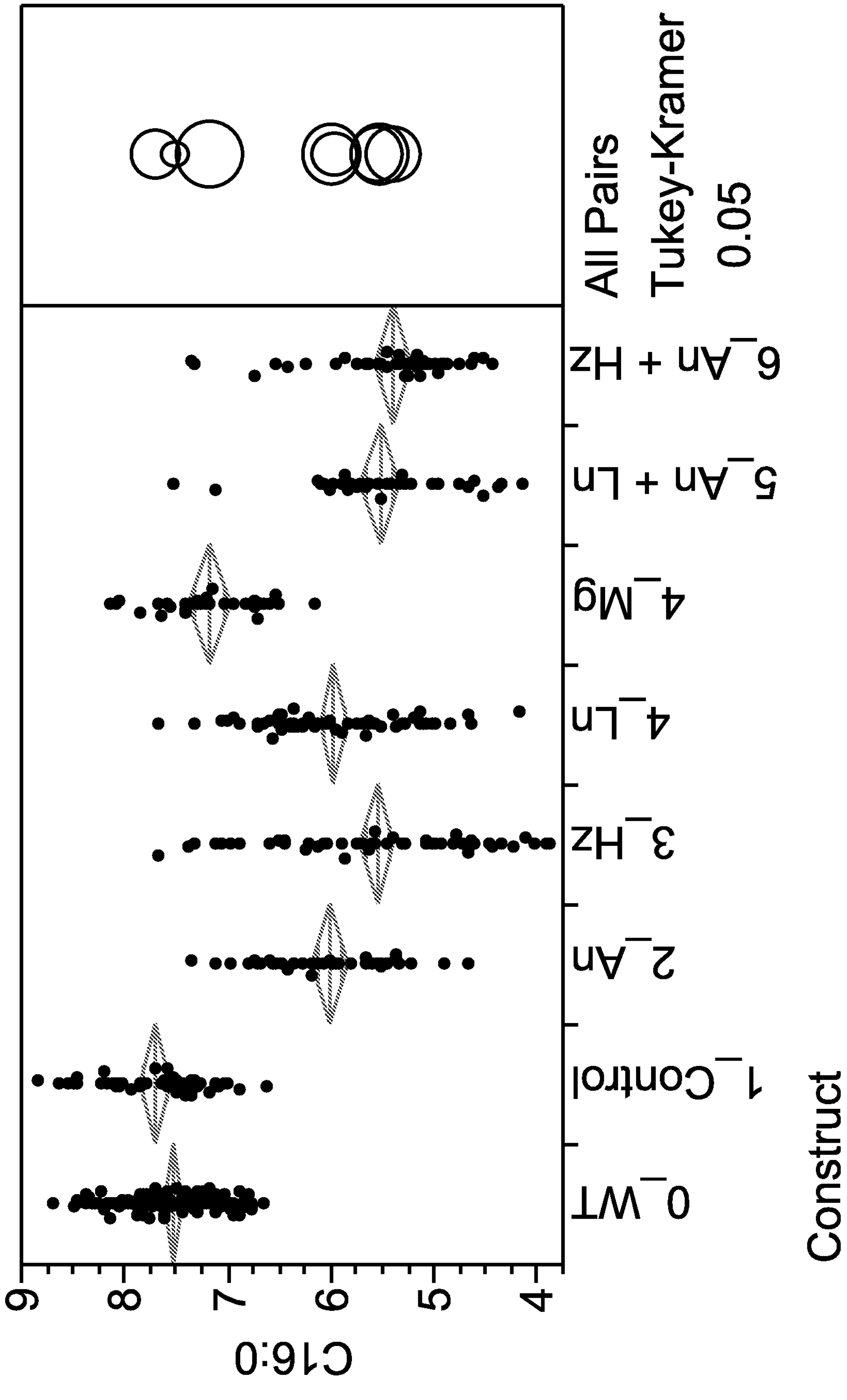
C16:0
9
8
7
6
5
4
0_WT
1_Control
2_An
3_Hz
4_Ln
4_Mg
5_An + Ln
6_An + Hz
Construct
All Pairs
Tukey-Kramer
0.05

Fig. 20

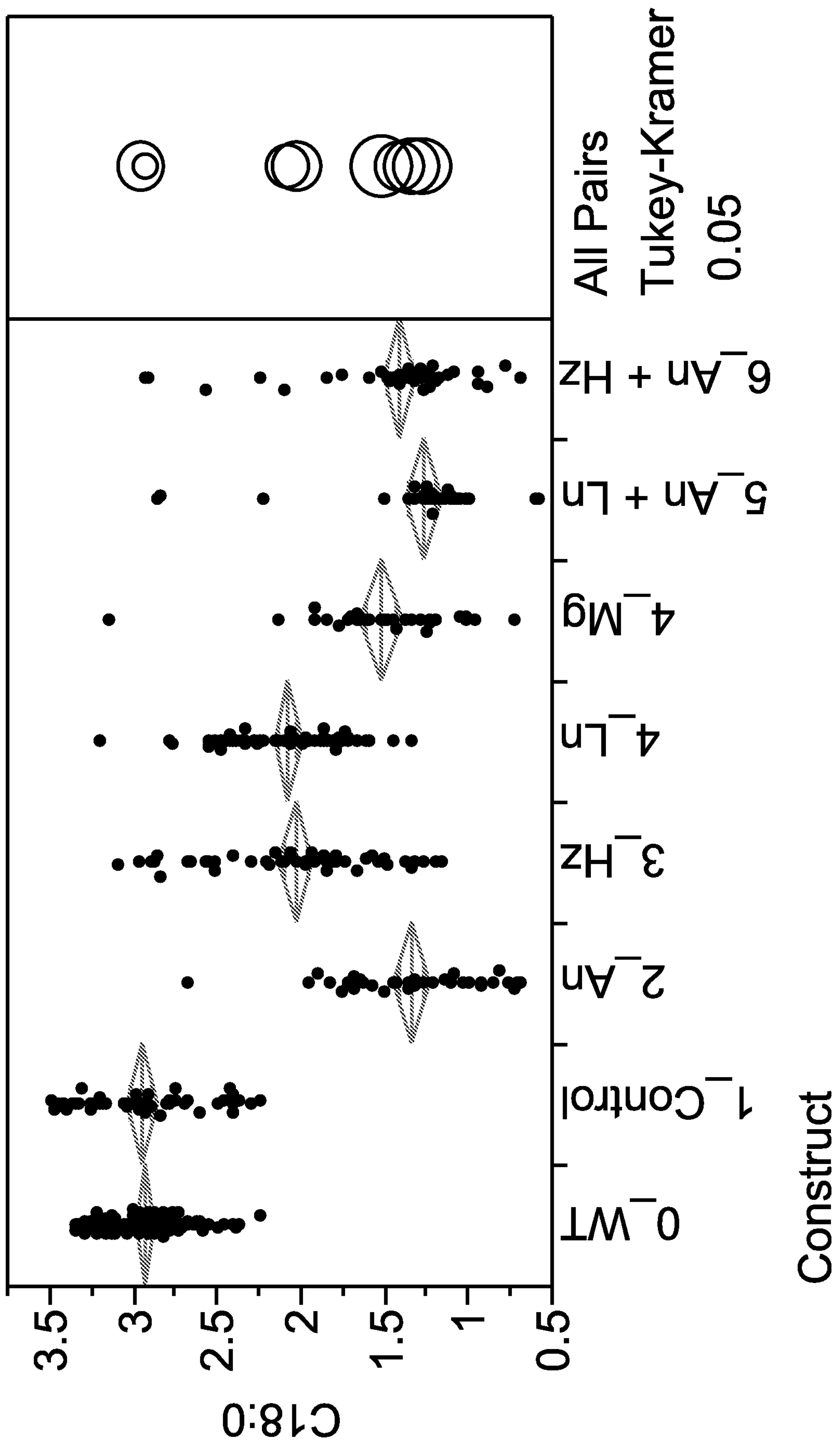
C18:0
3.5
3
2.5
2
1.5
1
0.5
0_WT
1_Control
2_An
3_Hz
4_Ln
4_Mg
5_An + Ln
6_An + Hz
Construct
All Pairs
Tukey-Kramer
0.05

Fig. 21

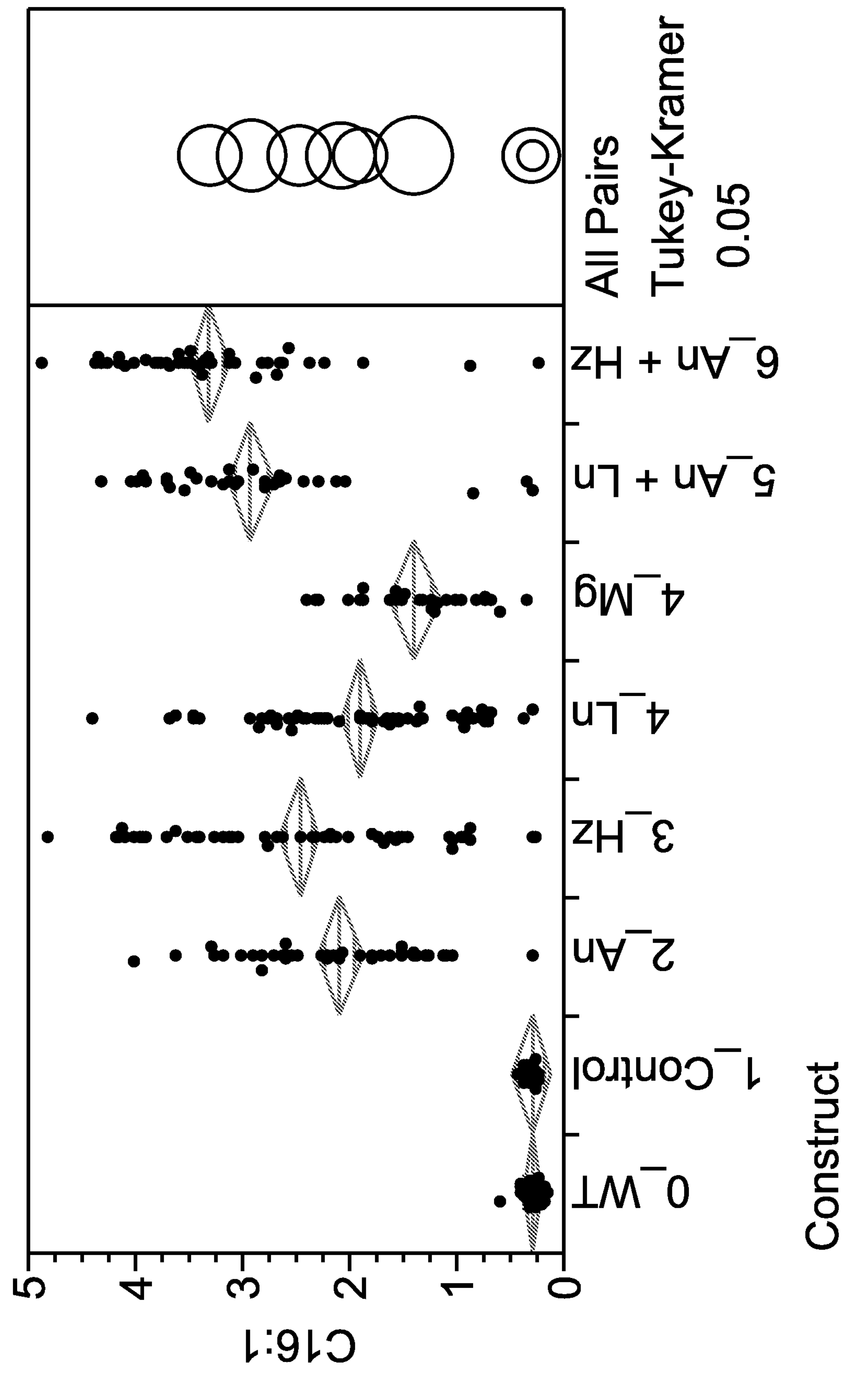
C16:1
0
1
2
3
4
5
0_WT
1_Control
2_An
3_Hz
4_Ln
4_Mg
5_An + Ln
6_An + Hz
Construct
All Pairs
Tukey-Kramer
0.05

Fig. 22

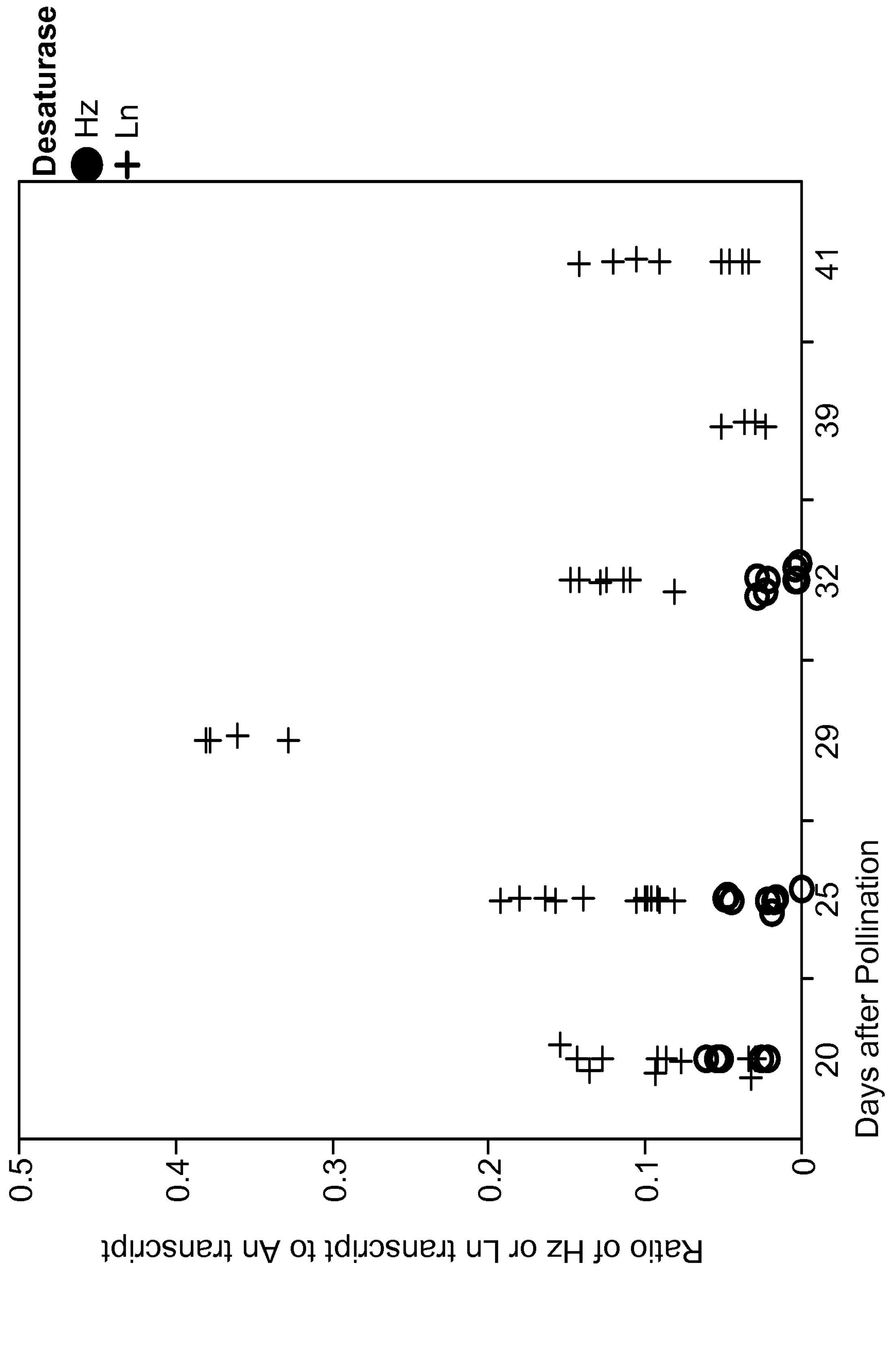
Desaturase
Hz
Ln
Ratio of Hz or Ln transcript to An transcript
0
0.1
0.2
0.3
0.4
0.5
Days after Pollination
20
25
29
32
39
41

**Fig. 23**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5723595 A
- US 6706950 B
- US 5777201 A
- US 20080260933 A1
- EP 0255378 A
- WO 9713402 A
- US 5380831 A
- US 5384253 A
- US 5508184 A
- US 5824877 A
- US 5591616 A
- US 5981840 A
- US 6384301 B
- US 5550318 A
- US 5538880 A
- US 6160208 A
- US 6399861 B
- US 6403865 B
- US 5004863 A
- US 5159135 A
- US 5518908 A
- US 5569834 A
- US 5416011 A
- US 5463174 A
- WO 1999050430 A
- WO 2000011012 A
- WO 2001025459A1 A
- US 5504200 A
- US 7253337 B
- US 6784342 B
- US 61358314 B


### Non-patent literature cited in the description


- **STUKEY et al.** *J. Biol. Chem.,* 1989, vol. 264, 16537-44
- **STUKEY et al.** *J. Biol. Chem.,* 1990, vol. 265, 20144-9
- **POLASHCOK et al.** *FASEB J.,* 1991, vol. 5, A1157
- **POLASHOK et al.** *Plant Physiol.,* 1992, vol. 100, 894-901
- **WANG et al.** *J. Agric. Food Chem.,* 1996, vol. 44, 3399-402
- **WANG et al.** *Phytochemistry,* 2001, vol. 58, 227-32
- **SMITH ; WATERMAN.** *Adv. Appl. math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **HIGGINS ; SHARP.** *Gene,* 1988, vol. 73, 237-44
- **HIGGINS ; SHARP.** *CABIOS,* 1989, vol. 5, 151-3
- **CORPET et al.** *Nucleic Acids Research,* 1988, vol. 16, 10881-10890
- **HUANG et al.** *Computer Applications in the Biosciences,* 1992, vol. 8, 155-65
- **PEARSON et al.** *Methods in Molecular Biology,* 1994, vol. 24, 307-31
- **TATIANA et al.** *FEMS Microbiol. Lett.,* 1990, vol. 174, 247-50
- **ALTSCHUL et al.** *J. Mol. Biol.,* 1990, vol. 215, 403-10
- **POSZKOWSKI et al.** *EMBO J.,* 1989, vol. 3, 2719
- **ODELL et al.** *Nature,* 1985, vol. 313, 810
- **CHAU et al.** *Science,* 1989, vol. 244, 174-81
- **MANDEL et al.** *Plant Mol. Biol.,* 1995, vol. 29, 995-1004
- **GRAHAM ; VAN DER EB.** *Virology,* 1973, vol. 54 (2), 536-9
- **ZATLOUKAL et al.** *Ann. N.Y. Acad. Sci.,* 1992, vol. 660, 136-53
- **CAPECHI.** *Cell,* 1980, vol. 22 (2), 479-88
- **WONG ; NEUMANN.** *Biochim. Biophys. Res. Commun.,* 1982, vol. 107 (2), 584-7
- **FROMM et al.** *Proc. Natl. Acad. Sci. USA,* 1985, vol. 82 (17), 5824-8
- **JOHNSTON ; TANG.** *Methods Cell Biol.,* 1994, vol. 43 (A), 353-65
- **FYNAN et al.** *Proc. Natl. Acad. Sci. USA,* 1993, vol. 90 (24), 11478-82
- **CLAPP.** *Clin. Perinatol.,* 1993, vol. 20 (1), 155-68
- **LU et al.** *J. Exp. Med.,* 1993, vol. 178 (6), 2089-96
- **EGLITIS ; ANDERSON.** *Biotechniques,* 1988, vol. 6 (7), 608-14

- **CURIEL et al.** *Hum. Gen. Ther.,* 1992, vol. 3 (2), 147-54
- **WAGNER et al.** *Proc. Natl. Acad. Sci. USA,* 1992, vol. 89 (13), 6099-103
- **ZHOU et al.** *Methods in Enzymology,* 1983, vol. 101, 433
- **HESS.** *Intern. Rev. Cytol.,* 1987, vol. 107, 367
- **LUO et al.** *Plant Mol. Biol. Reporter,* 1988, vol. 6, 165
- **PENA et al.** *Nature,* 1987, vol. 325, 274
- **NEUHAUS et al.** *Theor. Appl. Genet.,* 1987, vol. 75, 30
- **FRALEY et al.** *Proc. Natl. Acad. Sci. USA,* 1983, vol. 80, 4803
- **REAM.** *Ann. Rev. Phytopathol.,* 1989, vol. 27, 583-618
- **HOWARD ; CITOVSKY.** *Bioassays,* 1990, vol. 12, 103-8
- **KADO.** *Crit. Rev. Plant Sci.,* 1991, vol. 10, 1-32
- **ZAMBRYSKI.** *Annual Rev. Plant Physiol. Plant Mol. Biol.,* 1992, vol. 43, 465-90
- **GELVIN.** Transgenic Plants. Academic Press, 1993, 49-87
- **BINNS ; HOWITZ.** Bacterical Pathogenesis of Plants and Animals. Berlin: Springer Verlag, 1994, 119-38
- **HOOYKAAS ; BEIJERSBERGEN.** *Ann. Rev. Phytopathol.,* 1994, vol. 32, 157-79
- **LESSL ; LANKA ; 1994.** *Cell,* vol. 77, 321-4
- **ZUPAN ; ZAMBRYSKI.** *Annual Rev. Phytopathol.,* 1995, vol. 27, 583-618
- **DELLA-CIOPPA et al.** *Bio/Technology,* 1987, vol. 5, 579-84
- **JOERSBRO et al.** *Mol. Breed.,* 1998, vol. 4, 111-7
- **WEISSBACH ; WEISSBACH.** Methods for Plant Molecular Biology. cademic Press, Inc, 1988
- **HORSCH et al.** *Science,* 1985, vol. 227, 1229-31
- **FRALEY et al.** *Proc. Natl. Acad. Sci. USA,* 1993, vol. 80, 4803
- **MCCABE et al.** *Biotechnology,* 1988, vol. 6, 923
- **CHRISTOU et al.** *Plant Physiol.,* 1988, vol. 87, 671-4
- **CHENG et al.** *Plant Cell Rep.,* 1996, vol. 15, 653-7
- **MCKENTLY et al.** *Plant Cell Rep.,* 1995, vol. 14, 699-703
- **GRANT et al.** *Plant Cell Rep.,* 1995, vol. 15, 254-8
- **ROSENFIELD et al.** *Insect Biochem. Mol. Biol.,* 2001, vol. 31 (10), 949-64
- **TAMURA et al.** *Mol. Biol. and Evolution,* 2007, vol. 24, 1596-9
- **HUANG et al.** *J. Bacteriol.,* 1990, vol. 172, 1814-22
- **VERDAGUER et al.** *Plant Mol. Biol.,* 1996, vol. 31, 1129-39
- **WOHLLEBEN et al.** *Gene,* 1988, vol. 70, 25-37
- **TORO et al.** *Proc. Natl. Acad. Sci. U.S.A.,* 1988, vol. 85 (22), 8558-62
- **GARDNER et al.** *Science,* 1986, vol. 231, 725-7
- How to Transform Arabidopsis. **WEIGEL ; GLAZEBROOK.** Arabidopsis. Cold Spring Harbor Laboratory Press, 2002
- **HEPBURN et al.** *J. Gen. Microbiol.,* 1985, vol. 131, 2961-9
- **CLOUGH ; BENT.** *Plant J.,* 1998, vol. 16, 735-743
- **RAVID ; HOCHSTRASSER.** *Nat. Rev. Mol. Cell. Biol.,* 2008, vol. 9, 679-90
- **MCCARTNEY et al.** *Plant J.,* 2004, vol. 37, 156-73
- **MZIAUT et al.** *Proc. Natl. Acad. Sci. U.S.A.,* 2000, vol. 97, 8883-8
- **O'QUIN et al.** *Appl Microbiol Biotechnol,* 2009, vol. 83, 117-25
- **LIU et al.** *Nat. Biotechnol.,* 2003, vol. 21, 1222-8
- **GALLIE et al.** *Nucleic Acids Res.,* 1987, vol. 15, 8693-711
- **KOZIEL et al.** *Plant Mol. Biol.,* 1996, vol. 32, 393-405
- **VEMULA et al.** *J. Biol. Chem.,* 2003, vol. 278 (46), 45269-79

SEQUENCE LISTING

<110> Dow Agrosciences LLC
Merlo, Ann O
Gachotte, Daniel J
Thompson, Mark A
Walsh, Terence A

<120> Lowering Saturated Fatty Acid Content of Plant Seeds

<130> 2971-p10092.1US

<150> US 61/358,314
<151> 2010-06-24

<160> 78

<170> PatentIn version 3.4

<210> 1
<211> 44
<212> DNA
<213> Artificial

<220>
<223> Forward primer Mgdelta9F

<400> 1
gaagaattca tggcttcgtc atcttcctcc gtgccggagt tggc 44

<210> 2
<211> 41
<212> DNA
<213> Artificial

<220>
<223> Reverse primer Mg9deltaR

<400> 2
ctcgagctag ttagtcacgc ggcctgagca cccggaacag g 41

<210> 3
<211> 1523
<212> DNA
<213> Artificial

<220>
<223> PCR amplified fragment of MgD9Ds

<400> 3

gaattcatgg cttcgtcatc ttcctccgtg ccggagttgg ctgccgcctt ccctgatggc 60
actaccgact tcaagcccat gaggaacacc aagggctacg acgtcagcaa gccgcacatt 120
tccgagacac ctatgacact caagaactgg cataagcacg tcaactggct caacaccacc 180
ttcatcttgt ttgtgcccct ggctggtctc atatccactt actgggtccc tctgcagtgg 240
aagacggctg tatgggctgt cgtctactac ttcaacaccg gcctgggaat tactgccggt 300
aagtggctct tgaacaaacg agctaggccg ccgccctgta tccaatcatc tgtatccatc 360
cctagatgct aactagaaaa cttgcgggtt accaccgact ttgggctcac agctcgtaca 420
aggcctcgct tccgctcaaa atctaccttg ccgccgttgg cgctggtgcc gtcgagggct 480

```
ccatcagatg gtggtccaac ggtcaccgcg cacaccaccg atacaccgat accgagaagg    540
acccctactc agtccgcaag ggtctcctgt actcacacat gggatggatg cttctgaagc    600
agaaccccaa gaagcagggc cgcaccgaca tcaccgacct gaacgaggac cccgttgtcg    660
tttggcagca ccgcaacttc ctcaagtgtg ttatcttcat ggccctcgtc ttccccacac    720
ttgtggctgg ccttggctgg ggtgactact ggggaggttt catctacgga ggtattctgc    780
gtgtcttctt cgtccagcag gccaccttct gcgtcaactc gcttgcccac tggctcggtg    840
accagccttt cgacgatcgc aactcgccgc gtgatcacgt catcacagcc ctggtcaccc    900
ttggagaggg ataccacaac ttccaccacg agttcccttc ggactaccgc aacgctattg    960
agtggtacca gtatgacccc accaagtggt caatctggat ctggaagcag cttggtcttg   1020
cccacaacct gaagcagttc cgccaaaacg agattgagaa gggacgcgtc cagcagctgc   1080
agaagaagct cgaccagaag cgcgccaagc ttgattgggg tattcccttg gagcagcttc   1140
ccgttgttag ctgggatgac tttgttgagc agtccaagaa cggaaaggct tggattgcag   1200
ttgccggtgt catccacgat gttggtgact tcatcaagga ccaccctggt ggcagagctc   1260
tcatcaactc ggccattggc aaggacgcaa ccgcaatctt caacggcggt gtttacaacc   1320
actccaacgc cgctcacaac ctgctctcga ctatgcgtgt gggtgttttg cgtggcggct   1380
gcgaggttga gatctggaag cgcgcccagt ccgaaaacaa ggacgtctca accgtcgttg   1440
attcttcggg taaccgcatc gtccgcgcgg gtgggcaagc gaccaaggtc gtccagcctg   1500
ttccgggtgc tcaggccgcg tga                                           1523
```

<210> 4
<211> 1428
<212> DNA
<213> Artificial

<220>
<223> Intronless MgD9Ds clone

<400> 4

```
atggcttcgt catcttcctc cgtgccggag ttggctgccg ccttccctga tggcactacc      60
gacttcaagc ccatgaggaa caccaagggc tacgacgtca gcaagccgca catttccgag     120
acacctatga cactcaagaa ctggcataag cacgtcaact ggctcaacac caccttcatc     180
ttgtttgtgc ccctggctgg tctcatatcc acttactggg tccctctgca gtggaagacg     240
gctgtatggg ctgtcgtcta ctacttcaac accggcctgg gaattactgc cggttaccac     300
cgactttggg ctcacagctc gtacaaggcc tcgcttccgc tcaaaatcta ccttgccgcc     360
gttggcgctg gtgccgtcga gggctccatc agatggtggt ccaacggtca ccgcgcacac     420
caccgataca ccgataccga gaaggacccc tactcagtcc gcaagggtct cctgtactca     480
cacatgggat ggatgcttct gaagcagaac cccaagaagc agggccgcac cgacatcacc     540
gacctgaacg aggaccccgt tgtcgtttgg cagcaccgca acttcctcaa gtgtgttatc     600
ttcatggccc tcgtcttccc cacacttgtg gctggccttg gctggggtga ctactgggga     660

ggtttcatct acggaggtat tctgcgtgtc ttcttcgtcc agcaggccac cttctgcgtc     720
aactcgcttg cccactggct cggtgaccag cctttcgacg atcgcaactc gccgcgtgat     780
cacgtcatca cagccctggt caccettgga gagggatacc acaacttcca ccacgagttc     840
ccttcggact accgcaacgc tattgagtgg taccagtatg accccaccaa gtggtcaatc     900
tggatctgga agcagcttgg tcttgcccac aacctgaagc agttccgcca aaacgagatt     960
gagaagggac gcgtccagca gctgcagaag aagctcgacc agaagcgcgc caagcttgat    1020
tggggtattc ccttggagca gcttcccgtt gttagctggg atgactttgt tgagcagtcc    1080
aagaacggaa aggcttggat tgcagttgcc ggtgtcatcc acgatgttgg tgacttcatc    1140
aaggaccacc ctggtggcag agctctcatc aactcggcca ttggcaagga cgcaaccgca    1200
atcttcaacg gcggtgttta caaccactcc aacgccgctc acaacctgct ctcgactatg    1260
cgtgtgggtg ttttgcgtgg cggctgcgag gttgagatct ggaagcgcgc ccagtccgaa    1320
aacaaggacg tctcaaccgt cgttgattct tcgggtaacc gcatcgtccg cgcgggtggg    1380
caagcgacca aggtcgtcca gcctgttccg ggtgctcagg ccgcgtga                 1428
```

<210> 5
<211> 1997
<212> DNA
<213> Leptosphaeria nodorum

<400> 5

cccgattcat taatgcagct ggcacgacag gtttcccgac tggaaagcgg gcagtgagcg 60
caacgcaatt aatgtgagtt agctcactca ttaggcaccc caggctttac actttatgct 120
tccggctcgt atgttgtgtg gaattgtgag cggataacaa tttcacacag gaaacagcta 180
tgaccatgat tacgccaagc tcgaaattaa ccctcactaa agggaacaaa agctggagct 240
ccaccgcggt ggcggccgct ctagaactag tggatccccc gggctgcagg aattcggcac 300
gagtatgcct tcccaccagg ctgttgctgg catgcaggcc atcgaccccg agtttgtcaa 360
gcagccgtct cctatggcga gcacctcgga gcccaaccgc aactccaagt acgatcctaa 420
gaagccgcac attacagaca tgcccatcac gcggtcaaac tggtaccagc atgtcaactg 480
gctcaacgtc atcttcatca tcggcgtgcc tctcgctggc tgcgtcgccg ccttctggac 540
ccctctgcag tggaagaccg ctgcgtgggc tgtcatctac tatttctgga ctggcctcgg 600
tatcaccgcc ggataccatc gtctctgggc acacaagtca tacaacgccg gtcttcctct 660
gaggatctgg ctcgccgccg tcggcgctgg tgctgttgag ggttccatcc gctggtggag 720
ccgtgaccac cgcgcccacc accgctacac cgacaccaac aaggacccct acagtgtccg 780
caagggcctt ctctacagcc atctcggatg gatggtcatg aagcagaacc ccaagcgtat 840
cggccgcacc gacatcaccg acttgaacga ggaccccgtt gtcgtctggc agcacaagaa 900
ctacatcaag gccgtcgtca ccatgggctt gatctttccc tctgccgtcg ccggtctcat 960
gtggggcgat tggatgggtg gcttcatcta cgctggtatc ctccgtatct tcttcgtcca 1020
gcaggccacc ttctgcgtca actcgcttgc tcactggctc ggtgaccagc ccttcgacga 1080

ccgcaactct cctcgtgacc acgtcattac cgctcttgtc actctcggag agggctacca 1140
caacttccac cacgagttcc cctccgacta ccgcaacgcc atcgagtggc accagtacga 1200
ccctaccaag tggtccatct ggctgtggag caagctcggc ctcgcctcca acctcaagca 1260
gttccgctcc aacgaaatcg agaagggtcg tgtccagcag ctccagaaga agattgacca 1320
gaagcgcgcc aagctcgact ggggtgtccc tctcgaccag ctgcctgtca tagaatggga 1380
cgactatgtc gagcaggcca agaacggccg tggtctcatc gctgtcgctg gtgtcgttca 1440
tgacgttacc gacttcatca acgagcaccc cggtggcaag acgcttatca agagcggcgt 1500
tggcaaggat gccaccgcca tgttcaacgg cggtgtctac ttccactcca acggagccca 1560
caacctcctt tctaccatga gggttggtgt catccgcggt ggctgtgaag ttgagatctg 1620
gaagcgcgct cagcgtgaga acaaggatgt cggtctggtc ctggacgacg caggcaaccc 1680
aatcatcagg gctggtaacc agattaccaa ggttgcgcaa cccattcaga gtgctagtgc 1740
agcatagatt ggatcttcat cttcacgagc gatgtatggc gtttggttgt ctctcttcct 1800
tggcggacag agtaatattc aatttcttag cgatcgttag aaagcatcat ggttacgatg 1860
ctcagtcatg ttagatggcg tatgtttgta gccttcctcg agtgattggs tatgaaaagt 1920
agcctcacgg cctagaccaa gaatgaaaac attcacgatt tcagaaaaaa aaaaaaaaaa 1980
aaactcgagg gggggcc 1997

<210> 6
<211> 39
<212> DNA
<213> Artificial

<220>
<223> Forward primer Lnd9FAD2F

<400> 6
ggatccatgg cggccttgga cagcattcca gaggataag 39

<210> 7
<211> 38
<212> DNA
<213> Artificial

<220>
<223> Reverse primer Lnd9FAD2R

<400> 7
ccatggtcag ttagctacgc agccacagcc ccctcaac 38

<210> 8
<211> 1370
<212> DNA
<213> Leptosphaeria nodorum

<400> 8

ggatccatgg cggccttgga cagcattcca gaggataagg ctacctcgtc gaaatcgact 60

catattcaat atcaagaagt aacttttcgg aactggtata agaagataaa ttggctcaac 120

acgacgctgg tggtgctcat acccgctctt ggactctacc taacacgcac cacgccactt 180
acacgaccta cgctcatctg gtccgtcctg tactacttct gcacagcttt cggcatcaca 240
ggcggatatc atcgactatg gagtcatcgc agctactccg ctcgtctacc gctacgctta 300
ttcctagcct tcacaggcgc cggagccatc caaggtagtg ctcgatggtg gagcgcaaat 360
caccgcgccc accaccgatg gaccgacaca atgaaggacc cctactccgt tatgcgcggc 420
ctattattct cgcacatcgg atggatggta ttgaacagcg accccaaagt caaaggccga 480
acagacgtca gtgatctcga cagcgacccc gtcgtagtct ggcagcacaa gcactacggc 540
aagtgcctgc tgttcgccgc gtggatattc cccatgatcg tagccggcct cggatgggga 600
gattggtggg gaggccttgt ctacgccggc atcattcgag cgtgtttcgt ccagcaggcg 660
acattttgcg tgaactctct cgcgcattgg atcggcgagc agccgttcga cgacagacgc 720
acgcctcgag accacgtttt gacagcgttg gtaacgatgg gagaaggata tcataacttc 780
caccacgaat tcccaagcga ttatcgcaac gcgatcatct ggtaccaata cgaccctacc 840
aaatggctca tttacctctt ctccctcggc cccttccccc tcgcatactc gctcaaaacc 900
ttccggtcca atgagattga aaaagggcgg ttgcaacaac aacaaaaagc cctggacaag 960
aagcgctcag gacttgattg gggcctaccc ctcttccaac tccctgtcat atcgtgggac 1020
gacttccaag cgcgttgcaa agagtccggc gagatgctgg ttgctgtcgc aggtgtgatt 1080
cacgacgtca gccagtttat tgaagatcac cctggaggca ggagtttgat tcggagtgcg 1140
gtgggcaaag atgggacagg gatgtttaat ggaggcgtat atgagcacag taatgcggcg 1200
cataatctgt tgtcgacaat gagggtggga gtgcttagag gtgggcagga ggtggaggtg 1260
tggaagaagc agagagtgga tgttttaggg aagagcgaca ttttgagaca ggttacgcgg 1320
gtggagaggt tggttgaggg ggctgtggct gcgtagctaa ctgaccatgg 1370

<210> 9
<211> 1428
<212> DNA
<213> Magnaporthe grisea

<400> 9

atggcttcgt catcttcctc cgtgccggag ttggctgccg ccttccctga tggcactacc 60
gacttcaagc ccatgaggaa caccaagggc tacgacgtca gcaagccgca catttccgag 120
acacctatga cactcaagaa ctggcataag cacgtcaact ggctcaacac caccttcatc 180
ttgtttgtgc ccctggctgg tctcatatcc acttactggg tccctctgca gtggaagacg 240
gctgtatggg ctgtcgtcta ctacttcaac accggcctgg gaattactgc cggttaccac 300
cgactttggg ctcacagctc gtacaaggcc tcgcttccgc tcaaaatcta ccttgccgcc 360
gttggcgctg gtgccgtcga gggctccatc agatggtggt ccaacggtca ccgcgcacac 420
caccgataca ccgataccga gaaggacccc tactcagtcc gcaagggtct cctgtactca 480
cacatgggat ggatgcttct gaagcagaac cccaagaagc agggccgcac cgacatcacc 540
gacctgaacg aggaccccgt tgtcgtttgg cagcaccgca acttcctcaa gtgtgttatc 600

ttcatggccc tcgtcttccc cacacttgtg gctggccttg gctggggtga ctactgggga 660
ggtttcatct acggaggtat tctgcgtgtc ttcttcgtcc agcaggccac cttctgcgtc 720
aactcgcttg cccactggct cggtgaccag cctttcgacg atcgcaactc gccgcgtgat 780
cacgtcatca cagccctggt caccccttgga gagggatacc acaacttcca ccacgagttc 840
ccttcggact accgcaacgc tattgagtgg taccagtatg accccaccaa gtggtcaatc 900
tggatctgga agcagcttgg tcttgcccac aacctgaagc agttccgcca aaacgagatt 960
gagaagggac gcgtccagca gctgcagaag aagctcgacc agaagcgcgc caagcttgat 1020
tggggtattc ccttggagca gcttcccgtt gttagctggg atgactttgt tgagcagtcc 1080
aagaacggaa aggcttggat tgcagttgcc ggtgtcatcc acgatgttgg tgacttcatc 1140
aaggaccacc ctggtggcag agctctcatc aactcggcca ttggcaagga cgcaaccgca 1200
atcttcaacg gcggtgttta caaccactcc aacgccgctc acaacctgct ctcgactatg 1260
cgtgtgggtg ttttgcgtgg cggctgcgag gttgagatct ggaagcgcgc ccagtccgaa 1320
aacaaggacg tctcaaccgt cgttgattct tcgggtaacc gcatcgtccg cgcgggtggg 1380
caagcgacca aggtcgtcca gcctgttccg ggtgctcagg ccgcgtga 1428

<210> 10
<211> 1062
<212> DNA
<213> Helicoverpa zea

<400> 10

```
atggctccaa atatatcgga ggatgtgaac ggggtgctct tcgagagtga tgcagcgacg     60
ccggacctgg cgctgtccac gccgcctgtg cagaaggctg acaacaggcc caagcaactg    120
gtgtggagga acatactact gttcgcgtat cttcacttag cggctcttta cggaggttat    180
ctgttcctct tctcagctaa atggcagaca gacatatttg cctacatcct gtatgtgatc    240
tccgggcttg gtatcacggc tggagcacat cgcctgtggg cccacaagtc ctacaaagct    300
aaatggcctc tccgagttat cctggtcatc tttaacacag tggcattcca ggatgccgct    360
atggactggg cgcgcgacca ccgcatgcat cacaagtact cggaaaccga tgctgatcct    420
cataatgcga cccgaggatt cttcttctct cacattggct ggctgcttgt caggaaacat    480
cccgacctta aggagaaggg caagggactc gacatgagcg acttacttgc tgaccccatt    540
ctcaggttcc agaaaaaata ctacctgatc ctgatgccct tggcttgctt cgtgatgcct    600
accgtgattc ctgtgtactt ctggggtgaa acctggacca acgcattctt tgtggcggcc    660
atgttccgct acgcgttcat cctaaatgtg acgtggctcg tcaactctgc cgctcacaag    720
tggggagaca agccctacga caaaagcatt aagccttccg aaaacttgtc ggtcgccatg    780
ttcgctctcg gagaaggatt ccacaactac caccacactt tcccttggga ctacaaaact    840
gctgagctgg gcaacaacaa actcaacttc actaccacct ttattaactt cttcgctaaa    900
attggctggg cttacgacct gaagacagtg tctgatgata tcgtcaagaa cagggtgaag    960
cgcactggtg acggctccca ccacctgtgg ggctggggag acgaaaatca atccaaagaa   1020
gaaattgatg ccgctatcag aatcaatcct aaggacgatt aa                      1062
```

<210> 11
<211> 1350
<212> DNA
<213> Leptosphaeria nodorum

<400> 11

```
atggcggcct tggacagcat tccagaggat aaggctacct cgtcgaaatc gactcatatt      60
caatatcaag aagtaacttt tcggaactgg tataagaaga taaattggct caacacgacg     120
ctggtggtgc tcatacccgc tcttggactc tacctaacac gcaccacgcc acttacacga     180
cctacgctca tctggtccgt cctgtactac ttctgcacag ctttcggcat cacaggcgga     240
tatcatcgac tatggagtca tcgcagctac tccgctcgtc taccgctacg cttattccta     300
gccttcacag gcgccggagc catccaaggt agtgctcgat ggtggagcgc aaatcaccgc     360
gcccaccacc gatggaccga cacaatgaag gacccctact ccgttatgcg cggcctatta     420
ttctcgcaca tcggatggat ggtattgaac agcgacccca aagtcaaagg ccgaacagac     480
gtcagtgatc tcgacagcga ccccgtcgta gtctggcagc acaagcacta cggcaagtgc     540
ctgctgttcg ccgcgtggat attccccatg atcgtagccg gcctcggatg gggagattgg     600
tggggaggcc ttgtctacgc cggcatcatt cgagcgtgtt tcgtccagca ggcgacattt     660
tgcgtgaact ctctcgcgca ttggatcggc gagcagccgt tcgacgacag acgcacgcct     720
cgagaccacg ttttgacagc gttggtaacg atgggagaag gatatcataa cttccaccac     780
gaattcccaa gcgattatcg caacgcgatc atctggtacc aatacgaccc taccaaatgg     840
ctcatttacc tcttctccct cggccccttc cccctcgcat actcgctcaa aaccttccgg     900
tccaatgaga ttgaaaaagg gcggttgcaa caacaacaaa aagccctgga caagaagcgc     960
tcaggacttg attggggcct accccctcttc caactccctg tcatatcgtg ggacgacttc   1020
caagcgcgtt gcaaagagtc cggcgagatg ctggttgctg tcgcaggtgt gattcacgac    1080
gtcagccagt ttattgaaga tcaccctgga ggcaggagtt tgattcggag tgcggtgggc    1140
aaagatggga cagggatgtt taatggaggc gtatatgagc acagtaatgc ggcgcataat    1200
ctgttgtcga caatgagggt gggagtgctt agaggtgggc aggaggtgga ggtgtggaag    1260
aagcagagag tggatgtttt agggaagagc gacattttga gacaggttac gcgggtggag    1320
aggttggttg agggggctgt ggctgcgtag                                      1350
```

<210> 12
<211> 475
<212> PRT
<213> Magnaporthe grisea

<400> 12

```
Met Ala Ser Ser Ser Ser Ser Val Pro Glu Leu Ala Ala Ala Phe Pro
1               5                   10                  15
```

Asp Gly Thr Thr Asp Phe Lys Pro Met Arg Asn Thr Lys Gly Tyr Asp
20 25 30

Val Ser Lys Pro His Ile Ser Glu Thr Pro Met Thr Leu Lys Asn Trp
35 40 45

His Lys His Val Asn Trp Leu Asn Thr Thr Phe Ile Leu Phe Val Pro
50 55 60

Leu Ala Gly Leu Ile Ser Thr Tyr Trp Val Pro Leu Gln Trp Lys Thr
65 70 75 80

Ala Val Trp Ala Val Val Tyr Tyr Phe Asn Thr Gly Leu Gly Ile Thr
85 90 95

Ala Gly Tyr His Arg Leu Trp Ala His Ser Ser Tyr Lys Ala Ser Leu
100 105 110

Pro Leu Lys Ile Tyr Leu Ala Ala Val Gly Ala Gly Ala Val Glu Gly
115 120 125

Ser Ile Arg Trp Trp Ser Asn Gly His Arg Ala His His Arg Tyr Thr
130 135 140

Asp Thr Glu Lys Asp Pro Tyr Ser Val Arg Lys Gly Leu Leu Tyr Ser
145 150 155 160

His Met Gly Trp Met Leu Leu Lys Gln Asn Pro Lys Lys Gln Gly Arg
165 170 175

Thr Asp Ile Thr Asp Leu Asn Glu Asp Pro Val Val Val Trp Gln His
180 185 190

Arg Asn Phe Leu Lys Cys Val Ile Phe Met Ala Leu Val Phe Pro Thr
195 200 205

Leu Val Ala Gly Leu Gly Trp Gly Asp Tyr Trp Gly Gly Phe Ile Tyr
210 215 220

Gly Gly Ile Leu Arg Val Phe Phe Val Gln Gln Ala Thr Phe Cys Val
225 230 235 240

Asn Ser Leu Ala His Trp Leu Gly Asp Gln Pro Phe Asp Asp Arg Asn
245 250 255

Ser Pro Arg Asp His Val Ile Thr Ala Leu Val Thr Leu Gly Glu Gly
260 265 270

Tyr His Asn Phe His His Glu Phe Pro Ser Asp Tyr Arg Asn Ala Ile
275 280 285

```
Glu Trp Tyr Gln Tyr Asp Pro Thr Lys Trp Ser Ile Trp Ile Trp Lys
    290                 295                 300

Gln Leu Gly Leu Ala His Asn Leu Lys Gln Phe Arg Gln Asn Glu Ile
305                 310                 315                 320

Glu Lys Gly Arg Val Gln Gln Leu Gln Lys Lys Leu Asp Gln Lys Arg
                325                 330                 335

Ala Lys Leu Asp Trp Gly Ile Pro Leu Glu Gln Leu Pro Val Val Ser
            340                 345                 350

Trp Asp Asp Phe Val Glu Gln Ser Lys Asn Gly Lys Ala Trp Ile Ala
        355                 360                 365

Val Ala Gly Val Ile His Asp Val Gly Asp Phe Ile Lys Asp His Pro
    370                 375                 380

Gly Gly Arg Ala Leu Ile Asn Ser Ala Ile Gly Lys Asp Ala Thr Ala
385                 390                 395                 400

Ile Phe Asn Gly Gly Val Tyr Asn His Ser Asn Ala Ala His Asn Leu
                405                 410                 415

Leu Ser Thr Met Arg Val Gly Val Leu Arg Gly Gly Cys Glu Val Glu
            420                 425                 430

Ile Trp Lys Arg Ala Gln Ser Glu Asn Lys Asp Val Ser Thr Val Val
        435                 440                 445

Asp Ser Ser Gly Asn Arg Ile Val Arg Ala Gly Gly Gln Ala Thr Lys
    450                 455                 460

Val Val Gln Pro Val Pro Gly Ala Gln Ala Ala
465                 470                 475
```

<210> 13
<211> 353
<212> PRT
<213> Helicoverpa zea

<400> 13

```
Met Ala Pro Asn Ile Ser Glu Asp Val Asn Gly Val Leu Phe Glu Ser
1               5                   10                  15

Asp Ala Ala Thr Pro Asp Leu Ala Leu Ser Thr Pro Pro Val Gln Lys
            20                  25                  30

Ala Asp Asn Arg Pro Lys Gln Leu Val Trp Arg Asn Ile Leu Leu Phe
        35                  40                  45
```

```
Ala Tyr Leu His Leu Ala Ala Leu Tyr Gly Gly Tyr Leu Phe Leu Phe
    50                  55                  60

Ser Ala Lys Trp Gln Thr Asp Ile Phe Ala Tyr Ile Leu Tyr Val Ile
65                  70                  75                  80

Ser Gly Leu Gly Ile Thr Ala Gly Ala His Arg Leu Trp Ala His Lys
                85                  90                  95

Ser Tyr Lys Ala Lys Trp Pro Leu Arg Val Ile Leu Val Ile Phe Asn
            100                 105                 110

Thr Val Ala Phe Gln Asp Ala Ala Met Asp Trp Ala Arg Asp His Arg
        115                 120                 125

Met His His Lys Tyr Ser Glu Thr Asp Ala Asp Pro His Asn Ala Thr
    130                 135                 140

Arg Gly Phe Phe Phe Ser His Ile Gly Trp Leu Leu Val Arg Lys His
145                 150                 155                 160

Pro Asp Leu Lys Glu Lys Gly Lys Gly Leu Asp Met Ser Asp Leu Leu
                165                 170                 175

Ala Asp Pro Ile Leu Arg Phe Gln Lys Lys Tyr Tyr Leu Ile Leu Met
            180                 185                 190

Pro Leu Ala Cys Phe Val Met Pro Thr Val Ile Pro Val Tyr Phe Trp
        195                 200                 205

Gly Glu Thr Trp Thr Asn Ala Phe Phe Val Ala Ala Met Phe Arg Tyr
    210                 215                 220

Ala Phe Ile Leu Asn Val Thr Trp Leu Val Asn Ser Ala Ala His Lys
225                 230                 235                 240

Trp Gly Asp Lys Pro Tyr Asp Lys Ser Ile Lys Pro Ser Glu Asn Leu
                245                 250                 255

Ser Val Ala Met Phe Ala Leu Gly Glu Gly Phe His Asn Tyr His His
            260                 265                 270

Thr Phe Pro Trp Asp Tyr Lys Thr Ala Glu Leu Gly Asn Asn Lys Leu
        275                 280                 285

Asn Phe Thr Thr Thr Phe Ile Asn Phe Phe Ala Lys Ile Gly Trp Ala
    290                 295                 300

Tyr Asp Leu Lys Thr Val Ser Asp Asp Ile Val Lys Asn Arg Val Lys
305                 310                 315                 320
```

Arg Thr Gly Asp Gly Ser His His Leu Trp Gly Trp Gly Asp Glu Asn
325 330 335

Gln Ser Lys Glu Glu Ile Asp Ala Ala Ile Arg Ile Asn Pro Lys Asp
340 345 350

Asp

<210> 14
<211> 449
<212> PRT
<213> Leptosphaeria nodorum

<400> 14

```
Met Ala Ala Leu Asp Ser Ile Pro Glu Asp Lys Ala Thr Ser Ser Lys
1               5                   10                  15

Ser Thr His Ile Gln Tyr Gln Glu Val Thr Phe Arg Asn Trp Tyr Lys
            20                  25                  30

Lys Ile Asn Trp Leu Asn Thr Thr Leu Val Val Leu Ile Pro Ala Leu
        35                  40                  45

Gly Leu Tyr Leu Thr Arg Thr Thr Pro Leu Thr Arg Pro Thr Leu Ile
    50                  55                  60

Trp Ser Val Leu Tyr Tyr Phe Cys Thr Ala Phe Gly Ile Thr Gly Gly
65                  70                  75                  80

Tyr His Arg Leu Trp Ser His Arg Ser Tyr Ser Ala Arg Leu Pro Leu
                85                  90                  95

Arg Leu Phe Leu Ala Phe Thr Gly Ala Gly Ala Ile Gln Gly Ser Ala
            100                 105                 110

Arg Trp Trp Ser Ala Asn His Arg Ala His His Arg Trp Thr Asp Thr
        115                 120                 125

Met Lys Asp Pro Tyr Ser Val Met Arg Gly Leu Leu Phe Ser His Ile
    130                 135                 140

Gly Trp Met Val Leu Asn Ser Asp Pro Lys Val Lys Gly Arg Thr Asp
145                 150                 155                 160

Val Ser Asp Leu Asp Ser Asp Pro Val Val Val Trp Gln His Lys His
                165                 170                 175

Tyr Gly Lys Cys Leu Leu Phe Ala Ala Trp Ile Phe Pro Met Ile Val
            180                 185                 190

Ala Gly Leu Gly Trp Gly Asp Trp Trp Gly Gly Leu Val Tyr Ala Gly
```

195 200 205

Ile Ile Arg Ala Cys Phe Val Gln Gln Ala Thr Phe Cys Val Asn Ser
210 215 220

Leu Ala His Trp Ile Gly Glu Gln Pro Phe Asp Asp Arg Arg Thr Pro
225 230 235 240

Arg Asp His Val Leu Thr Ala Leu Val Thr Met Gly Glu Gly Tyr His
245 250 255

Asn Phe His His Glu Phe Pro Ser Asp Tyr Arg Asn Ala Ile Ile Trp
260 265 270

Tyr Gln Tyr Asp Pro Thr Lys Trp Leu Ile Tyr Leu Phe Ser Leu Gly
275 280 285

Pro Phe Pro Leu Ala Tyr Ser Leu Lys Thr Phe Arg Ser Asn Glu Ile
290 295 300

Glu Lys Gly Arg Leu Gln Gln Gln Gln Lys Ala Leu Asp Lys Lys Arg
305 310 315 320

Ser Gly Leu Asp Trp Gly Leu Pro Leu Phe Gln Leu Pro Val Ile Ser
325 330 335

Trp Asp Asp Phe Gln Ala Arg Cys Lys Glu Ser Gly Glu Met Leu Val
340 345 350

Ala Val Ala Gly Val Ile His Asp Val Ser Gln Phe Ile Glu Asp His
355 360 365

Pro Gly Gly Arg Ser Leu Ile Arg Ser Ala Val Gly Lys Asp Gly Thr
370 375 380

Gly Met Phe Asn Gly Gly Val Tyr Glu His Ser Asn Ala Ala His Asn
385 390 395 400

Leu Leu Ser Thr Met Arg Val Gly Val Leu Arg Gly Gly Gln Glu Val
405 410 415

Glu Val Trp Lys Lys Gln Arg Val Asp Val Leu Gly Lys Ser Asp Ile
420 425 430

Leu Arg Gln Val Thr Arg Val Glu Arg Leu Val Glu Gly Ala Val Ala
435 440 445

Ala

<210> 15
<211> 1428

<212> DNA
<213> Artificial

<220>
<223> canola-optimized desaturase sequence

<400> 15

```
atggccagca gttcttcaag tgtgccagaa cttgccgcag ctttccctga tgggacaacg      60
gacttcaaac ccatgaggaa caccaaaggc tatgatgtct ccaaacctca catctctgaa     120
acaccgatga ctttgaagaa ctggcacaaa catgtgaact ggctcaacac cacattcatt     180
ctctttgttc cactggctgg gttgatctca acctattggg ttcctcttca atggaaaact     240
gcagtgtggg cagttgtgta ctacttcaac actggacttg ggatcactgc tggctaccat     300
agattgtggg cacattcctc ttacaaggcc agcttgcctc tcaaaatcta ccttgccgca     360
gttggtgctg gagccgttga aggttccata agatggtgga gcaacggaca cagagcacat     420
cacagataca cagacacaga gaaagatcct tactcagtga ggaagggatt gctctacagc     480
cacatgggtt ggatgctctt gaagcagaat ccaaagaagc aagggaggac ggacattact     540
gatctgaatg aggacccagt tgtggtctgg caacatagga actttctcaa gtgtgtgatc     600
ttcatggctt tggtctttcc cacccttgtt gctggcctgg gatggggaga ctactgggga     660
ggtttcatct atggagggat cttgagagtg ttctttgttc agcaagccac cttctgtgtc     720
aactcacttg cacattggct tggtgatcaa ccgtttgatg acagaaactc tccacgtgac     780
catgtcataa ctgctcttgt cacgctgggt gaaggctatc acaactttca ccatgagttt     840
ccgtcagact atagaaatgc gattgagtgg tatcagtatg accccacgaa gtggagcatt     900
tggatttgga agcaacttgg acttgctcac aatctcaagc agttcagaca gaatgagata     960
gagaagggaa gggttcaaca gttgcagaag aaactggatc agaagagagc gaaacttgat    1020
tggggaatac cgttggaaca actccctgtt gtgtcttggg atgactttgt tgaacagtca    1080
aagaatggca aggcatggat tgctgttgct ggtgtcattc acgatgttgg tgacttcatc    1140
aaggatcatc ctggtggacg tgctctcatc aactctgcga ttggcaaaga tgccacagcg    1200
atcttcaatg gaggtgtcta caatcattca aatgccgcac acaaccttct ctccaccatg    1260
agggttggtg tcctccgtgg agggtgcgaa gtggagatat ggaaacgtgc tcaaagtgag    1320
aacaaagatg tctctactgt ggttgatagt tctggcaacc gtattgtgag agctggtgga    1380
caagctacca aagtggttca gccagtccct ggtgctcaag cagcttga                 1428
```

<210> 16
<211> 1062
<212> DNA
<213> Artificial

<220>
<223> Canola-optimized desaturase sequence

<400> 16

```
atggctccca acatttctga ggatgtcaat ggtgttcttt ttgagtcaga tgcggcaacc      60
cctgatttgg ctctttccac accacctgtg caaaaagctg acaacagacc caagcaactt     120
gtgtggagga acattttgct tttcgcttac ttgcacctcg cagctctcta cggaggctat     180
ttgtttctct tcagtgcaaa atggcagacc gacattttcg cttacattct ttatgtcatc     240
tctggactgg ggataactgc tggggcacat agactctggg ctcacaagtc atacaaagcc     300
aagtggccac tcagagttat actggtcatc ttcaacacgg ttgcctttca agacgctgct     360
atggattggg ctcgtgacca tagaatgcat cacaagtaca gcgagaccga cgcggaccca     420
cacaatgcaa cgagaggttt cttcttctct cacattggct ggcttcttgt taggaaacat     480
cctgatctga aagaaaaagg gaagggactc gacatgagtg atctccttgc tgatccaata     540
ctccgttttc agaagaagta ctatctgatc ctcatgcctc tggcctgttt tgtgatgcca     600
accgttatcc cggtttactt ttggggagaa acttggacaa atgctttctt cgtggcagcc     660
atgttccgtt atgctttcat cctgaatgtt acctggttgg tgaactctgc cgcacacaag     720
tggggagaca aaccctatga caagtccatc aagccttccg aaaacctttc agttgcgatg     780
tttgctttgg gagaaggatt tcacaattac catcacactt ttccgtggga ctacaagaca     840
gcagagcttg gaaacaacaa gttgaacttc acaacaacgt tcatcaattt ctttgcgaaa     900
atcggttggg cctatgattt gaagactgtg agtgatgaca ttgtcaagaa cagggtcaag     960
agaactggcg atggaagcca tcatctctgg ggctggggtg atgagaatca gagcaaagaa    1020
gagatagatg cagccattag gatcaaccct aaagacgatt ga                       1062
```

<210> 17
<211> 1350
<212> DNA
<213> Artificial

<220>
<223> Canola-optimized desaturase sequence

<400> 17

atggctgcac ttgatagcat ccctgaggac aaagcaacta gctccaagtc aacccacata 60
cagtaccaag aggtcacgtt taggaactgg tacaagaaaa tcaactggct caacacgacc 120
cttgttgtcc tcattcctgc tcttgggttg tacttgacga gaaccacacc tctcaccaga 180
cctaccctca tttggtctgt tctctactat ttctgtacag cgtttggcat cactggtggc 240
taccacagac tttggtccca taggtcttac agtgcgaggt tgccattgag actcttcctg 300
gctttcactg gagctggtgc gatccaaggt tctgcaagat ggtggtcagc caatcatagg 360
gcacatcacc gttggacgga caccatgaag gacccctact ctgtgatgag aggactgctg 420
ttctcccaca taggttggat ggttctcaac tctgatccaa aggtcaaagg cagaacagat 480
gtttctgatc ttgactctga tcccgtcgtt gtgtggcaac acaaacacta tggcaagtgt 540
ttgctctttg ccgcttggat ctttccgatg atagtggctg ggctgggttg gggagattgg 600
tggggtggac ttgtctatgc tggcatcata cgtgcctgct ttgttcagca agccactttc 660
tgtgtcaact cattggcaca ttggataggt gaacaaccgt ttgatgacag acgtactcca 720

agggatcatg ttctgactgc gttggtcaca atgggagaag gataccacaa cttccaccat 780
gagtttccga gtgactacag aaatgccatc atttggtatc agtatgaccc tacaaagtgg 840
ctcatctatc tcttcagctt gggtcccttc ccattggcct actctctcaa gaccttccgt 900
tccaatgaga ttgagaaagg aaggcttcag caacagcaaa aggctcttga caagaaaaga 960
agtggtcttg attggggact tcctctcttc cagcttccag tgatctcatg ggatgacttt 1020
caagctcgtt gcaaagaaag tggagagatg cttgttgctg ttgctggagt gatccatgat 1080
gtctcccagt tcattgaaga tcatcctggt gggaggagcc tcattagaag tgctgttggg 1140
aaagatggga ctggcatgtt caatggtgga gtgtatgaac attcaaacgc cgcacacaac 1200
ttgctgagca caatgagagt tggagtcttg agaggtggac aagaagtgga ggtttggaag 1260
aaacagaggg tggatgttct tgggaagtca gacattcttc gtcaagtgac aagggtggag 1320
cgtctggtgg aaggagctgt tgcagcgtga 1350

<210> 18
<211> 24
<212> DNA
<213> Artificial

<220>
<223> Primer MAS414

<400> 18
tgaagcattc cataagccgt cacg 24

<210> 19
<211> 24
<212> DNA
<213> Artificial

<220>

<223> Primer MAS415

<400> 19
gaaattatca cgcttccgca cacg 24

<210> 20
<211> 24
<212> DNA
<213> Artificial

<220>
<223> Primer MAS413

<400> 20
tgggctgaat tgaagacatg ctcc 24

<210> 21
<211> 25
<212> DNA
<213> Artificial

<220>
<223> Primer arw008

<400> 21
acacctctca ccagacctac cctca 25

<210> 22
<211> 22
<212> DNA
<213> Artificial

<220>
<223> Primer arw009

<400> 22
cacacaacga cgggatcaga gt 22

<210> 23
<211> 24
<212> DNA
<213> Artificial

<220>
<223> Primer arw010

<400> 23
caagtcatac aaagccaagt ggcc 24

<210> 24
<211> 22
<212> DNA
<213> Artificial

<220>
<223> Primer arw011

<400> 24

taacggaaca tggctgccac ga 22

<210> 25
<211> 33
<212> DNA
<213> Artificial

<220>
<223> Primer AntiLnD9DS2F

<400> 25
catatgttcg acgacagacg cacgcctcga gac 33

<210> 26
<211> 35
<212> DNA
<213> Artificial

<220>
<223> Primer AntiLnD9DS2Rh

<400> 26
ggatccgcag ccacagcccc ctcaaccaac ctctc 35

<210> 27
<211> 36
<212> DNA
<213> Artificial

<220>
<223> Primer AntiMgD9DSF

<400> 27
catatgttcg acgatcgcaa ctcgccgcgt gatcac 36

<210> 28
<211> 32
<212> DNA
<213> Artificial

<220>
<223> Primer AntiMgD9DSRh

<400> 28
ggatccgcgg cctgagcacc cggaacaggc tg 32

<210> 29
<211> 30
<212> DNA
<213> Artificial

<220>
<223> Primer AntiHzD9DSF

<400> 29
catatgtatg acaagtccat caagccttcc 30

<210> 30

<211> 35
<212> DNA
<213> Artificial

<220>
<223> Primer AntiHzD9DSRh

<400> 30
ggatcctcgt ctttagggtt gatcctaatg gctgc 35

<210> 31
<211> 24
<212> DNA
<213> Artificial

<220>
<223> Forward primer for target AnD9Ds

<400> 31
ggacttctct actctcacct tgga 24

<210> 32
<211> 20
<212> DNA
<213> Artificial

<220>
<223> Reverse primer for target AnD9Ds

<400> 32
tccgatcctc tttgggttct 20

<210> 33
<211> 18
<212> DNA
<213> Artificial

<220>
<223> Forward primer for target HzD9Ds

<400> 33
gacccacaca atgcaacg 18

<210> 34
<211> 21
<212> DNA
<213> Artificial

<220>
<223> Reverse primer for target HzD9Ds

<400> 34
cctaacaaga agccagccaa t 21

<210> 35
<211> 20
<212> DNA
<213> Artificial

<220>
<223> Forward primer for target LnD9Ds

<400> 35
gttctgactg cgttggtcac 20

<210> 36
<211> 20
<212> DNA
<213> Artificial

<220>
<223> Reverse primer for target LnD9Ds

<400> 36
cggaaactca tggtggaagt 20

<210> 37
<211> 21
<212> DNA
<213> Artificial

<220>
<223> Forward primer for target Actin

<400> 37
ctactggtat tgtgctcgac t 21

<210> 38
<211> 22
<212> DNA
<213> Artificial

<220>
<223> Reverse primer for target Actin

<400> 38
ctctctcggt gagaatcttc at 22

<210> 39
<211> 22
<212> DNA
<213> Artificial

<220>
<223> Actin probe

<400> 39
cacgctatcc tccgtctcga tc 22

<210> 40
<211> 13
<212> DNA
<213> Artificial

<220>
<223> Kozak sequence

<400> 40
ggatccaaca atg 13

<210> 41
<211> 13
<212> DNA
<213> Artificial

<220>
<223> Kozak sequence

<400> 41
acaaccaaaa atg 13

<210> 42
<211> 18
<212> DNA
<213> Artificial

<220>
<223> Kozak sequence

<400> 42
acaaccaacc taccatgg 18

<210> 43
<211> 14
<212> DNA
<213> Artificial

<220>
<223> Kozak sequence

<400> 43
acaaccaaaa aatg 14

<210> 44
<211> 1350
<212> DNA
<213> Artificial

<220>
<223> Canola-optimized desaturase

<400> 44

```
atggctgctc ttgattctat cccagaggat aaggctacct cttctaagtc tacccacatc      60
caataccaag aagttacctt caggaactgg tacaagaaga tcaactggct taacaccacc     120
cttgttgttc ttatcccagc tcttggactt taccttacca ggaccacccc acttaccagg     180
ccaaccctta tctggtctgt tctttactac ttctgcaccg ctttcggaat aaccggagga     240
taccacaggc tttggtctca caggtcttac tctgctaggc ttccacttag gcttttcctt     300
gctttcaccg gagctggagc tatccaagga tctgctagat ggtggtctgc taaccacagg     360
gctcaccaca ggtggaccga taccatgaag gacccatact ctgttatgag gggacttctt     420
ttctctcaca tcggatggat ggttcttaac tctgatccaa aggttaaggg aaggaccgat     480
gtttctgatc ttgattctga tccagttgtt gtttggcaac acaagcacta cggaaagtgc     540
cttcttttcg ctgcttggat cttcccaatg atcgttgctg gacttggatg gggagattgg     600
tggggaggac ttgtttacgc tggaatcatc agggcttgct tcgttcaaca agctaccttc     660
tgcgttaact ctcttgctca ctggatcgga gagcaaccat tcgacgatag gaggacccca     720
agggatcacg ttcttaccgc tcttgttacc atgggagagg gataccacaa cttccaccac     780
gagttcccat ctgattacag gaacgctatc atctggtacc aatacgatcc aaccaagtgg     840
cttatctacc ttttctctct tggaccattc ccacttgctt actctcttaa gaccttcagg     900
tctaacgaga tcgagaaggg aaggcttcaa caacaacaaa aggctcttga taagaagagg     960
tctggacttg attggggact tccacttttc caacttccag ttatctcttg ggatgatttc    1020
caagctaggt gcaaggagtc tggagagatg cttgttgctg ttgctggagt tatccacgat    1080
gtttctcaat tcatcgagga tcacccagga ggaaggtctc ttatcaggtc tgctgttgga    1140
aaggatggaa ccggaatgtt caacggagga gtttacgagc actctaacgc tgctcacaac    1200
cttctttcta ccatgagggt tggagttctt aggggaggac aagaggttga ggtttggaag    1260
aagcaaaggg ttgatgttct tggaaagtca gatatcctta ggcaagttac cagggttgag    1320
aggcttgttg agggagctgt tgctgcttga                                     1350
```

<210> 45
<211> 1062
<212> DNA
<213> Artificial

<220>
<223> Canola-optimized desaturase

<400> 45

```
atggctccaa acatctctga ggatgttaac ggagttcttt tcgagtctga tgctgctacc      60
ccagatcttg ctctttctac cccaccagtt caaaaggctg ataacaggcc aaagcaactt     120
gtttggagga acatccttct tttcgcttac cttcaccttg ctgctcttta cggaggatac     180
cttttccttt tctctgctaa gtggcaaacc gatatcttcg cttacatcct ttacgttatc     240
```

```
tctggacttg gaataaccgc tggagcacac aggctttggg ctcacaagtc ttacaaggct     300
aagtggccac ttagggttat ccttgttatc ttcaacaccg ttgctttcca agacgctgct     360
atggattggg ctagggatca caggatgcac cacaagtact ctgagaccga cgctgatcca     420
cacaacgcta ccaggggatt cttcttctct cacatcggat ggcttcttgt taggaagcac     480
ccagatctta aggagaaggg aaagggactt gatatgtctg atcttcttgc tgatccaatc     540
cttaggttcc aaaagaagta ctaccttatc cttatgccac ttgcttgctt cgttatgcca     600
accgttatcc cagtttactt ctggggagag acctggacca acgctttctt cgttgctgct     660
atgttcaggt acgctttcat ccttaacgtt acctggcttg ttaactctgc tgctcacaag     720
tggggagata agccatacga taagtctatc aagccatctg agaacctttc tgttgctatg     780
ttcgctcttg gagagggatt ccacaactac caccacacct tcccatggga ttacaagacc     840
gctgagcttg gaaacaacaa gcttaacttc accaccacct tcatcaactt cttcgctaag     900
atcggatggg cttacgatct taagaccgtt tctgatgata tcgttaagaa cagggttaag     960
aggaccggag atggatcaca ccacctttgg ggatggggag atgagaacca atctaaggag    1020
gagatcgatg ctgctatcag gatcaaccca aaggatgatt ga                       1062
```

<210> 46
<211> 10
<212> PRT
<213> Artificial

<220>
<223> Myc tag

<400> 46

```
Glu Gln Lys Leu Ile Ser Glu Glu Asp Leu
1               5                   10
```

<210> 47
<211> 9
<212> PRT
<213> Artificial

<220>
<223> Hemagglutin tag

<400> 47

```
Tyr Pro Tyr Asp Val Pro Asp Tyr Ala
1               5
```

<210> 48
<211> 1368
<212> DNA
<213> Aspergillus nidulans

<400> 48

```
atgtctgctc caaccgctga catcagggct agggctccag aggctaagaa ggttcacatc      60
gctgataccg ctatcaacag gcacaattgg tacaagcacg tgaactggct caacgtcttc     120
ctcatcatcg gaatcccact ctacggatgc atccaagctt tctgggttcc acttcaactc     180

aagaccgcta tctgggctgt gatctactac ttcttcaccg gacttggaat caccgctgga     240
taccacaggc tttgggctca ctgctcttac tctgctactc ttccacttag gatctggctt     300
gctgctgttg gaggaggagc tgttgaggga tctatcagat ggtgggctag ggatcacagg     360
gctcatcata ggtacaccga taccgacaag gacccatact ctgttaggaa gggacttctc     420
tactctcacc ttggatggat ggtgatgaag cagaacccaa agaggatcgg aaggaccgac     480
atctctgatc tcaacgagga cccagttgtt gtttggcaac acaggaacta cctcaaggtt     540
gtgttcacca tgggacttgc tgttccaatg cttgttgctg gacttggatg gggagattgg     600
cttggaggat tcgtgtacgc tggaatcctt aggatcttct tcgttcaaca agctaccttc     660
tgcgtgaact ctcttgctca ctggcttgga gatcaaccat tcgatgatag gaactctcct     720
agggatcacg tgatcaccgc tcttgttacc cttggagagg gataccacaa cttccaccac     780
gagttcccat ctgactacag gaacgctatc gagtggcacc agtacgatcc taccaagtgg     840
tctatctggg cttggaagca acttggattg gcttacgatc tcaagaagtt cagggctaac     900
gagatcgaga agggaagggt tcaacaactt cagaagaagc ttgataggaa gagggctact     960
cttgattggg gaaccccact tgatcaactt ccagtgatgg aatgggatga ctacgttgag    1020
caagctaaga acggaagggg acttgttgct atcgctggag ttgttcacga tgttaccgac    1080
ttcatcaagg atcacccagg aggaaaggct atgatctctt ctggaatcgg aaaggatgct    1140
accgctatgt tcaacggagg agtgtactac cactctaacg cagctcacaa ccttcttagc    1200
accatgaggg tgggagtgat caggggagga tgcgaggttg agatctggaa gagggctcag    1260
aaggagaacg ttgagtacgt tagggatgga tctggacaaa gggtgatcag ggctggagag    1320
caaccaacca agatcccaga gccaatccca accgctgatg ctgcttga                 1368
```

<210> 49
<211> 1368
<212> DNA
<213> Artificial

<220>
<223> AnD9DS v3 silent mutant

<400> 49

```
atgtctgctc caaccgctga catcagggct agggctccag aggctaagaa ggttcacatc      60
gctgataccg ctatcaacag gcacaattgg tacaagcacg tgaactggct caacgtcttc     120
ctcatcatcg gaatcccact ctacggatgc atccaagctt tctgggttcc acttcaactc     180
aagaccgcta tctgggctgt gatctactac ttcttcaccg gacttggaat caccgctgga     240
taccacaggc tttgggctca ctgctcatac tctgctactc ttccacttag gatctggctt     300
gctgctgttg gaggaggagc tgttgaggga tctatcagat ggtgggctag ggatcacagg     360
gctcatcata ggtacaccga taccgacaag gacccatact ctgttaggaa gggacttctc     420
tactctcacc ttggatggat ggtgatgaag cagaacccaa agaggatcgg aaggaccgac     480
atctctgatc tcaacgagga cccagttgtt gtttggcaac acaggaacta cctcaaggtt     540

gtgttcacca tgggacttgc tgttccaatg cttgttgctg gacttggatg gggagattgg     600
cttggaggat tcgtgtacgc tggaatcctt aggatcttct tcgttcaaca agctaccttc     660
tgcgtgaact ctcttgctca ctggcttgga gatcaaccat tcgatgatag gaactctcct     720
agggatcacg tgatcaccgc tcttgttacc cttggagagg gataccacaa cttccaccac     780
gagttcccat ctgactacag gaacgctatc gagtggcacc agtacgatcc taccaagtgg     840
tctatctggg cttggaagca acttggattg gcttacgatc tcaagaagtt cagggctaac     900
gagatcgaga agggaagggt tcaacaactt cagaagaagc ttgataggaa gagggctact     960
cttgattggg gaaccccact tgatcaactt ccagtgatgg aatgggatga ctacgttgag    1020
caagctaaga acggaagggg acttgttgct atcgctggag ttgttcacga tgttaccgac    1080
ttcatcaagg atcacccagg aggaaaggct atgatctctt ctggaatcgg aaaggatgct    1140
accgctatgt tcaacggagg agtgtactac cactctaacg cagctcacaa ccttcttagc    1200
accatgaggg tgggagtgat caggggagga tgcgaggttg agatctggaa gagggctcag    1260
aaggagaacg ttgagtacgt tagggatgga tctggacaaa gggtgatcag ggctggagag    1320
caaccaacca agatcccaga gccaatccca accgctgatg ctgcttga                 1368
```

<210> 50
<211> 455
<212> PRT
<213> Aspergillus nidulans

<400> 50

```
Met Ser Ala Pro Thr Ala Asp Ile Arg Ala Arg Ala Pro Glu Ala Lys
1               5                   10                  15

Lys Val His Ile Ala Asp Thr Ala Ile Asn Arg His Asn Trp Tyr Lys
            20                  25                  30

His Val Asn Trp Leu Asn Val Phe Leu Ile Ile Gly Ile Pro Leu Tyr
        35                  40                  45

Gly Cys Ile Gln Ala Phe Trp Val Pro Leu Gln Leu Lys Thr Ala Ile
    50                  55                  60

Trp Ala Val Ile Tyr Tyr Phe Phe Thr Gly Leu Gly Ile Thr Ala Gly
65                  70                  75                  80

Tyr His Arg Leu Trp Ala His Cys Ser Tyr Ser Ala Thr Leu Pro Leu
                85                  90                  95

Arg Ile Trp Leu Ala Ala Val Gly Gly Gly Ala Val Glu Gly Ser Ile
            100                 105                 110

Arg Trp Trp Ala Arg Asp His Arg Ala His His Arg Tyr Thr Asp Thr
        115                 120                 125
```

Asp Lys Asp Pro Tyr Ser Val Arg Lys Gly Leu Leu Tyr Ser His Leu
    130                 135                 140

Gly Trp Met Val Met Lys Gln Asn Pro Lys Arg Ile Gly Arg Thr Asp
145                 150                 155                 160

Ile Ser Asp Leu Asn Glu Asp Pro Val Val Val Trp Gln His Arg Asn
                165                 170                 175

Tyr Leu Lys Val Val Phe Thr Met Gly Leu Ala Val Pro Met Leu Val
            180                 185                 190

Ala Gly Leu Gly Trp Gly Asp Trp Leu Gly Gly Phe Val Tyr Ala Gly
        195                 200                 205

Ile Leu Arg Ile Phe Phe Val Gln Gln Ala Thr Phe Cys Val Asn Ser
    210                 215                 220

Leu Ala His Trp Leu Gly Asp Gln Pro Phe Asp Asp Arg Asn Ser Pro
225                 230                 235                 240

Arg Asp His Val Ile Thr Ala Leu Val Thr Leu Gly Glu Gly Tyr His
                245                 250                 255

Asn Phe His His Glu Phe Pro Ser Asp Tyr Arg Asn Ala Ile Glu Trp
            260                 265                 270

His Gln Tyr Asp Pro Thr Lys Trp Ser Ile Trp Ala Trp Lys Gln Leu
        275                 280                 285

Gly Leu Ala Tyr Asp Leu Lys Lys Phe Arg Ala Asn Glu Ile Glu Lys
    290                 295                 300

Gly Arg Val Gln Gln Leu Gln Lys Lys Leu Asp Arg Lys Arg Ala Thr
305                 310                 315                 320

Leu Asp Trp Gly Thr Pro Leu Asp Gln Leu Pro Val Met Glu Trp Asp
                325                 330                 335

Asp Tyr Val Glu Gln Ala Lys Asn Gly Arg Gly Leu Val Ala Ile Ala
            340                 345                 350

Gly Val Val His Asp Val Thr Asp Phe Ile Lys Asp His Pro Gly Gly
        355                 360                 365

Lys Ala Met Ile Ser Ser Gly Ile Gly Lys Asp Ala Thr Ala Met Phe
    370                 375                 380

Asn Gly Gly Val Tyr Tyr His Ser Asn Ala Ala His Asn Leu Leu Ser
385                 390                 395                 400

```
Thr Met Arg Val Gly Val Ile Arg Gly Gly Cys Glu Val Glu Ile Trp
                405                 410                 415

Lys Arg Ala Gln Lys Glu Asn Val Glu Tyr Val Arg Asp Gly Ser Gly
            420                 425                 430

Gln Arg Val Ile Arg Ala Gly Glu Gln Pro Thr Lys Ile Pro Glu Pro
        435                 440                 445

Ile Pro Thr Ala Asp Ala Ala
    450                 455
```

<210> 51
<211> 455
<212> PRT
<213> Aspergillus nidulans

<400> 51

```
Met Ser Ala Pro Thr Ala Asp Ile Arg Ala Arg Ala Pro Glu Ala Lys
1               5                   10                  15

Lys Val His Ile Ala Asp Thr Ala Ile Asn Arg His Asn Trp Tyr Lys
            20                  25                  30

His Val Asn Trp Leu Asn Val Phe Leu Ile Ile Gly Ile Pro Leu Tyr
        35                  40                  45

Gly Cys Ile Gln Ala Phe Trp Val Pro Leu Gln Leu Lys Thr Ala Ile
    50                  55                  60

Trp Ala Val Ile Tyr Tyr Phe Phe Thr Gly Leu Gly Ile Thr Ala Gly
65                  70                  75                  80

Tyr His Arg Leu Trp Ala His Cys Ser Tyr Ser Ala Thr Leu Pro Leu
                85                  90                  95

Arg Ile Trp Leu Ala Ala Val Gly Gly Gly Ala Val Glu Gly Ser Ile
            100                 105                 110

Arg Trp Trp Ala Arg Asp His Arg Ala His His Arg Tyr Thr Asp Thr
        115                 120                 125

Asp Lys Asp Pro Tyr Ser Val Arg Lys Gly Leu Leu Tyr Ser His Leu
    130                 135                 140

Gly Trp Met Val Met Lys Gln Asn Pro Lys Arg Ile Gly Arg Thr Asp
145                 150                 155                 160

Ile Ser Asp Leu Asn Glu Asp Pro Val Val Val Trp Gln His Arg Asn
                165                 170                 175
```

```
Tyr Leu Lys Val Val Phe Thr Met Gly Leu Ala Val Pro Met Leu Val
            180                 185                 190

Ala Gly Leu Gly Trp Gly Asp Trp Leu Gly Gly Phe Val Tyr Ala Gly
        195                 200                 205

Ile Leu Arg Ile Phe Phe Val Gln Gln Ala Thr Phe Cys Val Asn Ser
    210                 215                 220

Leu Ala Leu Trp Leu Gly Asp Gln Pro Phe Asp Asp Arg Asn Ser Pro
225                 230                 235                 240

Arg Asp His Val Ile Thr Ala Leu Val Thr Leu Gly Glu Gly Tyr His
                245                 250                 255

Asn Phe His His Glu Phe Pro Ser Asp Tyr Arg Asn Ala Ile Glu Trp
            260                 265                 270

His Gln Tyr Asp Pro Thr Lys Trp Ser Ile Trp Ala Trp Lys Gln Leu
        275                 280                 285

Gly Leu Ala Tyr Asp Leu Lys Lys Phe Arg Ala Asn Glu Ile Glu Lys
    290                 295                 300

Gly Arg Val Gln Gln Leu Gln Lys Lys Leu Asp Arg Lys Arg Ala Thr
305                 310                 315                 320

Leu Asp Trp Gly Thr Pro Leu Asp Gln Leu Pro Val Met Glu Trp Asp
                325                 330                 335

Asp Tyr Val Glu Gln Ala Lys Asn Gly Arg Gly Leu Val Ala Ile Ala
            340                 345                 350

Gly Val Val His Asp Val Thr Asp Phe Ile Lys Asp His Pro Gly Gly
        355                 360                 365

Lys Ala Met Ile Ser Ser Gly Ile Gly Lys Asp Ala Thr Ala Met Phe
    370                 375                 380

Asn Gly Gly Val Tyr Tyr His Ser Asn Ala Ala His Asn Leu Leu Ser
385                 390                 395                 400

Thr Met Arg Val Gly Val Ile Arg Gly Gly Cys Glu Val Glu Ile Trp
                405                 410                 415

Lys Arg Ala Gln Lys Glu Asn Val Glu Tyr Val Arg Asp Gly Ser Gly
            420                 425                 430

Gln Arg Val Ile Arg Ala Gly Glu Gln Pro Thr Lys Ile Pro Glu Pro
        435                 440                 445
```

Ile Pro Thr Ala Asp Ala Ala
450 455

<210> 52
<211> 510
<212> PRT
<213> Saccharomyces cerevisiae

<400> 52

```
Met Pro Thr Ser Gly Thr Thr Ile Glu Leu Ile Asp Asp Gln Phe Pro
1               5                   10                  15

Lys Asp Asp Ser Ala Ser Ser Gly Ile Val Asp Glu Val Asp Leu Thr
            20                  25                  30

Glu Ala Asn Ile Leu Ala Thr Gly Leu Asn Lys Lys Ala Pro Arg Ile
        35                  40                  45

Val Asn Gly Phe Gly Ser Leu Met Gly Ser Lys Glu Met Val Ser Val
    50                  55                  60

Glu Phe Asp Lys Lys Gly Asn Glu Lys Lys Ser Asn Leu Asp Arg Leu
65                  70                  75                  80

Leu Glu Lys Asp Asn Gln Glu Lys Glu Glu Ala Lys Thr Lys Ile His
                85                  90                  95

Ile Ser Glu Gln Pro Trp Thr Leu Asn Asn Trp His Gln His Leu Asn
            100                 105                 110

Trp Leu Asn Met Val Leu Val Cys Gly Met Pro Met Ile Gly Trp Tyr
        115                 120                 125

Phe Ala Leu Ser Gly Lys Val Pro Leu His Leu Asn Val Phe Leu Phe
    130                 135                 140

Ser Val Phe Tyr Tyr Ala Val Gly Gly Val Ser Ile Thr Ala Gly Tyr
145                 150                 155                 160

His Arg Leu Trp Ser His Arg Ser Tyr Ser Ala His Trp Pro Leu Arg
                165                 170                 175

Leu Phe Tyr Ala Ile Phe Gly Cys Ala Ser Val Glu Gly Ser Ala Lys
            180                 185                 190

Trp Trp Gly His Ser His Arg Ile His His Arg Tyr Thr Asp Thr Leu
        195                 200                 205

Arg Asp Pro Tyr Asp Ala Arg Arg Gly Leu Trp Tyr Ser His Met Gly
    210                 215                 220

Trp Met Leu Leu Lys Pro Asn Pro Lys Tyr Lys Ala Arg Ala Asp Ile
```

```
225                 230                 235                 240

Thr Asp Met Thr Asp Asp Trp Thr Ile Arg Phe Gln His Arg His Tyr
                245                 250                 255

Ile Leu Leu Met Leu Leu Thr Ala Phe Val Ile Pro Thr Leu Ile Cys
            260                 265                 270

Gly Tyr Phe Phe Asn Asp Tyr Met Gly Gly Leu Ile Tyr Ala Gly Phe
        275                 280                 285

Ile Arg Val Phe Val Ile Gln Gln Ala Thr Phe Cys Ile Asn Ser Met
    290                 295                 300

Ala His Tyr Ile Gly Thr Gln Pro Phe Asp Asp Arg Arg Thr Pro Arg
305                 310                 315                 320

Asp Asn Trp Ile Thr Ala Ile Val Thr Phe Gly Glu Gly Tyr His Asn
                325                 330                 335

Phe His His Glu Phe Pro Thr Asp Tyr Arg Asn Ala Ile Lys Trp Tyr
            340                 345                 350

Gln Tyr Asp Pro Thr Lys Val Ile Ile Tyr Leu Thr Ser Leu Val Gly
        355                 360                 365

Leu Ala Tyr Asp Leu Lys Lys Phe Ser Gln Asn Ala Ile Glu Glu Ala
    370                 375                 380

Leu Ile Gln Gln Glu Gln Lys Lys Ile Asn Lys Lys Lys Ala Lys Ile
385                 390                 395                 400

Asn Trp Gly Pro Val Leu Thr Asp Leu Pro Met Trp Asp Lys Gln Thr
                405                 410                 415

Phe Leu Ala Lys Ser Lys Glu Asn Lys Gly Leu Val Ile Ile Ser Gly
            420                 425                 430

Ile Val His Asp Val Ser Gly Tyr Ile Ser Glu His Pro Gly Gly Glu
        435                 440                 445

Thr Leu Ile Lys Thr Ala Leu Gly Lys Asp Ala Thr Lys Ala Phe Ser
    450                 455                 460

Gly Gly Val Tyr Arg His Ser Asn Ala Ala Gln Asn Val Leu Ala Asp
465                 470                 475                 480

Met Arg Val Ala Val Ile Lys Glu Ser Lys Asn Ser Ala Ile Arg Met
                485                 490                 495

Ala Ser Lys Arg Gly Glu Ile Tyr Glu Thr Gly Lys Phe Phe
```

500 505 510

<210> 53
<211> 13227
<212> DNA
<213> Artificial

<220>
<223> Plasmid pDAB7309

<400> 53

```
ggactagtcc agaaggtaat tatccaagat gtagcatcaa gaatccaatg tttacgggaa      60
aaactatgga agtattatgt aagctcagca agaagcagat caatatgcgg cacatatgca     120
acctatgttc aaaaatgaag aatgtacaga tacaagatcc tatactgcca gaatacgaag     180
aagaatacgt agaaattgaa aaagaagaac caggcgaaga aaagaatctt gaagacgtaa     240
gcactgacga caacaatgaa aagaagaaga taaggtcggt gattgtgaaa gagacataga     300
ggacacatgt aaggtggaaa atgtaagggc ggaaagtaac cttatcacaa aggaatctta     360
tcccccacta cttatccttt tatatttttc cgtgtcattt ttgcccttga gttttcctat     420
ataaggaacc aagttcggca tttgtgaaaa caagaaaaaa tttggtgtaa gctattttct     480
ttgaagtact gaggatacaa cttcagagaa atttgtaagt ttgtaggtac cagatctgga     540
tcccaaacca tgtctccgga gaggagacca gttgagatta ggccagctac agcagctgat     600
atggccgcgg tttgtgatat cgttaaccat tacattgaga cgtctacagt gaactttagg     660
acagagccac aaacaccaca agagtggatt gatgatctag agaggttgca agatagatac     720
ccttggttgg ttgctgaggt tgagggtgtt gtggctggta ttgcttacgc tgggccctgg     780
aaggctagga acgcttacga ttggacagtt gagagtactg tttacgtgtc acataggcat     840
caaaggttgg gcctaggatc tacattgtac acacatttgc ttaagtctat ggaggcgcaa     900
ggttttaagt ctgtggttgc tgttataggc cttccaaacg atccatctgt taggttgcat     960
gaggctttgg gatacacagc ccggggtaca ttgcgcgcag ctggatacaa gcatggtgga    1020
tggcatgatg ttggtttttg gcaaagggat tttgagttgc cagctcctcc aaggccagtt    1080
aggccagtta cccaaatctg agtagttagc ttaatcacct agagctcgat cggcggcaat    1140
agcttcttag cgccatcccg ggttgatcct atctgtgttg aaatagttgc ggtgggcaag    1200
gctctctttc agaaagacag gcggccaaag gaacccaagg tgaggtgggc tatggctctc    1260
agttccttgt ggaagcgctt ggtctaaggt gcagaggtgt tagcgggatg aagcaaaagt    1320
gtccgattgt aacaagatat gttgatccta cgtaaggata ttaaagtatg tattcatcac    1380
taatataatc agtgtattcc aatatgtact acgatttcca atgtctttat tgtcgccgta    1440
tgtaatcggc gtcacaaaat aatccccggt gactttcttt taatccagga tgaaataata    1500
tgttattata atttttgcga tttggtccgt tataggaatt gaagtgtgct tgaggtcggt    1560
cgccaccact cccatttcat aattttacat gtatttgaaa aataaaaatt tatggtattc    1620
aatttaaaca cgtatacttg taaagaatga tatcttgaaa gaaatatagt ttaaatattt    1680
```

attgataaaa taacaagtca ggtattatag tccaagcaaa aacataaatt tattgatgca 1740
agtttaaatt cagaaatatt tcaataactg attatatcag ctggtacatt gccgtagatg 1800
aaagactgag tgcgatatta tggtgtaata cataggaatt cgtttaaacg atctgcgtct 1860
aattttcggt ccaacttgca caggaaagac gtcgaccgcg gtagctcttg cccagcagac 1920
tgggcttcca gtcctttcgc tcgatcgggt ccaatgttgt cctcagctgt gaaccggaag 1980
cggacgacca acagtggaag aactgaaagg aacgagccgt ctataccttg atgatcggcc 2040
tctggtgaag ggtatcatcg cagccaagca agctcatgaa aggctgatgg gggaggtgta 2100
taattatgag gcccacggcg ggcttattct ttagggagga tctatctcgt tgctcaagtg 2160
catggcgcaa agcagttatt ggagtgcgga ttttcgttgg catattattc gccacgagtt 2220
agcagacgaa gagaccttca tgaacgtggc caaggccaga gttaagcaga tgttacgccc 2280
tgctgcaggc ctttctatta tccaatagtt ggttgatctt tggaaagagc ctcggctgag 2340
gcccatactg aaagagatcg atggatatcg atatgccatg ttgtttgcta gccagaacca 2400
gatcacatcc gatatgctat tgcagcttga cgcagatatg gaggataagt tgattcatgg 2460
gatcgctcag gagtagctca tccatgcacg ccgacaagaa cagaaattcc gtcgagttaa 2520
cgcagccgct tacgacggat tcgaaggtca tccattcgga atgtattagt ttgcaccagc 2580
tccgcgtcac acctgtcttc atttgaataa gatgttagca attgttttta gctttgtctt 2640
gttgtggcag ggcggcaagt gcttcagaca tcattctgtt ttcaaatttt atgctggaga 2700
acagcttctt aattcctttg gaaataatag actgcgtctt aaaattcaga tgtctggata 2760
tagatatgat tgtaaaataa cctatttaag tgtcatttag aacataagtt ttatgaatgt 2820
tcttccattt tcgtcatcga acgaataaga gtaaatacac ctttttttaac attacaaata 2880
agttcttata cgttgtttat acaccgggaa tcatttccat tattttcgcg caaaagtcac 2940
ggatattcgt gaaagcgaca taaactgcga aatttgcggg gagtgtcttg agtttgcctc 3000
gaggctagcg catgcacata gacacacaca tcatctcatt gatgcttggt aataattgtc 3060
attagattgt ttttatgcat agatgcactc gaaatcagcc aattttagac aagtatcaaa 3120
cggatgtgac ttcagtacat taaaaacgtc cgcaatgtgt tattaagttg tctaagcgtc 3180
aatttgattt acaattgaat atatcctgcc ccagccagcc aacagctcga tttacaattg 3240
aatatatcct gccggccggc ccacgcgtgt cgaggaattc tgatctggcc cccatttgga 3300
cgtgaatgta gacacgtcga aataaagatt tccgaattag aataatttgt ttattgcttt 3360
cgcctataaa tacgacggat cgtaatttgt cgttttatca aaatgtactt tcattttata 3420
ataacgctgc ggacatctac atttttgaat tgaaaaaaaa ttggtaatta ctctttcttt 3480
ttctccatat tgaccatcat actcattgct gatccatgta gatttcccgg acatgaagcc 3540
atttacaatt gaatatatcc tgccgccgct gccgctttgc acccggtgga gcttgcatgt 3600
tggtttctac gcagaactga gccggttagg cagataattt ccattgagaa ctgagccatg 3660
tgcaccttcc ccccaacacg gtgagcgacg gggcaacgga gtgatccaca tgggactttt 3720

aaacatcatc cgtcggatgg cgttgcgaga gaagcagtcg atccgtgaga tcagccgacg 3780
caccgggcag gcgcgcaaca cgatcgcaaa gtatttgaac gcaggtacaa tcgagccgac 3840
gttcacgcgg aacgaccaag caagcttggc tgccattttt ggggtgaggc cgttcgcggc 3900
cgaggggcgc agcccctggg gggatgggag gcccgcgtta gcgggccggg agggttcgag 3960
aagggggggc acccccccttc ggcgtgcgcg gtcacgcgca cagggcgcag ccctggttaa 4020
aaacaaggtt tataaatatt ggtttaaaag caggttaaaa gacaggttag cggtggccga 4080
aaaacgggcg gaaacccttg caaatgctgg attttctgcc tgtggacagc ccctcaaatg 4140
tcaataggtg cgcccctcat ctgtcagcac tctgcccctc aagtgtcaag gatcgcgccc 4200
ctcatctgtc agtagtcgcg cccctcaagt gtcaataccg cagggcactt atccccaggc 4260
ttgtccacat catctgtggg aaactcgcgt aaaatcaggc gttttcgccg atttgcgagg 4320
ctggccagct ccacgtcgcc ggccgaaatc gagcctgccc ctcatctgtc aacgccgcgc 4380
cgggtgagtc ggcccctcaa gtgtcaacgt ccgcccctca tctgtcagtg agggccaagt 4440
tttccgcgag gtatccacaa cgccggcggc cgcggtgtct cgcacacggc ttcgacggcg 4500
tttctggcgc gtttgcaggg ccatagacgg ccgccagccc agcggcgagg gcaaccagcc 4560
cggtgagcgt cggaaagggt cgacggatct tttccgctgc ataaccctgc ttcggggtca 4620
ttatagcgat tttttcggta tatccatcct ttttcgcacg atatacagga ttttgccaaa 4680
gggttcgtgt agactttcct tggtgtatcc aacggcgtca gccgggcagg ataggtgaag 4740
taggcccacc cgcgagcggg tgttccttct tcactgtccc ttattcgcac ctggcggtgc 4800
tcaacgggaa tcctgctctg cgaggctggc cggctaccgc cggcgtaaca gatgagggca 4860
agcggatggc tgatgaaacc aagccaacca ggaagggcag cccacctatc aaggtgtact 4920
gccttccaga cgaacgaaga gcgattgagg aaaaggcggc ggcggccggc atgagcctgt 4980
cggcctacct gctggccgtc ggccagggct acaaaatcac gggcgtcgtg gactatgagc 5040
acgtccgcga gctggcccgc atcaatggcg acctgggccg cctgggcggc ctgctgaaac 5100
tctggctcac cgacgacccg cgcacggcgc ggttcggtga tgccacgatc ctcgccctgc 5160
tggcgaagat cgaagagaag caggacgagc ttggcaaggt catgatgggc gtggtccgcc 5220
cgagggcaga gccatgactt ttttagccgc taaaacggcc ggggggtgcg cgtgattgcc 5280
aagcacgtcc ccatgcgctc catcaagaag agcgacttcg cggagctggt attcgtgcag 5340
ggcaagattc ggaataccaa gtacgagaag gacggccaga cggtctacgg gaccgacttc 5400
attgccgata aggtggatta tctggacacc aaggcaccag gcgggtcaaa tcaggaataa 5460
gggcacattg ccccggcgtg agtcggggca atcccgcaag gagggtgaat gaatcggacg 5520
tttgaccgga aggcatacag gcaagaactg atcgacgcgg ggttttccgc cgaggatgcc 5580
gaaaccatcg caagccgcac cgtcatgcgt gcgccccgcg aaaccttcca gtccgtcggc 5640
tcgatggtcc agcaagctac ggccaagatc gagcgcgaca gcgtgcaact ggctcccccct 5700
gccctgcccg cgccatcggc cgccgtggag cgttcgcgtc gtctcgaaca ggaggcggca 5760

ggtttggcga agtcgatgac catcgacacg cgaggaacta tgacgaccaa gaagcgaaaa 5820
accgccggcg aggacctggc aaaacaggtc agcgaggcca agcaggccgc gttgctgaaa 5880
cacacgaagc agcagatcaa ggaaatgcag ctttccttgt tcgatattgc gccgtggccg 5940
gacacgatgc gagcgatgcc aaacgacacg gcccgctctg ccctgttcac cacgcgcaac 6000
aagaaaatcc cgcgcgaggc gctgcaaaac aaggtcattt tccacgtcaa caaggacgtg 6060
aagatcacct acaccggcgt cgagctgcgg gccgacgatg acgaactggt gtggcagcag 6120
gtgttggagt acgcgaagcg cacccctatc ggcgagccga tcaccttcac gttctacgag 6180
ctttgccagg acctgggctg gtcgatcaat ggccggtatt acacgaaggc cgaggaatgc 6240
ctgtcgcgcc tacaggcgac ggcgatgggc ttcacgtccg accgcgttgg gcacctggaa 6300
tcggtgtcgc tgctgcaccg cttccgcgtc ctggaccgtg gcaagaaaac gtcccgttgc 6360
caggtcctga tcgacgagga aatcgtcgtg ctgtttgctg gcgaccacta cacgaaattc 6420
atatgggaga agtaccgcaa gctgtcgccg acggcccgac ggatgttcga ctatttcagc 6480
tcgcaccggg agccgtaccc gctcaagctg gaaaccttcc gcctcatgtg cggatcggat 6540
tccacccgcg tgaagaagtg gcgcgagcag gtcggcgaag cctgcgaaga gttgcgaggc 6600
agcggcctgg tggaacacgc ctgggtcaat gatgacctgg tgcattgcaa acgctagggc 6660
cttgtggggt cagttccggc tgggggttca gcagccagcg ctttactggc atttcaggaa 6720
caagcgggca ctgctcgacg cacttgcttc gctcagtatc gctcgggacg cacggcgcgc 6780
tctacgaact gccgataaac agaggattaa aattgacaat tgtgattaag gctcagattc 6840
gacggcttgg agcggccgac gtgcaggatt tccgcgagat ccgattgtcg gccctgaaga 6900
aagctccaga gatgttcggg tccgtttacg agcacgagga gaaaaagccc atggaggcgt 6960
tcgctgaacg gttgcgagat gccgtggcat tcggcgccta catcgacggc gagatcattg 7020
ggctgtcggt cttcaaacag gaggacggcc ccaaggacgc tcacaaggcg catctgtccg 7080
gcgttttcgt ggagcccgaa cagcgaggcc gaggggtcgc cggtatgctg ctgcgggcgt 7140
tgccggcggg tttattgctc gtgatgatcg tccgacagat tccaacggga atctggtgga 7200
tgcgcatctt catcctcggc gcacttaata tttcgctatt ctggagcttg ttgtttattt 7260
cggtctaccg cctgccgggc ggggtcgcgg cgacggtagg cgctgtgcag ccgctgatgg 7320
tcgtgttcat ctctgccgct ctgctaggta gcccgatacg attgatggcg gtcctggggg 7380
ctatttgcgg aactgcgggc gtggcgctgt tggtgttgac accaaacgca gcgctagatc 7440
ctgtcggcgt cgcagcgggc ctggcggggg cggtttccat ggcgttcgga accgtgctga 7500
cccgcaagtg gcaacctccc gtgcctctgc tcacctttac cgcctggcaa ctggcggccg 7560
gaggacttct gctcgttcca gtagctttag tgtttgatcc gccaatcccg atgcctacag 7620
gaaccaatgt tctcggcctg gcgtggctcg gcctgatcgg agcgggttta acctacttcc 7680
tttggttccg gggatctcg cgactcgaac ctacagttgt ttccttactg ggctttctca 7740
gcccccgagc gcttagtggg aatttgtacc ccttatcgaa ccgggagcac aggatgacgc 7800

ctaacaattc attcaagccg acaccgcttc gcggcgcggc ttaattcagg agttaaacat 7860
catgagggaa gcggtgatcg ccgaagtatc gactcaacta tcagaggtag ttggcgtcat 7920
cgagcgccat ctcgaaccga cgttgctggc cgtacatttg tacggctccg cagtggatgg 7980
cggcctgaag ccacacagtg atattgattt gctggttacg gtgaccgtaa ggcttgatga 8040
aacaacgcgg cgagctttga tcaacgacct tttggaaact tcggcttccc ctggagagag 8100
cgagattctc cgcgctgtag aagtcaccat tgttgtgcac gacgacatca ttccgtggcg 8160
ttatccagct aagcgcgaac tgcaatttgg agaatggcag cgcaatgaca ttcttgcagg 8220
tatcttcgag ccagccacga tcgacattga tctggctatc ttgctgacaa aagcaagaga 8280
acatagcgtt gccttggtag gtccagcggc ggaggaactc tttgatccgg ttcctgaaca 8340
ggatctattt gaggcgctaa atgaaacctt aacgctatgg aactcgccgc ccgactgggc 8400
tggcgatgag cgaaatgtag tgcttacgtt gtcccgcatt tggtacagcg cagtaaccgg 8460
caaaatcgcg ccgaaggatg tcgctgccga ctgggcaatg gagcgcctgc cggcccagta 8520
tcagcccgtc atacttgaag ctaggcaggc ttatcttgga caagaagatc gcttggcctc 8580
gcgcgcagat cagttggaag aatttgttca ctacgtgaaa ggcgagatca ccaaggtagt 8640
cggcaaataa tgtctaacaa ttcgttcaag ccgacgccgc ttcgcggcgc ggcttaactc 8700
aagcgttaga gagctgggga agactatgcg cgatctgttg aaggtggttc taagcctcgt 8760
cttgcgatgg catttcgatc cattcccatt ccgcgctcaa gatggcttcc cctcggcagt 8820
tcatcagggc taaatcaatc tagccgactt gtccggtgaa atgggctgca ctccaacaga 8880
aacaatcaaa caaacataca cagcgactta ttcacacgag ctcaaattac aacggtatat 8940
atcctgccag tcagcatcat cacaccaaaa gttaggcccg aatagtttga aattagaaag 9000
ctcgcaattg aggtctacag gccaaattcg ctcttagccg tacaatatta ctcaccggat 9060
cctaaccggt gtgatcatgg gccgcgatta aaaatctcaa ttatatttgg tctaatttag 9120
tttggtattg agtaaaacaa attcgaacca aaccaaaata taaatatata gtttttatat 9180
atatgccttt aagacttttt atagaatttt ctttaaaaaa tatctagaaa tatttgcgac 9240
tcttctggca tgtaatattt cgttaaatat gaagtgctcc atttttatta actttaaata 9300
attggttgta cgatcacttt cttatcaagt gttactaaaa tgcgtcaatc tctttgttct 9360
tccatattca tatgtcaaaa cctatcaaaa ttcttatata tctttttcga atttgaagtg 9420
aaatttcgat aatttaaaat taaatagaac atatcattat ttaggtatca tattgatttt 9480
tatacttaat tactaaattt ggttaacttt gaaagtgtac atcaacgaaa aattagtcaa 9540
acgactaaaa taaataaata tcatgtgtta ttaagaaaat tctcctataa gaatatttta 9600
atagatcata tgtttgtaaa aaaaattaat ttttactaac acatatattt acttatcaaa 9660
aatttgacaa agtaagatta aaataatatt catctaacaa aaaaaaaacc agaaaatgct 9720
gaaaacccgg caaaaccgaa ccaatccaaa ccgatatagt tggtttggtt tgattttgat 9780
ataaaccgaa ccaactcggt ccatttgcac ccctaatcat aatagcttta atatttcaag 9840

atattattaa gttaacgttg tcaatatcct ggaaattttg caaaatgaat caagcctata 9900
tggctgtaat atgaatttaa aagcagctcg atgtggtggt aatatgtaat ttacttgatt 9960
ctaaaaaaat atcccaagta ttaataattt ctgctaggaa gaaggttagc tacgatttac 10020
agcaaagcca gaatacaatg aaccataaag tgattgaagc tcgaaatata cgaaggaaca 10080
aatattttta aaaaaatacg caatgacttg gaacaaaaga aagtgatata ttttttgttc 10140
ttaaacaagc atcccctcta aagaatggca gttttccttt gcatgtaact attatgctcc 10200
cttcgttaca aaaattttgg actactattg ggaacttctt ctgaaaatag tggccaccgc 10260
ttaattaagg cgcgccatgc ccgggcaagc ggccgcacaa gtttgtacaa aaaagctgaa 10320
cgagaaacgt aaaatgatat aaatatcaat atattaaatt agattttgca taaaaaacag 10380
actacataat actgtaaaac acaacatatc cagtcactat gaatcaacta cttagatggt 10440
attagtgacc tgtagtcgac cgacagcctt ccaaatgttc ttcgggtgat gctgccaact 10500
tagtcgaccg acagccttcc aaatgttctt ctcaaacgga atcgtcgtat ccagcctact 10560
cgctattgtc ctcaatgccg tattaaatca taaaaagaaa taagaaaaag aggtgcgagc 10620
ctcttttttg tgtgacaaaa taaaaacatc tacctattca tatacgctag tgtcatagtc 10680
ctgaaaatca tctgcatcaa gaacaatttc acaactctta tacttttctc ttacaagtcg 10740
ttcggcttca tctggatttt cagcctctat acttactaaa cgtgataaag tttctgtaat 10800
ttctactgta tcgacctgca gactggctgt gtataaggga gcctgacatt tatattcccc 10860
agaacatcag gttaatggcg tttttgatgt cattttcgcg gtggctgaga tcagccactt 10920
cttccccgat aacggagacc ggcacactgg ccatatcggt ggtcatcatg cgccagcttt 10980
catccccgat atgcaccacc gggtaaagtt cacgggagac tttatctgac agcagacgtg 11040
cactggccag ggggatcacc atccgtcgcc cgggcgtgtc aataatatca ctctgtacat 11100
ccacaaacag acgataacgg ctctctcttt tataggtgta aaccttaaac tgcatttcac 11160
cagcccctgt tctcgtcagc aaaagagccg ttcatttcaa taaaccgggc gacctcagcc 11220
atcccttcct gattttccgc tttccagcgt tcggcacgca gacgacgggc ttcattctgc 11280
atggttgtgc ttaccagacc ggagatattg acatcatata tgccttgagc aactgatagc 11340
tgtcgctgtc aactgtcact gtaatacgct gcttcatagc atacctcttt ttgacatact 11400
tcgggtatac atatcagtat atattcttat accgcaaaaa tcagcgcgca aatacgcata 11460
ctgttatctg gcttttagta agccggatcc acgcggcgtt tacgcccccc ctgccactca 11520
tcgcagtact gttgtaattc attaagcatt ctgccgacat ggaagccatc acaaacggca 11580
tgatgaacct gaatcgccag cggcatcagc accttgtcgc cttgcgtata atatttgccc 11640
atggtgaaaa cgggggcgaa gaagttgtcc atattggcca cgtttaaatc aaaactggtg 11700
aaactcaccc agggattggc tgagacgaaa aacatattct caataaaccc tttagggaaa 11760
taggccaggt tttcaccgta acacgccaca tcttgcgaat atatgtgtag aaactgccgg 11820
aaatcgtcgt ggtattcact ccagagcgat gaaaacgttt cagtttgctc atggaaaacg 11880

gtgtaacaag ggtgaacact atcccatatc accagctcac cgtctttcat tgccatacgg 11940
aattccggat gagcattcat caggcgggca agaatgtgaa taaaggccgg ataaaacttg 12000
tgcttatttt tctttacggt ctttaaaaag gccgtaatat ccagctgaac ggtctggtta 12060
taggtacatt gagcaactga ctgaaatgcc tcaaaatgtt ctttacgatg ccattgggat 12120
atatcaacgg tggtatatcc agtgattttt ttctccattt tagcttcctt agctcctgaa 12180
aatctcgata actcaaaaaa tacgcccggt agtgatctta tttcattatg gtgaaagttg 12240
gaacctctta cgtgccgatc aacgtctcat tttcgccaaa agttggccca gggcttcccg 12300
gtatcaacag ggacaccagg atttatttat tctgcgaagt gatcttccgt cacaggtatt 12360
tattcggcgc aaagtgcgtc gggtgatgct gccaacttag tcgactacag gtcactaata 12420
ccatctaagt agttgattca tagtgactgg atatgttgtg ttttacagta ttatgtagtc 12480
tgttttttat gcaaaatcta atttaatata ttgatattta tatcatttta cgtttctcgt 12540
tcagctttct tgtacaaagt ggttgcggcc gcttaattaa atttaaattc aattaatgca 12600
atcttgattt tcaacaacga aggtaatggc gtaaaagaaa aaatgtatgt tattgtattg 12660
atctttcatg atgttgaagc gtgccataat atgatgatgt ataattaaaa tattaactgt 12720
cgcattttat tgaaatggca ctgttatttc aaccatatct ttgattctgt tacatgacac 12780
gactgcaaga agtaaataat agacgccgtt gttaaagaat tgctatcata tgtgcctaac 12840
tagagggaat ttgagcgtca gacctaatca aatattacaa aatatctcac tctgtcgcca 12900
gcaatggtgt aatcagcgca gacaaatggc gtaaagatcg cggaaaaacc tccccgagtg 12960
gcatgatagc tgcctctgta ttgctgattt agtcagcctt atttgactta agggtgccct 13020
cgttagtgac aaattgcttt caaggagaca gccatgcccc acactttgtt gaaaaacaaa 13080
ttgcctttgg ggagacggta aagccagttg ctcttcaata aggaatgtcg aggaggcaat 13140
gtaaccgcct ctggtagtac acttctctaa tccaaaaatc aatttgtatt caagataccg 13200
caaaaaactt atggtttaaa ccctgca 13227

<210> 54
<211> 10247
<212> DNA
<213> Artificial

<220>
<223> Plasmid pDAB7318

<400> 54

```
cgcgccgacc cagctttctt gtacaaagtt ggcattataa gaaagcattg cttatcaatt     60
tgttgcaacg aacaggtcac tatcagtcaa aataaaatca ttatttgcca tccagctgat    120
atcccctata gtgagtcgta ttacatggtc atagctgttt cctggcagct ctggcccgtg    180
tctcaaaatc tctgatgtta cattgcacaa gataaaaata tatcatcatg aacaataaaa    240
ctgtctgctt acataaacag taatacaagg ggtgttatga gccatattca acgggaaacg    300
tcgaggccgc gattaaattc caacatggat gctgatttat atgggtataa atgggctcgc    360
```

gataatgtcg ggcaatcagg tgcgacaatc tatcgcttgt atgggaagcc cgatgcgcca 420
gagttgtttc tgaaacatgg caaaggtagc gttgccaatg atgttacaga tgagatggtc 480
agactaaact ggctgacgga atttatgcct cttccgacca tcaagcattt tatccgtact 540
cctgatgatg catggttact caccactgcg atccccggaa aaacagcatt ccaggtatta 600
gaagaatatc ctgattcagg tgaaaatatt gttgatgcgc tggcagtgtt cctgcgccgg 660
ttgcattcga ttcctgtttg taattgtcct tttaacagcg atcgcgtatt tcgtctcgct 720
caggcgcaat cacgaatgaa taacggtttg gttgatgcga gtgattttga tgacgagcgt 780
aatggctggc ctgttgaaca agtctggaaa gaaatgcata aacttttgcc attctcaccg 840
gattcagtcg tcactcatgg tgatttctca cttgataacc ttatttttga cgaggggaaa 900
ttaataggtt gtattgatgt tggacgagtc ggaatcgcag accgatacca ggatcttgcc 960
atcctatgga actgcctcgg tgagttttct ccttcattac agaaacggct ttttcaaaaa 1020
tatggtattg ataatcctga tatgaataaa ttgcagtttc atttgatgct cgatgagttt 1080
ttctaatcag aattggttaa ttggttgtaa cactggcaga gcattacgct gacttgacgg 1140
gacggcgcaa gctcatgacc aaaatccctt aacgtgagtt acgcgtcgtt ccactgagcg 1200
tcagaccccg tagaaaagat caaaggatct tcttgagatc ctttttttct gcgcgtaatc 1260
tgctgcttgc aaacaaaaaa accaccgcta ccagcggtgg tttgtttgcc ggatcaagag 1320
ctaccaactc tttttccgaa ggtaactggc ttcagcagag cgcagatacc aaatactgtc 1380
cttctagtgt agccgtagtt aggccaccac ttcaagaact ctgtagcacc gcctacatac 1440
ctcgctctgc taatcctgtt accagtggct gctgccagtg gcgataagtc gtgtcttacc 1500
gggttggact caagacgata gttaccggat aaggcgcagc ggtcgggctg aacggggggt 1560
tcgtgcacac agcccagctt ggagcgaacg acctacaccg aactgagata cctacagcgt 1620
gagcattgag aaagcgccac gcttcccgaa gggagaaagg cggacaggta tccggtaagc 1680
ggcagggtcg gaacaggaga gcgcacgagg gagcttccag ggggaaacgc ctggtatctt 1740
tatagtcctg tcgggtttcg ccacctctga cttgagcgtc gatttttgtg atgctcgtca 1800
ggggggcgga gcctatggaa aaacgccagc aacgcggcct ttttacggtt cctggccttt 1860
tgctggcctt ttgctcacat gttctttcct gcgttatccc ctgattctgt ggataaccgt 1920
attaccgcct ttgagtgagc tgataccgct cgccgcagcc gaacgaccga gcgcagcgag 1980
tcagtgagcg aggaagcgga agagcgccca atacgcaaac cgcctctccc cgcgcgttgg 2040
ccgattcatt aatgcagctg gcacgacagg tttcccgact ggaaagcggg cagtgagcgc 2100
aacgcaatta atacgcgtac cgctagccag gaagagtttg tagaaacgca aaaaggccat 2160
ccgtcaggat ggccttctgc ttagtttgat gcctggcagt ttatggcggg cgtcctgccc 2220
gccaccctcc gggccgttgc ttcacaacgt tcaaatccgc tcccggcgga tttgtcctac 2280
tcaggagagc gttcaccgac aaacaacaga taaaacgaaa ggcccagtct tccgactgag 2340
cctttcgttt tatttgatgc ctggcagttc cctactctcg cgttaacgct agcatggatg 2400

ttttcccagt cacgacgttg taaaacgacg gccagtctta agctcgggcc ccaaataatg 2460
attttatttt gactgatagt gacctgttcg ttgcaacaaa ttgatgagca atgctttttt 2520
ataatgccaa ctttgtacaa aaaagcaggc tccgcggccg cactaggttt aaactctaga 2580
agctaggaat tcaaacaaag aagcgatcgc gcggccgcca ttgtactccc agtatcatta 2640
tagtgaaagt tttggctctc tcgccggtgg ttttttacct ctatttaaag ggttttcca 2700
cctaaaaatt ctggtatcat tctcacttta cttgttactt taatttctca taatctttgg 2760
ttgaaattat cacgcttccg cacacgatat ccctacaaat ttattatttg ttaaacattt 2820
tcaaaccgca taaaatttta tgaagtcccg tctatcttta atgtagtcta acattttcat 2880
attgaaatat ataatttact taattttagc gttggtagaa agcataatga tttattctta 2940
ttcttcttca tataaatgtt taatatacaa tataaacaaa ttctttacct taagaaggat 3000
ttcccatttt atattttaaa aatatattta tcaaatattt ttcaaccacg taaatctcat 3060
aataataagt tgtttcaaaa gtaataaaat ttaactccat aattttttta ttcgactgat 3120
cttaaagcaa cacccagtga cacaactagc catttttttc tttgaataaa aaaatccaat 3180
tatcattgta ttttttttat acaatgaaaa tttcaccaaa caatgatttg tggtatttct 3240
gaagcaagtc atgttatgca aaattctata attcccattt gacactacgg aagtaactga 3300
agatctgctt ttacatgcga gacacatctt ctaaagtaat tttaataata gttactatat 3360
tcaagatttc atatatcaaa tactcaatat tacttctaaa aaattaatta gatataatta 3420
aaatattact tttttaattt taagtttaat tgttgaattt gtgactattg atttattatt 3480
ctactatgtt taaattgttt tatagatagt ttaaagtaaa tataagtaat gtagtagagt 3540
gttagagtgt taccctaaac cataaactat aagatttatg gtggactaat tttcatatat 3600
ttcttattgc ttttaccttt tcttggtatg taagtccgta actggaatta ctgtgggttg 3660
ccatgacact ctgtggtctt ttggttcatg catggatgct tgcgcaagaa aaagacaaag 3720
aacaaagaaa aaagacaaaa cagagagaca aaacgcaatc acacaaccaa ctcaaattag 3780
tcactggctg atcaagatcg ccgcgtccat gtatgtctaa atgccatgca aagcaacacg 3840
tgcttaacat gcactttaaa tggctcaccc atctcaaccc acacacaaac acattgcctt 3900
tttcttcatc atcaccacaa ccacctgtat atattcattc tcttccgcca cctcaatttc 3960
ttcacttcaa cacacgtcaa cctgcatatg cgtgtcatcc catgcccaaa tctccatgca 4020
tgttccaacc accttctctc ttatataata cctataaata cctctaatat cactcacttc 4080
tttcatcatc catccatcca gagtactact actctactac tataataccc caacccaact 4140
catattcaat actactctag gatccaacaa tgtctgctcc aaccgctgac atcagggcta 4200
gggctccaga ggctaagaag gttcacatcg ctgataccgc tatcaacagg cacaattggt 4260
acaagcacgt gaactggctc aacgtcttcc tcatcatcgg aatcccactc tacggatgca 4320
tccaagcttt ctgggttcca cttcaactca agaccgctat ctgggctgtg atctactact 4380
tcttcaccgg acttggaatc accgctggat accacaggct ttgggctcac tgctcatact 4440

ctgctactct tccacttagg atctggcttg ctgctgttgg aggaggagct gttgagggat 4500
ctatcagatg gtgggctagg gatcacaggg ctcatcatag gtacaccgat accgacaagg 4560
acccatactc tgttaggaag ggacttctct actctcacct tggatggatg gtgatgaagc 4620
agaacccaaa gaggatcgga aggaccgaca tctctgatct caacgaggac ccagttgttg 4680
tttggcaaca caggaactac ctcaaggttg tgttcaccat gggacttgct gttccaatgc 4740
ttgttgctgg acttggatgg ggagattggc ttggaggatt cgtgtacgct ggaatcctta 4800
ggatcttctt cgttcaacaa gctaccttct gcgtgaactc tcttgctcac tggcttggag 4860
atcaaccatt cgatgatagg aactctccta gggatcacgt gatcaccgct cttgttaccc 4920
ttggagaggg ataccacaac ttccaccacg agttcccatc tgactacagg aacgctatcg 4980
agtggcacca gtacgatcct accaagtggt ctatctgggc ttggaagcaa cttggattgg 5040
cttacgatct caagaagttc agggctaacg agatcgagaa gggaagggtt caacaacttc 5100
agaagaagct tgataggaag agggctactc ttgattgggg aaccccactt gatcaacttc 5160
cagtgatgga atgggatgac tacgttgagc aagctaagaa cggaagggga cttgttgcta 5220
tcgctggagt tgttcacgat gttaccgact tcatcaagga tcacccagga ggaaaggcta 5280
tgatctcttc tggaatcgga aaggatgcta ccgctatgtt caacggagga gtgtactacc 5340
actctaacgc agctcacaac cttcttagca ccatgagggt gggagtgatc aggggaggat 5400
gcgaggttga gatctggaag agggctcaga aggagaacgt tgagtacgtt agggatggat 5460
ctggacaaag ggtgatcagg gctggagagc aaccaaccaa gatcccagag ccaatcccaa 5520
ccgctgatgc tgcttgagta gttagcttaa tcacctaggt caccagtatg aactaaaatg 5580
catgtaggtg taagagctca tggagagcat ggaatattgt atccgaccat gtaacagtat 5640
aataactgag ctccatctca cttcttctat gaataaacaa aggatgttat gatatattaa 5700
cactctatct atgcacctta ttgttctatg ataaatttcc tcttattatt ataaatcatc 5760
tgaatcgtga cggcttatgg aatgcttcaa atagtacaaa aacaaatgtg tactataaga 5820
ctttctaaac aattctaact ttagcattgt gaacgagaca taagtgttaa gaagacataa 5880
caattataat ggaagaagtt tgtctccatt tatatattat atattaccca cttatgtatt 5940
atattaggat gttaaggaga cataacaatt ataaagagag aagtttgtat ccatttatat 6000
attatatact acccatttat atattatact tatccactta tttaatgtct ttataaggtt 6060
tgatccatga tatttctaat attttagttg atatgtatat gaaaaggtac tatttgaact 6120
ctcttactct gtataaaggt tggatcatcc ttaaagtggg tctatttaat tttattgctt 6180
cttacagata aaaaaaaaat tatgagttgg tttgataaaa tattgaagga tttaaaataa 6240
taataaataa taaataacat ataatatatg tatataaatt tattataata taacatttat 6300
ctataaaaaa gtaaatattg tcataaatct atacaatcgt ttagccttgc tggaacgaat 6360
ctcaattatt taaacgagag taaacatatt tgactttttg gttatttaac aaattattat 6420
ttaacactat atgaaatttt ttttttttat cagcaaagaa taaaattaaa ttaagaagga 6480

caatggtgtc ccaatcctta tacaaccaac ttccacaaga aagtcaagtc agagacaaca 6540
aaaaaacaag caaaggaaat tttttaattt gagttgtctt gtttgctgca taatttatgc 6600
agtaaaacac tacacataac ccttttagca gtagagcaat ggttgaccgt gtgcttagct 6660
tcttttattt tattttttta tcagcaaaga ataaataaaa taaaatgaga cacttcaggg 6720
atgtttcaac ccttatacaa aaccccaaaa acaagtttcc tagcacccta ccaacgaatt 6780
cgcggccgct ttcctgcatg acatcgtcct gcagagccaa gcgcatgctt aattaaacta 6840
gtctcccagt atcattatag tgaaagtttt ggctctctcg ccggtggttt tttacctcta 6900
tttaaagggg ttttccacct aaaaattctg gtatcattct cactttactt gttactttaa 6960
tttctcataa tctttggttg aaattatcac gcttccgcac acgatatccc tacaaattta 7020
ttatttgtta aacattttca aaccgcataa aattttatga agtcccgtct atctttaatg 7080
tagtctaaca ttttcatatt gaaatatata atttacttaa ttttagcgtt ggtagaaagc 7140
ataatgattt attcttattc ttcttcatat aaatgtttaa tatacaatat aaacaaattc 7200
tttaccttaa gaaggatttc ccattttata ttttaaaaat atatttatca aatatttttc 7260
aaccacgtaa atctcataat aataagttgt ttcaaaagta ataaaattta actccataat 7320
ttttttattc gactgatctt aaagcaacac ccagtgacac aactagccat ttttttcttt 7380
gaataaaaaa atccaattat cattgtattt tttttataca atgaaaattt caccaaacaa 7440
tgatttgtgg tatttctgaa gcaagtcatg ttatgcaaaa ttctataatt cccatttgac 7500
actacggaag taactgaaga tctgctttta catgcgagac acatcttcta aagtaatttt 7560
aataatagtt actatattca agatttcata tatcaaatac tcaatattac ttctaaaaaa 7620
ttaattagat ataattaaaa tattactttt ttaattttaa gtttaattgt tgaatttgtg 7680
actattgatt tattattcta ctatgtttaa attgttttat agatagttta aagtaaatat 7740
aagtaatgta gtagagtgtt agagtgttac cctaaaccat aaactataag atttatggtg 7800
gactaatttt catatatttc ttattgcttt taccttttct tggtatgtaa gtccgtaact 7860
ggaattactg tgggttgcca tgacactctg tggtcttttg gttcatgcat ggatcttgcg 7920
caagaaaaag acaaagaaca aagaaaaaag acaaaacaga gagacaaaac gcaatcacac 7980
aaccaactca aattagtcac tggctgatca agatcgccgc gtccatgtat gtctaaatgc 8040
catgcaaagc aacacgtgct taacatgcac tttaaatggc tcacccatct caacccacac 8100
acaaacacat tgcctttttc ttcatcatca ccacaaccac ctgtatatat tcattctctt 8160
ccgccacctc aatttcttca cttcaacaca cgtcaacctg catatgcgtg tcatcccatg 8220
cccaaatctc catgcatgtt ccaaccacct tctctcttat ataataccta taaatacctc 8280
taatatcact cacttctttc atcatccatc catccagagt actactactc tactactata 8340
ataccccaac ccaactcata ttcaatacta ctctaggtac cctgcaggga tccaacaatg 8400
gctgcacttg atagcatccc tgaggacaaa gcaactagct ccaagtcaac ccacatacag 8460
taccaagagg tcacgtttag gaactggtac aagaaaatca actggctcaa cacgaccctt 8520

gttgtcctca ttcctgctct tgggttgtac ttgacgagaa ccacacctct caccagacct 8580
accctcattt ggtctgttct ctactatttc tgtacagcgt ttggcatcac tggtggctac 8640
cacagacttt ggtcccatag gtcttacagt gcgaggttgc cattgagact cttcctggct 8700
ttcactggag ctggtgcgat ccaaggttct gcaagatggt ggtcagccaa tcatagggca 8760
catcaccgtt ggacggacac catgaaggac ccctactctg tgatgagagg actgctgttc 8820
tcccacatag gttggatggt tctcaactct gatccaaagg tcaaaggcag aacagatgtt 8880
tctgatcttg actctgatcc cgtcgttgtg tggcaacaca aacactatgg caagtgtttg 8940
ctctttgccg cttggatctt tccgatgata gtggctgggc tgggttgggg agattggtgg 9000
ggtggacttg tctatgctgg catcatacgt gcctgctttg ttcagcaagc cactttctgt 9060
gtcaactcat tggcacattg gataggtgaa caaccgtttg atgacagacg tactccaagg 9120
gatcatgttc tgactgcgtt ggtcacaatg ggagaaggat accacaactt ccaccatgag 9180
tttccgagtg actacagaaa tgccatcatt tggtatcagt atgaccctac aaagtggctc 9240
atctatctct tcagcttggg tcccttccca ttggcctact ctctcaagac cttccgttcc 9300
aatgagattg agaaaggaag gcttcagcaa cagcaaaagg ctcttgacaa gaaaagaagt 9360
ggtcttgatt ggggacttcc tctcttccag cttccagtga tctcatggga tgactttcaa 9420
gctcgttgca aagaaagtgg agagatgctt gttgctgttg ctggagtgat ccatgatgtc 9480
tcccagttca ttgaagatca tcctggtggg aggagcctca ttagaagtgc tgttgggaaa 9540
gatgggactg gcatgttcaa tggtggagtg tatgaacatt caaacgccgc acacaacttg 9600
ctgagcacaa tgagagttgg agtcttgaga ggtggacaag aagtggaggt ttggaagaaa 9660
cagagggtgg atgttcttgg gaagtcagac attcttcgtc aagtgacaag ggtggagcgt 9720
ctggtggaag gagctgttgc agcgtgatga gtagttagct taatcaccta gagctcggtc 9780
acctcgagta tcaaaatcta tttagaaata cacaatattt tgttgcaggc ttgctggaga 9840
atcgatctgc tatcataaaa attacaaaaa aattttattt gcctcaatta ttttaggatt 9900
ggtattaagg acgcttaaat tatttgtcgg gtcactacgc atcattgtga ttgagaagat 9960
cagcgatacg aaatattcgt agtactatcg ataatttatt tgaaaattca taagaaaagc 10020
aaacgttaca tgaattgatg aaacaataca aagacagata aagccacgca catttaggat 10080
attggccgag attactgaat attgagtaag atcacggaat ttctgacagg agcatgtctt 10140
caattcagcc caaatggcag ttgaaatact caaaccgccc catatgcagg agcggatcat 10200
tcattgtttg tttggttgcc tttgccaaca tgggagtcca aggttgg 10247

<210> 55
<211> 6058
<212> DNA
<213> Artificial

<220>
<223> Plasmid pDAB7320

<400> 55

```
aattcgcggc cgctttcctg catgacatcg tcctgcagag ccaagcgcat gcttaattaa      60
actagtctcc cagtatcatt atagtgaaag ttttggctct ctcgccggtg gtttttttacc     120
tctatttaaa ggggttttcc acctaaaaat tctggtatca ttctcacttt acttgttact     180
ttaatttctc ataatctttg gttgaaatta tcacgcttcc gcacacgata tccctacaaa     240
tttattattt gttaaacatt ttcaaaccgc ataaaatttt atgaagtccc gtctatcttt     300
aatgtagtct aacattttca tattgaaata tataatttac ttaattttag cgttggtaga     360
aagcataatg atttattctt attcttcttc atataaatgt ttaatataca atataaacaa     420
attctttacc ttaagaagga tttcccattt tatattttaa aaatatattt atcaaatatt     480
tttcaaccac gtaaatctca taataataag ttgtttcaaa agtaataaaa tttaactcca     540
taattttttt attcgactga tcttaaagca acacccagtg acacaactag ccattttttt     600
ctttgaataa aaaaatccaa ttatcattgt attttttttta tacaatgaaa atttcaccaa    660
acaatgattt gtggtatttc tgaagcaagt catgttatgc aaaattctat aattcccatt     720
tgacactacg gaagtaactg aagatctgct tttacatgcg agacacatct tctaaagtaa     780
ttttaataat agttactata ttcaagattt catatatcaa atactcaata ttacttctaa     840
aaaattaatt agatataatt aaaatattac ttttttaatt ttaagtttaa ttgttgaatt     900
tgtgactatt gatttattat tctactatgt ttaaattgtt ttatagatag tttaaagtaa     960
atataagtaa tgtagtagag tgttagagtg ttaccctaaa ccataaacta taagatttat    1020
ggtggactaa ttttcatata tttcttattg cttttacctt ttcttggtat gtaagtccgt    1080
aactggaatt actgtgggtt gccatgacac tctgtggtct tttggttcat gcatggatct    1140
tgcgcaagaa aaagacaaag aacaaagaaa aaagacaaaa cagagagaca aaacgcaatc    1200
acacaaccaa ctcaaattag tcactggctg atcaagatcg ccgcgtccat gtatgtctaa    1260
atgccatgca aagcaacacg tgcttaacat gcactttaaa tggctcaccc atctcaaccc    1320
acacacaaac acattgcctt tttcttcatc atcaccacaa ccacctgtat atattcattc    1380
tcttccgcca cctcaatttc ttcacttcaa cacacgtcaa cctgcatatg cgtgtcatcc    1440
catgcccaaa tctccatgca tgttccaacc accttctctc ttatataata cctataaata    1500
cctctaatat cactcacttc tttcatcatc catccatcca gagtactact actctactac    1560
tataataccc caacccaact catattcaat actactctag gtaccctgca gggatccaac    1620
aatggctgca cttgatagca tccctgagga caaagcaact agctccaagt caacccacat    1680
acagtaccaa gaggtcacgt ttaggaactg gtacaagaaa atcaactggc tcaacacgac    1740
ccttgttgtc ctcattcctg ctcttgggtt gtacttgacg agaaccacac ctctcaccag    1800
acctaccctc atttggtctg ttctctacta tttctgtaca gcgtttggca tcactggtgg    1860
ctaccacaga ctttggtccc ataggtctta cagtgcgagg ttgccattga gactcttcct    1920
ggctttcact ggagctggtg cgatccaagg ttctgcaaga tggtggtcag ccaatcatag    1980
ggcacatcac cgttggacgg acaccatgaa ggacccctac tctgtgatga gaggactgct    2040
```

gttctcccac ataggttgga tggttctcaa ctctgatcca aaggtcaaag gcagaacaga 2100
tgtttctgat cttgactctg atcccgtcgt tgtgtggcaa cacaaacact atggcaagtg 2160
tttgctcttt gccgcttgga tctttccgat gatagtggct gggctgggtt ggggagattg 2220
gtggggtgga cttgtctatg ctggcatcat acgtgcctgc tttgttcagc aagccacttt 2280
ctgtgtcaac tcattggcac attggatagg tgaacaaccg tttgatgaca gacgtactcc 2340
aagggatcat gttctgactg cgttggtcac aatgggagaa ggataccaca acttccacca 2400
tgagtttccg agtgactaca gaaatgccat catttggtat cagtatgacc ctacaaagtg 2460
gctcatctat ctcttcagct tgggtccctt cccattggcc tactctctca agaccttccg 2520
ttccaatgag attgagaaag gaaggcttca gcaacagcaa aaggctcttg acaagaaaag 2580
aagtggtctt gattggggac ttcctctctt ccagcttcca gtgatctcat gggatgactt 2640
tcaagctcgt tgcaaagaaa gtggagagat gcttgttgct gttgctggag tgatccatga 2700
tgtctcccag ttcattgaag atcatcctgg tgggaggagc ctcattagaa gtgctgttgg 2760
gaaagatggg actggcatgt tcaatggtgg agtgtatgaa cattcaaacg ccgcacacaa 2820
cttgctgagc acaatgagag ttggagtctt gagaggtgga caagaagtgg aggtttggaa 2880
gaaacagagg gtggatgttc ttgggaagtc agacattctt cgtcaagtga caagggtgga 2940
gcgtctggtg gaaggagctg ttgcagcgtg atgagtagtt agcttaatca cctagagctc 3000
ggtcacctcg agtatcaaaa tctatttaga aatacacaat attttgttgc aggcttgctg 3060
gagaatcgat ctgctatcat aaaaattaca aaaaaatttt atttgcctca attattttag 3120
gattggtatt aaggacgctt aaattatttg tcgggtcact acgcatcatt gtgattgaga 3180
agatcagcga tacgaaatat tcgtagtact atcgataatt tatttgaaaa ttcataagaa 3240
aagcaaacgt tacatgaatt gatgaaacaa tacaaagaca gataaagcca cgcacattta 3300
ggatattggc cgagattact gaatattgag taagatcacg gaatttctga caggagcatg 3360
tcttcaattc agcccaaatg gcagttgaaa tactcaaacc gccccatatg caggagcgga 3420
tcattcattg tttgtttggt tgcctttgcc aacatgggag tccaaggttg gcgcgccgac 3480
ccagctttct tgtacaaagt tggcattata agaaagcatt gcttatcaat ttgttgcaac 3540
gaacaggtca ctatcagtca aaataaaatc attatttgcc atccagctga tatcccctat 3600
agtgagtcgt attacatggt catagctgtt tcctggcagc tctggcccgt gtctcaaaat 3660
ctctgatgtt acattgcaca agataaaaat atatcatcat gaacaataaa actgtctgct 3720
tacataaaca gtaatacaag gggtgttatg agccatattc aacgggaaac gtcgaggccg 3780
cgattaaatt ccaacatgga tgctgattta tatgggtata aatgggctcg cgataatgtc 3840
gggcaatcag gtgcgacaat ctatcgcttg tatgggaagc ccgatgcgcc agagttgttt 3900
ctgaaacatg gcaaaggtag cgttgccaat gatgttacag atgagatggt cagactaaac 3960
tggctgacgg aatttatgcc tcttccgacc atcaagcatt ttatccgtac tcctgatgat 4020
gcatggttac tcaccactgc gatccccgga aaaacagcat tccaggtatt agaagaatat 4080

cctgattcag gtgaaaatat tgttgatgcg ctggcagtgt tcctgcgccg gttgcattcg 4140
attcctgttt gtaattgtcc ttttaacagc gatcgcgtat ttcgtctcgc tcaggcgcaa 4200
tcacgaatga ataacggttt ggttgatgcg agtgattttg atgacgagcg taatggctgg 4260
cctgttgaac aagtctggaa agaaatgcat aaacttttgc cattctcacc ggattcagtc 4320
gtcactcatg gtgatttctc acttgataac cttatttttg acgaggggaa attaataggt 4380
tgtattgatg ttggacgagt cggaatcgca gaccgatacc aggatcttgc catcctatgg 4440
aactgcctcg gtgagttttc tccttcatta cagaaacggc tttttcaaaa atatggtatt 4500
gataatcctg atatgaataa attgcagttt catttgatgc tcgatgagtt tttctaatca 4560
gaattggtta attggttgta acactggcag agcattacgc tgacttgacg ggacggcgca 4620
agctcatgac caaaatccct taacgtgagt tacgcgtcgt tccactgagc gtcagacccc 4680
gtagaaaaga tcaaaggatc ttcttgagat cctttttttc tgcgcgtaat ctgctgcttg 4740
caaacaaaaa aaccaccgct accagcggtg gtttgtttgc cggatcaaga gctaccaact 4800
ctttttccga aggtaactgg cttcagcaga gcgcagatac caaatactgt ccttctagtg 4860
tagccgtagt taggccacca cttcaagaac tctgtagcac cgcctacata cctcgctctg 4920
ctaatcctgt taccagtggc tgctgccagt ggcgataagt cgtgtcttac cgggttggac 4980
tcaagacgat agttaccgga taaggcgcag cggtcgggct gaacgggggg ttcgtgcaca 5040
cagcccagct tggagcgaac gacctacacc gaactgagat acctacagcg tgagcattga 5100
gaaagcgcca cgcttcccga agggagaaag gcggacaggt atccggtaag cggcagggtc 5160
ggaacaggag agcgcacgag ggagcttcca gggggaaacg cctggtatct ttatagtcct 5220
gtcgggtttc gccacctctg acttgagcgt cgatttttgt gatgctcgtc aggggggcgg 5280
agcctatgga aaaacgccag caacgcggcc tttttacggt tcctggcctt ttgctggcct 5340
tttgctcaca tgttctttcc tgcgttatcc cctgattctg tggataaccg tattaccgcc 5400
tttgagtgag ctgataccgc tcgccgcagc cgaacgaccg agcgcagcga gtcagtgagc 5460
gaggaagcgg aagagcgccc aatacgcaaa ccgcctctcc ccgcgcgttg gccgattcat 5520
taatgcagct ggcacgacag gtttcccgac tggaaagcgg gcagtgagcg caacgcaatt 5580
aatacgcgta ccgctagcca ggaagagttt gtagaaacgc aaaaaggcca tccgtcagga 5640
tggccttctg cttagtttga tgcctggcag tttatggcgg gcgtcctgcc cgccaccctc 5700
cgggccgttg cttcacaacg ttcaaatccg ctcccggcgg atttgtccta ctcaggagag 5760
cgttcaccga caaacaacag ataaaacgaa aggcccagtc ttccgactga gcctttcgtt 5820
ttatttgatg cctggcagtt ccctactctc gcgttaacgc tagcatggat gttttcccag 5880
tcacgacgtt gtaaaacgac ggccagtctt aagctcgggc cccaaataat gattttattt 5940
tgactgatag tgacctgttc gttgcaacaa attgatgagc aatgcttttt tataatgcca 6000
actttgtaca aaaaagcagg ctccgcggcc gcactaggtt taaactctag aagctagg 6058

<210> 56

<211> 9956
<212> DNA
<213> Artificial

<220>
<223> Plasmid pDAB7323

<400> 56

```
ctagtctccc agtatcatta tagtgaaagt tttggctctc tcgccggtgg ttttttacct      60
ctatttaaag gggttttcca cctaaaaatt ctggtatcat tctcacttta cttgttactt     120
taatttctca taatctttgg ttgaaattat cacgcttccg cacacgatat ccctacaaat     180
ttattatttg ttaaacattt tcaaaccgca taaaatttta tgaagtcccg tctatcttta     240
atgtagtcta acattttcat attgaaatat ataatttact taattttagc gttggtagaa     300
agcataatga tttattctta ttcttcttca tataaatgtt taatatacaa tataaacaaa     360
ttctttacct taagaaggat ttcccatttt atattttaaa aatatattta tcaaatattt     420
ttcaaccacg taaatctcat aataataagt tgtttcaaaa gtaataaaat ttaactccat     480
aatttttta ttcgactgat cttaaagcaa cacccagtga cacaactagc cattttttc       540
tttgaataaa aaaatccaat tatcattgta ttttttttat acaatgaaaa tttcaccaaa     600
caatgatttg tggtatttct gaagcaagtc atgttatgca aaattctata attcccattt     660
gacactacgg aagtaactga agatctgctt ttacatgcga gacacatctt ctaaagtaat     720
tttaataata gttactatat tcaagatttc atatatcaaa tactcaatat tacttctaaa     780
aaattaatta gatataatta aaatattact tttttaattt taagtttaat tgttgaattt     840
gtgactattg atttattatt ctactatgtt taaattgttt tatagatagt ttaaagtaaa     900
tataagtaat gtagtagagt gttagagtgt taccctaaac cataaactat aagatttatg     960
gtggactaat tttcatatat ttcttattgc ttttaccttt tcttggtatg taagtccgta    1020
actggaatta ctgtgggttg ccatgacact ctgtggtctt ttggttcatg catggatctt    1080
gcgcaagaaa aagacaaaga acaaagaaaa aagacaaaac agagagacaa aacgcaatca    1140
cacaaccaac tcaaattagt cactggctga tcaagatcgc cgcgtccatg tatgtctaaa    1200
tgccatgcaa agcaacacgt gcttaacatg cactttaaat ggctcaccca tctcaaccca    1260
cacacaaaca cattgccttt ttcttcatca tcaccacaac cacctgtata tattcattct    1320
cttccgccac ctcaatttct tcacttcaac acacgtcaac ctgcatatgc gtgtcatccc    1380
atgcccaaat ctccatgcat gttccaacca ccttctctct tatataatac ctataaatac    1440
ctctaatatc actcacttct ttcatcatcc atccatccag agtactacta ctctactact    1500
ataatacccc aacccaactc atattcaata ctactctagg taccctgcag ggatccaaca    1560
atggctccca acatttctga ggatgtcaat ggtgttcttt ttgagtcaga tgcggcaacc    1620
cctgatttgg ctctttccac accacctgtg caaaaagctg acaacagacc caagcaactt    1680
gtgtggagga acattttgct tttcgcttac ttgcacctcg cagctctcta cggaggctat    1740
ttgtttctct tcagtgcaaa atggcagacc gacattttcg cttacattct ttatgtcatc    1800
```

tctggactgg ggataactgc tggggcacat agactctggg ctcacaagtc atacaaagcc 1860
aagtggccac tcagagttat actggtcatc ttcaacacgg ttgcctttca agacgctgct 1920
atggattggg ctcgtgacca tagaatgcat cacaagtaca gcgagaccga cgcggaccca 1980
cacaatgcaa cgagaggttt cttcttctct cacattggct ggcttcttgt taggaaacat 2040
cctgatctga aagaaaaagg gaagggactc gacatgagtg atctccttgc tgatccaata 2100
ctccgttttc agaagaagta ctatctgatc ctcatgcctc tggcctgttt tgtgatgcca 2160
accgttatcc cggtttactt ttggggagaa acttggacaa atgctttctt cgtggcagcc 2220
atgttccgtt atgctttcat cctgaatgtt acctggttgg tgaactctgc cgcacacaag 2280
tggggagaca aaccctatga caagtccatc aagccttccg aaaacctttc agttgcgatg 2340
tttgctttgg gagaaggatt tcacaattac catcacactt ttccgtggga ctacaagaca 2400
gcagagcttg gaaacaacaa gttgaacttc acaacaacgt tcatcaattt ctttgcgaaa 2460
atcggttggg cctatgattt gaagactgtg agtgatgaca ttgtcaagaa cagggtcaag 2520
agaactggcg atggaagcca tcatctctgg ggctggggtg atgagaatca gagcaaagaa 2580
gagatagatg cagccattag gatcaaccct aaagacgatt gagtagttag cttaatcacc 2640
tagagctcgg tcacctcgag tatcaaaatc tatttagaaa tacacaatat tttgttgcag 2700
gcttgctgga gaatcgatct gctatcataa aaattacaaa aaaattttat ttgcctcaat 2760
tattttagga ttggtattaa ggacgcttaa attatttgtc gggtcactac gcatcattgt 2820
gattgagaag atcagcgata cgaaatattc gtagtactat cgataattta tttgaaaatt 2880
cataagaaaa gcaaacgtta catgaattga tgaaacaata caaagacaga taaagccacg 2940
cacatttagg atattggccg agattactga atattgagta agatcacgga atttctgaca 3000
ggagcatgtc ttcaattcag cccaaatggc agttgaaata ctcaaaccgc cccatatgca 3060
ggagcggatc attcattgtt tgtttggttg cctttgccaa catgggagtc caaggttggc 3120
gcgccgaccc agctttcttg tacaaagttg gcattataag aaagcattgc ttatcaattt 3180
gttgcaacga acaggtcact atcagtcaaa ataaaatcat tatttgccat ccagctgata 3240
tcccctatag tgagtcgtat tacatggtca tagctgtttc ctggcagctc tggcccgtgt 3300
ctcaaaatct ctgatgttac attgcacaag ataaaaatat atcatcatga acaataaaac 3360
tgtctgctta cataaacagt aatacaaggg gtgttatgag ccatattcaa cgggaaacgt 3420
cgaggccgcg attaaattcc aacatggatg ctgatttata tgggtataaa tgggctcgcg 3480
ataatgtcgg gcaatcaggt gcgacaatct atcgcttgta tgggaagccc gatgcgccag 3540
agttgtttct gaaacatggc aaaggtagcg ttgccaatga tgttacagat gagatggtca 3600
gactaaactg gctgacggaa tttatgcctc ttccgaccat caagcatttt atccgtactc 3660
ctgatgatgc atggttactc accactgcga tccccggaaa aacagcattc caggtattag 3720
aagaatatcc tgattcaggt gaaaatattg ttgatgcgct ggcagtgttc ctgcgccggt 3780
tgcattcgat tcctgtttgt aattgtcctt ttaacagcga tcgcgtattt cgtctcgctc 3840

aggcgcaatc acgaatgaat aacggtttgg ttgatgcgag tgattttgat gacgagcgta 3900
atggctggcc tgttgaacaa gtctggaaag aaatgcataa acttttgcca ttctcaccgg 3960
attcagtcgt cactcatggt gatttctcac ttgataacct tatttttgac gaggggaaat 4020
taataggttg tattgatgtt ggacgagtcg gaatcgcaga ccgataccag gatcttgcca 4080
tcctatggaa ctgcctcggt gagttttctc cttcattaca gaaacggctt tttcaaaaat 4140
atggtattga taatcctgat atgaataaat tgcagtttca tttgatgctc gatgagtttt 4200
tctaatcaga attggttaat tggttgtaac actggcagag cattacgctg acttgacggg 4260
acggcgcaag ctcatgacca aaatccctta acgtgagtta cgcgtcgttc cactgagcgt 4320
cagaccccgt agaaaagatc aaaggatctt cttgagatcc tttttttctg cgcgtaatct 4380
gctgcttgca aacaaaaaaa ccaccgctac cagcggtggt ttgtttgccg gatcaagagc 4440
taccaactct ttttccgaag gtaactggct tcagcagagc gcagatacca aatactgtcc 4500
ttctagtgta gccgtagtta ggccaccact tcaagaactc tgtagcaccg cctacatacc 4560
tcgctctgct aatcctgtta ccagtggctg ctgccagtgg cgataagtcg tgtcttaccg 4620
ggttggactc aagacgatag ttaccggata aggcgcagcg gtcgggctga acggggggtt 4680
cgtgcacaca gcccagcttg gagcgaacga cctacaccga actgagatac ctacagcgtg 4740
agcattgaga aagcgccacg cttcccgaag ggagaaaggc ggacaggtat ccggtaagcg 4800
gcagggtcgg aacaggagag cgcacgaggg agcttccagg gggaaacgcc tggtatcttt 4860
atagtcctgt cgggtttcgc cacctctgac ttgagcgtcg atttttgtga tgctcgtcag 4920
gggggcggag cctatggaaa aacgccagca acgcggcctt tttacggttc ctggcctttt 4980
gctggccttt tgctcacatg ttctttcctg cgttatcccc tgattctgtg gataaccgta 5040
ttaccgcctt tgagtgagct gataccgctc gccgcagccg aacgaccgag cgcagcgagt 5100
cagtgagcga ggaagcggaa gagcgcccaa tacgcaaacc gcctctcccc gcgcgttggc 5160
cgattcatta atgcagctgg cacgacaggt ttcccgactg gaaagcgggc agtgagcgca 5220
acgcaattaa tacgcgtacc gctagccagg aagagtttgt agaaacgcaa aaaggccatc 5280
cgtcaggatg gccttctgct tagtttgatg cctggcagtt tatggcgggc gtcctgcccg 5340
ccaccctccg ggccgttgct tcacaacgtt caaatccgct cccggcggat ttgtcctact 5400
caggagagcg ttcaccgaca aacaacagat aaaacgaaag gcccagtctt ccgactgagc 5460
ctttcgtttt atttgatgcc tggcagttcc ctactctcgc gttaacgcta gcatggatgt 5520
tttcccagtc acgacgttgt aaaacgacgg ccagtcttaa gctcgggccc caaataatga 5580
ttttattttg actgatagtg acctgttcgt tgcaacaaat tgatgagcaa tgctttttta 5640
taatgccaac tttgtacaaa aaagcaggct ccgcggccgc actaggttta aactctagaa 5700
gctaggaatt caaacaaaga agcgatcgcg cggccgccat tgtactccca gtatcattat 5760
agtgaaagtt ttggctctct cgccggtggt tttttacctc tatttaaagg ggttttccac 5820
ctaaaaattc tggtatcatt ctcactttac ttgttacttt aatttctcat aatctttggt 5880

tgaaattatc acgcttccgc acacgatatc cctacaaatt tattatttgt taaacatttt 5940
caaaccgcat aaaattttat gaagtcccgt ctatctttaa tgtagtctaa cattttcata 6000
ttgaaatata taatttactt aattttagcg ttggtagaaa gcataatgat ttattcttat 6060
tcttcttcat ataaatgttt aatatacaat ataaacaaat tctttacctt aagaaggatt 6120
tcccatttta tattttaaaa atatatttat caaatatttt tcaaccacgt aaatctcata 6180
ataataagtt gtttcaaaag taataaaatt taactccata atttttttat tcgactgatc 6240
ttaaagcaac acccagtgac acaactagcc atttttttct ttgaataaaa aaatccaatt 6300
atcattgtat tttttttata caatgaaaat ttcaccaaac aatgatttgt ggtatttctg 6360
aagcaagtca tgttatgcaa aattctataa ttcccatttg acactacgga agtaactgaa 6420
gatctgcttt tacatgcgag acacatcttc taaagtaatt ttaataatag ttactatatt 6480
caagatttca tatatcaaat actcaatatt acttctaaaa aattaattag atataattaa 6540
aatattactt ttttaatttt aagtttaatt gttgaatttg tgactattga tttattattc 6600
tactatgttt aaattgtttt atagatagtt taaagtaaat ataagtaatg tagtagagtg 6660
ttagagtgtt accctaaacc ataaactata agatttatgg tggactaatt ttcatatatt 6720
tcttattgct tttacctttt cttggtatgt aagtccgtaa ctggaattac tgtgggttgc 6780
catgacactc tgtggtcttt tggttcatgc atggatgctt gcgcaagaaa aagacaaaga 6840
acaaagaaaa aagacaaaac agagagacaa aacgcaatca cacaaccaac tcaaattagt 6900
cactggctga tcaagatcgc cgcgtccatg tatgtctaaa tgccatgcaa agcaacacgt 6960
gcttaacatg cactttaaat ggctcaccca tctcaaccca cacacaaaca cattgccttt 7020
ttcttcatca tcaccacaac cacctgtata tattcattct cttccgccac ctcaatttct 7080
tcacttcaac acacgtcaac ctgcatatgc gtgtcatccc atgcccaaat ctccatgcat 7140
gttccaacca ccttctctct tatataatac ctataaatac ctctaatatc actcacttct 7200
ttcatcatcc atccatccag agtactacta ctctactact ataataccccc aacccaactc 7260
atattcaata ctactctagg atccaacaat gtctgctcca accgctgaca tcagggctag 7320
ggctccagag gctaagaagg ttcacatcgc tgataccgct atcaacaggc acaattggta 7380
caagcacgtg aactggctca acgtcttcct catcatcgga atcccactct acggatgcat 7440
ccaagctttc tgggttccac ttcaactcaa gaccgctatc tgggctgtga tctactactt 7500
cttcaccgga cttggaatca ccgctggata ccacaggctt tgggctcact gctcatactc 7560
tgctactctt ccacttagga tctggcttgc tgctgttgga ggaggagctg ttgagggatc 7620
tatcagatgg tgggctaggg atcacagggc tcatcatagg tacaccgata ccgacaagga 7680
cccatactct gttaggaagg gacttctcta ctctcacctt ggatggatgg tgatgaagca 7740
gaacccaaag aggatcggaa ggaccgacat ctctgatctc aacgaggacc cagttgttgt 7800
ttggcaacac aggaactacc tcaaggttgt gttcaccatg ggacttgctg ttccaatgct 7860
tgttgctgga cttggatggg gagattggct tggaggattc gtgtacgctg gaatccttag 7920

```
gatcttcttc gttcaacaag ctaccttctg cgtgaactct cttgctcact ggcttggaga    7980
tcaaccattc gatgatagga actctcctag ggatcacgtg atcaccgctc ttgttaccct    8040
tggagaggga taccacaact tccaccacga gttcccatct gactacagga acgctatcga    8100
gtggcaccag tacgatccta ccaagtggtc tatctgggct tggaagcaac ttggattggc    8160
ttacgatctc aagaagttca gggctaacga gatcgagaag ggaagggttc aacaacttca    8220
gaagaagctt gataggaaga gggctactct tgattgggga accccacttg atcaacttcc    8280
agtgatggaa tgggatgact acgttgagca agctaagaac ggaaggggac ttgttgctat    8340
cgctggagtt gttcacgatg ttaccgactt catcaaggat cacccaggag gaaaggctat    8400
gatctcttct ggaatcggaa aggatgctac cgctatgttc aacggaggag tgtactacca    8460
ctctaacgca gctcacaacc ttcttagcac catgagggtg ggagtgatca ggggaggatg    8520
cgaggttgag atctggaaga gggctcagaa ggagaacgtt gagtacgtta gggatggatc    8580
tggacaaagg gtgatcaggg ctggagagca accaaccaag atcccagagc caatcccaac    8640
cgctgatgct gcttgagtag ttagcttaat cacctaggtc accagtatga actaaaatgc    8700
atgtaggtgt aagagctcat ggagagcatg gaatattgta tccgaccatg taacagtata    8760
ataactgagc tccatctcac ttcttctatg aataaacaaa ggatgttatg atatattaac    8820
actctatcta tgcaccttat tgttctatga taaatttcct cttattatta taaatcatct    8880
gaatcgtgac ggcttatgga atgcttcaaa tagtacaaaa acaaatgtgt actataagac    8940
tttctaaaca attctaactt tagcattgtg aacgagacat aagtgttaag aagacataac    9000
aattataatg gaagaagttt gtctccattt atatattata tattacccac ttatgtatta    9060
tattaggatg ttaaggagac ataacaatta taaagagaga agtttgtatc catttatata    9120
ttatatacta cccatttata tattatactt atccacttat ttaatgtctt tataaggttt    9180
gatccatgat atttctaata ttttagttga tatgtatatg aaaaggtact atttgaactc    9240
tcttactctg tataaaggtt ggatcatcct taaagtgggt ctatttaatt ttattgcttc    9300
ttacagataa aaaaaaaatt atgagttggt ttgataaaat attgaaggat ttaaaataat    9360
aataaataat aaataacata taatatatgt atataaattt attataatat aacatttatc    9420
tataaaaaag taaatattgt cataaatcta tacaatcgtt tagccttgct ggaacgaatc    9480
tcaattattt aaacgagagt aaacatattt gactttttgg ttatttaaca aattattatt    9540
taacactata tgaaattttt ttttttatc agcaaagaat aaaattaaat taagaaggac    9600
aatggtgtcc caatccttat acaaccaact tccacaagaa agtcaagtca gagacaacaa    9660
aaaaacaagc aaaggaaatt ttttaatttg agttgtcttg tttgctgcat aatttatgca    9720
gtaaaacact acacataacc cttttagcag tagagcaatg gttgaccgtg tgcttagctt    9780
cttttatttt attttttat cagcaaagaa taaataaaat aaaatgagac acttcaggga    9840
tgtttcaacc cttatacaaa accccaaaaa caagtttcct agcaccctac caacgaattc    9900
gcggccgctt tcctgcatga catcgtcctg cagagccaag cgcatgctta attaaa        9956
```

<210> 57
<211> 5767
<212> DNA
<213> Artificial

<220>
<223> Plasmid pDAB7325

<400> 57

```
aattcgcggc cgctttcctg catgacatcg tcctgcagag ccaagcgcat gcttaattaa      60
actagtctcc cagtatcatt atagtgaaag ttttggctct ctcgccggtg gtttttacc      120
tctatttaaa ggggttttcc acctaaaaat tctggtatca ttctcacttt acttgttact     180
ttaatttctc ataatctttg gttgaaatta tcacgcttcc gcacacgata tccctacaaa     240
tttattattt gttaaacatt ttcaaaccgc ataaaatttt atgaagtccc gtctatcttt     300
aatgtagtct aacattttca tattgaaata tataatttac ttaattttag cgttggtaga     360
aagcataatg atttattctt attcttcttc atataaatgt ttaatataca atataaacaa     420
attctttacc ttaagaagga tttcccattt tatattttaa aaatatattt atcaaatatt     480
tttcaaccac gtaaatctca taataataag ttgtttcaaa agtaataaaa tttaactcca     540
taattttttt attcgactga tcttaaagca acacccagtg acacaactag ccattttttt     600
ctttgaataa aaaaatccaa ttatcattgt attttttttta tacaatgaaa atttcaccaa    660
acaatgattt gtggtatttc tgaagcaagt catgttatgc aaaattctat aattcccatt     720
tgacactacg gaagtaactg aagatctgct tttacatgcg agacacatct tctaaagtaa     780
ttttaataat agttactata ttcaagattt catatatcaa atactcaata ttacttctaa     840
aaaattaatt agatataatt aaaatattac ttttttaatt ttaagtttaa ttgttgaatt     900
tgtgactatt gatttattat tctactatgt ttaaattgtt ttatagatag tttaaagtaa     960
atataagtaa tgtagtagag tgttagagtg ttaccctaaa ccataaacta taagatttat    1020
ggtggactaa ttttcatata tttcttattg cttttacctt ttcttggtat gtaagtccgt    1080
aactggaatt actgtgggtt gccatgacac tctgtggtct tttggttcat gcatggatct    1140
tgcgcaagaa aaagacaaag aacaaagaaa aaagacaaaa cagagagaca aaacgcaatc    1200
acacaaccaa ctcaaattag tcactggctg atcaagatcg ccgcgtccat gtatgtctaa    1260
atgccatgca aagcaacacg tgcttaacat gcactttaaa tggctcaccc atctcaaccc    1320
acacacaaac acattgcctt tttcttcatc atcaccacaa ccacctgtat atattcattc    1380
tcttccgcca cctcaatttc ttcacttcaa cacacgtcaa cctgcatatg cgtgtcatcc    1440
catgcccaaa tctccatgca tgttccaacc accttctctc ttatataata cctataaata    1500
cctctaatat cactcacttc tttcatcatc catccatcca gagtactact actctactac    1560
tataataccc caacccaact catattcaat actactctag gtaccctgca gggatccaac    1620
aatggctccc aacatttctg aggatgtcaa tggtgttctt tttgagtcag atgcggcaac    1680
ccctgatttg gctctttcca caccacctgt gcaaaaagct gacaacagac ccaagcaact    1740
```

tgtgtggagg aacattttgc ttttcgctta cttgcacctc gcagctctct acggaggcta 1800
tttgtttctc ttcagtgcaa aatggcagac cgacattttc gcttacattc tttatgtcat 1860
ctctggactg gggataactg ctggggcaca tagactctgg gctcacaagt catacaaagc 1920
caagtggcca ctcagagtta tactggtcat cttcaacacg gttgcctttc aagacgctgc 1980
tatggattgg gctcgtgacc atagaatgca tcacaagtac agcgagaccg acgcggaccc 2040
acacaatgca acgagaggtt tcttcttctc tcacattggc tggcttcttg ttaggaaaca 2100
tcctgatctg aaagaaaaag ggaagggact cgacatgagt gatctccttg ctgatccaat 2160
actccgtttt cagaagaagt actatctgat cctcatgcct ctggcctgtt ttgtgatgcc 2220
aaccgttatc ccggtttact tttggggaga aacttggaca aatgctttct tcgtggcagc 2280
catgttccgt tatgctttca tcctgaatgt tacctggttg gtgaactctg ccgcacacaa 2340
gtggggagac aaaccctatg acaagtccat caagccttcc gaaaaccttt cagttgcgat 2400
gtttgctttg ggagaaggat ttcacaatta ccatcacact tttccgtggg actacaagac 2460
agcagagctt ggaaacaaca agttgaactt cacaacaacg ttcatcaatt tctttgcgaa 2520
aatcggttgg gcctatgatt tgaagactgt gagtgatgac attgtcaaga acagggtcaa 2580
gagaactggc gatggaagcc atcatctctg gggctggggt gatgagaatc agagcaaaga 2640
agagatagat gcagccatta ggatcaaccc taaagacgat tgagtagtta gcttaatcac 2700
ctagagctcg gtcacctcga gtatcaaaat ctatttagaa atacacaata ttttgttgca 2760
ggcttgctgg agaatcgatc tgctatcata aaaattacaa aaaaatttta tttgcctcaa 2820
ttattttagg attggtatta aggacgctta aattatttgt cgggtcacta cgcatcattg 2880
tgattgagaa gatcagcgat acgaaatatt cgtagtacta tcgataattt atttgaaaat 2940
tcataagaaa agcaaacgtt acatgaattg atgaaacaat acaaagacag ataaagccac 3000
gcacatttag gatattggcc gagattactg aatattgagt aagatcacgg aatttctgac 3060
aggagcatgt cttcaattca gcccaaatgg cagttgaaat actcaaaccg ccccatatgc 3120
aggagcggat cattcattgt ttgtttggtt gcctttgcca acatgggagt ccaaggttgg 3180
cgcgccgacc cagctttctt gtacaaagtt ggcattataa gaaagcattg cttatcaatt 3240
tgttgcaacg aacaggtcac tatcagtcaa aataaaatca ttatttgcca tccagctgat 3300
atcccctata gtgagtcgta ttacatggtc atagctgttt cctggcagct ctggcccgtg 3360
tctcaaaatc tctgatgtta cattgcacaa gataaaaata tatcatcatg aacaataaaa 3420
ctgtctgctt acataaacag taatacaagg ggtgttatga gccatattca acgggaaacg 3480
tcgaggccgc gattaaattc caacatggat gctgatttat atgggtataa atgggctcgc 3540
gataatgtcg ggcaatcagg tgcgacaatc tatcgcttgt atgggaagcc cgatgcgcca 3600
gagttgtttc tgaaacatgg caaaggtagc gttgccaatg atgttacaga tgagatggtc 3660
agactaaact ggctgacgga atttatgcct cttccgacca tcaagcattt tatccgtact 3720
cctgatgatg catggttact caccactgcg atccccggaa aaacagcatt ccaggtatta 3780

gaagaatatc ctgattcagg tgaaaatatt gttgatgcgc tggcagtgtt cctgcgccgg 3840
ttgcattcga ttcctgtttg taattgtcct tttaacagcg atcgcgtatt tcgtctcgct 3900
caggcgcaat cacgaatgaa taacggtttg gttgatgcga gtgattttga tgacgagcgt 3960
aatggctggc ctgttgaaca agtctggaaa gaaatgcata aacttttgcc attctcaccg 4020
gattcagtcg tcactcatgg tgatttctca cttgataacc ttatttttga cgaggggaaa 4080
ttaataggtt gtattgatgt tggacgagtc ggaatcgcag accgatacca ggatcttgcc 4140
atcctatgga actgcctcgg tgagttttct ccttcattac agaaacggct ttttcaaaaa 4200
tatggtattg ataatcctga tatgaataaa ttgcagtttc atttgatgct cgatgagttt 4260
ttctaatcag aattggttaa ttggttgtaa cactggcaga gcattacgct gacttgacgg 4320
gacggcgcaa gctcatgacc aaaatccctt aacgtgagtt acgcgtcgtt ccactgagcg 4380
tcagaccccg tagaaaagat caaaggatct tcttgagatc ctttttttct gcgcgtaatc 4440
tgctgcttgc aaacaaaaaa accaccgcta ccagcggtgg tttgtttgcc ggatcaagag 4500
ctaccaactc tttttccgaa ggtaactggc ttcagcagag cgcagatacc aaatactgtc 4560
cttctagtgt agccgtagtt aggccaccac ttcaagaact ctgtagcacc gcctacatac 4620
ctcgctctgc taatcctgtt accagtggct gctgccagtg gcgataagtc gtgtcttacc 4680
gggttggact caagacgata gttaccggat aaggcgcagc ggtcgggctg aacggggggt 4740
tcgtgcacac agcccagctt ggagcgaacg acctacaccg aactgagata cctacagcgt 4800
gagcattgag aaagcgccac gcttcccgaa gggagaaagg cggacaggta tccggtaagc 4860
ggcagggtcg gaacaggaga gcgcacgagg gagcttccag ggggaaacgc ctggtatctt 4920
tatagtcctg tcgggtttcg ccacctctga cttgagcgtc gatttttgtg atgctcgtca 4980
ggggggcgga gcctatggaa aaacgccagc aacgcggcct ttttacggtt cctggccttt 5040
tgctggcctt ttgctcacat gttctttcct gcgttatccc ctgattctgt ggataaccgt 5100
attaccgcct ttgagtgagc tgataccgct cgccgcagcc gaacgaccga gcgcagcgag 5160
tcagtgagcg aggaagcgga agagcgccca atacgcaaac cgcctctccc cgcgcgttgg 5220
ccgattcatt aatgcagctg gcacgacagg tttcccgact ggaaagcggg cagtgagcgc 5280
aacgcaatta atacgcgtac cgctagccag gaagagtttg tagaaacgca aaaaggccat 5340
ccgtcaggat ggccttctgc ttagtttgat gcctggcagt ttatggcggg cgtcctgccc 5400
gccaccctcc gggccgttgc ttcacaacgt tcaaatccgc tcccggcgga tttgtcctac 5460
tcaggagagc gttcaccgac aaacaacaga taaaacgaaa ggcccagtct tccgactgag 5520
cctttcgttt tatttgatgc ctggcagttc cctactctcg cgttaacgct agcatggatg 5580
ttttcccagt cacgacgttg taaaacgacg gccagtctta agctcgggcc ccaaataatg 5640
attttatttt gactgatagt gacctgttcg ttgcaacaaa ttgatgagca atgctttttt 5700
ataatgccaa ctttgtacaa aaaagcaggc tccgcggccg cactaggttt aaactctaga 5760
agctagg 5767

<210> 58
<211> 6109
<212> DNA
<213> Artificial

<220>
<223> Plasmid pDAB7327

<400> 58

```
cggtcacctc gagtatcaaa atctatttag aaatacacaa tattttgttg caggcttgct      60
ggagaatcga tctgctatca taaaaattac aaaaaaattt tatttgcctc aattatttta     120
ggattggtat taaggacgct taaattattt gtcgggtcac tacgcatcat tgtgattgag     180
aagatcagcg atacgaaata ttcgtagtac tatcgataat ttatttgaaa attcataaga     240
aaagcaaacg ttacatgaat tgatgaaaca atacaaagac agataaagcc acgcacattt     300
aggatattgg ccgagattac tgaatattga gtaagatcac ggaatttctg acaggagcat     360
gtcttcaatt cagcccaaat ggcagttgaa atactcaaac cgccccatat gcaggagcgg     420
atcattcatt gtttgtttgg ttgcctttgc caacatggga gtccaaggtt ggcgcgccga     480
cccagctttc ttgtacaaag ttggcattat aagaaagcat tgcttatcaa tttgttgcaa     540
cgaacaggtc actatcagtc aaaataaaat cattatttgc catccagctg atatccccta     600
tagtgagtcg tattacatgg tcatagctgt ttcctggcag ctctggcccg tgtctcaaaa     660
tctctgatgt tacattgcac aagataaaaa tatatcatca tgaacaataa aactgtctgc     720
ttacataaac agtaatacaa ggggtgttat gagccatatt caacgggaaa cgtcgaggcc     780
gcgattaaat tccaacatgg atgctgattt atatgggtat aaatgggctc gcgataatgt     840
cgggcaatca ggtgcgacaa tctatcgctt gtatgggaag cccgatgcgc cagagttgtt     900
tctgaaacat ggcaaaggta gcgttgccaa tgatgttaca gatgagatgg tcagactaaa     960
ctggctgacg gaatttatgc ctcttccgac catcaagcat tttatccgta ctcctgatga    1020
tgcatggtta ctcaccactg cgatccccgg aaaaacagca ttccaggtat tagaagaata    1080
tcctgattca ggtgaaaata ttgttgatgc gctggcagtg ttcctgcgcc ggttgcattc    1140
gattcctgtt tgtaattgtc cttttaacag cgatcgcgta tttcgtctcg ctcaggcgca    1200
atcacgaatg aataacggtt tggttgatgc gagtgatttt gatgacgagc gtaatggctg    1260
gcctgttgaa caagtctgga aagaaatgca taaacttttg ccattctcac cggattcagt    1320
cgtcactcat ggtgatttct cacttgataa ccttattttt gacgagggga aattaatagg    1380
ttgtattgat gttggacgag tcggaatcgc agaccgatac caggatcttg ccatcctatg    1440
gaactgcctc ggtgagtttt ctccttcatt acagaaacgg ctttttcaaa aatatggtat    1500
tgataatcct gatatgaata aattgcagtt tcatttgatg ctcgatgagt ttttctaatc    1560
agaattggtt aattggttgt aacactggca gagcattacg ctgacttgac gggacggcgc    1620
aagctcatga ccaaaatccc ttaacgtgag ttacgcgtcg ttccactgag cgtcagaccc    1680
cgtagaaaag atcaaaggat cttcttgaga tccttttttt ctgcgcgtaa tctgctgctt    1740
```

gcaaacaaaa aaaccaccgc taccagcggt ggtttgtttg ccggatcaag agctaccaac 1800
tctttttccg aaggtaactg gcttcagcag agcgcagata ccaaatactg tccttctagt 1860
gtagccgtag ttaggccacc acttcaagaa ctctgtagca ccgcctacat acctcgctct 1920
gctaatcctg ttaccagtgg ctgctgccag tggcgataag tcgtgtctta ccgggttgga 1980
ctcaagacga tagttaccgg ataaggcgca gcggtcgggc tgaacggggg gttcgtgcac 2040
acagcccagc ttggagcgaa cgacctacac cgaactgaga tacctacagc gtgagcattg 2100
agaaagcgcc acgcttcccg aagggagaaa ggcggacagg tatccggtaa gcggcagggt 2160
cggaacagga gagcgcacga gggagcttcc agggggaaac gcctggtatc tttatagtcc 2220
tgtcgggttt cgccacctct gacttgagcg tcgatttttg tgatgctcgt caggggggcg 2280
gagcctatgg aaaaacgcca gcaacgcggc ctttttacgg ttcctggcct tttgctggcc 2340
ttttgctcac atgttctttc ctgcgttatc ccctgattct gtggataacc gtattaccgc 2400
ctttgagtga gctgataccg ctcgccgcag ccgaacgacc gagcgcagcg agtcagtgag 2460
cgaggaagcg gaagagcgcc caatacgcaa accgcctctc cccgcgcgtt ggccgattca 2520
ttaatgcagc tggcacgaca ggtttcccga ctggaaagcg ggcagtgagc gcaacgcaat 2580
taatacgcgt accgctagcc aggaagagtt tgtagaaacg caaaaaggcc atccgtcagg 2640
atggccttct gcttagtttg atgcctggca gtttatggcg ggcgtcctgc ccgccaccct 2700
ccgggccgtt gcttcacaac gttcaaatcc gctcccggcg gatttgtcct actcaggaga 2760
gcgttcaccg acaaacaaca gataaaacga aaggcccagt cttccgactg agcctttcgt 2820
tttatttgat gcctggcagt tccctactct cgcgttaacg ctagcatgga tgttttccca 2880
gtcacgacgt tgtaaaacga cggccagtct taagctcggg ccccaaataa tgattttatt 2940
ttgactgata gtgacctgtt cgttgcaaca aattgatgag caatgctttt ttataatgcc 3000
aactttgtac aaaaaagcag gctccgcggc cgcactaggt ttaaactcta gaagctagga 3060
attcgcggcc gctttcctgc atgacatcgt cctgcagagc caagcgcatg cttaattaaa 3120
ctagtctccc agtatcatta tagtgaaagt tttggctctc tcgccggtgg ttttttacct 3180
ctatttaaag ggttttccca cctaaaaatt ctggtatcat tctcacttta cttgttactt 3240
taatttctca taatctttgg ttgaaattat cacgcttccg cacacgatat ccctacaaat 3300
ttattatttg ttaaacattt tcaaaccgca taaaatttta tgaagtcccg tctatcttta 3360
atgtagtcta acattttcat attgaaatat ataatttact taattttagc gttggtagaa 3420
agcataatga tttattctta ttcttcttca tataaatgtt taatatacaa tataaacaaa 3480
ttctttacct taagaaggat ttcccatttt atattttaaa aatatattta tcaaatattt 3540
ttcaaccacg taaatctcat aataataagt tgtttcaaaa gtaataaaat ttaactccat 3600
aatttttttta ttcgactgat cttaaagcaa cacccagtga cacaactagc cattttttttc 3660
tttgaataaa aaaatccaat tatcattgta ttttttttat acaatgaaaa tttcaccaaa 3720
caatgatttg tggtatttct gaagcaagtc atgttatgca aaattctata attcccattt 3780

```
gacactacgg aagtaactga agatctgctt ttacatgcga gacacatctt ctaaagtaat 3840
tttaataata gttactatat tcaagatttc atatatcaaa tactcaatat tacttctaaa 3900
aaattaatta gatataatta aaatattact tttttaattt taagtttaat tgttgaattt 3960
gtgactattg atttattatt ctactatgtt taaattgttt tatagatagt ttaaagtaaa 4020
tataagtaat gtagtagagt gttagagtgt taccctaaac cataaactat aagatttatg 4080
gtggactaat tttcatatat ttcttattgc ttttaccttt tcttggtatg taagtccgta 4140
actggaatta ctgtgggttg ccatgacact ctgtggtctt ttggttcatg catggatctt 4200
gcgcaagaaa aagacaaaga acaaagaaaa aagacaaaac agagagacaa aacgcaatca 4260
cacaaccaac tcaaattagt cactggctga tcaagatcgc cgcgtccatg tatgtctaaa 4320
tgccatgcaa agcaacacgt gcttaacatg cactttaaat ggctcaccca tctcaaccca 4380
cacacaaaca cattgccttt ttcttcatca tcaccacaac cacctgtata tattcattct 4440
cttccgccac ctcaatttct tcacttcaac acacgtcaac ctgcatatgc gtgtcatccc 4500
atgcccaaat ctccatgcat gttccaacca ccttctctct tatataatac ctataaatac 4560
ctctaatatc actcacttct ttcatcatcc atccatccag agtactacta ctctactact 4620
ataatacccc aacccaactc atattcaata ctactctagg taccctgcag ggatccaaca 4680
atgtctgctc caaccgctga catcagggct agggctccag aggctaagaa ggttcacatc 4740
gctgataccg ctatcaacag gcacaattgg tacaagcacg tgaactggct caacgtcttc 4800
ctcatcatcg gaatcccact ctacggatgc atccaagctt tctgggttcc acttcaactc 4860
aagaccgcta tctgggctgt gatctactac ttcttcaccg gacttggaat caccgctgga 4920
taccacaggc tttgggctca ctgctcatac tctgctactc ttccacttag gatctggctt 4980
gctgctgttg gaggaggagc tgttgaggga tctatcagat ggtgggctag ggatcacagg 5040
gctcatcata ggtacaccga taccgacaag gacccatact ctgttaggaa gggacttctc 5100
tactctcacc ttggatggat ggtgatgaag cagaacccaa agaggatcgg aaggaccgac 5160
atctctgatc tcaacgagga cccagttgtt gtttggcaac acaggaacta cctcaaggtt 5220
gtgttcacca tgggacttgc tgttccaatg cttgttgctg gacttggatg gggagattgg 5280
cttggaggat tcgtgtacgc tggaatcctt aggatcttct tcgttcaaca agctaccttc 5340
tgcgtgaact ctcttgctca ctggcttgga gatcaaccat tcgatgatag gaactctcct 5400
agggatcacg tgatcaccgc tcttgttacc cttggagagg gataccacaa cttccaccac 5460
gagttcccat ctgactacag gaacgctatc gagtggcacc agtacgatcc taccaagtgg 5520
tctatctggg cttggaagca acttggattg gcttacgatc tcaagaagtt cagggctaac 5580
gagatcgaga agggaagggt tcaacaactt cagaagaagc ttgataggaa gagggctact 5640
cttgattggg gaaccccact tgatcaactt ccagtgatgg aatgggatga ctacgttgag 5700
caagctaaga acggaagggg acttgttgct atcgctggag ttgttcacga tgttaccgac 5760
ttcatcaagg atcacccagg aggaaaggct atgatctctt ctggaatcgg aaaggatgct 5820
```

accgctatgt tcaacggagg agtgtactac cactctaacg cagctcacaa ccttcttagc 5880
accatgaggg tgggagtgat caggggagga tgcgaggttg agatctggaa gagggctcag 5940
aaggagaacg ttgagtacgt tagggatgga tctggacaaa gggtgatcag ggctggagag 6000
caaccaacca agatcccaga gccaatccca accgctgatg ctgcttgagt agttagctta 6060
atcacctagg tcaccagtat gaactaaaat gcatgtaggt gtaagagct 6109

<210> 59
<211> 6136
<212> DNA
<213> Artificial

<220>
<223> Plasmid pDAB7329

<400> 59

```
cggtcacctc gagtatcaaa atctatttag aaatacacaa tattttgttg caggcttgct     60
ggagaatcga tctgctatca taaaaattac aaaaaaattt tatttgcctc aattatttta    120
ggattggtat taaggacgct taaattattt gtcgggtcac tacgcatcat tgtgattgag    180
aagatcagcg atacgaaata ttcgtagtac tatcgataat ttatttgaaa attcataaga    240
aaagcaaacg ttacatgaat tgatgaaaca atacaaagac agataaagcc acgcacattt    300
aggatattgg ccgagattac tgaatattga gtaagatcac ggaatttctg acaggagcat    360
gtcttcaatt cagcccaaat ggcagttgaa atactcaaac cgccccatat gcaggagcgg    420
atcattcatt gtttgtttgg ttgcctttgc caacatggga gtccaaggtt ggcgcgccga    480
cccagctttc ttgtacaaag ttggcattat aagaaagcat tgcttatcaa tttgttgcaa    540
cgaacaggtc actatcagtc aaaataaaat cattatttgc catccagctg atatccccta    600
tagtgagtcg tattacatgg tcatagctgt ttcctggcag ctctggcccg tgtctcaaaa    660
tctctgatgt tacattgcac aagataaaaa tatatcatca tgaacaataa aactgtctgc    720
ttacataaac agtaatacaa ggggtgttat gagccatatt caacgggaaa cgtcgaggcc    780
gcgattaaat tccaacatgg atgctgattt atatgggtat aaatgggctc gcgataatgt    840
cgggcaatca ggtgcgacaa tctatcgctt gtatgggaag cccgatgcgc cagagttgtt    900
tctgaaacat ggcaaaggta gcgttgccaa tgatgttaca gatgagatgg tcagactaaa    960
ctggctgacg gaatttatgc ctcttccgac catcaagcat tttatccgta ctcctgatga   1020
tgcatggtta ctcaccactg cgatccccgg aaaaacagca ttccaggtat tagaagaata   1080
tcctgattca ggtgaaaata ttgttgatgc gctggcagtg ttcctgcgcc ggttgcattc   1140
gattcctgtt tgtaattgtc cttttaacag cgatcgcgta tttcgtctcg ctcaggcgca   1200
atcacgaatg aataacggtt tggttgatgc gagtgatttt gatgacgagc gtaatggctg   1260
gcctgttgaa caagtctgga aagaaatgca taaacttttg ccattctcac cggattcagt   1320
cgtcactcat ggtgatttct cacttgataa ccttattttt gacgagggga aattaatagg   1380
ttgtattgat gttggacgag tcggaatcgc agaccgatac caggatcttg ccatcctatg   1440
```

gaactgcctc ggtgagtttt ctccttcatt acagaaacgg ctttttcaaa aatatggtat 1500
tgataatcct gatatgaata aattgcagtt tcatttgatg ctcgatgagt ttttctaatc 1560
agaattggtt aattggttgt aacactggca gagcattacg ctgacttgac gggacggcgc 1620
aagctcatga ccaaaatccc ttaacgtgag ttacgcgtcg ttccactgag cgtcagaccc 1680
cgtagaaaag atcaaaggat cttcttgaga tccttttttt ctgcgcgtaa tctgctgctt 1740
gcaaacaaaa aaaccaccgc taccagcggt ggtttgtttg ccggatcaag agctaccaac 1800
tctttttccg aaggtaactg gcttcagcag agcgcagata ccaaatactg tccttctagt 1860
gtagccgtag ttaggccacc acttcaagaa ctctgtagca ccgcctacat acctcgctct 1920
gctaatcctg ttaccagtgg ctgctgccag tggcgataag tcgtgtctta ccgggttgga 1980
ctcaagacga tagttaccgg ataaggcgca gcggtcgggc tgaacggggg gttcgtgcac 2040
acagcccagc ttggagcgaa cgacctacac cgaactgaga tacctacagc gtgagcattg 2100
agaaagcgcc acgcttcccg aagggagaaa ggcggacagg tatccggtaa gcggcagggt 2160
cggaacagga gagcgcacga gggagcttcc agggggaaac gcctggtatc tttatagtcc 2220
tgtcgggttt cgccacctct gacttgagcg tcgatttttg tgatgctcgt caggggggcg 2280
gagcctatgg aaaaacgcca gcaacgcggc ctttttacgg ttcctggcct tttgctggcc 2340
ttttgctcac atgttctttc ctgcgttatc ccctgattct gtggataacc gtattaccgc 2400
ctttgagtga gctgataccg ctcgccgcag ccgaacgacc gagcgcagcg agtcagtgag 2460
cgaggaagcg gaagagcgcc caatacgcaa accgcctctc cccgcgcgtt ggccgattca 2520
ttaatgcagc tggcacgaca ggtttcccga ctggaaagcg ggcagtgagc gcaacgcaat 2580
taatacgcgt accgctagcc aggaagagtt tgtagaaacg caaaaaggcc atccgtcagg 2640
atggccttct gcttagtttg atgcctggca gtttatggcg ggcgtcctgc ccgccaccct 2700
ccgggccgtt gcttcacaac gttcaaatcc gctcccggcg gatttgtcct actcaggaga 2760
gcgttcaccg acaaacaaca gataaaacga aaggcccagt cttccgactg agcctttcgt 2820
tttatttgat gcctggcagt tccctactct cgcgttaacg ctagcatgga tgttttccca 2880
gtcacgacgt tgtaaaacga cggccagtct taagctcggg ccccaaataa tgattttatt 2940
ttgactgata gtgacctgtt cgttgcaaca aattgatgag caatgctttt ttataatgcc 3000
aactttgtac aaaaaagcag gctccgcggc cgcactaggt ttaaactcta gaagctagga 3060
attcgcggcc gctttcctgc atgacatcgt cctgcagagc caagcgcatg cttaattaaa 3120
ctagtctccc agtatcatta tagtgaaagt tttggctctc tcgccggtgg ttttttacct 3180
ctatttaaag ggttttcca cctaaaaatt ctggtatcat tctcacttta cttgttactt 3240
taatttctca taatctttgg ttgaaattat cacgcttccg cacacgatat ccctacaaat 3300
ttattatttg ttaaacattt tcaaaccgca taaaatttta tgaagtcccg tctatcttta 3360
atgtagtcta acattttcat attgaaatat ataatttact taattttagc gttggtagaa 3420
agcataatga tttattctta ttcttcttca tataaatgtt taatatacaa tataaacaaa 3480

ttctttacct taagaaggat ttcccatttt atattttaaa aatatattta tcaaatattt 3540
ttcaaccacg taaatctcat aataataagt tgtttcaaaa gtaataaaat ttaactccat 3600
aatttttta ttcgactgat cttaaagcaa cacccagtga cacaactagc catttttttc 3660
tttgaataaa aaaatccaat tatcattgta tttttttat acaatgaaaa tttcaccaaa 3720
caatgatttg tggtatttct gaagcaagtc atgttatgca aaattctata attcccattt 3780
gacactacgg aagtaactga agatctgctt ttacatgcga gacacatctt ctaaagtaat 3840
tttaataata gttactatat tcaagatttc atatatcaaa tactcaatat tacttctaaa 3900
aaattaatta gatataatta aaatattact tttttaattt taagtttaat tgttgaattt 3960
gtgactattg atttattatt ctactatgtt taaattgttt tatagatagt ttaaagtaaa 4020
tataagtaat gtagtagagt gttagagtgt taccctaaac cataaactat aagatttatg 4080
gtggactaat tttcatatat ttcttattgc ttttaccttt tcttggtatg taagtccgta 4140
actggaatta ctgtgggttg ccatgacact ctgtggtctt ttggttcatg catggatctt 4200
gcgcaagaaa aagacaaaga acaaagaaaa aagacaaaac agagagacaa aacgcaatca 4260
cacaaccaac tcaaattagt cactggctga tcaagatcgc cgcgtccatg tatgtctaaa 4320
tgccatgcaa agcaacacgt gcttaacatg cactttaaat ggctcaccca tctcaaccca 4380
cacacaaaca cattgccttt ttcttcatca tcaccacaac cacctgtata tattcattct 4440
cttccgccac ctcaatttct tcacttcaac acacgtcaac ctgcatatgc gtgtcatccc 4500
atgcccaaat ctccatgcat gttccaacca ccttctctct tatataatac ctataaatac 4560
ctctaatatc actcacttct ttcatcatcc atccatccag agtactacta ctctactact 4620
ataataccccc aacccaactc atattcaata ctactctagg taccctgcag ggatccaaca 4680
atggccagca gttcttcaag tgtgccagaa cttgccgcag ctttccctga tgggacaacg 4740
gacttcaaac ccatgaggaa caccaaaggc tatgatgtct ccaaacctca catctctgaa 4800
acaccgatga ctttgaagaa ctggcacaaa catgtgaact ggctcaacac cacattcatt 4860
ctctttgttc cactggctgg gttgatctca acctattggg ttcctcttca atggaaaact 4920
gcagtgtggg cagttgtgta ctacttcaac actggacttg ggatcactgc tggctaccat 4980
agattgtggg cacattcctc ttacaaggcc agcttgcctc tcaaaatcta ccttgccgca 5040
gttggtgctg gagccgttga aggttccata agatggtgga gcaacggaca cagagcacat 5100
cacagataca cagacacaga gaaagatcct tactcagtga ggaagggatt gctctacagc 5160
cacatgggtt ggatgctctt gaagcagaat ccaaagaagc aagggaggac ggacattact 5220
gatctgaatg aggacccagt tgtggtctgg caacatagga actttctcaa gtgtgtgatc 5280
ttcatggctt tggtctttcc caccccttgtt gctggcctgg gatggggaga ctactgggga 5340
ggtttcatct atggagggat cttgagagtg ttctttgttc agcaagccac cttctgtgtc 5400
aactcacttg cacattggct tggtgatcaa ccgtttgatg acagaaactc tccacgtgac 5460
catgtcataa ctgctcttgt cacgctgggt gaaggctatc acaactttca ccatgagttt 5520

```
ccgtcagact atagaaatgc gattgagtgg tatcagtatg accccacgaa gtggagcatt    5580
tggatttgga agcaacttgg acttgctcac aatctcaagc agttcagaca gaatgagata    5640
gagaagggaa gggttcaaca gttgcagaag aaactggatc agaagagagc gaaacttgat    5700
tggggaatac cgttggaaca actccctgtt gtgtcttggg atgactttgt tgaacagtca    5760
aagaatggca aggcatggat tgctgttgct ggtgtcattc acgatgttgg tgacttcatc    5820
aaggatcatc ctggtggacg tgctctcatc aactctgcga ttggcaaaga tgccacagcg    5880
atcttcaatg gaggtgtcta caatcattca aatgccgcac acaaccttct ctccaccatg    5940
agggttggtg tcctccgtgg agggtgcgaa gtggagatat ggaaacgtgc tcaaagtgag    6000
aacaaagatg tctctactgt ggttgatagt tctggcaacc gtattgtgag agctggtgga    6060
caagctacca aagtggttca gccagtccct ggtgctcaag cagcttgatg agtagttagc    6120
ttaatcacct agagct                                                    6136
```

<210> 60
<211> 18713
<212> DNA
<213> Artificial

<220>
<223> Plasmid pDAB7319

<400> 60

```
ttgtacaaag tggttgcggc cgcttaatta aatttaaatt caattaatgc aatcttgatt      60
ttcaacaacg aaggtaatgg cgtaaaagaa aaaatgtatg ttattgtatt gatctttcat     120
gatgttgaag cgtgccataa tatgatgatg tataattaaa atattaactg tcgcatttta     180
ttgaaatggc actgttattt caaccatatc tttgattctg ttacatgaca cgactgcaag     240
aagtaaataa tagacgccgt tgttaaagaa ttgctatcat atgtgcctaa ctagagggaa     300
tttgagcgtc agacctaatc aaatattaca aaatatctca ctctgtcgcc agcaatggtg     360
taatcagcgc agacaaatgg cgtaaagatc gcggaaaaac ctccccgagt ggcatgatag     420
ctgcctctgt attgctgatt tagtcagcct tatttgactt aagggtgccc tcgttagtga     480
caaattgctt tcaaggagac agccatgccc cacactttgt tgaaaaacaa attgcctttg     540
gggagacggt aaagccagtt gctcttcaat aaggaatgtc gaggaggcaa tgtaaccgcc     600
tctggtagta cacttctcta atccaaaaat caatttgtat tcaagatacc gcaaaaaact     660
tatggtttaa accctgcagg actagtccag aaggtaatta tccaagatgt agcatcaaga     720
atccaatgtt tacgggaaaa actatggaag tattatgtaa gctcagcaag aagcagatca     780
atatgcggca catatgcaac ctatgttcaa aaatgaagaa tgtacagata caagatccta     840
tactgccaga atacgaagaa gaatacgtag aaattgaaaa agaagaacca ggcgaagaaa     900
agaatcttga agacgtaagc actgacgaca acaatgaaaa gaagaagata aggtcggtga     960
ttgtgaaaga gacatagagg acacatgtaa ggtggaaaat gtaagggcgg aaagtaacct    1020
tatcacaaag gaatcttatc ccccactact tatcctttta tatttttccg tgtcattttt    1080
```

```
gcccttgagt tttcctatat aaggaaccaa gttcggcatt tgtgaaaaca agaaaaaatt   1140
tggtgtaagc tattttcttt gaagtactga ggatacaact tcagagaaat ttgtaagttt   1200
gtaggtacca gatctggatc ccaaaccatg tctccggaga ggagaccagt tgagattagg   1260
ccagctacag cagctgatat ggccgcggtt tgtgatatcg ttaaccatta cattgagacg   1320
tctacagtga actttaggac agagccacaa acaccacaag agtggattga tgatctagag   1380
aggttgcaag atagataccc ttggttggtt gctgaggttg agggtgttgt ggctggtatt   1440
gcttacgctg ggccctggaa ggctaggaac gcttacgatt ggacagttga gagtactgtt   1500
tacgtgtcac ataggcatca aaggttgggc ctaggatcta cattgtacac acatttgctt   1560
aagtctatgg aggcgcaagg ttttaagtct gtggttgctg ttataggcct tccaaacgat   1620
ccatctgtta ggttgcatga ggctttggga tacacagccc ggggtacatt gcgcgcagct   1680
ggatacaagc atggtggatg gcatgatgtt ggtttttggc aaagggattt tgagttgcca   1740
gctcctccaa ggccagttag gccagttacc caaatctgag tagttagctt aatcacctag   1800
agctcgatcg gcggcaatag cttcttagcg ccatcccggg ttgatcctat ctgtgttgaa   1860
atagttgcgg tgggcaaggc tctctttcag aaagacaggc ggccaaagga acccaaggtg   1920
aggtgggcta tggctctcag ttccttgtgg aagcgcttgg tctaaggtgc agaggtgtta   1980
gcgggatgaa gcaaaagtgt ccgattgtaa caagatatgt tgatcctacg taaggatatt   2040
aaagtatgta ttcatcacta atataatcag tgtattccaa tatgtactac gatttccaat   2100
gtctttattg tcgccgtatg taatcggcgt cacaaaataa tccccggtga ctttctttta   2160
atccaggatg aaataatatg ttattataat ttttgcgatt tggtccgtta taggaattga   2220
agtgtgcttg aggtcggtcg ccaccactcc catttcataa ttttacatgt atttgaaaaa   2280
taaaaattta tggtattcaa tttaaacacg tatacttgta aagaatgata tcttgaaaga   2340
aatatagttt aaatatttat tgataaaata acaagtcagg tattatagtc caagcaaaaa   2400
cataaattta ttgatgcaag tttaaattca gaaatatttc aataactgat tatatcagct   2460
ggtacattgc cgtagatgaa agactgagtg cgatattatg gtgtaataca taggaattcg   2520
tttaaacgat ctgcgtctaa ttttcggtcc aacttgcaca ggaaagacgt cgaccgcggt   2580
agctcttgcc cagcagactg ggcttccagt cctttcgctc gatcgggtcc aatgttgtcc   2640
tcagctgtga accggaagcg gacgaccaac agtggaagaa ctgaaaggaa cgagccgtct   2700
ataccttgat gatcggcctc tggtgaaggg tatcatcgca gccaagcaag ctcatgaaag   2760
gctgatgggg gaggtgtata attatgaggc ccacggcggg cttattcttt agggaggatc   2820
tatctcgttg ctcaagtgca tggcgcaaag cagttattgg agtgcggatt ttcgttggca   2880
tattattcgc cacgagttag cagacgaaga gaccttcatg aacgtggcca aggccagagt   2940
taagcagatg ttacgccctg ctgcaggcct ttctattatc caatagttgg ttgatctttg   3000
gaaagagcct cggctgaggc ccatactgaa agagatcgat ggatatcgat atgccatgtt   3060
gtttgctagc cagaaccaga tcacatccga tatgctattg cagcttgacg cagatatgga   3120
```

ggataagttg attcatggga tcgctcagga gtagctcatc catgcacgcc gacaagaaca 3180
gaaattccgt cgagttaacg cagccgctta cgacggattc gaaggtcatc cattcggaat 3240
gtattagttt gcaccagctc cgcgtcacac ctgtcttcat ttgaataaga tgttagcaat 3300
tgtttttagc tttgtcttgt tgtggcaggg cggcaagtgc ttcagacatc attctgtttt 3360
caaattttat gctggagaac agcttcttaa ttcctttgga aataatagac tgcgtcttaa 3420
aattcagatg tctggatata gatatgattg taaaataacc tatttaagtg tcatttagaa 3480
cataagtttt atgaatgttc ttccattttc gtcatcgaac gaataagagt aaatacacct 3540
tttttaacat tacaaataag ttcttatacg ttgtttatac accgggaatc atttccatta 3600
ttttcgcgca aaagtcacgg atattcgtga aagcgacata aactgcgaaa tttgcgggga 3660
gtgtcttgag tttgcctcga ggctagcgca tgcacataga cacacacatc atctcattga 3720
tgcttggtaa taattgtcat tagattgttt ttatgcatag atgcactcga aatcagccaa 3780
ttttagacaa gtatcaaacg gatgtgactt cagtacatta aaaacgtccg caatgtgtta 3840
ttaagttgtc taagcgtcaa tttgatttac aattgaatat atcctgcccc agccagccaa 3900
cagctcgatt tacaattgaa tatatcctgc cggccggccc acgcgtgtcg aggaattctg 3960
atctggcccc catttggacg tgaatgtaga cacgtcgaaa taaagatttc cgaattagaa 4020
taatttgttt attgctttcg cctataaata cgacggatcg taatttgtcg ttttatcaaa 4080
atgtactttc attttataat aacgctgcgg acatctacat ttttgaattg aaaaaaaatt 4140
ggtaattact ctttcttttt ctccatattg accatcatac tcattgctga tccatgtaga 4200
tttcccggac atgaagccat ttacaattga atatatcctg ccgccgctgc cgctttgcac 4260
ccggtggagc ttgcatgttg gtttctacgc agaactgagc cggttaggca gataatttcc 4320
attgagaact gagccatgtg caccttcccc ccaacacggt gagcgacggg gcaacggagt 4380
gatccacatg ggacttttaa acatcatccg tcggatggcg ttgcgagaga agcagtcgat 4440
ccgtgagatc agccgacgca ccgggcaggc gcgcaacacg atcgcaaagt atttgaacgc 4500
aggtacaatc gagccgacgt tcacgcggaa cgaccaagca agcttggctg ccatttttgg 4560
ggtgaggccg ttcgcggccg aggggcgcag cccctggggg gatgggaggc ccgcgttagc 4620
gggccgggag ggttcgagaa ggggggcac ccccttcgg cgtgcgcggt cacgcgcaca 4680
gggcgcagcc ctggttaaaa acaaggttta taaatattgg tttaaaagca ggttaaaaga 4740
caggttagcg gtggccgaaa aacgggcgga aacccttgca aatgctggat tttctgcctg 4800
tggacagccc ctcaaatgtc aataggtgcg cccctcatct gtcagcactc tgcccctcaa 4860
gtgtcaagga tcgcgcccct catctgtcag tagtcgcgcc cctcaagtgt caataccgca 4920
gggcacttat ccccaggctt gtccacatca tctgtgggaa actcgcgtaa aatcaggcgt 4980
tttcgccgat ttgcgaggct ggccagctcc acgtcgccgg ccgaaatcga gcctgcccct 5040
catctgtcaa cgccgcgccg ggtgagtcgg cccctcaagt gtcaacgtcc gcccctcatc 5100
tgtcagtgag ggccaagttt tccgcgaggt atccacaacg ccggcggccg cggtgtctcg 5160

cacacggctt cgacggcgtt tctggcgcgt ttgcagggcc atagacggcc gccagcccag 5220
cggcgagggc aaccagcccg gtgagcgtcg gaaagggtcg acggatcttt tccgctgcat 5280
aaccctgctt cggggtcatt atagcgattt tttcggtata tccatccttt ttcgcacgat 5340
atacaggatt ttgccaaagg gttcgtgtag actttccttg gtgtatccaa cggcgtcagc 5400
cgggcaggat aggtgaagta ggcccacccg cgagcgggtg ttccttcttc actgtccctt 5460
attcgcacct ggcggtgctc aacgggaatc ctgctctgcg aggctggccg gctaccgccg 5520
gcgtaacaga tgagggcaag cggatggctg atgaaaccaa gccaaccagg aagggcagcc 5580
cacctatcaa ggtgtactgc cttccagacg aacgaagagc gattgaggaa aaggcggcgg 5640
cggccggcat gagcctgtcg gcctacctgc tggccgtcgg ccagggctac aaaatcacgg 5700
gcgtcgtgga ctatgagcac gtccgcgagc tggcccgcat caatggcgac ctgggccgcc 5760
tgggcggcct gctgaaactc tggctcaccg acgacccgcg cacggcgcgg ttcggtgatg 5820
ccacgatcct cgccctgctg gcgaagatcg aagagaagca ggacgagctt ggcaaggtca 5880
tgatgggcgt ggtccgcccg agggcagagc catgactttt ttagccgcta aaacggccgg 5940
ggggtgcgcg tgattgccaa gcacgtcccc atgcgctcca tcaagaagag cgacttcgcg 6000
gagctggtat tcgtgcaggg caagattcgg aataccaagt acgagaagga cggccagacg 6060
gtctacggga ccgacttcat tgccgataag gtggattatc tggacaccaa ggcaccaggc 6120
gggtcaaatc aggaataagg gcacattgcc ccggcgtgag tcggggcaat cccgcaagga 6180
gggtgaatga atcggacgtt tgaccggaag gcatacaggc aagaactgat cgacgcgggg 6240
ttttccgccg aggatgccga aaccatcgca agccgcaccg tcatgcgtgc gccccgcgaa 6300
accttccagt ccgtcggctc gatggtccag caagctacgg ccaagatcga gcgcgacagc 6360
gtgcaactgg ctcccccctgc cctgcccgcg ccatcggccg ccgtggagcg ttcgcgtcgt 6420
ctcgaacagg aggcggcagg tttggcgaag tcgatgacca tcgacacgcg aggaactatg 6480
acgaccaaga agcgaaaaac cgccggcgag gacctggcaa aacaggtcag cgaggccaag 6540
caggccgcgt tgctgaaaca cacgaagcag cagatcaagg aaatgcagct ttccttgttc 6600
gatattgcgc cgtggccgga cacgatgcga gcgatgccaa acgacacggc ccgctctgcc 6660
ctgttcacca cgcgcaacaa gaaaatcccg cgcgaggcgc tgcaaaacaa ggtcattttc 6720
cacgtcaaca aggacgtgaa gatcacctac accggcgtcg agctgcgggc cgacgatgac 6780
gaactggtgt ggcagcaggt gttggagtac gcgaagcgca cccctatcgg cgagccgatc 6840
accttcacgt tctacgagct ttgccaggac ctgggctggt cgatcaatgg ccggtattac 6900
acgaaggccg aggaatgcct gtcgcgccta caggcgacgg cgatgggctt cacgtccgac 6960
cgcgttgggc acctggaatc ggtgtcgctg ctgcaccgct tccgcgtcct ggaccgtggc 7020
aagaaaacgt cccgttgcca ggtcctgatc gacgaggaaa tcgtcgtgct gtttgctggc 7080
gaccactaca cgaaattcat atgggagaag taccgcaagc tgtcgccgac ggcccgacgg 7140
atgttcgact atttcagctc gcaccgggag ccgtacccgc tcaagctgga aaccttccgc 7200

ctcatgtgcg gatcggattc cacccgcgtg aagaagtggc gcgagcaggt cggcgaagcc 7260
tgcgaagagt tgcgaggcag cggcctggtg gaacacgcct gggtcaatga tgacctggtg 7320
cattgcaaac gctagggcct tgtggggtca gttccggctg gggggttcagc agccagcgct 7380
ttactggcat ttcaggaaca agcgggcact gctcgacgca cttgcttcgc tcagtatcgc 7440
tcgggacgca cggcgcgctc tacgaactgc cgataaacag aggattaaaa ttgacaattg 7500
tgattaaggc tcagattcga cggcttggag cggccgacgt gcaggatttc cgcgagatcc 7560
gattgtcggc cctgaagaaa gctccagaga tgttcgggtc cgtttacgag cacgaggaga 7620
aaaagcccat ggaggcgttc gctgaacggt tgcgagatgc cgtggcattc ggcgcctaca 7680
tcgacggcga gatcattggg ctgtcggtct tcaaacagga ggacggcccc aaggacgctc 7740
acaaggcgca tctgtccggc gttttcgtgg agcccgaaca gcgaggccga ggggtcgccg 7800
gtatgctgct gcgggcgttg ccggcgggtt tattgctcgt gatgatcgtc cgacagattc 7860
caacgggaat ctggtggatg cgcatcttca tcctcggcgc acttaatatt tcgctattct 7920
ggagcttgtt gtttatttcg gtctaccgcc tgccgggcgg ggtcgcggcg acggtaggcg 7980
ctgtgcagcc gctgatggtc gtgttcatct ctgccgctct gctaggtagc ccgatacgat 8040
tgatggcggt cctgggggct atttgcggaa ctgcgggcgt ggcgctgttg gtgttgacac 8100
caaacgcagc gctagatcct gtcggcgtcg cagcgggcct ggcgggggcg gtttccatgg 8160
cgttcggaac cgtgctgacc cgcaagtggc aacctcccgt gcctctgctc acctttaccg 8220
cctggcaact ggcggccgga ggacttctgc tcgttccagt agctttagtg tttgatccgc 8280
caatcccgat gcctacagga accaatgttc tcggcctggc gtggctcggc ctgatcggag 8340
cgggtttaac ctacttcctt tggttccggg ggatctcgcg actcgaacct acagttgttt 8400
ccttactggg ctttctcagc cccgagcgc ttagtgggaa tttgtacccc ttatcgaacc 8460
gggagcacag gatgacgcct aacaattcat tcaagccgac accgcttcgc ggcgcggctt 8520
aattcaggag ttaaacatca tgagggaagc ggtgatcgcc gaagtatcga ctcaactatc 8580
agaggtagtt ggcgtcatcg agcgccatct cgaaccgacg ttgctggccg tacatttgta 8640
cggctccgca gtggatggcg gcctgaagcc acacagtgat attgatttgc tggttacggt 8700
gaccgtaagg cttgatgaaa caacgcggcg agctttgatc aacgaccttt tggaaacttc 8760
ggcttcccct ggagagagcg agattctccg cgctgtagaa gtcaccattg ttgtgcacga 8820
cgacatcatt ccgtggcgtt atccagctaa gcgcgaactg caatttggag aatggcagcg 8880
caatgacatt cttgcaggta tcttcgagcc agccacgatc gacattgatc tggctatctt 8940
gctgacaaaa gcaagagaac atagcgttgc cttggtaggt ccagcggcgg aggaactctt 9000
tgatccggtt cctgaacagg atctatttga ggcgctaaat gaaaccttaa cgctatggaa 9060
ctcgccgccc gactgggctg gcgatgagcg aaatgtagtg cttacgttgt cccgcatttg 9120
gtacagcgca gtaaccggca aaatcgcgcc gaaggatgtc gctgccgact gggcaatgga 9180
gcgcctgccg gcccagtatc agcccgtcat acttgaagct aggcaggctt atcttggaca 9240

agaagatcgc ttggcctcgc gcgcagatca gttggaagaa tttgttcact acgtgaaagg 9300
cgagatcacc aaggtagtcg gcaaataatg tctaacaatt cgttcaagcc gacgccgctt 9360
cgcggcgcgg cttaactcaa gcgttagaga gctggggaag actatgcgcg atctgttgaa 9420
ggtggttcta agcctcgtct tgcgatggca tttcgatcca ttcccattcc gcgctcaaga 9480
tggcttcccc tcggcagttc atcagggcta aatcaatcta gccgacttgt ccggtgaaat 9540
gggctgcact ccaacagaaa caatcaaaca aacatacaca gcgacttatt cacacgagct 9600
caaattacaa cggtatatat cctgccagtc agcatcatca caccaaaagt taggcccgaa 9660
tagtttgaaa ttagaaagct cgcaattgag gtctacaggc caaattcgct cttagccgta 9720
caatattact caccggatcc taaccggtgt gatcatgggc cgcgattaaa aatctcaatt 9780
atatttggtc taatttagtt tggtattgag taaaacaaat tcgaaccaaa ccaaaatata 9840
aatatatagt ttttatatat atgcctttaa gactttttat agaattttct ttaaaaaata 9900
tctagaaata tttgcgactc ttctggcatg taatatttcg ttaaatatga agtgctccat 9960
ttttattaac tttaaataat tggttgtacg atcactttct tatcaagtgt tactaaaatg 10020
cgtcaatctc tttgttcttc catattcata tgtcaaaacc tatcaaaatt cttatatatc 10080
tttttcgaat ttgaagtgaa atttcgataa tttaaaatta aatagaacat atcattattt 10140
aggtatcata ttgattttta tacttaatta ctaaatttgg ttaactttga aagtgtacat 10200
caacgaaaaa ttagtcaaac gactaaaata aataaatatc atgtgttatt aagaaaattc 10260
tcctataaga atattttaat agatcatatg tttgtaaaaa aaattaattt ttactaacac 10320
atatatttac ttatcaaaaa tttgacaaag taagattaaa ataatattca tctaacaaaa 10380
aaaaaaccag aaaatgctga aaacccggca aaaccgaacc aatccaaacc gatatagttg 10440
gtttggtttg attttgatat aaaccgaacc aactcggtcc atttgcaccc ctaatcataa 10500
tagctttaat atttcaagat attattaagt taacgttgtc aatatcctgg aaattttgca 10560
aaatgaatca agcctatatg gctgtaatat gaatttaaaa gcagctcgat gtggtggtaa 10620
tatgtaattt acttgattct aaaaaaatat cccaagtatt aataatttct gctaggaaga 10680
aggttagcta cgatttacag caaagccaga atacaatgaa ccataaagtg attgaagctc 10740
gaaatatacg aaggaacaaa tatttttaaa aaaatacgca atgacttgga acaaaagaaa 10800
gtgatatatt ttttgttctt aaacaagcat cccctctaaa gaatggcagt tttcctttgc 10860
atgtaactat tatgctccct tcgttacaaa aattttggac tactattggg aacttcttct 10920
gaaaatagtg gccaccgctt aattaaggcg cgccatgccc gggcaagcgg ccgcacaagt 10980
ttgtacaaaa aagcaggctc cgcggccgca ctaggtttaa actctagaag ctaggaattc 11040
aaacaaagaa gcgatcgcgc ggccgccatt gtactcccag tatcattata gtgaaagttt 11100
tggctctctc gccggtggtt ttttacctct atttaaaggg gttttccacc taaaaattct 11160
ggtatcattc tcactttact tgttacttta atttctcata atctttggtt gaaattatca 11220
cgcttccgca cacgatatcc ctacaaattt attatttgtt aaacattttc aaaccgcata 11280

aaattttatg aagtcccgtc tatctttaat gtagtctaac attttcatat tgaaatatat 11340
aatttactta attttagcgt tggtagaaag cataatgatt tattcttatt cttcttcata 11400
taaatgttta atatacaata taaacaaatt ctttacctta agaaggattt cccattttat 11460
attttaaaaa tatatttatc aaatattttt caaccacgta aatctcataa taataagttg 11520
tttcaaaagt aataaaattt aactccataa ttttttttatt cgactgatct taaagcaaca 11580
cccagtgaca caactagcca ttttttttctt tgaataaaaa aatccaatta tcattgtatt 11640
ttttttatac aatgaaaatt tcaccaaaca atgatttgtg gtatttctga agcaagtcat 11700
gttatgcaaa attctataat tcccatttga cactacggaa gtaactgaag atctgctttt 11760
acatgcgaga cacatcttct aaagtaattt taataatagt tactatattc aagatttcat 11820
atatcaaata ctcaatatta cttctaaaaa attaattaga tataattaaa atattacttt 11880
tttaatttta agtttaattg ttgaatttgt gactattgat ttattattct actatgttta 11940
aattgtttta tagatagttt aaagtaaata taagtaatgt agtagagtgt tagagtgtta 12000
ccctaaacca taaactataa gatttatggt ggactaattt tcatatattt cttattgctt 12060
ttaccttttc ttggtatgta agtccgtaac tggaattact gtgggttgcc atgacactct 12120
gtggtctttt ggttcatgca tggatgcttg cgcaagaaaa agacaaagaa caaagaaaaa 12180
agacaaaaca gagagacaaa acgcaatcac acaaccaact caaattagtc actggctgat 12240
caagatcgcc gcgtccatgt atgtctaaat gccatgcaaa gcaacacgtg cttaacatgc 12300
actttaaatg gctcacccat ctcaacccac acacaaacac attgcctttt tcttcatcat 12360
caccacaacc acctgtatat attcattctc ttccgccacc tcaatttctt cacttcaaca 12420
cacgtcaacc tgcatatgcg tgtcatccca tgcccaaatc tccatgcatg ttccaaccac 12480
cttctctctt atataatacc tataaatacc tctaatatca ctcacttctt tcatcatcca 12540
tccatccaga gtactactac tctactacta taatacccca acccaactca tattcaatac 12600
tactctagga tccaacaatg tctgctccaa ccgctgacat cagggctagg gctccagagg 12660
ctaagaaggt tcacatcgct gataccgcta tcaacaggca caattggtac aagcacgtga 12720
actggctcaa cgtcttcctc atcatcggaa tcccactcta cggatgcatc caagctttct 12780
gggttccact tcaactcaag accgctatct gggctgtgat ctactacttc ttcaccggac 12840
ttggaatcac cgctggatac cacaggcttt gggctcactg ctcatactct gctactcttc 12900
cacttaggat ctggcttgct gctgttggag gaggagctgt tgagggatct atcagatggt 12960
gggctaggga tcacagggct catcataggt acaccgatac cgacaaggac ccatactctg 13020
ttaggaaggg acttctctac tctcaccttg gatggatggt gatgaagcag aacccaaaga 13080
ggatcggaag gaccgacatc tctgatctca acgaggaccc agttgttgtt tggcaacaca 13140
ggaactacct caaggttgtg ttcaccatgg gacttgctgt tccaatgctt gttgctggac 13200
ttggatgggg agattggctt ggaggattcg tgtacgctgg aatccttagg atcttcttcg 13260
ttcaacaagc taccttctgc gtgaactctc ttgctcactg gcttggagat caaccattcg 13320

atgataggaa ctctcctagg gatcacgtga tcaccgctct tgttaccctt ggagagggat 13380
accacaactt ccaccacgag ttcccatctg actacaggaa cgctatcgag tggcaccagt 13440
acgatcctac caagtggtct atctgggctt ggaagcaact tggattggct tacgatctca 13500
agaagttcag ggctaacgag atcgagaagg gaagggttca acaacttcag aagaagcttg 13560
ataggaagag ggctactctt gattggggaa ccccacttga tcaacttcca gtgatggaat 13620
gggatgacta cgttgagcaa gctaagaacg gaaggggact tgttgctatc gctggagttg 13680
ttcacgatgt taccgacttc atcaaggatc acccaggagg aaaggctatg atctcttctg 13740
gaatcggaaa ggatgctacc gctatgttca acggaggagt gtactaccac tctaacgcag 13800
ctcacaacct tcttagcacc atgagggtgg gagtgatcag gggaggatgc gaggttgaga 13860
tctggaagag ggctcagaag gagaacgttg agtacgttag ggatggatct ggacaaaggg 13920
tgatcagggc tggagagcaa ccaaccaaga tcccagagcc aatcccaacc gctgatgctg 13980
cttgagtagt tagcttaatc acctaggtca ccagtatgaa ctaaaatgca tgtaggtgta 14040
agagctcatg gagagcatgg aatattgtat ccgaccatgt aacagtataa taactgagct 14100
ccatctcact tcttctatga ataaacaaag gatgttatga tatattaaca ctctatctat 14160
gcaccttatt gttctatgat aaatttcctc ttattattat aaatcatctg aatcgtgacg 14220
gcttatggaa tgcttcaaat agtacaaaaa caaatgtgta ctataagact ttctaaacaa 14280
ttctaacttt agcattgtga acgagacata agtgttaaga agacataaca attataatgg 14340
aagaagtttg tctccattta tatattatat attacccact tatgtattat attaggatgt 14400
taaggagaca taacaattat aaagagagaa gtttgtatcc atttatatat tatatactac 14460
ccatttatat attatactta tccacttatt taatgtcttt ataaggtttg atccatgata 14520
tttctaatat tttagttgat atgtatatga aaaggtacta tttgaactct cttactctgt 14580
ataaaggttg gatcatcctt aaagtgggtc tatttaattt tattgcttct tacagataaa 14640
aaaaaaatta tgagttggtt tgataaaata ttgaaggatt taaaataata ataaataata 14700
aataacatat aatatatgta tataaattta ttataatata acatttatct ataaaaaagt 14760
aaatattgtc ataaatctat acaatcgttt agccttgctg gaacgaatct caattattta 14820
aacgagagta aacatatttg actttttggt tatttaacaa attattattt aacactatat 14880
gaaatttttt ttttttatca gcaaagaata aaattaaatt aagaaggaca atggtgtccc 14940
aatccttata caaccaactt ccacaagaaa gtcaagtcag agacaacaaa aaaacaagca 15000
aaggaaattt tttaatttga gttgtcttgt ttgctgcata atttatgcag taaaacacta 15060
cacataaccc ttttagcagt agagcaatgg ttgaccgtgt gcttagcttc ttttatttta 15120
ttttttttatc agcaaagaat aaataaaata aaatgagaca cttcagggat gtttcaaccc 15180
ttatacaaaa ccccaaaaac aagtttccta gcaccctacc aacgaattcg cggccgcttt 15240
cctgcatgac atcgtcctgc agagccaagc gcatgcttaa ttaaactagt ctcccagtat 15300
cattatagtg aaagttttgg ctctctcgcc ggtggttttt tacctctatt taaaggggtt 15360

ttccacctaa aaattctggt atcattctca ctttacttgt tactttaatt tctcataatc 15420
tttggttgaa attatcacgc ttccgcacac gatatcccta caaatttatt atttgttaaa 15480
cattttcaaa ccgcataaaa ttttatgaag tcccgtctat ctttaatgta gtctaacatt 15540
ttcatattga aatatataat ttacttaatt ttagcgttgg tagaaagcat aatgatttat 15600
tcttattctt cttcatataa atgtttaata tacaatataa acaaattctt taccttaaga 15660
aggatttccc attttatatt ttaaaaatat atttatcaaa tatttttcaa ccacgtaaat 15720
ctcataataa taagttgttt caaaagtaat aaaatttaac tccataattt ttttattcga 15780
ctgatcttaa agcaacaccc agtgacacaa ctagccattt ttttctttga ataaaaaaat 15840
ccaattatca ttgtattttt tttatacaat gaaaatttca ccaaacaatg atttgtggta 15900
tttctgaagc aagtcatgtt atgcaaaatt ctataattcc catttgacac tacggaagta 15960
actgaagatc tgcttttaca tgcgagacac atcttctaaa gtaattttaa taatagttac 16020
tatattcaag atttcatata tcaaatactc aatattactt ctaaaaaatt aattagatat 16080
aattaaaata ttactttttt aattttaagt ttaattgttg aatttgtgac tattgattta 16140
ttattctact atgtttaaat tgttttatag atagtttaaa gtaaatataa gtaatgtagt 16200
agagtgttag agtgttaccc taaaccataa actataagat ttatggtgga ctaattttca 16260
tatatttctt attgctttta ccttttcttg gtatgtaagt ccgtaactgg aattactgtg 16320
ggttgccatg acactctgtg gtcttttggt tcatgcatgg atcttgcgca agaaaaagac 16380
aaagaacaaa gaaaaaagac aaaacagaga gacaaaacgc aatcacacaa ccaactcaaa 16440
ttagtcactg gctgatcaag atcgccgcgt ccatgtatgt ctaaatgcca tgcaaagcaa 16500
cacgtgctta acatgcactt taaatggctc acccatctca acccacacac aaacacattg 16560
cctttttctt catcatcacc acaaccacct gtatatattc attctcttcc gccacctcaa 16620
tttcttcact tcaacacacg tcaacctgca tatgcgtgtc atcccatgcc caaatctcca 16680
tgcatgttcc aaccaccttc tctcttatat aatacctata aatacctcta atatcactca 16740
cttctttcat catccatcca tccagagtac tactactcta ctactataat accccaaccc 16800
aactcatatt caatactact ctaggtaccc tgcagggatc caacaatggc tgcacttgat 16860
agcatccctg aggacaaagc aactagctcc aagtcaaccc acatacagta ccaagaggtc 16920
acgtttagga actggtacaa gaaaatcaac tggctcaaca cgacccttgt tgtcctcatt 16980
cctgctcttg ggttgtactt gacgagaacc acacctctca ccagacctac cctcatttgg 17040
tctgttctct actatttctg tacagcgttt ggcatcactg gtggctacca cagactttgg 17100
tcccataggt cttacagtgc gaggttgcca ttgagactct tcctggcttt cactggagct 17160
ggtgcgatcc aaggttctgc aagatggtgg tcagccaatc atagggcaca tcaccgttgg 17220
acggacacca tgaaggaccc ctactctgtg atgagaggac tgctgttctc ccacataggt 17280
tggatggttc tcaactctga tccaaaggtc aaaggcagaa cagatgtttc tgatcttgac 17340
tctgatcccg tcgttgtgtg gcaacacaaa cactatggca agtgtttgct ctttgccgct 17400

tggatctttc cgatgatagt ggctgggctg ggttggggag attggtgggg tggacttgtc 17460
tatgctggca tcatacgtgc ctgctttgtt cagcaagcca ctttctgtgt caactcattg 17520
gcacattgga taggtgaaca accgtttgat gacagacgta ctccaaggga tcatgttctg 17580
actgcgttgg tcacaatggg agaaggatac cacaacttcc accatgagtt tccgagtgac 17640
tacagaaatg ccatcatttg gtatcagtat gaccctacaa agtggctcat ctatctcttc 17700
agcttgggtc ccttcccatt ggcctactct ctcaagacct tccgttccaa tgagattgag 17760
aaaggaaggc ttcagcaaca gcaaaaggct cttgacaaga aaagaagtgg tcttgattgg 17820
ggacttcctc tcttccagct tccagtgatc tcatgggatg actttcaagc tcgttgcaaa 17880
gaaagtggag agatgcttgt tgctgttgct ggagtgatcc atgatgtctc ccagttcatt 17940
gaagatcatc ctggtgggag gagcctcatt agaagtgctg ttgggaaaga tgggactggc 18000
atgttcaatg gtggagtgta tgaacattca aacgccgcac acaacttgct gagcacaatg 18060
agagttggag tcttgagagg tggacaagaa gtggaggttt ggaagaaaca gagggtggat 18120
gttcttggga agtcagacat tcttcgtcaa gtgacaaggg tggagcgtct ggtggaagga 18180
gctgttgcag cgtgatgagt agttagctta atcacctaga gctcggtcac ctcgagtatc 18240
aaaatctatt tagaaataca caatattttg ttgcaggctt gctggagaat cgatctgcta 18300
tcataaaaat tacaaaaaaa ttttatttgc ctcaattatt ttaggattgg tattaaggac 18360
gcttaaatta tttgtcgggt cactacgcat cattgtgatt gagaagatca gcgatacgaa 18420
atattcgtag tactatcgat aatttatttg aaaattcata agaaaagcaa acgttacatg 18480
aattgatgaa acaatacaaa gacagataaa gccacgcaca tttaggatat tggccgagat 18540
tactgaatat tgagtaagat cacggaattt ctgacaggag catgtcttca attcagccca 18600
aatggcagtt gaaatactca aaccgcccca tatgcaggag cggatcattc attgtttgtt 18660
tggttgcctt tgccaacatg ggagtccaag gttggcgcgc cgacccagct ttc 18713

<210> 61
<211> 14524
<212> DNA
<213> Artificial

<220>
<223> Plasmid pDAB7321

<400> 61

```
ttgtacaaag tggttgcggc cgcttaatta aatttaaatt caattaatgc aatcttgatt      60
ttcaacaacg aaggtaatgg cgtaaaagaa aaaatgtatg ttattgtatt gatctttcat     120
gatgttgaag cgtgccataa tatgatgatg tataattaaa atattaactg tcgcatttta     180
ttgaaatggc actgttattt caaccatatc tttgattctg ttacatgaca cgactgcaag     240
aagtaaataa tagacgccgt tgttaaagaa ttgctatcat atgtgcctaa ctagagggaa     300
tttgagcgtc agacctaatc aaatattaca aaatatctca ctctgtcgcc agcaatggtg     360
taatcagcgc agacaaatgg cgtaaagatc gcggaaaaac ctccccgagt ggcatgatag     420
```

```
ctgcctctgt attgctgatt tagtcagcct tatttgactt aagggtgccc tcgttagtga    480
caaattgctt tcaaggagac agccatgccc cacactttgt tgaaaaacaa attgcctttg    540
gggagacggt aaagccagtt gctcttcaat aaggaatgtc gaggaggcaa tgtaaccgcc    600
tctggtagta cacttctcta atccaaaaat caatttgtat tcaagatacc gcaaaaaact    660
tatggtttaa accctgcagg actagtccag aaggtaatta tccaagatgt agcatcaaga    720
atccaatgtt tacgggaaaa actatggaag tattatgtaa gctcagcaag aagcagatca    780
atatgcggca catatgcaac ctatgttcaa aaatgaagaa tgtacagata caagatccta    840
tactgccaga atacgaagaa gaatacgtag aaattgaaaa agaagaacca ggcgaagaaa    900
agaatcttga agacgtaagc actgacgaca acaatgaaaa gaagaagata aggtcggtga    960
ttgtgaaaga gacatagagg acacatgtaa ggtggaaaat gtaagggcgg aaagtaacct   1020
tatcacaaag gaatcttatc ccccactact tatcctttta tatttttccg tgtcattttt   1080
gcccttgagt tttcctatat aaggaaccaa gttcggcatt tgtgaaaaca agaaaaaatt   1140
tggtgtaagc tattttcttt gaagtactga ggatacaact tcagagaaat ttgtaagttt   1200
gtaggtacca gatctggatc ccaaaccatg tctccggaga ggagaccagt tgagattagg   1260
ccagctacag cagctgatat ggccgcggtt tgtgatatcg ttaaccatta cattgagacg   1320
tctacagtga actttaggac agagccacaa acaccacaag agtggattga tgatctagag   1380
aggttgcaag atagataccc ttggttggtt gctgaggttg agggtgttgt ggctggtatt   1440
gcttacgctg ggccctggaa ggctaggaac gcttacgatt ggacagttga gagtactgtt   1500
tacgtgtcac ataggcatca aaggttgggc ctaggatcta cattgtacac acatttgctt   1560
aagtctatgg aggcgcaagg ttttaagtct gtggttgctg ttataggcct tccaaacgat   1620
ccatctgtta ggttgcatga ggctttggga tacacagccc ggggtacatt gcgcgcagct   1680
ggatacaagc atggtggatg gcatgatgtt ggtttttggc aaagggattt tgagttgcca   1740
gctcctccaa ggccagttag gccagttacc caaatctgag tagttagctt aatcacctag   1800
agctcgatcg gcggcaatag cttcttagcg ccatcccggg ttgatcctat ctgtgttgaa   1860
atagttgcgg tgggcaaggc tctctttcag aaagacaggc ggccaaagga acccaaggtg   1920
aggtgggcta tggctctcag ttccttgtgg aagcgcttgg tctaaggtgc agaggtgtta   1980
gcgggatgaa gcaaaagtgt ccgattgtaa caagatatgt tgatcctacg taaggatatt   2040
aaagtatgta ttcatcacta atataatcag tgtattccaa tatgtactac gatttccaat   2100
gtctttattg tcgccgtatg taatcggcgt cacaaaataa tccccggtga ctttctttta   2160
atccaggatg aaataatatg ttattataat ttttgcgatt tggtccgtta taggaattga   2220
agtgtgcttg aggtcggtcg ccaccactcc catttcataa ttttacatgt atttgaaaaa   2280
taaaaattta tggtattcaa tttaaacacg tatacttgta aagaatgata tcttgaaaga   2340
aatatagttt aaatatttat tgataaaata acaagtcagg tattatagtc caagcaaaaa   2400
cataaattta ttgatgcaag tttaaattca gaaatatttc aataactgat tatatcagct   2460
```

```
ggtacattgc cgtagatgaa agactgagtg cgatattatg gtgtaataca taggaattcg   2520
tttaaacgat ctgcgtctaa ttttcggtcc aacttgcaca ggaaagacgt cgaccgcggt   2580
agctcttgcc cagcagactg ggcttccagt cctttcgctc gatcgggtcc aatgttgtcc   2640
tcagctgtga accggaagcg gacgaccaac agtggaagaa ctgaaaggaa cgagccgtct   2700
ataccttgat gatcggcctc tggtgaaggg tatcatcgca gccaagcaag ctcatgaaag   2760
gctgatgggg gaggtgtata attatgaggc ccacggcggg cttattcttt agggaggatc   2820
tatctcgttg ctcaagtgca tggcgcaaag cagttattgg agtgcggatt ttcgttggca   2880
tattattcgc cacgagttag cagacgaaga gaccttcatg aacgtggcca aggccagagt   2940
taagcagatg ttacgccctg ctgcaggcct ttctattatc caatagttgg ttgatctttg   3000
gaaagagcct cggctgaggc ccatactgaa agagatcgat ggatatcgat atgccatgtt   3060
gtttgctagc cagaaccaga tcacatccga tatgctattg cagcttgacg cagatatgga   3120
ggataagttg attcatggga tcgctcagga gtagctcatc catgcacgcc gacaagaaca   3180
gaaattccgt cgagttaacg cagccgctta cgacggattc gaaggtcatc cattcggaat   3240
gtattagttt gcaccagctc cgcgtcacac ctgtcttcat ttgaataaga tgttagcaat   3300
tgtttttagc tttgtcttgt tgtggcaggg cggcaagtgc ttcagacatc attctgtttt   3360
caaattttat gctggagaac agcttcttaa ttcctttgga aataatagac tgcgtcttaa   3420
aattcagatg tctggatata gatatgattg taaaataacc tatttaagtg tcatttagaa   3480
cataagtttt atgaatgttc ttccattttc gtcatcgaac gaataagagt aaatacacct   3540
tttttaacat tacaaataag ttcttatacg ttgtttatac accgggaatc atttccatta   3600
ttttcgcgca aaagtcacgg atattcgtga aagcgacata aactgcgaaa tttgcgggga   3660
gtgtcttgag tttgcctcga ggctagcgca tgcacataga cacacacatc atctcattga   3720
tgcttggtaa taattgtcat tagattgttt ttatgcatag atgcactcga aatcagccaa   3780
ttttagacaa gtatcaaacg gatgtgactt cagtacatta aaaacgtccg caatgtgtta   3840
ttaagttgtc taagcgtcaa tttgatttac aattgaatat atcctgcccc agccagccaa   3900
cagctcgatt tacaattgaa tatatcctgc cggccggccc acgcgtgtcg aggaattctg   3960
atctggcccc catttggacg tgaatgtaga cacgtcgaaa taaagatttc cgaattagaa   4020
taatttgttt attgctttcg cctataaata cgacggatcg taatttgtcg ttttatcaaa   4080
atgtactttc attttataat aacgctgcgg acatctacat ttttgaattg aaaaaaaatt   4140
ggtaattact ctttcttttt ctccatattg accatcatac tcattgctga tccatgtaga   4200
tttcccggac atgaagccat ttacaattga atatatcctg ccgccgctgc cgctttgcac   4260
ccggtggagc ttgcatgttg gtttctacgc agaactgagc cggttaggca gataatttcc   4320
attgagaact gagccatgtg caccttcccc ccaacacggt gagcgacggg gcaacggagt   4380
gatccacatg ggacttttaa acatcatccg tcggatggcg ttgcgagaga agcagtcgat   4440
ccgtgagatc agccgacgca ccgggcaggc gcgcaacacg atcgcaaagt atttgaacgc   4500
```

aggtacaatc gagccgacgt tcacgcggaa cgaccaagca agcttggctg ccatttttgg 4560
ggtgaggccg ttcgcggccg aggggcgcag cccctggggg gatgggaggc ccgcgttagc 4620
gggccgggag ggttcgagaa ggggggcac cccccttcgg cgtgcgcggt cacgcgcaca 4680
gggcgcagcc ctggttaaaa acaaggttta taaatattgg tttaaaagca ggttaaaaga 4740
caggttagcg gtggccgaaa aacgggcgga aacccttgca aatgctggat tttctgcctg 4800
tggacagccc ctcaaatgtc aataggtgcg cccctcatct gtcagcactc tgcccctcaa 4860
gtgtcaagga tcgcgcccct catctgtcag tagtcgcgcc cctcaagtgt caataccgca 4920
gggcacttat ccccaggctt gtccacatca tctgtgggaa actcgcgtaa aatcaggcgt 4980
tttcgccgat ttgcgaggct ggccagctcc acgtcgccgg ccgaaatcga gcctgcccct 5040
catctgtcaa cgccgcgccg ggtgagtcgg cccctcaagt gtcaacgtcc gcccctcatc 5100
tgtcagtgag ggccaagttt tccgcgaggt atccacaacg ccggcggccg cggtgtctcg 5160
cacacggctt cgacggcgtt tctggcgcgt ttgcagggcc atagacggcc gccagcccag 5220
cggcgagggc aaccagcccg gtgagcgtcg gaaagggtcg acggatcttt tccgctgcat 5280
aaccctgctt cggggtcatt atagcgattt tttcggtata tccatccttt ttcgcacgat 5340
atacaggatt ttgccaaagg gttcgtgtag actttccttg gtgtatccaa cggcgtcagc 5400
cgggcaggat aggtgaagta ggcccacccg cgagcgggtg ttccttcttc actgtccctt 5460
attcgcacct ggcggtgctc aacgggaatc ctgctctgcg aggctggccg gctaccgccg 5520
gcgtaacaga tgagggcaag cggatggctg atgaaaccaa gccaaccagg aagggcagcc 5580
cacctatcaa ggtgtactgc cttccagacg aacgaagagc gattgaggaa aaggcggcgg 5640
cggccggcat gagcctgtcg gcctacctgc tggccgtcgg ccagggctac aaaatcacgg 5700
gcgtcgtgga ctatgagcac gtccgcgagc tggcccgcat caatggcgac ctgggccgcc 5760
tgggcggcct gctgaaactc tggctcaccg acgacccgcg cacggcgcgg ttcggtgatg 5820
ccacgatcct cgccctgctg gcgaagatcg aagagaagca ggacgagctt ggcaaggtca 5880
tgatgggcgt ggtccgcccg agggcagagc catgactttt ttagccgcta aaacggccgg 5940
ggggtgcgcg tgattgccaa gcacgtcccc atgcgctcca tcaagaagag cgacttcgcg 6000
gagctggtat tcgtgcaggg caagattcgg aataccaagt acgagaagga cggccagacg 6060
gtctacggga ccgacttcat tgccgataag gtggattatc tggacaccaa ggcaccaggc 6120
gggtcaaatc aggaataagg gcacattgcc ccggcgtgag tcggggcaat cccgcaagga 6180
gggtgaatga atcggacgtt tgaccggaag gcatacaggc aagaactgat cgacgcgggg 6240
ttttccgccg aggatgccga aaccatcgca agccgcaccg tcatgcgtgc gccccgcgaa 6300
accttccagt ccgtcggctc gatggtccag caagctacgg ccaagatcga gcgcgacagc 6360
gtgcaactgg ctccccctgc cctgcccgcg ccatcggccg ccgtggagcg ttcgcgtcgt 6420
ctcgaacagg aggcggcagg tttggcgaag tcgatgacca tcgacacgcg aggaactatg 6480
acgaccaaga agcgaaaaac cgccggcgag gacctggcaa aacaggtcag cgaggccaag 6540

caggccgcgt tgctgaaaca cacgaagcag cagatcaagg aaatgcagct ttccttgttc 6600
gatattgcgc cgtggccgga cacgatgcga gcgatgccaa acgacacggc ccgctctgcc 6660
ctgttcacca cgcgcaacaa gaaaatcccg cgcgaggcgc tgcaaaacaa ggtcattttc 6720
cacgtcaaca aggacgtgaa gatcacctac accggcgtcg agctgcgggc cgacgatgac 6780
gaactggtgt ggcagcaggt gttggagtac gcgaagcgca cccctatcgg cgagccgatc 6840
accttcacgt tctacgagct ttgccaggac ctgggctggt cgatcaatgg ccggtattac 6900
acgaaggccg aggaatgcct gtcgcgccta caggcgacgg cgatgggctt cacgtccgac 6960
cgcgttgggc acctggaatc ggtgtcgctg ctgcaccgct tccgcgtcct ggaccgtggc 7020
aagaaaacgt cccgttgcca ggtcctgatc gacgaggaaa tcgtcgtgct gtttgctggc 7080
gaccactaca cgaaattcat atgggagaag taccgcaagc tgtcgccgac ggcccgacgg 7140
atgttcgact atttcagctc gcaccgggag ccgtacccgc tcaagctgga aaccttccgc 7200
ctcatgtgcg gatcggattc cacccgcgtg aagaagtggc gcgagcaggt cggcgaagcc 7260
tgcgaagagt tgcgaggcag cggcctggtg gaacacgcct gggtcaatga tgacctggtg 7320
cattgcaaac gctagggcct tgtggggtca gttccggctg ggggttcagc agccagcgct 7380
ttactggcat ttcaggaaca agcgggcact gctcgacgca cttgcttcgc tcagtatcgc 7440
tcgggacgca cggcgcgctc tacgaactgc cgataaacag aggattaaaa ttgacaattg 7500
tgattaaggc tcagattcga cggcttggag cggccgacgt gcaggatttc cgcgagatcc 7560
gattgtcggc cctgaagaaa gctccagaga tgttcgggtc cgtttacgag cacgaggaga 7620
aaaagcccat ggaggcgttc gctgaacggt tgcgagatgc cgtggcattc ggcgcctaca 7680
tcgacggcga gatcattggg ctgtcggtct tcaaacagga ggacggcccc aaggacgctc 7740
acaaggcgca tctgtccggc gttttcgtgg agcccgaaca gcgaggccga ggggtcgccg 7800
gtatgctgct gcgggcgttg ccggcgggtt tattgctcgt gatgatcgtc cgacagattc 7860
caacgggaat ctggtggatg cgcatcttca tcctcggcgc acttaatatt tcgctattct 7920
ggagcttgtt gtttatttcg gtctaccgcc tgccgggcgg ggtcgcggcg acggtaggcg 7980
ctgtgcagcc gctgatggtc gtgttcatct ctgccgctct gctaggtagc ccgatacgat 8040
tgatggcggt cctgggggct atttgcggaa ctgcgggcgt ggcgctgttg gtgttgacac 8100
caaacgcagc gctagatcct gtcggcgtcg cagcgggcct ggcgggggcg gtttccatgg 8160
cgttcggaac cgtgctgacc cgcaagtggc aacctcccgt gcctctgctc acctttaccg 8220
cctggcaact ggcggccgga ggacttctgc tcgttccagt agctttagtg tttgatccgc 8280
caatcccgat gcctacagga accaatgttc tcggcctggc gtggctcggc ctgatcggag 8340
cgggtttaac ctacttcctt tggttccggg ggatctcgcg actcgaacct acagttgttt 8400
ccttactggg ctttctcagc cccgagcgc ttagtgggaa tttgtacccc ttatcgaacc 8460
gggagcacag gatgacgcct aacaattcat tcaagccgac accgcttcgc ggcgcggctt 8520
aattcaggag ttaaacatca tgagggaagc ggtgatcgcc gaagtatcga ctcaactatc 8580

agaggtagtt ggcgtcatcg agcgccatct cgaaccgacg ttgctggccg tacatttgta 8640
cggctccgca gtggatggcg gcctgaagcc acacagtgat attgatttgc tggttacggt 8700
gaccgtaagg cttgatgaaa caacgcggcg agctttgatc aacgaccttt tggaaacttc 8760
ggcttcccct ggagagagcg agattctccg cgctgtagaa gtcaccattg ttgtgcacga 8820
cgacatcatt ccgtggcgtt atccagctaa gcgcgaactg caatttggag aatggcagcg 8880
caatgacatt cttgcaggta tcttcgagcc agccacgatc gacattgatc tggctatctt 8940
gctgacaaaa gcaagagaac atagcgttgc cttggtaggt ccagcggcgg aggaactctt 9000
tgatccggtt cctgaacagg atctatttga ggcgctaaat gaaaccttaa cgctatggaa 9060
ctcgccgccc gactgggctg gcgatgagcg aaatgtagtg cttacgttgt cccgcatttg 9120
gtacagcgca gtaaccggca aaatcgcgcc gaaggatgtc gctgccgact gggcaatgga 9180
gcgcctgccg gcccagtatc agcccgtcat acttgaagct aggcaggctt atcttggaca 9240
agaagatcgc ttggcctcgc gcgcagatca gttggaagaa tttgttcact acgtgaaagg 9300
cgagatcacc aaggtagtcg gcaaataatg tctaacaatt cgttcaagcc gacgccgctt 9360
cgcggcgcgg cttaactcaa gcgttagaga gctggggaag actatgcgcg atctgttgaa 9420
ggtggttcta agcctcgtct tgcgatggca tttcgatcca ttcccattcc gcgctcaaga 9480
tggcttcccc tcggcagttc atcagggcta aatcaatcta gccgacttgt ccggtgaaat 9540
gggctgcact ccaacagaaa caatcaaaca aacatacaca gcgacttatt cacacgagct 9600
caaattacaa cggtatatat cctgccagtc agcatcatca caccaaaagt taggcccgaa 9660
tagtttgaaa ttagaaagct cgcaattgag gtctacaggc caaattcgct cttagccgta 9720
caatattact caccggatcc taaccggtgt gatcatgggc cgcgattaaa aatctcaatt 9780
atatttggtc taatttagtt tggtattgag taaaacaaat tcgaaccaaa ccaaaatata 9840
aatatatagt ttttatatat atgcctttaa gactttttat agaattttct ttaaaaaata 9900
tctagaaata tttgcgactc ttctggcatg taatatttcg ttaaatatga agtgctccat 9960
ttttattaac tttaaataat tggttgtacg atcactttct tatcaagtgt tactaaaatg 10020
cgtcaatctc tttgttcttc catattcata tgtcaaaacc tatcaaaatt cttatatatc 10080
tttttcgaat ttgaagtgaa atttcgataa tttaaaatta aatagaacat atcattattt 10140
aggtatcata ttgattttta tacttaatta ctaaatttgg ttaactttga aagtgtacat 10200
caacgaaaaa ttagtcaaac gactaaaata aataaatatc atgtgttatt aagaaaattc 10260
tcctataaga atattttaat agatcatatg tttgtaaaaa aaattaattt ttactaacac 10320
atatatttac ttatcaaaaa tttgacaaag taagattaaa ataatattca tctaacaaaa 10380
aaaaaaccag aaaatgctga aaacccggca aaaccgaacc aatccaaacc gatatagttg 10440
gtttggtttg attttgatat aaaccgaacc aactcggtcc atttgcaccc ctaatcataa 10500
tagctttaat atttcaagat attattaagt taacgttgtc aatatcctgg aaattttgca 10560
aaatgaatca agcctatatg gctgtaatat gaatttaaaa gcagctcgat gtggtggtaa 10620

tatgtaattt acttgattct aaaaaaatat cccaagtatt aataatttct gctaggaaga 10680
aggttagcta cgatttacag caaagccaga atacaatgaa ccataaagtg attgaagctc 10740
gaaatatacg aaggaacaaa tatttttaaa aaaatacgca atgacttgga acaaaagaaa 10800
gtgatatatt ttttgttctt aaacaagcat cccctctaaa gaatggcagt tttcctttgc 10860
atgtaactat tatgctccct tcgttacaaa aattttggac tactattggg aacttcttct 10920
gaaaatagtg gccaccgctt aattaaggcg cgccatgccc gggcaagcgg ccgcacaagt 10980
ttgtacaaaa aagcaggctc cgcggccgca ctaggtttaa actctagaag ctaggaattc 11040
gcggccgctt tcctgcatga catcgtcctg cagagccaag cgcatgctta attaaactag 11100
tctcccagta tcattatagt gaaagttttg gctctctcgc cggtggtttt ttacctctat 11160
ttaaaggggt tttccaccta aaaattctgg tatcattctc actttacttg ttactttaat 11220
ttctcataat ctttggttga aattatcacg cttccgcaca cgatatccct acaaatttat 11280
tatttgttaa acattttcaa accgcataaa attttatgaa gtcccgtcta tctttaatgt 11340
agtctaacat tttcatattg aaatatataa tttacttaat tttagcgttg gtagaaagca 11400
taatgattta ttcttattct tcttcatata aatgtttaat atacaatata aacaaattct 11460
ttaccttaag aaggatttcc cattttatat tttaaaaata tatttatcaa atatttttca 11520
accacgtaaa tctcataata ataagttgtt tcaaaagtaa taaaatttaa ctccataatt 11580
tttttattcg actgatctta aagcaacacc cagtgacaca actagccatt tttttctttg 11640
aataaaaaaa tccaattatc attgtatttt ttttatacaa tgaaaatttc accaaacaat 11700
gatttgtggt atttctgaag caagtcatgt tatgcaaaat tctataattc ccatttgaca 11760
ctacggaagt aactgaagat ctgcttttac atgcgagaca catcttctaa agtaatttta 11820
ataatagtta ctatattcaa gatttcatat atcaaatact caatattact tctaaaaaat 11880
taattagata taattaaaat attacttttt taattttaag tttaattgtt gaatttgtga 11940
ctattgattt attattctac tatgtttaaa ttgttttata gatagtttaa agtaaatata 12000
agtaatgtag tagagtgtta gagtgttacc ctaaaccata aactataaga tttatggtgg 12060
actaattttc atatatttct tattgctttt accttttctt ggtatgtaag tccgtaactg 12120
gaattactgt gggttgccat gacactctgt ggtcttttgg ttcatgcatg gatcttgcgc 12180
aagaaaaaga caaagaacaa agaaaaaaga caaaacagag agacaaaacg caatcacaca 12240
accaactcaa attagtcact ggctgatcaa gatcgccgcg tccatgtatg tctaaatgcc 12300
atgcaaagca acacgtgctt aacatgcact ttaaatggct cacccatctc aacccacaca 12360
caaacacatt gcctttttct tcatcatcac cacaaccacc tgtatatatt cattctcttc 12420
cgccacctca atttcttcac ttcaacacac gtcaacctgc atatgcgtgt catcccatgc 12480
ccaaatctcc atgcatgttc caaccacctt ctctcttata taatacctat aaatacctct 12540
aatatcactc acttctttca tcatccatcc atccagagta ctactactct actactataa 12600
taccccaacc caactcatat tcaatactac tctaggtacc ctgcagggat ccaacaatgg 12660

ctgcacttga tagcatccct gaggacaaag caactagctc caagtcaacc cacatacagt 12720
accaagaggt cacgtttagg aactggtaca agaaaatcaa ctggctcaac acgacccttg 12780
ttgtcctcat tcctgctctt gggttgtact tgacgagaac cacacctctc accagaccta 12840
ccctcatttg gtctgttctc tactatttct gtacagcgtt tggcatcact ggtggctacc 12900
acagactttg gtcccatagg tcttacagtg cgaggttgcc attgagactc ttcctggctt 12960
tcactggagc tggtgcgatc caaggttctg caagatggtg gtcagccaat catagggcac 13020
atcaccgttg gacggacacc atgaaggacc cctactctgt gatgagagga ctgctgttct 13080
cccacatagg ttggatggtt ctcaactctg atccaaaggt caaaggcaga acagatgttt 13140
ctgatcttga ctctgatccc gtcgttgtgt ggcaacacaa acactatggc aagtgtttgc 13200
tctttgccgc ttggatcttt ccgatgatag tggctgggct gggttgggga gattggtggg 13260
gtggacttgt ctatgctggc atcatacgtg cctgctttgt tcagcaagcc actttctgtg 13320
tcaactcatt ggcacattgg ataggtgaac aaccgtttga tgacagacgt actccaaggg 13380
atcatgttct gactgcgttg gtcacaatgg gagaaggata ccacaacttc caccatgagt 13440
ttccgagtga ctacagaaat gccatcattt ggtatcagta tgaccctaca aagtggctca 13500
tctatctctt cagcttgggt cccttcccat tggcctactc tctcaagacc ttccgttcca 13560
atgagattga gaaaggaagg cttcagcaac agcaaaaggc tcttgacaag aaaagaagtg 13620
gtcttgattg gggacttcct ctcttccagc ttccagtgat ctcatgggat gactttcaag 13680
ctcgttgcaa agaaagtgga gagatgcttg ttgctgttgc tggagtgatc catgatgtct 13740
cccagttcat tgaagatcat cctggtggga ggagcctcat tagaagtgct gttgggaaag 13800
atgggactgg catgttcaat ggtggagtgt atgaacattc aaacgccgca cacaacttgc 13860
tgagcacaat gagagttgga gtcttgagag gtggacaaga agtggaggtt tggaagaaac 13920
agagggtgga tgttcttggg aagtcagaca ttcttcgtca agtgacaagg gtggagcgtc 13980
tggtggaagg agctgttgca gcgtgatgag tagttagctt aatcacctag agctcggtca 14040
cctcgagtat caaaatctat ttagaaatac acaatatttt gttgcaggct tgctggagaa 14100
tcgatctgct atcataaaaa ttacaaaaaa attttatttg cctcaattat tttaggattg 14160
gtattaagga cgcttaaatt atttgtcggg tcactacgca tcattgtgat tgagaagatc 14220
agcgatacga aatattcgta gtactatcga taatttattt gaaaattcat aagaaaagca 14280
aacgttacat gaattgatga aacaatacaa agacagataa agccacgcac atttaggata 14340
ttggccgaga ttactgaata ttgagtaaga tcacggaatt tctgacagga gcatgtcttc 14400
aattcagccc aaatggcagt tgaaatactc aaaccgcccc atatgcagga gcggatcatt 14460
cattgtttgt ttggttgcct ttgccaacat gggagtccaa ggttggcgcg ccgacccagc 14520
tttc 14524

<210> 62
<211> 18422
<212> DNA

<213> Artificial

<220>
<223> Plasmid pDAB7324

<400> 62

ttgtacaaag tggttgcggc cgcttaatta aatttaaatt caattaatgc aatcttgatt 60
ttcaacaacg aaggtaatgg cgtaaaagaa aaaatgtatg ttattgtatt gatctttcat 120
gatgttgaag cgtgccataa tatgatgatg tataattaaa atattaactg tcgcatttta 180
ttgaaatggc actgttattt caaccatatc tttgattctg ttacatgaca cgactgcaag 240
aagtaaataa tagacgccgt tgttaaagaa ttgctatcat atgtgcctaa ctagagggaa 300
tttgagcgtc agacctaatc aaatattaca aaatatctca ctctgtcgcc agcaatggtg 360
taatcagcgc agacaaatgg cgtaaagatc gcggaaaaac ctccccgagt ggcatgatag 420
ctgcctctgt attgctgatt tagtcagcct tatttgactt aagggtgccc tcgttagtga 480
caaattgctt tcaaggagac agccatgccc cacactttgt tgaaaaacaa attgcctttg 540
gggagacggt aaagccagtt gctcttcaat aaggaatgtc gaggaggcaa tgtaaccgcc 600
tctggtagta cacttctcta atccaaaaat caatttgtat tcaagatacc gcaaaaaact 660
tatggtttaa accctgcagg actagtccag aaggtaatta tccaagatgt agcatcaaga 720
atccaatgtt tacgggaaaa actatggaag tattatgtaa gctcagcaag aagcagatca 780
atatgcggca catatgcaac ctatgttcaa aaatgaagaa tgtacagata caagatccta 840
tactgccaga atacgaagaa gaatacgtag aaattgaaaa agaagaacca ggcgaagaaa 900
agaatcttga agacgtaagc actgacgaca acaatgaaaa gaagaagata aggtcggtga 960
ttgtgaaaga gacatagagg acacatgtaa ggtggaaaat gtaagggcgg aaagtaacct 1020
tatcacaaag gaatcttatc ccccactact tatcctttta tatttttccg tgtcattttt 1080
gcccttgagt tttcctatat aaggaaccaa gttcggcatt tgtgaaaaca agaaaaaatt 1140
tggtgtaagc tattttcttt gaagtactga ggatacaact tcagagaaat ttgtaagttt 1200
gtaggtacca gatctggatc ccaaaccatg tctccggaga ggagaccagt tgagattagg 1260
ccagctacag cagctgatat ggccgcggtt tgtgatatcg ttaaccatta cattgagacg 1320
tctacagtga actttaggac agagccacaa acaccacaag agtggattga tgatctagag 1380
aggttgcaag atagataccc ttggttggtt gctgaggttg agggtgttgt ggctggtatt 1440
gcttacgctg ggccctggaa ggctaggaac gcttacgatt ggacagttga gagtactgtt 1500
tacgtgtcac ataggcatca aaggttgggc ctaggatcta cattgtacac acatttgctt 1560
aagtctatgg aggcgcaagg ttttaagtct gtggttgctg ttataggcct tccaaacgat 1620
ccatctgtta ggttgcatga ggctttggga tacacagccc ggggtacatt gcgcgcagct 1680
ggatacaagc atggtggatg gcatgatgtt ggtttttggc aaagggattt tgagttgcca 1740
gctcctccaa ggccagttag gccagttacc caaatctgag tagttagctt aatcacctag 1800
agctcgatcg gcggcaatag cttcttagcg ccatcccggg ttgatcctat ctgtgttgaa 1860

atagttgcgg tgggcaaggc tctctttcag aaagacaggc ggccaaagga acccaaggtg 1920
aggtgggcta tggctctcag ttccttgtgg aagcgcttgg tctaaggtgc agaggtgtta 1980
gcgggatgaa gcaaaagtgt ccgattgtaa caagatatgt tgatcctacg taaggatatt 2040
aaagtatgta ttcatcacta atataatcag tgtattccaa tatgtactac gatttccaat 2100
gtctttattg tcgccgtatg taatcggcgt cacaaaataa tccccggtga ctttctttta 2160
atccaggatg aaataatatg ttattataat ttttgcgatt tggtccgtta taggaattga 2220
agtgtgcttg aggtcggtcg ccaccactcc catttcataa ttttacatgt atttgaaaaa 2280
taaaaattta tggtattcaa tttaaacacg tatacttgta aagaatgata tcttgaaaga 2340
aatatagttt aaatatttat tgataaaata acaagtcagg tattatagtc caagcaaaaa 2400
cataaattta ttgatgcaag tttaaattca gaaatatttc aataactgat tatatcagct 2460
ggtacattgc cgtagatgaa agactgagtg cgatattatg gtgtaataca taggaattcg 2520
tttaaacgat ctgcgtctaa ttttcggtcc aacttgcaca ggaaagacgt cgaccgcggt 2580
agctcttgcc cagcagactg ggcttccagt cctttcgctc gatcgggtcc aatgttgtcc 2640
tcagctgtga accggaagcg gacgaccaac agtggaagaa ctgaaaggaa cgagccgtct 2700
ataccttgat gatcggcctc tggtgaaggg tatcatcgca gccaagcaag ctcatgaaag 2760
gctgatgggg gaggtgtata attatgaggc ccacggcggg cttattcttt agggaggatc 2820
tatctcgttg ctcaagtgca tggcgcaaag cagttattgg agtgcggatt ttcgttggca 2880
tattattcgc cacgagttag cagacgaaga gaccttcatg aacgtggcca aggccagagt 2940
taagcagatg ttacgccctg ctgcaggcct ttctattatc caatagttgg ttgatctttg 3000
gaaagagcct cggctgaggc ccatactgaa agagatcgat ggatatcgat atgccatgtt 3060
gtttgctagc cagaaccaga tcacatccga tatgctattg cagcttgacg cagatatgga 3120
ggataagttg attcatggga tcgctcagga gtagctcatc catgcacgcc gacaagaaca 3180
gaaattccgt cgagttaacg cagccgctta cgacggattc gaaggtcatc cattcggaat 3240
gtattagttt gcaccagctc cgcgtcacac ctgtcttcat ttgaataaga tgttagcaat 3300
tgtttttagc tttgtcttgt tgtggcaggg cggcaagtgc ttcagacatc attctgtttt 3360
caaattttat gctggagaac agcttcttaa ttcctttgga aataatagac tgcgtcttaa 3420
aattcagatg tctggatata gatatgattg taaaataacc tatttaagtg tcatttagaa 3480
cataagtttt atgaatgttc ttccattttc gtcatcgaac gaataagagt aaatacacct 3540
tttttaacat tacaaataag ttcttatacg ttgtttatac accgggaatc atttccatta 3600
ttttcgcgca aaagtcacgg atattcgtga aagcgacata aactgcgaaa tttgcgggga 3660
gtgtcttgag tttgcctcga ggctagcgca tgcacataga cacacacatc atctcattga 3720
tgcttggtaa taattgtcat tagattgttt ttatgcatag atgcactcga aatcagccaa 3780
ttttagacaa gtatcaaacg gatgtgactt cagtacatta aaaacgtccg caatgtgtta 3840
ttaagttgtc taagcgtcaa tttgatttac aattgaatat atcctgcccc agccagccaa 3900

cagctcgatt tacaattgaa tatatcctgc cggccggccc acgcgtgtcg aggaattctg 3960
atctggcccc catttggacg tgaatgtaga cacgtcgaaa taaagatttc cgaattagaa 4020
taatttgttt attgctttcg cctataaata cgacggatcg taatttgtcg ttttatcaaa 4080
atgtactttc attttataat aacgctgcgg acatctacat ttttgaattg aaaaaaaatt 4140
ggtaattact ctttcttttt ctccatattg accatcatac tcattgctga tccatgtaga 4200
tttcccggac atgaagccat ttacaattga atatatcctg ccgccgctgc cgctttgcac 4260
ccggtggagc ttgcatgttg gtttctacgc agaactgagc cggttaggca gataatttcc 4320
attgagaact gagccatgtg caccttcccc ccaacacggt gagcgacggg gcaacggagt 4380
gatccacatg ggacttttaa acatcatccg tcggatggcg ttgcgagaga agcagtcgat 4440
ccgtgagatc agccgacgca ccgggcaggc gcgcaacacg atcgcaaagt atttgaacgc 4500
aggtacaatc gagccgacgt tcacgcggaa cgaccaagca agcttggctg ccatttttgg 4560
ggtgaggccg ttcgcggccg aggggcgcag cccctggggg gatgggaggc ccgcgttagc 4620
gggccgggag ggttcgagaa ggggggcac cccccttcgg cgtgcgcggt cacgcgcaca 4680
gggcgcagcc ctggttaaaa acaaggttta taaatattgg tttaaaagca ggttaaaaga 4740
caggttagcg gtggccgaaa aacgggcgga aacccttgca aatgctggat ttctgcctg 4800
tggacagccc ctcaaatgtc aataggtgcg cccctcatct gtcagcactc tgcccctcaa 4860
gtgtcaagga tcgcgcccct catctgtcag tagtcgcgcc cctcaagtgt caataccgca 4920
gggcacttat ccccaggctt gtccacatca tctgtgggaa actcgcgtaa aatcaggcgt 4980
tttcgccgat ttgcgaggct ggccagctcc acgtcgccgg ccgaaatcga gcctgcccct 5040
catctgtcaa cgccgcgccg ggtgagtcgg cccctcaagt gtcaacgtcc gcccctcatc 5100
tgtcagtgag ggccaagttt tccgcgaggt atccacaacg ccggcggccg cggtgtctcg 5160
cacacggctt cgacggcgtt tctggcgcgt ttgcagggcc atagacggcc gccagcccag 5220
cggcgagggc aaccagcccg gtgagcgtcg gaaagggtcg acggatcttt tccgctgcat 5280
aaccctgctt cggggtcatt atagcgattt tttcggtata tccatccttt ttcgcacgat 5340
atacaggatt ttgccaaagg gttcgtgtag actttccttg gtgtatccaa cggcgtcagc 5400
cgggcaggat aggtgaagta ggcccacccg cgagcgggtg ttccttcttc actgtccctt 5460
attcgcacct ggcggtgctc aacgggaatc ctgctctgcg aggctggccg gctaccgccg 5520
gcgtaacaga tgagggcaag cggatggctg atgaaaccaa gccaaccagg aagggcagcc 5580
cacctatcaa ggtgtactgc cttccagacg aacgaagagc gattgaggaa aaggcggcgg 5640
cggccggcat gagcctgtcg gcctacctgc tggccgtcgg ccagggctac aaaatcacgg 5700
gcgtcgtgga ctatgagcac gtccgcgagc tggcccgcat caatggcgac ctgggccgcc 5760
tgggcggcct gctgaaactc tggctcaccg acgacccgcg cacggcgcgg ttcggtgatg 5820
ccacgatcct cgccctgctg gcgaagatcg aagagaagca ggacgagctt ggcaaggtca 5880
tgatgggcgt ggtccgcccg agggcagagc catgactttt ttagccgcta aaacggccgg 5940

ggggtgcgcg tgattgccaa gcacgtcccc atgcgctcca tcaagaagag cgacttcgcg 6000
gagctggtat tcgtgcaggg caagattcgg aataccaagt acgagaagga cggccagacg 6060
gtctacggga ccgacttcat tgccgataag gtggattatc tggacaccaa ggcaccaggc 6120
gggtcaaatc aggaataagg gcacattgcc ccggcgtgag tcggggcaat cccgcaagga 6180
gggtgaatga atcggacgtt tgaccggaag gcatacaggc aagaactgat cgacgcgggg 6240
ttttccgccg aggatgccga aaccatcgca agccgcaccg tcatgcgtgc gccccgcgaa 6300
accttccagt ccgtcggctc gatggtccag caagctacgg ccaagatcga gcgcgacagc 6360
gtgcaactgg ctccccctgc cctgcccgcg ccatcggccg ccgtggagcg ttcgcgtcgt 6420
ctcgaacagg aggcggcagg tttggcgaag tcgatgacca tcgacacgcg aggaactatg 6480
acgaccaaga agcgaaaaac cgccggcgag gacctggcaa aacaggtcag cgaggccaag 6540
caggccgcgt tgctgaaaca cacgaagcag cagatcaagg aaatgcagct ttccttgttc 6600
gatattgcgc cgtggccgga cacgatgcga gcgatgccaa acgacacggc ccgctctgcc 6660
ctgttcacca cgcgcaacaa gaaaatcccg cgcgaggcgc tgcaaaacaa ggtcattttc 6720
cacgtcaaca aggacgtgaa gatcacctac accggcgtcg agctgcgggc cgacgatgac 6780
gaactggtgt ggcagcaggt gttggagtac gcgaagcgca cccctatcgg cgagccgatc 6840
accttcacgt tctacgagct ttgccaggac ctgggctggt cgatcaatgg ccggtattac 6900
acgaaggccg aggaatgcct gtcgcgccta caggcgacgg cgatgggctt cacgtccgac 6960
cgcgttgggc acctggaatc ggtgtcgctg ctgcaccgct tccgcgtcct ggaccgtggc 7020
aagaaaacgt cccgttgcca ggtcctgatc gacgaggaaa tcgtcgtgct gtttgctggc 7080
gaccactaca cgaaattcat atgggagaag taccgcaagc tgtcgccgac ggcccgacgg 7140
atgttcgact atttcagctc gcaccgggag ccgtacccgc tcaagctgga aaccttccgc 7200
ctcatgtgcg gatcggattc cacccgcgtg aagaagtggc gcgagcaggt cggcgaagcc 7260
tgcgaagagt tgcgaggcag cggcctggtg gaacacgcct gggtcaatga tgacctggtg 7320
cattgcaaac gctagggcct tgtggggtca gttccggctg ggggttcagc agccagcgct 7380
ttactggcat ttcaggaaca agcgggcact gctcgacgca cttgcttcgc tcagtatcgc 7440
tcgggacgca cggcgcgctc tacgaactgc cgataaacag aggattaaaa ttgacaattg 7500
tgattaaggc tcagattcga cggcttggag cggccgacgt gcaggatttc cgcgagatcc 7560
gattgtcggc cctgaagaaa gctccagaga tgttcgggtc cgtttacgag cacgaggaga 7620
aaaagcccat ggaggcgttc gctgaacggt tgcgagatgc cgtggcattc ggcgcctaca 7680
tcgacggcga gatcattggg ctgtcggtct tcaaacagga ggacggcccc aaggacgctc 7740
acaaggcgca tctgtccggc gttttcgtgg agcccgaaca gcgaggccga ggggtcgccg 7800
gtatgctgct gcgggcgttg ccggcgggtt tattgctcgt gatgatcgtc cgacagattc 7860
caacgggaat ctggtggatg cgcatcttca tcctcggcgc acttaatatt tcgctattct 7920
ggagcttgtt gtttatttcg gtctaccgcc tgccgggcgg ggtcgcggcg acggtaggcg 7980

ctgtgcagcc gctgatggtc gtgttcatct ctgccgctct gctaggtagc ccgatacgat 8040
tgatggcggt cctgggggct atttgcggaa ctgcgggcgt ggcgctgttg gtgttgacac 8100
caaacgcagc gctagatcct gtcggcgtcg cagcgggcct ggcgggggcg gtttccatgg 8160
cgttcggaac cgtgctgacc cgcaagtggc aacctcccgt gcctctgctc acctttaccg 8220
cctggcaact ggcggccgga ggacttctgc tcgttccagt agctttagtg tttgatccgc 8280
caatcccgat gcctacagga accaatgttc tcggcctggc gtggctcggc ctgatcggag 8340
cgggtttaac ctacttcctt tggttccggg ggatctcgcg actcgaacct acagttgttt 8400
ccttactggg ctttctcagc cccgagcgc ttagtgggaa tttgtacccc ttatcgaacc 8460
gggagcacag gatgacgcct aacaattcat tcaagccgac accgcttcgc ggcgcggctt 8520
aattcaggag ttaaacatca tgagggaagc ggtgatcgcc gaagtatcga ctcaactatc 8580
agaggtagtt ggcgtcatcg agcgccatct cgaaccgacg ttgctggccg tacatttgta 8640
cggctccgca gtggatggcg gcctgaagcc acacagtgat attgatttgc tggttacggt 8700
gaccgtaagg cttgatgaaa caacgcggcg agctttgatc aacgaccttt tggaaacttc 8760
ggcttcccct ggagagagcg agattctccg cgctgtagaa gtcaccattg ttgtgcacga 8820
cgacatcatt ccgtggcgtt atccagctaa gcgcgaactg caatttggag aatggcagcg 8880
caatgacatt cttgcaggta tcttcgagcc agccacgatc gacattgatc tggctatctt 8940
gctgacaaaa gcaagagaac atagcgttgc cttggtaggt ccagcggcgg aggaactctt 9000
tgatccggtt cctgaacagg atctatttga ggcgctaaat gaaaccttaa cgctatggaa 9060
ctcgccgccc gactgggctg gcgatgagcg aaatgtagtg cttacgttgt cccgcatttg 9120
gtacagcgca gtaaccggca aaatcgcgcc gaaggatgtc gctgccgact gggcaatgga 9180
gcgcctgccg gcccagtatc agcccgtcat acttgaagct aggcaggctt atcttggaca 9240
agaagatcgc ttggcctcgc gcgcagatca gttggaagaa tttgttcact acgtgaaagg 9300
cgagatcacc aaggtagtcg gcaaataatg tctaacaatt cgttcaagcc gacgccgctt 9360
cgcggcgcgg cttaactcaa gcgttagaga gctggggaag actatgcgcg atctgttgaa 9420
ggtggttcta agcctcgtct tgcgatggca tttcgatcca ttcccattcc gcgctcaaga 9480
tggcttcccc tcggcagttc atcagggcta aatcaatcta gccgacttgt ccggtgaaat 9540
gggctgcact ccaacagaaa caatcaaaca aacatacaca gcgacttatt cacacgagct 9600
caaattacaa cggtatatat cctgccagtc agcatcatca caccaaaagt taggcccgaa 9660
tagtttgaaa ttagaaagct cgcaattgag gtctacaggc caaattcgct cttagccgta 9720
caatattact caccggatcc taaccggtgt gatcatgggc cgcgattaaa aatctcaatt 9780
atatttggtc taatttagtt tggtattgag taaaacaaat tcgaaccaaa ccaaaatata 9840
aatatatagt ttttatatat atgcctttaa gactttttat agaattttct ttaaaaaata 9900
tctagaaata tttgcgactc ttctggcatg taatatttcg ttaaatatga agtgctccat 9960
ttttattaac tttaaataat tggttgtacg atcactttct tatcaagtgt tactaaaatg 10020

cgtcaatctc tttgttcttc catattcata tgtcaaaacc tatcaaaatt cttatatatc 10080
tttttcgaat ttgaagtgaa atttcgataa tttaaaatta aatagaacat atcattattt 10140
aggtatcata ttgattttta tacttaatta ctaaatttgg ttaactttga aagtgtacat 10200
caacgaaaaa ttagtcaaac gactaaaata aataaatatc atgtgttatt aagaaaattc 10260
tcctataaga atattttaat agatcatatg tttgtaaaaa aaattaattt ttactaacac 10320
atatatttac ttatcaaaaa tttgacaaag taagattaaa ataatattca tctaacaaaa 10380
aaaaaaccag aaaatgctga aaacccggca aaaccgaacc aatccaaacc gatatagttg 10440
gtttggtttg attttgatat aaaccgaacc aactcggtcc atttgcaccc ctaatcataa 10500
tagctttaat atttcaagat attattaagt taacgttgtc aatatcctgg aaattttgca 10560
aaatgaatca agcctatatg gctgtaatat gaatttaaaa gcagctcgat gtggtggtaa 10620
tatgtaattt acttgattct aaaaaaatat cccaagtatt aataatttct gctaggaaga 10680
aggttagcta cgatttacag caaagccaga atacaatgaa ccataaagtg attgaagctc 10740
gaaatatacg aaggaacaaa tatttttaaa aaaatacgca atgacttgga acaaaagaaa 10800
gtgatatatt ttttgttctt aaacaagcat cccctctaaa gaatggcagt tttcctttgc 10860
atgtaactat tatgctccct tcgttacaaa aattttggac tactattggg aacttcttct 10920
gaaaatagtg gccaccgctt aattaaggcg cgccatgccc gggcaagcgg ccgcacaagt 10980
ttgtacaaaa aagcaggctc cgcggccgca ctaggtttaa actctagaag ctaggaattc 11040
aaacaaagaa gcgatcgcgc ggccgccatt gtactcccag tatcattata gtgaaagttt 11100
tggctctctc gccggtggtt ttttacctct atttaaaggg gttttccacc taaaaattct 11160
ggtatcattc tcactttact tgttacttta atttctcata atctttggtt gaaattatca 11220
cgcttccgca cacgatatcc ctacaaattt attatttgtt aaacattttc aaaccgcata 11280
aaattttatg aagtcccgtc tatctttaat gtagtctaac attttcatat tgaaatatat 11340
aatttactta attttagcgt tggtagaaag cataatgatt tattcttatt cttcttcata 11400
taaatgttta atatacaata taaacaaatt ctttacctta agaaggattt cccattttat 11460
attttaaaaa tatatttatc aaatattttt caaccacgta aatctcataa taataagttg 11520
tttcaaaagt aataaaattt aactccataa ttttttttatt cgactgatct taaagcaaca 11580
cccagtgaca caactagcca tttttttctt tgaataaaaa aatccaatta tcattgtatt 11640
ttttttatac aatgaaaatt tcaccaaaca atgatttgtg gtatttctga agcaagtcat 11700
gttatgcaaa attctataat tcccatttga cactacggaa gtaactgaag atctgctttt 11760
acatgcgaga cacatcttct aaagtaattt taataatagt tactatattc aagatttcat 11820
atatcaaata ctcaatatta cttctaaaaa attaattaga tataattaaa atattacttt 11880
tttaatttta agtttaattg ttgaatttgt gactattgat ttattattct actatgttta 11940
aattgtttta tagatagttt aaagtaaata taagtaatgt agtagagtgt tagagtgtta 12000
ccctaaacca taaactataa gatttatggt ggactaattt tcatatattt cttattgctt 12060

ttacctttc ttggtatgta agtccgtaac tggaattact gtgggttgcc atgacactct 12120
gtggtctttt ggttcatgca tggatgcttg cgcaagaaaa agacaaagaa caaagaaaaa 12180
agacaaaaca gagagacaaa acgcaatcac acaaccaact caaattagtc actggctgat 12240
caagatcgcc gcgtccatgt atgtctaaat gccatgcaaa gcaacacgtg cttaacatgc 12300
actttaaatg gctcacccat ctcaacccac acacaaacac attgcctttt tcttcatcat 12360
caccacaacc acctgtatat attcattctc ttccgccacc tcaatttctt cacttcaaca 12420
cacgtcaacc tgcatatgcg tgtcatccca tgcccaaatc tccatgcatg ttccaaccac 12480
cttctctctt atataatacc tataaatacc tctaatatca ctcacttctt tcatcatcca 12540
tccatccaga gtactactac tctactacta taatacccca acccaactca tattcaatac 12600
tactctagga tccaacaatg tctgctccaa ccgctgacat cagggctagg gctccagagg 12660
ctaagaaggt tcacatcgct gataccgcta tcaacaggca caattggtac aagcacgtga 12720
actggctcaa cgtcttcctc atcatcggaa tcccactcta cggatgcatc caagctttct 12780
gggttccact tcaactcaag accgctatct gggctgtgat ctactacttc ttcaccggac 12840
ttggaatcac cgctggatac cacaggcttt gggctcactg ctcatactct gctactcttc 12900
cacttaggat ctggcttgct gctgttggag gaggagctgt tgagggatct atcagatggt 12960
gggctaggga tcacagggct catcataggt acaccgatac cgacaaggac ccatactctg 13020
ttaggaaggg acttctctac tctcaccttg gatggatggt gatgaagcag aacccaaaga 13080
ggatcggaag gaccgacatc tctgatctca acgaggaccc agttgttgtt tggcaacaca 13140
ggaactacct caaggttgtg ttcaccatgg gacttgctgt tccaatgctt gttgctggac 13200
ttggatgggg agattggctt ggaggattcg tgtacgctgg aatccttagg atcttcttcg 13260
ttcaacaagc taccttctgc gtgaactctc ttgctcactg gcttggagat caaccattcg 13320
atgataggaa ctctcctagg gatcacgtga tcaccgctct tgttaccctt ggagagggat 13380
accacaactt ccaccacgag ttcccatctg actacaggaa cgctatcgag tggcaccagt 13440
acgatcctac caagtggtct atctgggctt ggaagcaact tggattggct tacgatctca 13500
agaagttcag ggctaacgag atcgagaagg gaagggttca acaacttcag aagaagcttg 13560
ataggaagag ggctactctt gattggggaa ccccacttga tcaacttcca gtgatggaat 13620
gggatgacta cgttgagcaa gctaagaacg gaaggggact tgttgctatc gctggagttg 13680
ttcacgatgt taccgacttc atcaaggatc acccaggagg aaaggctatg atctcttctg 13740
gaatcggaaa ggatgctacc gctatgttca acggaggagt gtactaccac tctaacgcag 13800
ctcacaacct tcttagcacc atgagggtgg gagtgatcag gggaggatgc gaggttgaga 13860
tctggaagag ggctcagaag gagaacgttg agtacgttag ggatggatct ggacaaaggg 13920
tgatcagggc tggagagcaa ccaaccaaga tcccagagcc aatcccaacc gctgatgctg 13980
cttgagtagt tagcttaatc acctaggtca ccagtatgaa ctaaaatgca tgtaggtgta 14040
agagctcatg gagagcatgg aatattgtat ccgaccatgt aacagtataa taactgagct 14100

ccatctcact tcttctatga ataaacaaag gatgttatga tatattaaca ctctatctat 14160
gcaccttatt gttctatgat aaatttcctc ttattattat aaatcatctg aatcgtgacg 14220
gcttatggaa tgcttcaaat agtacaaaaa caaatgtgta ctataagact ttctaaacaa 14280
ttctaacttt agcattgtga acgagacata agtgttaaga agacataaca attataatgg 14340
aagaagtttg tctccattta tatattatat attacccact tatgtattat attaggatgt 14400
taaggagaca taacaattat aaagagagaa gtttgtatcc atttatatat tatatactac 14460
ccatttatat attatactta tccacttatt taatgtcttt ataaggtttg atccatgata 14520
tttctaatat tttagttgat atgtatatga aaaggtacta tttgaactct cttactctgt 14580
ataaaggttg gatcatcctt aaagtgggtc tatttaattt tattgcttct tacagataaa 14640
aaaaaaatta tgagttggtt tgataaaata ttgaaggatt taaaataata ataaataata 14700
aataacatat aatatatgta tataaattta ttataatata acatttatct ataaaaaagt 14760
aaatattgtc ataaatctat acaatcgttt agccttgctg gaacgaatct caattattta 14820
aacgagagta aacatatttg actttttggt tatttaacaa attattattt aacactatat 14880
gaaatttttt tttttatca gcaaagaata aaattaaatt aagaaggaca atggtgtccc 14940
aatccttata caaccaactt ccacaagaaa gtcaagtcag agacaacaaa aaaacaagca 15000
aaggaaattt tttaatttga gttgtcttgt ttgctgcata atttatgcag taaaacacta 15060
cacataaccc ttttagcagt agagcaatgg ttgaccgtgt gcttagcttc ttttatttta 15120
ttttttatc agcaaagaat aaataaaata aaatgagaca cttcagggat gtttcaaccc 15180
ttatacaaaa ccccaaaaac aagtttccta gcaccctacc aacgaattcg cggccgcttt 15240
cctgcatgac atcgtcctgc agagccaagc gcatgcttaa ttaaactagt ctcccagtat 15300
cattatagtg aaagttttgg ctctctcgcc ggtggttttt tacctctatt taaaggggtt 15360
ttccacctaa aaattctggt atcattctca ctttacttgt tactttaatt tctcataatc 15420
tttggttgaa attatcacgc ttccgcacac gatatcccta caaatttatt atttgttaaa 15480
cattttcaaa ccgcataaaa ttttatgaag tcccgtctat ctttaatgta gtctaacatt 15540
ttcatattga aatatataat ttacttaatt ttagcgttgg tagaaagcat aatgatttat 15600
tcttattctt cttcatataa atgtttaata tacaatataa acaaattctt taccttaaga 15660
aggatttccc attttatatt ttaaaaatat atttatcaaa tatttttcaa ccacgtaaat 15720
ctcataataa taagttgttt caaaagtaat aaaatttaac tccataattt ttttattcga 15780
ctgatcttaa agcaacaccc agtgacacaa ctagccattt ttttctttga ataaaaaaat 15840
ccaattatca ttgtattttt tttatacaat gaaaatttca ccaaacaatg atttgtggta 15900
tttctgaagc aagtcatgtt atgcaaaatt ctataattcc catttgacac tacggaagta 15960
actgaagatc tgcttttaca tgcgagacac atcttctaaa gtaattttaa taatagttac 16020
tatattcaag atttcatata tcaaatactc aatattactt ctaaaaaatt aattagatat 16080
aattaaaata ttactttttt aattttaagt ttaattgttg aatttgtgac tattgattta 16140

ttattctact atgtttaaat tgttttatag atagtttaaa gtaaatataa gtaatgtagt 16200
agagtgttag agtgttaccc taaaccataa actataagat ttatggtgga ctaattttca 16260
tatatttctt attgctttta ccttttcttg gtatgtaagt ccgtaactgg aattactgtg 16320
ggttgccatg acactctgtg gtcttttggt tcatgcatgg atcttgcgca agaaaaagac 16380
aaagaacaaa gaaaaaagac aaaacagaga gacaaaacgc aatcacacaa ccaactcaaa 16440
ttagtcactg gctgatcaag atcgccgcgt ccatgtatgt ctaaatgcca tgcaaagcaa 16500
cacgtgctta acatgcactt taaatggctc acccatctca acccacacac aaacacattg 16560
ccttttcttt catcatcacc acaaccacct gtatatattc attctcttcc gccacctcaa 16620
tttcttcact tcaacacacg tcaacctgca tatgcgtgtc atcccatgcc caaatctcca 16680
tgcatgttcc aaccaccttc tctcttatat aatacctata aatacctcta atatcactca 16740
cttctttcat catccatcca tccagagtac tactactcta ctactataat accccaaccc 16800
aactcatatt caatactact ctaggtaccc tgcagggatc caacaatggc tcccaacatt 16860
tctgaggatg tcaatggtgt tctttttgag tcagatgcgg caacccctga tttggctctt 16920
tccacaccac ctgtgcaaaa agctgacaac agacccaagc aacttgtgtg gaggaacatt 16980
ttgcttttcg cttacttgca cctcgcagct ctctacggag gctatttgtt tctcttcagt 17040
gcaaaatggc agaccgacat tttcgcttac attctttatg tcatctctgg actggggata 17100
actgctgggg cacatagact ctgggctcac aagtcataca aagccaagtg gccactcaga 17160
gttatactgg tcatcttcaa cacggttgcc tttcaagacg ctgctatgga ttgggctcgt 17220
gaccatagaa tgcatcacaa gtacagcgag accgacgcgg acccacacaa tgcaacgaga 17280
ggtttcttct tctctcacat tggctggctt cttgttagga aacatcctga tctgaaagaa 17340
aaagggaagg gactcgacat gagtgatctc cttgctgatc caatactccg ttttcagaag 17400
aagtactatc tgatcctcat gcctctggcc tgttttgtga tgccaaccgt tatcccggtt 17460
tacttttggg gagaaacttg gacaaatgct ttcttcgtgg cagccatgtt ccgttatgct 17520
ttcatcctga atgttacctg gttggtgaac tctgccgcac acaagtgggg agacaaaccc 17580
tatgacaagt ccatcaagcc ttccgaaaac ctttcagttg cgatgtttgc tttgggagaa 17640
ggatttcaca attaccatca cacttttccg tgggactaca agacagcaga gcttggaaac 17700
aacaagttga acttcacaac aacgttcatc aatttctttg cgaaaatcgg ttgggcctat 17760
gatttgaaga ctgtgagtga tgacattgtc aagaacaggg tcaagagaac tggcgatgga 17820
agccatcatc tctggggctg gggtgatgag aatcagagca aagaagagat agatgcagcc 17880
attaggatca accctaaaga cgattgagta gttagcttaa tcacctagag ctcggtcacc 17940
tcgagtatca aaatctattt agaaatacac aatattttgt tgcaggcttg ctggagaatc 18000
gatctgctat cataaaaatt acaaaaaaat tttatttgcc tcaattattt taggattggt 18060
attaaggacg cttaaattat ttgtcgggtc actacgcatc attgtgattg agaagatcag 18120
cgatacgaaa tattcgtagt actatcgata atttatttga aaattcataa gaaaagcaaa 18180

cgttacatga attgatgaaa caatacaaag acagataaag ccacgcacat ttaggatatt 18240

ggccgagatt actgaatatt gagtaagatc acggaatttc tgacaggagc atgtcttcaa 18300

ttcagcccaa atggcagttg aaatactcaa accgccccat atgcaggagc ggatcattca 18360

ttgtttgttt ggttgccttt gccaacatgg gagtccaagg ttggcgcgcc gacccagctt 18420

tc 18422

<210> 63
<211> 14233
<212> DNA
<213> Artificial

<220>
<223> Plasmid pDAB7326

<400> 63

ttgtacaaag tggttgcggc cgcttaatta aatttaaatt caattaatgc aatcttgatt 60
ttcaacaacg aaggtaatgg cgtaaaagaa aaaatgtatg ttattgtatt gatctttcat 120
gatgttgaag cgtgccataa tatgatgatg tataattaaa atattaactg tcgcatttta 180
ttgaaatggc actgttattt caaccatatc tttgattctg ttacatgaca cgactgcaag 240
aagtaaataa tagacgccgt tgttaaagaa ttgctatcat atgtgcctaa ctagagggaa 300
tttgagcgtc agacctaatc aaatattaca aaatatctca ctctgtcgcc agcaatggtg 360
taatcagcgc agacaaatgg cgtaaagatc gcggaaaaac ctccccgagt ggcatgatag 420
ctgcctctgt attgctgatt tagtcagcct tatttgactt aagggtgccc tcgttagtga 480
caaattgctt tcaaggagac agccatgccc cacactttgt tgaaaaacaa attgcctttg 540
gggagacggt aaagccagtt gctcttcaat aaggaatgtc gaggaggcaa tgtaaccgcc 600
tctggtagta cacttctcta atccaaaaat caatttgtat tcaagatacc gcaaaaaact 660
tatggtttaa accctgcagg actagtccag aaggtaatta tccaagatgt agcatcaaga 720
atccaatgtt tacgggaaaa actatggaag tattatgtaa gctcagcaag aagcagatca 780
atatgcggca catatgcaac ctatgttcaa aaatgaagaa tgtacagata caagatccta 840
tactgccaga atacgaagaa gaatacgtag aaattgaaaa agaagaacca ggcgaagaaa 900
agaatcttga agacgtaagc actgacgaca acaatgaaaa gaagaagata aggtcggtga 960
ttgtgaaaga gacatagagg acacatgtaa ggtggaaaat gtaagggcgg aaagtaacct 1020
tatcacaaag gaatcttatc ccccactact tatcctttta tatttttccg tgtcattttt 1080
gcccttgagt tttcctatat aaggaaccaa gttcggcatt tgtgaaaaca agaaaaaatt 1140
tggtgtaagc tattttcttt gaagtactga ggatacaact tcagagaaat ttgtaagttt 1200
gtaggtacca gatctggatc ccaaaccatg tctccggaga ggagaccagt tgagattagg 1260
ccagctacag cagctgatat ggccgcggtt tgtgatatcg ttaaccatta cattgagacg 1320
tctacagtga actttaggac agagccacaa acaccacaag agtggattga tgatctagag 1380
aggttgcaag atagataccc ttggttggtt gctgaggttg agggtgttgt ggctggtatt 1440

gcttacgctg ggccctggaa ggctaggaac gcttacgatt ggacagttga gagtactgtt 1500
tacgtgtcac ataggcatca aaggttgggc ctaggatcta cattgtacac acatttgctt 1560
aagtctatgg aggcgcaagg ttttaagtct gtggttgctg ttataggcct tccaaacgat 1620
ccatctgtta ggttgcatga ggctttggga tacacagccc ggggtacatt gcgcgcagct 1680
ggatacaagc atggtggatg gcatgatgtt ggtttttggc aaagggattt tgagttgcca 1740
gctcctccaa ggccagttag gccagttacc caaatctgag tagttagctt aatcacctag 1800
agctcgatcg gcggcaatag cttcttagcg ccatcccggg ttgatcctat ctgtgttgaa 1860
atagttgcgg tgggcaaggc tctctttcag aaagacaggc ggccaaagga acccaaggtg 1920
aggtgggcta tggctctcag ttccttgtgg aagcgcttgg tctaaggtgc agaggtgtta 1980
gcgggatgaa gcaaaagtgt ccgattgtaa caagatatgt tgatcctacg taaggatatt 2040
aaagtatgta ttcatcacta atataatcag tgtattccaa tatgtactac gatttccaat 2100
gtctttattg tcgccgtatg taatcggcgt cacaaaataa tccccggtga ctttctttta 2160
atccaggatg aaataatatg ttattataat ttttgcgatt tggtccgtta taggaattga 2220
agtgtgcttg aggtcggtcg ccaccactcc catttcataa ttttacatgt atttgaaaaa 2280
taaaaattta tggtattcaa tttaaacacg tatacttgta aagaatgata tcttgaaaga 2340
aatatagttt aaatatttat tgataaaata acaagtcagg tattatagtc caagcaaaaa 2400
cataaattta ttgatgcaag tttaaattca gaaatatttc aataactgat tatatcagct 2460
ggtacattgc cgtagatgaa agactgagtg cgatattatg gtgtaataca taggaattcg 2520
tttaaacgat ctgcgtctaa ttttcggtcc aacttgcaca ggaaagacgt cgaccgcggt 2580
agctcttgcc cagcagactg ggcttccagt cctttcgctc gatcgggtcc aatgttgtcc 2640
tcagctgtga accggaagcg gacgaccaac agtggaagaa ctgaaaggaa cgagccgtct 2700
ataccttgat gatcggcctc tggtgaaggg tatcatcgca gccaagcaag ctcatgaaag 2760
gctgatgggg gaggtgtata attatgaggc ccacggcggg cttattcttt agggaggatc 2820
tatctcgttg ctcaagtgca tggcgcaaag cagttattgg agtgcggatt ttcgttggca 2880
tattattcgc cacgagttag cagacgaaga gaccttcatg aacgtggcca aggccagagt 2940
taagcagatg ttacgccctg ctgcaggcct ttctattatc caatagttgg ttgatctttg 3000
gaaagagcct cggctgaggc ccatactgaa agagatcgat ggatatcgat atgccatgtt 3060
gtttgctagc cagaaccaga tcacatccga tatgctattg cagcttgacg cagatatgga 3120
ggataagttg attcatggga tcgctcagga gtagctcatc catgcacgcc gacaagaaca 3180
gaaattccgt cgagttaacg cagccgctta cgacggattc gaaggtcatc cattcggaat 3240
gtattagttt gcaccagctc cgcgtcacac ctgtcttcat ttgaataaga tgttagcaat 3300
tgtttttagc tttgtcttgt tgtggcaggg cggcaagtgc ttcagacatc attctgtttt 3360
caaattttat gctggagaac agcttcttaa ttcctttgga aataatagac tgcgtcttaa 3420
aattcagatg tctggatata gatatgattg taaaataacc tatttaagtg tcatttagaa 3480

cataagtttt atgaatgttc ttccattttc gtcatcgaac gaataagagt aaatacacct 3540
tttttaacat tacaaataag ttcttatacg ttgtttatac accgggaatc atttccatta 3600
ttttcgcgca aaagtcacgg atattcgtga aagcgacata aactgcgaaa tttgcgggga 3660
gtgtcttgag tttgcctcga ggctagcgca tgcacataga cacacacatc atctcattga 3720
tgcttggtaa taattgtcat tagattgttt ttatgcatag atgcactcga aatcagccaa 3780
ttttagacaa gtatcaaacg gatgtgactt cagtacatta aaaacgtccg caatgtgtta 3840
ttaagttgtc taagcgtcaa tttgatttac aattgaatat atcctgcccc agccagccaa 3900
cagctcgatt tacaattgaa tatatcctgc cggccggccc acgcgtgtcg aggaattctg 3960
atctggcccc catttggacg tgaatgtaga cacgtcgaaa taaagatttc cgaattagaa 4020
taatttgttt attgctttcg cctataaata cgacggatcg taatttgtcg ttttatcaaa 4080
atgtactttc attttataat aacgctgcgg acatctacat ttttgaattg aaaaaaaatt 4140
ggtaattact ctttcttttt ctccatattg accatcatac tcattgctga tccatgtaga 4200
tttcccggac atgaagccat ttacaattga atatatcctg ccgccgctgc cgctttgcac 4260
ccggtggagc ttgcatgttg gtttctacgc agaactgagc cggttaggca gataatttcc 4320
attgagaact gagccatgtg caccttcccc ccaacacggt gagcgacggg gcaacggagt 4380
gatccacatg ggacttttaa acatcatccg tcggatggcg ttgcgagaga agcagtcgat 4440
ccgtgagatc agccgacgca ccgggcaggc gcgcaacacg atcgcaaagt atttgaacgc 4500
aggtacaatc gagccgacgt tcacgcggaa cgaccaagca agcttggctg ccatttttgg 4560
ggtgaggccg ttcgcggccg aggggcgcag cccctggggg gatgggaggc ccgcgttagc 4620
gggccgggag ggttcgagaa gggggggcac cccccttcgg cgtgcgcggt cacgcgcaca 4680
gggcgcagcc ctggttaaaa acaaggttta taaatattgg tttaaaagca ggttaaaaga 4740
caggttagcg gtggccgaaa aacgggcgga aacccttgca aatgctggat ttctgcctg 4800
tggacagccc ctcaaatgtc aataggtgcg cccctcatct gtcagcactc tgcccctcaa 4860
gtgtcaagga tcgcgcccct catctgtcag tagtcgcgcc cctcaagtgt caataccgca 4920
gggcacttat ccccaggctt gtccacatca tctgtgggaa actcgcgtaa aatcaggcgt 4980
tttcgccgat ttgcgaggct ggccagctcc acgtcgccgg ccgaaatcga gcctgcccct 5040
catctgtcaa cgccgcgccg ggtgagtcgg cccctcaagt gtcaacgtcc gcccctcatc 5100
tgtcagtgag ggccaagttt tccgcgaggt atccacaacg ccggcggccg cggtgtctcg 5160
cacacggctt cgacggcgtt tctggcgcgt ttgcagggcc atagacggcc gccagcccag 5220
cggcgagggc aaccagcccg gtgagcgtcg gaaagggtcg acggatcttt tccgctgcat 5280
aaccctgctt cggggtcatt atagcgattt tttcggtata tccatccttt ttcgcacgat 5340
atacaggatt ttgccaaagg gttcgtgtag actttccttg gtgtatccaa cggcgtcagc 5400
cgggcaggat aggtgaagta ggcccacccg cgagcgggtg ttccttcttc actgtccctt 5460
attcgcacct ggcggtgctc aacgggaatc ctgctctgcg aggctggccg gctaccgccg 5520

gcgtaacaga tgagggcaag cggatggctg atgaaaccaa gccaaccagg aagggcagcc 5580
cacctatcaa ggtgtactgc cttccagacg aacgaagagc gattgaggaa aaggcggcgg 5640
cggccggcat gagcctgtcg gcctacctgc tggccgtcgg ccagggctac aaaatcacgg 5700
gcgtcgtgga ctatgagcac gtccgcgagc tggcccgcat caatggcgac ctgggccgcc 5760
tgggcggcct gctgaaactc tggctcaccg acgacccgcg cacggcgcgg ttcggtgatg 5820
ccacgatcct cgccctgctg gcgaagatcg aagagaagca ggacgagctt ggcaaggtca 5880
tgatgggcgt ggtccgcccg agggcagagc catgactttt ttagccgcta aaacggccgg 5940
ggggtgcgcg tgattgccaa gcacgtcccc atgcgctcca tcaagaagag cgacttcgcg 6000
gagctggtat tcgtgcaggg caagattcgg aataccaagt acgagaagga cggccagacg 6060
gtctacggga ccgacttcat tgccgataag gtggattatc tggacaccaa ggcaccaggc 6120
gggtcaaatc aggaataagg gcacattgcc ccggcgtgag tcggggcaat cccgcaagga 6180
gggtgaatga atcggacgtt tgaccggaag gcatacaggc aagaactgat cgacgcgggg 6240
ttttccgccg aggatgccga aaccatcgca agccgcaccg tcatgcgtgc gccccgcgaa 6300
accttccagt ccgtcggctc gatggtccag caagctacgg ccaagatcga gcgcgacagc 6360
gtgcaactgg ctccccctgc cctgcccgcg ccatcggccg ccgtggagcg ttcgcgtcgt 6420
ctcgaacagg aggcggcagg tttggcgaag tcgatgacca tcgacacgcg aggaactatg 6480
acgaccaaga agcgaaaaac cgccggcgag gacctggcaa aacaggtcag cgaggccaag 6540
caggccgcgt tgctgaaaca cacgaagcag cagatcaagg aaatgcagct ttccttgttc 6600
gatattgcgc cgtggccgga cacgatgcga gcgatgccaa acgacacggc ccgctctgcc 6660
ctgttcacca cgcgcaacaa gaaaatcccg cgcgaggcgc tgcaaaacaa ggtcattttc 6720
cacgtcaaca aggacgtgaa gatcacctac accggcgtcg agctgcgggc cgacgatgac 6780
gaactggtgt ggcagcaggt gttggagtac gcgaagcgca cccctatcgg cgagccgatc 6840
accttcacgt tctacgagct ttgccaggac ctgggctggt cgatcaatgg ccggtattac 6900
acgaaggccg aggaatgcct gtcgcgccta caggcgacgg cgatgggctt cacgtccgac 6960
cgcgttgggc acctggaatc ggtgtcgctg ctgcaccgct tccgcgtcct ggaccgtggc 7020
aagaaaacgt cccgttgcca ggtcctgatc gacgaggaaa tcgtcgtgct gtttgctggc 7080
gaccactaca cgaaattcat atgggagaag taccgcaagc tgtcgccgac ggcccgacgg 7140
atgttcgact atttcagctc gcaccgggag ccgtacccgc tcaagctgga aaccttccgc 7200
ctcatgtgcg gatcggattc cacccgcgtg aagaagtggc gcgagcaggt cggcgaagcc 7260
tgcgaagagt tgcgaggcag cggcctggtg gaacacgcct gggtcaatga tgacctggtg 7320
cattgcaaac gctagggcct tgtggggtca gttccggctg ggggttcagc agccagcgct 7380
ttactggcat ttcaggaaca agcgggcact gctcgacgca cttgcttcgc tcagtatcgc 7440
tcgggacgca cggcgcgctc tacgaactgc cgataaacag aggattaaaa ttgacaattg 7500
tgattaaggc tcagattcga cggcttggag cggccgacgt gcaggatttc cgcgagatcc 7560

gattgtcggc cctgaagaaa gctccagaga tgttcgggtc cgtttacgag cacgaggaga 7620
aaaagcccat ggaggcgttc gctgaacggt tgcgagatgc cgtggcattc ggcgcctaca 7680
tcgacggcga gatcattggg ctgtcggtct tcaaacagga ggacggcccc aaggacgctc 7740
acaaggcgca tctgtccggc gttttcgtgg agcccgaaca gcgaggccga ggggtcgccg 7800
gtatgctgct gcgggcgttg ccggcgggtt tattgctcgt gatgatcgtc cgacagattc 7860
caacgggaat ctggtggatg cgcatcttca tcctcggcgc acttaatatt tcgctattct 7920
ggagcttgtt gtttatttcg gtctaccgcc tgccgggcgg ggtcgcggcg acggtaggcg 7980
ctgtgcagcc gctgatggtc gtgttcatct ctgccgctct gctaggtagc ccgatacgat 8040
tgatggcggt cctgggggct atttgcggaa ctgcgggcgt ggcgctgttg gtgttgacac 8100
caaacgcagc gctagatcct gtcggcgtcg cagcgggcct ggcgggggcg gtttccatgg 8160
cgttcggaac cgtgctgacc cgcaagtggc aacctcccgt gcctctgctc acctttaccg 8220
cctggcaact ggcggccgga ggacttctgc tcgttccagt agctttagtg tttgatccgc 8280
caatcccgat gcctacagga accaatgttc tcggcctggc gtggctcggc ctgatcggag 8340
cgggtttaac ctacttcctt tggttccggg ggatctcgcg actcgaacct acagttgttt 8400
ccttactggg ctttctcagc cccgagcgc ttagtgggaa tttgtacccc ttatcgaacc 8460
gggagcacag gatgacgcct aacaattcat tcaagccgac accgcttcgc ggcgcggctt 8520
aattcaggag ttaaacatca tgagggaagc ggtgatcgcc gaagtatcga ctcaactatc 8580
agaggtagtt ggcgtcatcg agcgccatct cgaaccgacg ttgctggccg tacatttgta 8640
cggctccgca gtggatggcg gcctgaagcc acacagtgat attgatttgc tggttacggt 8700
gaccgtaagg cttgatgaaa caacgcggcg agctttgatc aacgaccttt tggaaacttc 8760
ggcttcccct ggagagagcg agattctccg cgctgtagaa gtcaccattg ttgtgcacga 8820
cgacatcatt ccgtggcgtt atccagctaa gcgcgaactg caatttggag aatggcagcg 8880
caatgacatt cttgcaggta tcttcgagcc agccacgatc gacattgatc tggctatctt 8940
gctgacaaaa gcaagagaac atagcgttgc cttggtaggt ccagcggcgg aggaactctt 9000
tgatccggtt cctgaacagg atctatttga ggcgctaaat gaaaccttaa cgctatggaa 9060
ctcgccgccc gactgggctg gcgatgagcg aaatgtagtg cttacgttgt cccgcatttg 9120
gtacagcgca gtaaccggca aaatcgcgcc gaaggatgtc gctgccgact gggcaatgga 9180
gcgcctgccg gcccagtatc agcccgtcat acttgaagct aggcaggctt atcttggaca 9240
agaagatcgc ttggcctcgc gcgcagatca gttggaagaa tttgttcact acgtgaaagg 9300
cgagatcacc aaggtagtcg gcaaataatg tctaacaatt cgttcaagcc gacgccgctt 9360
cgcggcgcgg cttaactcaa gcgttagaga gctggggaag actatgcgcg atctgttgaa 9420
ggtggttcta agcctcgtct tgcgatggca tttcgatcca ttcccattcc gcgctcaaga 9480
tggcttcccc tcggcagttc atcagggcta aatcaatcta gccgacttgt ccggtgaaat 9540
gggctgcact ccaacagaaa caatcaaaca aacatacaca gcgacttatt cacacgagct 9600

caaattacaa cggtatatat cctgccagtc agcatcatca caccaaaagt taggcccgaa 9660
tagtttgaaa ttagaaagct cgcaattgag gtctacaggc caaattcgct cttagccgta 9720
caatattact caccggatcc taaccggtgt gatcatgggc cgcgattaaa aatctcaatt 9780
atatttggtc taatttagtt tggtattgag taaaacaaat tcgaaccaaa ccaaaatata 9840
aatatatagt ttttatatat atgcctttaa gactttttat agaattttct ttaaaaaata 9900
tctagaaata tttgcgactc ttctggcatg taatatttcg ttaaatatga agtgctccat 9960
ttttattaac tttaaataat tggttgtacg atcactttct tatcaagtgt tactaaaatg 10020
cgtcaatctc tttgttcttc catattcata tgtcaaaacc tatcaaaatt cttatatatc 10080
tttttcgaat ttgaagtgaa atttcgataa tttaaaatta aatagaacat atcattattt 10140
aggtatcata ttgattttta tacttaatta ctaaatttgg ttaactttga aagtgtacat 10200
caacgaaaaa ttagtcaaac gactaaaata aataaatatc atgtgttatt aagaaaattc 10260
tcctataaga atattttaat agatcatatg tttgtaaaaa aaattaattt ttactaacac 10320
atatatttac ttatcaaaaa tttgacaaag taagattaaa ataatattca tctaacaaaa 10380
aaaaaaccag aaaatgctga aaacccggca aaaccgaacc aatccaaacc gatatagttg 10440
gtttggtttg attttgatat aaaccgaacc aactcggtcc atttgcaccc ctaatcataa 10500
tagctttaat atttcaagat attattaagt taacgttgtc aatatcctgg aaattttgca 10560
aaatgaatca agcctatatg gctgtaatat gaatttaaaa gcagctcgat gtggtggtaa 10620
tatgtaattt acttgattct aaaaaaatat cccaagtatt aataatttct gctaggaaga 10680
aggttagcta cgatttacag caaagccaga atacaatgaa ccataaagtg attgaagctc 10740
gaaatatacg aaggaacaaa tatttttaaa aaaatacgca atgacttgga acaaaagaaa 10800
gtgatatatt ttttgttctt aaacaagcat cccctctaaa gaatggcagt tttcctttgc 10860
atgtaactat tatgctccct tcgttacaaa aattttggac tactattggg aacttcttct 10920
gaaaatagtg gccaccgctt aattaaggcg cgccatgccc gggcaagcgg ccgcacaagt 10980
ttgtacaaaa aagcaggctc cgcggccgca ctaggtttaa actctagaag ctaggaattc 11040
gcggccgctt tcctgcatga catcgtcctg cagagccaag cgcatgctta attaaactag 11100
tctcccagta tcattatagt gaaagttttg gctctctcgc cggtggtttt ttacctctat 11160
ttaaaggggt tttccaccta aaaattctgg tatcattctc actttacttg ttactttaat 11220
ttctcataat ctttggttga aattatcacg cttccgcaca cgatatccct acaaatttat 11280
tatttgttaa acattttcaa accgcataaa attttatgaa gtcccgtcta tctttaatgt 11340
agtctaacat tttcatattg aaatatataa tttacttaat tttagcgttg gtagaaagca 11400
taatgattta ttcttattct tcttcatata aatgtttaat atacaatata aacaaattct 11460
ttaccttaag aaggatttcc cattttatat tttaaaaata tatttatcaa atatttttca 11520
accacgtaaa tctcataata ataagttgtt tcaaaagtaa taaaatttaa ctccataatt 11580
tttttattcg actgatctta aagcaacacc cagtgacaca actagccatt tttttctttg 11640

aataaaaaaa tccaattatc attgtatttt ttttatacaa tgaaaatttc accaaacaat 11700
gatttgtggt atttctgaag caagtcatgt tatgcaaaat tctataattc ccatttgaca 11760
ctacggaagt aactgaagat ctgcttttac atgcgagaca catcttctaa agtaatttta 11820
ataatagtta ctatattcaa gatttcatat atcaaatact caatattact tctaaaaaat 11880
taattagata taattaaaat attacttttt taattttaag tttaattgtt gaatttgtga 11940
ctattgattt attattctac tatgtttaaa ttgttttata gatagtttaa agtaaatata 12000
agtaatgtag tagagtgtta gagtgttacc ctaaaccata aactataaga tttatggtgg 12060
actaattttc atatatttct tattgctttt accttttctt ggtatgtaag tccgtaactg 12120
gaattactgt gggttgccat gacactctgt ggtcttttgg ttcatgcatg gatcttgcgc 12180
aagaaaaaga caaagaacaa agaaaaaaga caaaacagag agacaaaacg caatcacaca 12240
accaactcaa attagtcact ggctgatcaa gatcgccgcg tccatgtatg tctaaatgcc 12300
atgcaaagca acacgtgctt aacatgcact ttaaatggct cacccatctc aacccacaca 12360
caaacacatt gccttttttct tcatcatcac cacaaccacc tgtatatatt cattctcttc 12420
cgccacctca atttcttcac ttcaacacac gtcaacctgc atatgcgtgt catcccatgc 12480
ccaaatctcc atgcatgttc caaccacctt ctctcttata taatacctat aaatacctct 12540
aatatcactc acttctttca tcatccatcc atccagagta ctactactct actactataa 12600
taccccaacc caactcatat tcaatactac tctaggtacc ctgcagggat ccaacaatgg 12660
ctcccaacat ttctgaggat gtcaatggtg ttctttttga gtcagatgcg gcaacccctg 12720
atttggctct ttccacacca cctgtgcaaa aagctgacaa cagacccaag caacttgtgt 12780
ggaggaacat tttgcttttc gcttacttgc acctcgcagc tctctacgga ggctatttgt 12840
ttctcttcag tgcaaaatgg cagaccgaca ttttcgctta cattctttat gtcatctctg 12900
gactggggat aactgctggg gcacatagac tctgggctca caagtcatac aaagccaagt 12960
ggccactcag agttatactg gtcatcttca acacggttgc ctttcaagac gctgctatgg 13020
attgggctcg tgaccataga atgcatcaca agtacagcga gaccgacgcg gacccacaca 13080
atgcaacgag aggtttcttc ttctctcaca ttggctggct tcttgttagg aaacatcctg 13140
atctgaaaga aaaagggaag ggactcgaca tgagtgatct ccttgctgat ccaatactcc 13200
gttttcagaa gaagtactat ctgatcctca tgcctctggc ctgttttgtg atgccaaccg 13260
ttatcccggt ttacttttgg ggagaaactt ggacaaatgc tttcttcgtg gcagccatgt 13320
tccgttatgc tttcatcctg aatgttacct ggttggtgaa ctctgccgca cacaagtggg 13380
gagacaaacc ctatgacaag tccatcaagc cttccgaaaa cctttcagtt gcgatgtttg 13440
ctttgggaga aggatttcac aattaccatc acacttttcc gtgggactac aagacagcag 13500
agcttggaaa caacaagttg aacttcacaa caacgttcat caatttcttt gcgaaaatcg 13560
gttgggccta tgatttgaag actgtgagtg atgacattgt caagaacagg gtcaagagaa 13620
ctggcgatgg aagccatcat ctctggggct ggggtgatga gaatcagagc aaagaagaga 13680

tagatgcagc cattaggatc aaccctaaag acgattgagt agttagctta atcacctaga 13740
gctcggtcac ctcgagtatc aaaatctatt tagaaataca caatattttg ttgcaggctt 13800
gctggagaat cgatctgcta tcataaaaat tacaaaaaaa ttttatttgc ctcaattatt 13860
ttaggattgg tattaaggac gcttaaatta tttgtcgggt cactacgcat cattgtgatt 13920
gagaagatca gcgatacgaa atattcgtag tactatcgat aatttatttg aaaattcata 13980
agaaaagcaa acgttacatg aattgatgaa acaatacaaa gacagataaa gccacgcaca 14040
tttaggatat tggccgagat tactgaatat tgagtaagat cacggaattt ctgacaggag 14100
catgtcttca attcagccca aatggcagtt gaaatactca aaccgcccca tatgcaggag 14160
cggatcattc attgtttgtt tggttgcctt tgccaacatg ggagtccaag gttggcgcgc 14220
cgacccagct ttc 14233

<210> 64
<211> 14575
<212> DNA
<213> Artificial

<220>
<223> Plasmid pDAB7328

<400> 64

```
ttgtacaaag tggttgcggc cgcttaatta aatttaaatt caattaatgc aatcttgatt      60
ttcaacaacg aaggtaatgg cgtaaaagaa aaaatgtatg ttattgtatt gatctttcat     120
gatgttgaag cgtgccataa tatgatgatg tataattaaa atattaactg tcgcatttta     180
ttgaaatggc actgttattt caaccatatc tttgattctg ttacatgaca cgactgcaag     240
aagtaaataa tagacgccgt tgttaaagaa ttgctatcat atgtgcctaa ctagagggaa     300
tttgagcgtc agacctaatc aaatattaca aaatatctca ctctgtcgcc agcaatggtg     360
taatcagcgc agacaaatgg cgtaaagatc gcggaaaaac ctccccgagt ggcatgatag     420
ctgcctctgt attgctgatt tagtcagcct tatttgactt aagggtgccc tcgttagtga     480
caaattgctt tcaaggagac agccatgccc cacactttgt tgaaaaacaa attgcctttg     540
gggagacggt aaagccagtt gctcttcaat aaggaatgtc gaggaggcaa tgtaaccgcc     600
tctggtagta cacttctcta atccaaaaat caatttgtat tcaagatacc gcaaaaaact     660
tatggtttaa accctgcagg actagtccag aaggtaatta tccaagatgt agcatcaaga     720
atccaatgtt tacgggaaaa actatggaag tattatgtaa gctcagcaag aagcagatca     780
atatgcggca catatgcaac ctatgttcaa aaatgaagaa tgtacagata caagatccta     840
tactgccaga atacgaagaa gaatacgtag aaattgaaaa agaagaacca ggcgaagaaa     900
agaatcttga agacgtaagc actgacgaca acaatgaaaa gaagaagata aggtcggtga     960
ttgtgaaaga gacatagagg acacatgtaa ggtggaaaat gtaagggcgg aaagtaacct    1020
tatcacaaag gaatcttatc ccccactact tatcctttta tatttttccg tgtcattttt    1080
gcccttgagt tttcctatat aaggaaccaa gttcggcatt tgtgaaaaca agaaaaaatt    1140
```

tggtgtaagc tattttcttt gaagtactga ggatacaact tcagagaaat ttgtaagttt 1200
gtaggtacca gatctggatc ccaaaccatg tctccggaga ggagaccagt tgagattagg 1260
ccagctacag cagctgatat ggccgcggtt tgtgatatcg ttaaccatta cattgagacg 1320
tctacagtga actttaggac agagccacaa acaccacaag agtggattga tgatctagag 1380
aggttgcaag atagataccc ttggttggtt gctgaggttg agggtgttgt ggctggtatt 1440
gcttacgctg ggccctggaa ggctaggaac gcttacgatt ggacagttga gagtactgtt 1500
tacgtgtcac ataggcatca aaggttgggc ctaggatcta cattgtacac acatttgctt 1560
aagtctatgg aggcgcaagg ttttaagtct gtggttgctg ttataggcct tccaaacgat 1620
ccatctgtta ggttgcatga ggctttggga tacacagccc ggggtacatt gcgcgcagct 1680
ggatacaagc atggtggatg gcatgatgtt ggtttttggc aaagggattt tgagttgcca 1740
gctcctccaa ggccagttag gccagttacc caaatctgag tagttagctt aatcacctag 1800
agctcgatcg gcggcaatag cttcttagcg ccatcccggg ttgatcctat ctgtgttgaa 1860
atagttgcgg tgggcaaggc tctctttcag aaagacaggc ggccaaagga acccaaggtg 1920
aggtgggcta tggctctcag ttccttgtgg aagcgcttgg tctaaggtgc agaggtgtta 1980
gcgggatgaa gcaaaagtgt ccgattgtaa caagatatgt tgatcctacg taaggatatt 2040
aaagtatgta ttcatcacta atataatcag tgtattccaa tatgtactac gatttccaat 2100
gtctttattg tcgccgtatg taatcggcgt cacaaaataa tccccggtga ctttctttta 2160
atccaggatg aaataatatg ttattataat ttttgcgatt tggtccgtta taggaattga 2220
agtgtgcttg aggtcggtcg ccaccactcc catttcataa ttttacatgt atttgaaaaa 2280
taaaaattta tggtattcaa tttaaacacg tatacttgta aagaatgata tcttgaaaga 2340
aatatagttt aaatatttat tgataaaata acaagtcagg tattatagtc caagcaaaaa 2400
cataaattta ttgatgcaag tttaaattca gaaatatttc aataactgat tatatcagct 2460
ggtacattgc cgtagatgaa agactgagtg cgatattatg gtgtaataca taggaattcg 2520
tttaaacgat ctgcgtctaa ttttcggtcc aacttgcaca ggaaagacgt cgaccgcggt 2580
agctcttgcc cagcagactg ggcttccagt cctttcgctc gatcgggtcc aatgttgtcc 2640
tcagctgtga accggaagcg gacgaccaac agtggaagaa ctgaaaggaa cgagccgtct 2700
ataccttgat gatcggcctc tggtgaaggg tatcatcgca gccaagcaag ctcatgaaag 2760
gctgatgggg gaggtgtata attatgaggc ccacggcggg cttattcttt agggaggatc 2820
tatctcgttg ctcaagtgca tggcgcaaag cagttattgg agtgcggatt ttcgttggca 2880
tattattcgc cacgagttag cagacgaaga gaccttcatg aacgtggcca aggccagagt 2940
taagcagatg ttacgccctg ctgcaggcct ttctattatc caatagttgg ttgatctttg 3000
gaaagagcct cggctgaggc ccatactgaa agagatcgat ggatatcgat atgccatgtt 3060
gtttgctagc cagaaccaga tcacatccga tatgctattg cagcttgacg cagatatgga 3120
ggataagttg attcatggga tcgctcagga gtagctcatc catgcacgcc gacaagaaca 3180

gaaattccgt cgagttaacg cagccgctta cgacggattc gaaggtcatc cattcggaat 3240
gtattagttt gcaccagctc cgcgtcacac ctgtcttcat ttgaataaga tgttagcaat 3300
tgtttttagc tttgtcttgt tgtggcaggg cggcaagtgc ttcagacatc attctgtttt 3360
caaattttat gctggagaac agcttcttaa ttcctttgga aataatagac tgcgtcttaa 3420
aattcagatg tctggatata gatatgattg taaaataacc tatttaagtg tcatttagaa 3480
cataagtttt atgaatgttc ttccattttc gtcatcgaac gaataagagt aaatacacct 3540
tttttaacat tacaaataag ttcttatacg ttgtttatac accgggaatc atttccatta 3600
ttttcgcgca aaagtcacgg atattcgtga aagcgacata aactgcgaaa tttgcgggga 3660
gtgtcttgag tttgcctcga ggctagcgca tgcacataga cacacacatc atctcattga 3720
tgcttggtaa taattgtcat tagattgttt ttatgcatag atgcactcga aatcagccaa 3780
ttttagacaa gtatcaaacg gatgtgactt cagtacatta aaaacgtccg caatgtgtta 3840
ttaagttgtc taagcgtcaa tttgatttac aattgaatat atcctgcccc agccagccaa 3900
cagctcgatt tacaattgaa tatatcctgc cggccggccc acgcgtgtcg aggaattctg 3960
atctggcccc catttggacg tgaatgtaga cacgtcgaaa taaagatttc cgaattagaa 4020
taatttgttt attgctttcg cctataaata cgacggatcg taatttgtcg ttttatcaaa 4080
atgtactttc attttataat aacgctgcgg acatctacat ttttgaattg aaaaaaaatt 4140
ggtaattact ctttcttttt ctccatattg accatcatac tcattgctga tccatgtaga 4200
tttcccggac atgaagccat ttacaattga atatatcctg ccgccgctgc cgctttgcac 4260
ccggtggagc ttgcatgttg gtttctacgc agaactgagc cggttaggca gataatttcc 4320
attgagaact gagccatgtg caccttcccc ccaacacggt gagcgacggg gcaacggagt 4380
gatccacatg ggacttttaa acatcatccg tcggatggcg ttgcgagaga agcagtcgat 4440
ccgtgagatc agccgacgca ccgggcaggc gcgcaacacg atcgcaaagt atttgaacgc 4500
aggtacaatc gagccgacgt tcacgcggaa cgaccaagca agcttggctg ccatttttgg 4560
ggtgaggccg ttcgcggccg aggggcgcag cccctggggg gatgggaggc ccgcgttagc 4620
gggccgggag ggttcgagaa ggggggcac ccccttcgg cgtgcgcggt cacgcgcaca 4680
gggcgcagcc ctggttaaaa acaaggttta taaatattgg tttaaaagca ggttaaaaga 4740
caggttagcg gtggccgaaa aacgggcgga aacccttgca aatgctggat tttctgcctg 4800
tggacagccc ctcaaatgtc aataggtgcg cccctcatct gtcagcactc tgcccctcaa 4860
gtgtcaagga tcgcgcccct catctgtcag tagtcgcgcc cctcaagtgt caataccgca 4920
gggcacttat ccccaggctt gtccacatca tctgtgggaa actcgcgtaa aatcaggcgt 4980
tttcgccgat ttgcgaggct ggccagctcc acgtcgccgg ccgaaatcga gcctgcccct 5040
catctgtcaa cgccgcgccg ggtgagtcgg cccctcaagt gtcaacgtcc gcccctcatc 5100
tgtcagtgag ggccaagttt tccgcgaggt atccacaacg ccggcggccg cggtgtctcg 5160
cacacggctt cgacggcgtt tctggcgcgt ttgcagggcc atagacggcc gccagcccag 5220

cggcgagggc aaccagcccg gtgagcgtcg gaaagggtcg acggatcttt tccgctgcat 5280
aaccctgctt cggggtcatt atagcgattt tttcggtata tccatccttt ttcgcacgat 5340
atacaggatt ttgccaaagg gttcgtgtag actttccttg gtgtatccaa cggcgtcagc 5400
cgggcaggat aggtgaagta ggcccacccg cgagcgggtg ttccttcttc actgtccctt 5460
attcgcacct ggcggtgctc aacgggaatc ctgctctgcg aggctggccg gctaccgccg 5520
gcgtaacaga tgagggcaag cggatggctg atgaaaccaa gccaaccagg aagggcagcc 5580
cacctatcaa ggtgtactgc cttccagacg aacgaagagc gattgaggaa aaggcggcgg 5640
cggccggcat gagcctgtcg gcctacctgc tggccgtcgg ccagggctac aaaatcacgg 5700
gcgtcgtgga ctatgagcac gtccgcgagc tggcccgcat caatggcgac ctgggccgcc 5760
tgggcggcct gctgaaactc tggctcaccg acgacccgcg cacggcgcgg ttcggtgatg 5820
ccacgatcct cgccctgctg gcgaagatcg aagagaagca ggacgagctt ggcaaggtca 5880
tgatgggcgt ggtccgcccg agggcagagc catgactttt ttagccgcta aaacggccgg 5940
ggggtgcgcg tgattgccaa gcacgtcccc atgcgctcca tcaagaagag cgacttcgcg 6000
gagctggtat tcgtgcaggg caagattcgg aataccaagt acgagaagga cggccagacg 6060
gtctacggga ccgacttcat tgccgataag gtggattatc tggacaccaa ggcaccaggc 6120
gggtcaaatc aggaataagg gcacattgcc ccggcgtgag tcggggcaat cccgcaagga 6180
gggtgaatga atcggacgtt tgaccggaag gcatacaggc aagaactgat cgacgcgggg 6240
ttttccgccg aggatgccga aaccatcgca agccgcaccg tcatgcgtgc gccccgcgaa 6300
accttccagt ccgtcggctc gatggtccag caagctacgg ccaagatcga gcgcgacagc 6360
gtgcaactgg ctccccctgc cctgcccgcg ccatcggccg ccgtggagcg ttcgcgtcgt 6420
ctcgaacagg aggcggcagg tttggcgaag tcgatgacca tcgacacgcg aggaactatg 6480
acgaccaaga agcgaaaaac cgccggcgag gacctggcaa aacaggtcag cgaggccaag 6540
caggccgcgt tgctgaaaca cacgaagcag cagatcaagg aaatgcagct ttccttgttc 6600
gatattgcgc cgtggccgga cacgatgcga gcgatgccaa acgacacggc ccgctctgcc 6660
ctgttcacca cgcgcaacaa gaaaatcccg cgcgaggcgc tgcaaaacaa ggtcattttc 6720
cacgtcaaca aggacgtgaa gatcacctac accggcgtcg agctgcgggc cgacgatgac 6780
gaactggtgt ggcagcaggt gttggagtac gcgaagcgca cccctatcgg cgagccgatc 6840
accttcacgt tctacgagct ttgccaggac ctgggctggt cgatcaatgg ccggtattac 6900
acgaaggccg aggaatgcct gtcgcgccta caggcgacgg cgatgggctt cacgtccgac 6960
cgcgttgggc acctggaatc ggtgtcgctg ctgcaccgct tccgcgtcct ggaccgtggc 7020
aagaaaacgt cccgttgcca ggtcctgatc gacgaggaaa tcgtcgtgct gtttgctggc 7080
gaccactaca cgaaattcat atgggagaag taccgcaagc tgtcgccgac ggcccgacgg 7140
atgttcgact atttcagctc gcaccgggag ccgtacccgc tcaagctgga aaccttccgc 7200
ctcatgtgcg gatcggattc cacccgcgtg aagaagtggc gcgagcaggt cggcgaagcc 7260

```
tgcgaagagt tgcgaggcag cggcctggtg gaacacgcct gggtcaatga tgacctggtg    7320
cattgcaaac gctagggcct tgtggggtca gttccggctg gggttcagc agccagcgct     7380
ttactggcat ttcaggaaca agcgggcact gctcgacgca cttgcttcgc tcagtatcgc    7440
tcgggacgca cggcgcgctc tacgaactgc cgataaacag aggattaaaa ttgacaattg    7500
tgattaaggc tcagattcga cggcttggag cggccgacgt gcaggatttc cgcgagatcc    7560
gattgtcggc cctgaagaaa gctccagaga tgttcgggtc cgtttacgag cacgaggaga    7620
aaaagcccat ggaggcgttc gctgaacggt tgcgagatgc cgtggcattc ggcgcctaca    7680
tcgacggcga gatcattggg ctgtcggtct tcaaacagga ggacggcccc aaggacgctc    7740
acaaggcgca tctgtccggc gttttcgtgg agcccgaaca gcgaggccga ggggtcgccg    7800
gtatgctgct gcgggcgttg ccggcgggtt tattgctcgt gatgatcgtc cgacagattc    7860
caacgggaat ctggtggatg cgcatcttca tcctcggcgc acttaatatt tcgctattct    7920
ggagcttgtt gtttatttcg gtctaccgcc tgccgggcgg ggtcgcggcg acggtaggcg    7980
ctgtgcagcc gctgatggtc gtgttcatct ctgccgctct gctaggtagc ccgatacgat    8040
tgatggcggt cctgggggct atttgcggaa ctgcgggcgt ggcgctgttg gtgttgacac    8100
caaacgcagc gctagatcct gtcggcgtcg cagcgggcct ggcgggggcg gtttccatgg    8160
cgttcggaac cgtgctgacc cgcaagtggc aacctcccgt gcctctgctc acctttaccg    8220
cctggcaact ggcggccgga ggacttctgc tcgttccagt agctttagtg tttgatccgc    8280
caatcccgat gcctacagga accaatgttc tcggcctggc gtggctcggc ctgatcggag    8340
cgggtttaac ctacttcctt tggttccggg ggatctcgcg actcgaacct acagttgttt    8400
ccttactggg ctttctcagc cccgagcgc ttagtgggaa tttgtacccc ttatcgaacc     8460
gggagcacag gatgacgcct aacaattcat tcaagccgac accgcttcgc ggcgcggctt    8520
aattcaggag ttaaacatca tgagggaagc ggtgatcgcc gaagtatcga ctcaactatc    8580
agaggtagtt ggcgtcatcg agcgccatct cgaaccgacg ttgctggccg tacatttgta    8640
cggctccgca gtggatggcg gcctgaagcc acacagtgat attgatttgc tggttacggt    8700
gaccgtaagg cttgatgaaa caacgcggcg agctttgatc aacgaccttt tggaaacttc    8760
ggcttcccct ggagagagcg agattctccg cgctgtagaa gtcaccattg ttgtgcacga    8820
cgacatcatt ccgtggcgtt atccagctaa gcgcgaactg caatttggag aatggcagcg    8880
caatgacatt cttgcaggta tcttcgagcc agccacgatc gacattgatc tggctatctt    8940
gctgacaaaa gcaagagaac atagcgttgc cttggtaggt ccagcggcgg aggaactctt    9000
tgatccggtt cctgaacagg atctatttga ggcgctaaat gaaaccttaa cgctatggaa    9060
ctcgccgccc gactgggctg gcgatgagcg aaatgtagtg cttacgttgt cccgcatttg    9120
gtacagcgca gtaaccggca aaatcgcgcc gaaggatgtc gctgccgact gggcaatgga    9180
gcgcctgccg gcccagtatc agcccgtcat acttgaagct aggcaggctt atcttggaca    9240
agaagatcgc ttggcctcgc gcgcagatca gttggaagaa tttgttcact acgtgaaagg    9300
```

cgagatcacc aaggtagtcg gcaaataatg tctaacaatt cgttcaagcc gacgccgctt 9360
cgcggcgcgg cttaactcaa gcgttagaga gctggggaag actatgcgcg atctgttgaa 9420
ggtggttcta agcctcgtct tgcgatggca tttcgatcca ttcccattcc gcgctcaaga 9480
tggcttcccc tcggcagttc atcagggcta aatcaatcta gccgacttgt ccggtgaaat 9540
gggctgcact ccaacagaaa caatcaaaca aacatacaca gcgacttatt cacacgagct 9600
caaattacaa cggtatatat cctgccagtc agcatcatca caccaaaagt taggcccgaa 9660
tagtttgaaa ttagaaagct cgcaattgag gtctacaggc caaattcgct cttagccgta 9720
caatattact caccggatcc taaccggtgt gatcatgggc cgcgattaaa aatctcaatt 9780
atatttggtc taatttagtt tggtattgag taaaacaaat tcgaaccaaa ccaaaatata 9840
aatatatagt ttttatatat atgcctttaa gactttttat agaattttct ttaaaaaata 9900
tctagaaata tttgcgactc ttctggcatg taatatttcg ttaaatatga agtgctccat 9960
ttttattaac tttaaataat tggttgtacg atcactttct tatcaagtgt tactaaaatg 10020
cgtcaatctc tttgttcttc catattcata tgtcaaaacc tatcaaaatt cttatatatc 10080
tttttcgaat ttgaagtgaa atttcgataa tttaaaatta aatagaacat atcattattt 10140
aggtatcata ttgattttta tacttaatta ctaaatttgg ttaactttga aagtgtacat 10200
caacgaaaaa ttagtcaaac gactaaaata aataaatatc atgtgttatt aagaaaattc 10260
tcctataaga atattttaat agatcatatg tttgtaaaaa aaattaattt ttactaacac 10320
atatatttac ttatcaaaaa tttgacaaag taagattaaa ataatattca tctaacaaaa 10380
aaaaaaccag aaaatgctga aaacccggca aaaccgaacc aatccaaacc gatatagttg 10440
gtttggtttg attttgatat aaaccgaacc aactcggtcc atttgcaccc ctaatcataa 10500
tagctttaat atttcaagat attattaagt taacgttgtc aatatcctgg aaattttgca 10560
aaatgaatca agcctatatg gctgtaatat gaatttaaaa gcagctcgat gtggtggtaa 10620
tatgtaattt acttgattct aaaaaaatat cccaagtatt aataatttct gctaggaaga 10680
aggttagcta cgatttacag caaagccaga atacaatgaa ccataaagtg attgaagctc 10740
gaaatatacg aaggaacaaa tatttttaaa aaaatacgca atgacttgga acaaaagaaa 10800
gtgatatatt ttttgttctt aaacaagcat cccctctaaa gaatggcagt tttcctttgc 10860
atgtaactat tatgctccct tcgttacaaa aattttggac tactattggg aacttcttct 10920
gaaaatagtg gccaccgctt aattaaggcg cgccatgccc gggcaagcgg ccgcacaagt 10980
ttgtacaaaa aagcaggctc cgcggccgca ctaggtttaa actctagaag ctaggaattc 11040
gcggccgctt tcctgcatga catcgtcctg cagagccaag cgcatgctta attaaactag 11100
tctcccagta tcattatagt gaaagttttg gctctctcgc cggtggtttt ttacctctat 11160
ttaaaggggt tttccaccta aaaattctgg tatcattctc actttacttg ttactttaat 11220
ttctcataat ctttggttga aattatcacg cttccgcaca cgatatccct acaaatttat 11280
tatttgttaa acattttcaa accgcataaa attttatgaa gtcccgtcta tctttaatgt 11340

agtctaacat tttcatattg aaatatataa tttacttaat tttagcgttg gtagaaagca 11400
taatgattta ttcttattct tcttcatata aatgtttaat atacaatata aacaaattct 11460
ttaccttaag aaggatttcc cattttatat tttaaaaata tatttatcaa atatttttca 11520
accacgtaaa tctcataata ataagttgtt tcaaaagtaa taaaatttaa ctccataatt 11580
tttttattcg actgatctta aagcaacacc cagtgacaca actagccatt tttttctttg 11640
aataaaaaaa tccaattatc attgtatttt ttttatacaa tgaaaatttc accaaacaat 11700
gatttgtggt atttctgaag caagtcatgt tatgcaaaat tctataattc ccatttgaca 11760
ctacggaagt aactgaagat ctgcttttac atgcgagaca catcttctaa agtaatttta 11820
ataatagtta ctatattcaa gatttcatat atcaaatact caatattact tctaaaaaat 11880
taattagata taattaaaat attacttttt taattttaag tttaattgtt gaatttgtga 11940
ctattgattt attattctac tatgtttaaa ttgttttata gatagtttaa agtaaatata 12000
agtaatgtag tagagtgtta gagtgttacc ctaaaccata aactataaga tttatggtgg 12060
actaattttc atatatttct tattgctttt accttttctt ggtatgtaag tccgtaactg 12120
gaattactgt gggttgccat gacactctgt ggtcttttgg ttcatgcatg gatcttgcgc 12180
aagaaaaaga caaagaacaa agaaaaaaga caaaacagag agacaaaacg caatcacaca 12240
accaactcaa attagtcact ggctgatcaa gatcgccgcg tccatgtatg tctaaatgcc 12300
atgcaaagca acacgtgctt aacatgcact ttaaatggct cacccatctc aacccacaca 12360
caaacacatt gccttttttct tcatcatcac cacaaccacc tgtatatatt cattctcttc 12420
cgccacctca atttcttcac ttcaacacac gtcaacctgc atatgcgtgt catcccatgc 12480
ccaaatctcc atgcatgttc caaccacctt ctctcttata taatacctat aaatacctct 12540
aatatcactc acttctttca tcatccatcc atccagagta ctactactct actactataa 12600
taccccaacc caactcatat tcaatactac tctaggtacc ctgcagggat ccaacaatgt 12660
ctgctccaac cgctgacatc agggctaggg ctccagaggc taagaaggtt cacatcgctg 12720
ataccgctat caacaggcac aattggtaca agcacgtgaa ctggctcaac gtcttcctca 12780
tcatcggaat cccactctac ggatgcatcc aagctttctg ggttccactt caactcaaga 12840
ccgctatctg ggctgtgatc tactacttct tcaccggact tggaatcacc gctggatacc 12900
acaggctttg ggctcactgc tcatactctg ctactcttcc acttaggatc tggcttgctg 12960
ctgttggagg aggagctgtt gagggatcta tcagatggtg ggctagggat cacagggctc 13020
atcataggta caccgatacc gacaaggacc catactctgt taggaaggga cttctctact 13080
ctcaccttgg atggatggtg atgaagcaga acccaaagag gatcggaagg accgacatct 13140
ctgatctcaa cgaggaccca gttgttgttt ggcaacacag gaactacctc aaggttgtgt 13200
tcaccatggg acttgctgtt ccaatgcttg ttgctggact tggatgggga gattggcttg 13260
gaggattcgt gtacgctgga atccttagga tcttcttcgt tcaacaagct accttctgcg 13320
tgaactctct tgctcactgg cttggagatc aaccattcga tgataggaac tctcctaggg 13380

atcacgtgat caccgctctt gttacccttg gagagggata ccacaacttc caccacgagt 13440
tcccatctga ctacaggaac gctatcgagt ggcaccagta cgatcctacc aagtggtcta 13500
tctgggcttg gaagcaactt ggattggctt acgatctcaa gaagttcagg gctaacgaga 13560
tcgagaaggg aagggttcaa caacttcaga agaagcttga taggaagagg gctactcttg 13620
attggggaac cccacttgat caacttccag tgatggaatg ggatgactac gttgagcaag 13680
ctaagaacgg aaggggactt gttgctatcg ctggagttgt tcacgatgtt accgacttca 13740
tcaaggatca cccaggagga aaggctatga tctcttctgg aatcggaaag gatgctaccg 13800
ctatgttcaa cggaggagtg tactaccact ctaacgcagc tcacaacctt cttagcacca 13860
tgagggtggg agtgatcagg ggaggatgcg aggttgagat ctggaagagg gctcagaagg 13920
agaacgttga gtacgttagg gatggatctg gacaaagggt gatcagggct ggagagcaac 13980
caaccaagat cccagagcca atcccaaccg ctgatgctgc ttgagtagtt agcttaatca 14040
cctaggtcac cagtatgaac taaaatgcat gtaggtgtaa gagctcggtc acctcgagta 14100
tcaaaatcta tttagaaata cacaatattt tgttgcaggc ttgctggaga atcgatctgc 14160
tatcataaaa attacaaaaa aattttattt gcctcaatta ttttaggatt ggtattaagg 14220
acgcttaaat tatttgtcgg gtcactacgc atcattgtga ttgagaagat cagcgatacg 14280
aaatattcgt agtactatcg ataatttatt tgaaaattca taagaaaagc aaacgttaca 14340
tgaattgatg aaacaataca aagacagata aagccacgca catttaggat attggccgag 14400
attactgaat attgagtaag atcacggaat ttctgacagg agcatgtctt caattcagcc 14460
caaatggcag ttgaaatact caaaccgccc catatgcagg agcggatcat tcattgtttg 14520
tttggttgcc tttgccaaca tgggagtcca aggttggcgc gccgacccag ctttc 14575

<210> 65
<211> 14602
<212> DNA
<213> Artificial

<220>
<223> Plasmid pDAB7330

<400> 65

```
ttgtacaaag tggttgcggc cgcttaatta aatttaaatt caattaatgc aatcttgatt      60
ttcaacaacg aaggtaatgg cgtaaaagaa aaaatgtatg ttattgtatt gatctttcat     120
gatgttgaag cgtgccataa tatgatgatg tataattaaa atattaactg tcgcatttta     180
ttgaaatggc actgttattt caaccatatc tttgattctg ttacatgaca cgactgcaag     240
aagtaaataa tagacgccgt tgttaaagaa ttgctatcat atgtgcctaa ctagagggaa     300
tttgagcgtc agacctaatc aaatattaca aaatatctca ctctgtcgcc agcaatggtg     360
taatcagcgc agacaaatgg cgtaaagatc gcggaaaaac ctccccgagt ggcatgatag     420
ctgcctctgt attgctgatt tagtcagcct tatttgactt aagggtgccc tcgttagtga     480
caaattgctt tcaaggagac agccatgccc cacactttgt tgaaaaacaa attgcctttg     540
```

gggagacggt aaagccagtt gctcttcaat aaggaatgtc gaggaggcaa tgtaaccgcc 600
tctggtagta cacttctcta atccaaaaat caatttgtat tcaagatacc gcaaaaaact 660
tatggtttaa accctgcagg actagtccag aaggtaatta tccaagatgt agcatcaaga 720
atccaatgtt tacgggaaaa actatggaag tattatgtaa gctcagcaag aagcagatca 780
atatgcggca catatgcaac ctatgttcaa aaatgaagaa tgtacagata caagatccta 840
tactgccaga atacgaagaa gaatacgtag aaattgaaaa agaagaacca ggcgaagaaa 900
agaatcttga agacgtaagc actgacgaca acaatgaaaa gaagaagata aggtcggtga 960
ttgtgaaaga gacatagagg acacatgtaa ggtggaaaat gtaagggcgg aaagtaacct 1020
tatcacaaag gaatcttatc ccccactact tatcctttta tatttttccg tgtcattttt 1080
gcccttgagt tttcctatat aaggaaccaa gttcggcatt tgtgaaaaca agaaaaaatt 1140
tggtgtaagc tattttcttt gaagtactga ggatacaact tcagagaaat ttgtaagttt 1200
gtaggtacca gatctggatc ccaaaccatg tctccggaga ggagaccagt tgagattagg 1260
ccagctacag cagctgatat ggccgcggtt tgtgatatcg ttaaccatta cattgagacg 1320
tctacagtga actttaggac agagccacaa acaccacaag agtggattga tgatctagag 1380
aggttgcaag atagataccc ttggttggtt gctgaggttg agggtgttgt ggctggtatt 1440
gcttacgctg ggccctggaa ggctaggaac gcttacgatt ggacagttga gagtactgtt 1500
tacgtgtcac ataggcatca aaggttgggc ctaggatcta cattgtacac acatttgctt 1560
aagtctatgg aggcgcaagg ttttaagtct gtggttgctg ttataggcct tccaaacgat 1620
ccatctgtta ggttgcatga ggctttggga tacacagccc ggggtacatt gcgcgcagct 1680
ggatacaagc atggtggatg gcatgatgtt ggtttttggc aaagggattt tgagttgcca 1740
gctcctccaa ggccagttag gccagttacc caaatctgag tagttagctt aatcacctag 1800
agctcgatcg gcggcaatag cttcttagcg ccatcccggg ttgatcctat ctgtgttgaa 1860
atagttgcgg tgggcaaggc tctctttcag aaagacaggc ggccaaagga acccaaggtg 1920
aggtgggcta tggctctcag ttccttgtgg aagcgcttgg tctaaggtgc agaggtgtta 1980
gcgggatgaa gcaaaagtgt ccgattgtaa caagatatgt tgatcctacg taaggatatt 2040
aaagtatgta ttcatcacta atataatcag tgtattccaa tatgtactac gatttccaat 2100
gtctttattg tcgccgtatg taatcggcgt cacaaaataa tccccggtga ctttctttta 2160
atccaggatg aaataatatg ttattataat ttttgcgatt tggtccgtta taggaattga 2220
agtgtgcttg aggtcggtcg ccaccactcc catttcataa ttttacatgt atttgaaaaa 2280
taaaaattta tggtattcaa tttaaacacg tatacttgta aagaatgata tcttgaaaga 2340
aatatagttt aaatatttat tgataaaata acaagtcagg tattatagtc caagcaaaaa 2400
cataaattta ttgatgcaag tttaaattca gaaatatttc aataactgat tatatcagct 2460
ggtacattgc cgtagatgaa agactgagtg cgatattatg gtgtaataca taggaattcg 2520
tttaaacgat ctgcgtctaa ttttcggtcc aacttgcaca ggaaagacgt cgaccgcggt 2580

agctcttgcc cagcagactg ggcttccagt cctttcgctc gatcgggtcc aatgttgtcc 2640
tcagctgtga accggaagcg gacgaccaac agtggaagaa ctgaaaggaa cgagccgtct 2700
ataccttgat gatcggcctc tggtgaaggg tatcatcgca gccaagcaag ctcatgaaag 2760
gctgatgggg gaggtgtata attatgaggc ccacggcggg cttattcttt agggaggatc 2820
tatctcgttg ctcaagtgca tggcgcaaag cagttattgg agtgcggatt ttcgttggca 2880
tattattcgc cacgagttag cagacgaaga gaccttcatg aacgtggcca aggccagagt 2940
taagcagatg ttacgccctg ctgcaggcct ttctattatc caatagttgg ttgatctttg 3000
gaaagagcct cggctgaggc ccatactgaa agagatcgat ggatatcgat atgccatgtt 3060
gtttgctagc cagaaccaga tcacatccga tatgctattg cagcttgacg cagatatgga 3120
ggataagttg attcatggga tcgctcagga gtagctcatc catgcacgcc gacaagaaca 3180
gaaattccgt cgagttaacg cagccgctta cgacggattc gaaggtcatc cattcggaat 3240
gtattagttt gcaccagctc cgcgtcacac ctgtcttcat ttgaataaga tgttagcaat 3300
tgtttttagc tttgtcttgt tgtggcaggg cggcaagtgc ttcagacatc attctgtttt 3360
caaattttat gctggagaac agcttcttaa ttcctttgga aataatagac tgcgtcttaa 3420
aattcagatg tctggatata gatatgattg taaaataacc tatttaagtg tcatttagaa 3480
cataagtttt atgaatgttc ttccattttc gtcatcgaac gaataagagt aaatacacct 3540
tttttaacat tacaaataag ttcttatacg ttgtttatac accgggaatc atttccatta 3600
ttttcgcgca aaagtcacgg atattcgtga aagcgacata aactgcgaaa tttgcgggga 3660
gtgtcttgag tttgcctcga ggctagcgca tgcacataga cacacacatc atctcattga 3720
tgcttggtaa taattgtcat tagattgttt ttatgcatag atgcactcga aatcagccaa 3780
ttttagacaa gtatcaaacg gatgtgactt cagtacatta aaaacgtccg caatgtgtta 3840
ttaagttgtc taagcgtcaa tttgatttac aattgaatat atcctgcccc agccagccaa 3900
cagctcgatt tacaattgaa tatatcctgc cggccggccc acgcgtgtcg aggaattctg 3960
atctggcccc catttggacg tgaatgtaga cacgtcgaaa taaagatttc cgaattagaa 4020
taatttgttt attgctttcg cctataaata cgacggatcg taatttgtcg ttttatcaaa 4080
atgtactttc attttataat aacgctgcgg acatctacat ttttgaattg aaaaaaaatt 4140
ggtaattact ctttcttttt ctccatattg accatcatac tcattgctga tccatgtaga 4200
tttcccggac atgaagccat ttacaattga atatatcctg ccgccgctgc cgctttgcac 4260
ccggtggagc ttgcatgttg gtttctacgc agaactgagc cggttaggca gataatttcc 4320
attgagaact gagccatgtg caccttcccc ccaacacggt gagcgacggg gcaacggagt 4380
gatccacatg ggacttttaa acatcatccg tcggatggcg ttgcgagaga agcagtcgat 4440
ccgtgagatc agccgacgca ccgggcaggc gcgcaacacg atcgcaaagt atttgaacgc 4500
aggtacaatc gagccgacgt tcacgcggaa cgaccaagca agcttggctg ccatttttgg 4560
ggtgaggccg ttcgcggccg aggggcgcag cccctggggg gatgggaggc ccgcgttagc 4620

gggccgggag ggttcgagaa gggggggcac ccccccttcgg cgtgcgcggt cacgcgcaca 4680
gggcgcagcc ctggttaaaa acaaggttta taaatattgg tttaaaagca ggttaaaaga 4740
caggttagcg gtggccgaaa aacgggcgga aacccttgca aatgctggat tttctgcctg 4800
tggacagccc ctcaaatgtc aataggtgcg cccctcatct gtcagcactc tgcccctcaa 4860
gtgtcaagga tcgcgcccct catctgtcag tagtcgcgcc cctcaagtgt caataccgca 4920
gggcacttat ccccaggctt gtccacatca tctgtgggaa actcgcgtaa aatcaggcgt 4980
tttcgccgat ttgcgaggct ggccagctcc acgtcgccgg ccgaaatcga gcctgcccct 5040
catctgtcaa cgccgcgccg ggtgagtcgg cccctcaagt gtcaacgtcc gcccctcatc 5100
tgtcagtgag ggccaagttt tccgcgaggt atccacaacg ccggcggccg cggtgtctcg 5160
cacacggctt cgacggcgtt tctggcgcgt ttgcagggcc atagacggcc gccagcccag 5220
cggcgagggc aaccagcccg gtgagcgtcg gaaagggtcg acggatcttt tccgctgcat 5280
aaccctgctt cggggtcatt atagcgattt tttcggtata tccatccttt ttcgcacgat 5340
atacaggatt ttgccaaagg gttcgtgtag actttccttg gtgtatccaa cggcgtcagc 5400
cgggcaggat aggtgaagta ggcccacccg cgagcgggtg ttccttcttc actgtccctt 5460
attcgcacct ggcggtgctc aacgggaatc ctgctctgcg aggctggccg gctaccgccg 5520
gcgtaacaga tgagggcaag cggatggctg atgaaaccaa gccaaccagg aagggcagcc 5580
cacctatcaa ggtgtactgc cttccagacg aacgaagagc gattgaggaa aaggcggcgg 5640
cggccggcat gagcctgtcg gcctacctgc tggccgtcgg ccagggctac aaaatcacgg 5700
gcgtcgtgga ctatgagcac gtccgcgagc tggcccgcat caatggcgac ctgggccgcc 5760
tgggcggcct gctgaaactc tggctcaccg acgacccgcg cacggcgcgg ttcggtgatg 5820
ccacgatcct cgccctgctg gcgaagatcg aagagaagca ggacgagctt ggcaaggtca 5880
tgatgggcgt ggtccgcccg agggcagagc catgactttt ttagccgcta aaacggccgg 5940
ggggtgcgcg tgattgccaa gcacgtcccc atgcgctcca tcaagaagag cgacttcgcg 6000
gagctggtat tcgtgcaggg caagattcgg aataccaagt acgagaagga cggccagacg 6060
gtctacggga ccgacttcat tgccgataag gtggattatc tggacaccaa ggcaccaggc 6120
gggtcaaatc aggaataagg gcacattgcc ccggcgtgag tcggggcaat cccgcaagga 6180
gggtgaatga atcggacgtt tgaccggaag gcatacaggc aagaactgat cgacgcgggg 6240
ttttccgccg aggatgccga aaccatcgca agccgcaccg tcatgcgtgc gccccgcgaa 6300
accttccagt ccgtcggctc gatggtccag caagctacgg ccaagatcga gcgcgacagc 6360
gtgcaactgg ctccccctgc cctgcccgcg ccatcggccg ccgtggagcg ttcgcgtcgt 6420
ctcgaacagg aggcggcagg tttggcgaag tcgatgacca tcgacacgcg aggaactatg 6480
acgaccaaga agcgaaaaac cgccggcgag gacctggcaa aacaggtcag cgaggccaag 6540
caggccgcgt tgctgaaaca cacgaagcag cagatcaagg aaatgcagct ttccttgttc 6600
gatattgcgc cgtggccgga cacgatgcga gcgatgccaa acgacacggc ccgctctgcc 6660

ctgttcacca cgcgcaacaa gaaaatcccg cgcgaggcgc tgcaaaacaa ggtcattttc 6720
cacgtcaaca aggacgtgaa gatcacctac accggcgtcg agctgcgggc cgacgatgac 6780
gaactggtgt ggcagcaggt gttggagtac gcgaagcgca cccctatcgg cgagccgatc 6840
accttcacgt tctacgagct ttgccaggac ctgggctggt cgatcaatgg ccggtattac 6900
acgaaggccg aggaatgcct gtcgcgccta caggcgacgg cgatgggctt cacgtccgac 6960
cgcgttgggc acctggaatc ggtgtcgctg ctgcaccgct tccgcgtcct ggaccgtggc 7020
aagaaaacgt cccgttgcca ggtcctgatc gacgaggaaa tcgtcgtgct gtttgctggc 7080
gaccactaca cgaaattcat atgggagaag taccgcaagc tgtcgccgac ggcccgacgg 7140
atgttcgact atttcagctc gcaccgggag ccgtacccgc tcaagctgga aaccttccgc 7200
ctcatgtgcg gatcggattc cacccgcgtg aagaagtggc gcgagcaggt cggcgaagcc 7260
tgcgaagagt tgcgaggcag cggcctggtg gaacacgcct gggtcaatga tgacctggtg 7320
cattgcaaac gctagggcct tgtggggtca gttccggctg ggggttcagc agccagcgct 7380
ttactggcat ttcaggaaca agcgggcact gctcgacgca cttgcttcgc tcagtatcgc 7440
tcgggacgca cggcgcgctc tacgaactgc cgataaacag aggattaaaa ttgacaattg 7500
tgattaaggc tcagattcga cggcttggag cggccgacgt gcaggatttc cgcgagatcc 7560
gattgtcggc cctgaagaaa gctccagaga tgttcgggtc cgtttacgag cacgaggaga 7620
aaaagcccat ggaggcgttc gctgaacggt tgcgagatgc cgtggcattc ggcgcctaca 7680
tcgacggcga gatcattggg ctgtcggtct tcaaacagga ggacggcccc aaggacgctc 7740
acaaggcgca tctgtccggc gttttcgtgg agcccgaaca gcgaggccga ggggtcgccg 7800
gtatgctgct gcgggcgttg ccggcgggtt tattgctcgt gatgatcgtc cgacagattc 7860
caacgggaat ctggtggatg cgcatcttca tcctcggcgc acttaatatt tcgctattct 7920
ggagcttgtt gtttatttcg gtctaccgcc tgccgggcgg ggtcgcggcg acggtaggcg 7980
ctgtgcagcc gctgatggtc gtgttcatct ctgccgctct gctaggtagc ccgatacgat 8040
tgatggcggt cctgggggct atttgcggaa ctgcgggcgt ggcgctgttg gtgttgacac 8100
caaacgcagc gctagatcct gtcggcgtcg cagcgggcct ggcgggggcg gtttccatgg 8160
cgttcggaac cgtgctgacc cgcaagtggc aacctcccgt gcctctgctc acctttaccg 8220
cctggcaact ggcggccgga ggacttctgc tcgttccagt agctttagtg tttgatccgc 8280
caatcccgat gcctacagga accaatgttc tcggcctggc gtggctcggc ctgatcggag 8340
cgggtttaac ctacttcctt tggttccggg ggatctcgcg actcgaacct acagttgttt 8400
ccttactggg ctttctcagc cccccgagcgc ttagtgggaa tttgtacccc ttatcgaacc 8460
gggagcacag gatgacgcct aacaattcat tcaagccgac accgcttcgc ggcgcggctt 8520
aattcaggag ttaaacatca tgagggaagc ggtgatcgcc gaagtatcga ctcaactatc 8580
agaggtagtt ggcgtcatcg agcgccatct cgaaccgacg ttgctggccg tacatttgta 8640
cggctccgca gtggatggcg gcctgaagcc acacagtgat attgatttgc tggttacggt 8700

gaccgtaagg cttgatgaaa caacgcggcg agctttgatc aacgaccttt tggaaacttc 8760
ggcttcccct ggagagagcg agattctccg cgctgtagaa gtcaccattg ttgtgcacga 8820
cgacatcatt ccgtggcgtt atccagctaa gcgcgaactg caatttggag aatggcagcg 8880
caatgacatt cttgcaggta tcttcgagcc agccacgatc gacattgatc tggctatctt 8940
gctgacaaaa gcaagagaac atagcgttgc cttggtaggt ccagcggcgg aggaactctt 9000
tgatccggtt cctgaacagg atctatttga ggcgctaaat gaaaccttaa cgctatggaa 9060
ctcgccgccc gactgggctg gcgatgagcg aaatgtagtg cttacgttgt cccgcatttg 9120
gtacagcgca gtaaccggca aaatcgcgcc gaaggatgtc gctgccgact gggcaatgga 9180
gcgcctgccg gcccagtatc agcccgtcat acttgaagct aggcaggctt atcttggaca 9240
agaagatcgc ttggcctcgc gcgcagatca gttggaagaa tttgttcact acgtgaaagg 9300
cgagatcacc aaggtagtcg gcaaataatg tctaacaatt cgttcaagcc gacgccgctt 9360
cgcggcgcgg cttaactcaa gcgttagaga gctggggaag actatgcgcg atctgttgaa 9420
ggtggttcta agcctcgtct tgcgatggca tttcgatcca ttcccattcc gcgctcaaga 9480
tggcttcccc tcggcagttc atcagggcta aatcaatcta gccgacttgt ccggtgaaat 9540
gggctgcact ccaacagaaa caatcaaaca aacatacaca gcgacttatt cacacgagct 9600
caaattacaa cggtatatat cctgccagtc agcatcatca caccaaaagt taggcccgaa 9660
tagtttgaaa ttagaaagct cgcaattgag gtctacaggc caaattcgct cttagccgta 9720
caatattact caccggatcc taaccggtgt gatcatgggc cgcgattaaa aatctcaatt 9780
atatttggtc taatttagtt tggtattgag taaaacaaat tcgaaccaaa ccaaaatata 9840
aatatatagt ttttatatat atgcctttaa gactttttat agaattttct ttaaaaaata 9900
tctagaaata tttgcgactc ttctggcatg taatatttcg ttaaatatga agtgctccat 9960
ttttattaac tttaaataat tggttgtacg atcactttct tatcaagtgt tactaaaatg 10020
cgtcaatctc tttgttcttc catattcata tgtcaaaacc tatcaaaatt cttatatatc 10080
tttttcgaat ttgaagtgaa atttcgataa tttaaaatta aatagaacat atcattattt 10140
aggtatcata ttgattttta tacttaatta ctaaatttgg ttaactttga aagtgtacat 10200
caacgaaaaa ttagtcaaac gactaaaata aataaatatc atgtgttatt aagaaaattc 10260
tcctataaga atattttaat agatcatatg tttgtaaaaa aaattaattt ttactaacac 10320
atatatttac ttatcaaaaa tttgacaaag taagattaaa ataatattca tctaacaaaa 10380
aaaaaaccag aaaatgctga aaacccggca aaaccgaacc aatccaaacc gatatagttg 10440
gtttggtttg attttgatat aaaccgaacc aactcggtcc atttgcaccc ctaatcataa 10500
tagctttaat atttcaagat attattaagt taacgttgtc aatatcctgg aaattttgca 10560
aaatgaatca agcctatatg gctgtaatat gaatttaaaa gcagctcgat gtggtggtaa 10620
tatgtaattt acttgattct aaaaaaatat cccaagtatt aataatttct gctaggaaga 10680
aggttagcta cgatttacag caaagccaga atacaatgaa ccataaagtg attgaagctc 10740

gaaatatacg aaggaacaaa tatttttaaa aaaatacgca atgacttgga acaaaagaaa 10800
gtgatatatt ttttgttctt aaacaagcat cccctctaaa gaatggcagt tttcctttgc 10860
atgtaactat tatgctccct tcgttacaaa aattttggac tactattggg aacttcttct 10920
gaaaatagtg gccaccgctt aattaaggcg cgccatgccc gggcaagcgg ccgcacaagt 10980
ttgtacaaaa aagcaggctc cgcggccgca ctaggtttaa actctagaag ctaggaattc 11040
gcggccgctt tcctgcatga catcgtcctg cagagccaag cgcatgctta attaaactag 11100
tctcccagta tcattatagt gaaagttttg gctctctcgc cggtggtttt ttacctctat 11160
ttaaaggggt tttccaccta aaaattctgg tatcattctc actttacttg ttactttaat 11220
ttctcataat ctttggttga aattatcacg cttccgcaca cgatatccct acaaatttat 11280
tatttgttaa acattttcaa accgcataaa attttatgaa gtcccgtcta tctttaatgt 11340
agtctaacat tttcatattg aaatatataa tttacttaat tttagcgttg gtagaaagca 11400
taatgattta ttcttattct tcttcatata aatgtttaat atacaatata aacaaattct 11460
ttaccttaag aaggatttcc cattttatat tttaaaaata tatttatcaa atatttttca 11520
accacgtaaa tctcataata ataagttgtt tcaaaagtaa taaaatttaa ctccataatt 11580
tttttattcg actgatctta aagcaacacc cagtgacaca actagccatt tttttctttg 11640
aataaaaaaa tccaattatc attgtatttt ttttatacaa tgaaaatttc accaaacaat 11700
gatttgtggt atttctgaag caagtcatgt tatgcaaaat tctataattc ccatttgaca 11760
ctacggaagt aactgaagat ctgcttttac atgcgagaca catcttctaa agtaatttta 11820
ataatagtta ctatattcaa gatttcatat atcaaatact caatattact tctaaaaaat 11880
taattagata taattaaaat attacttttt taattttaag tttaattgtt gaatttgtga 11940
ctattgattt attattctac tatgtttaaa ttgttttata gatagtttaa agtaaatata 12000
agtaatgtag tagagtgtta gagtgttacc ctaaaccata aactataaga tttatggtgg 12060
actaattttc atatatttct tattgctttt accttttctt ggtatgtaag tccgtaactg 12120
gaattactgt gggttgccat gacactctgt ggtcttttgg ttcatgcatg gatcttgcgc 12180
aagaaaaaga caaagaacaa agaaaaaaga caaaacagag agacaaaacg caatcacaca 12240
accaactcaa attagtcact ggctgatcaa gatcgccgcg tccatgtatg tctaaatgcc 12300
atgcaaagca acacgtgctt aacatgcact ttaaatggct cacccatctc aacccacaca 12360
caaacacatt gcctttttct tcatcatcac cacaaccacc tgtatatatt cattctcttc 12420
cgccacctca atttcttcac ttcaacacac gtcaacctgc atatgcgtgt catcccatgc 12480
ccaaatctcc atgcatgttc caaccacctt ctctcttata taatacctat aaatacctct 12540
aatatcactc acttctttca tcatccatcc atccagagta ctactactct actactataa 12600
taccccaacc caactcatat tcaatactac tctaggtacc ctgcagggat ccaacaatgg 12660
ccagcagttc ttcaagtgtg ccagaacttg ccgcagcttt ccctgatggg acaacggact 12720
tcaaacccat gaggaacacc aaaggctatg atgtctccaa acctcacatc tctgaaacac 12780

cgatgacttt gaagaactgg cacaaacatg tgaactggct caacaccaca ttcattctct 12840
ttgttccact ggctgggttg atctcaacct attgggttcc tcttcaatgg aaaactgcag 12900
tgtgggcagt tgtgtactac ttcaacactg gacttgggat cactgctggc taccatagat 12960
tgtgggcaca ttcctcttac aaggccagct tgcctctcaa aatctacctt gccgcagttg 13020
gtgctggagc cgttgaaggt tccataagat ggtggagcaa cggacacaga gcacatcaca 13080
gatacacaga cacagagaaa gatccttact cagtgaggaa gggattgctc tacagccaca 13140
tgggttggat gctcttgaag cagaatccaa agaagcaagg gaggacggac attactgatc 13200
tgaatgagga cccagttgtg gtctggcaac ataggaactt tctcaagtgt gtgatcttca 13260
tggctttggt ctttcccacc cttgttgctg gcctgggatg gggagactac tggggaggtt 13320
tcatctatgg agggatcttg agagtgttct ttgttcagca agccaccttc tgtgtcaact 13380
cacttgcaca ttggcttggt gatcaaccgt ttgatgacag aaactctcca cgtgaccatg 13440
tcataactgc tcttgtcacg ctgggtgaag gctatcacaa ctttcaccat gagtttccgt 13500
cagactatag aaatgcgatt gagtggtatc agtatgaccc cacgaagtgg agcatttgga 13560
tttggaagca acttggactt gctcacaatc tcaagcagtt cagacagaat gagatagaga 13620
agggaagggt tcaacagttg cagaagaaac tggatcagaa gagagcgaaa cttgattggg 13680
gaataccgtt ggaacaactc cctgttgtgt cttgggatga ctttgttgaa cagtcaaaga 13740
atggcaaggc atggattgct gttgctggtg tcattcacga tgttggtgac ttcatcaagg 13800
atcatcctgg tggacgtgct ctcatcaact ctgcgattgg caaagatgcc acagcgatct 13860
tcaatggagg tgtctacaat cattcaaatg ccgcacacaa ccttctctcc accatgaggg 13920
ttggtgtcct ccgtggaggg tgcgaagtgg agatatggaa acgtgctcaa agtgagaaca 13980
aagatgtctc tactgtggtt gatagttctg gcaaccgtat tgtgagagct ggtggacaag 14040
ctaccaaagt ggttcagcca gtccctggtg ctcaagcagc ttgatgagta gttagcttaa 14100
tcacctagag ctcggtcacc tcgagtatca aaatctattt agaaatacac aatattttgt 14160
tgcaggcttg ctggagaatc gatctgctat cataaaaatt acaaaaaaat tttatttgcc 14220
tcaattattt taggattggt attaaggacg cttaaattat ttgtcgggtc actacgcatc 14280
attgtgattg agaagatcag cgatacgaaa tattcgtagt actatcgata atttatttga 14340
aaattcataa gaaaagcaaa cgttacatga attgatgaaa caatacaaag acagataaag 14400
ccacgcacat ttaggatatt ggccgagatt actgaatatt gagtaagatc acggaatttc 14460
tgacaggagc atgtcttcaa ttcagcccaa atggcagttg aaatactcaa accgccccat 14520
atgcaggagc ggatcattca ttgtttgttt ggttgccttt gccaacatgg gagtccaagg 14580
ttggcgcgcc gacccagctt tc 14602

<210> 66
<211> 10915
<212> DNA
<213> Artificial

<220>
<223> Plasmid pDAB7331

<400> 66

taaatttaaa ttcaattaat gcaatcttga ttttcaacaa cgaaggtaat ggcgtaaaag 60
aaaaaatgta tgttattgta ttgatctttc atgatgttga agcgtgccat aatatgatga 120
tgtataatta aaatattaac tgtcgcattt tattgaaatg gcactgttat ttcaaccata 180
tctttgattc tgttacatga cacgactgca agaagtaaat aatagacgcc gttgttaaag 240
aattgctatc atatgtgcct aactagaggg aatttgagcg tcagacctaa tcaaatatta 300
caaaatatct cactctgtcg ccagcaatgg tgtaatcagc gcagacaaat ggcgtaaaga 360
tcgcggaaaa acctccccga gtggcatgat agctgcctct gtattgctga tttagtcagc 420
cttatttgac ttaagggtgc cctcgttagt gacaaattgc tttcaaggag acagccatgc 480
cccacacttt gttgaaaaac aaattgcctt tggggagacg gtaaagccag ttgctcttca 540
ataaggaatg tcgaggaggc aatgtaaccg cctctggtag tacacttctc taatccaaaa 600
atcaatttgt attcaagata ccgcaaaaaa cttatggttt aaaccctgca ggactagtcc 660
agaaggtaat tatccaagat gtagcatcaa gaatccaatg tttacgggaa aaactatgga 720
agtattatgt aagctcagca agaagcagat caatatgcgg cacatatgca acctatgttc 780
aaaaatgaag aatgtacaga tacaagatcc tatactgcca gaatacgaag aagaatacgt 840
agaaattgaa aaagaagaac caggcgaaga aaagaatctt gaagacgtaa gcactgacga 900
caacaatgaa aagaagaaga taaggtcggt gattgtgaaa gagacataga ggacacatgt 960
aaggtggaaa atgtaagggc ggaaagtaac cttatcacaa aggaatctta tcccccacta 1020
cttatccttt tatatttttc cgtgtcattt ttgcccttga gttttcctat ataaggaacc 1080
aagttcggca tttgtgaaaa caagaaaaaa tttggtgtaa gctattttct ttgaagtact 1140
gaggatacaa cttcagagaa atttgtaagt ttgtaggtac cagatctgga tcccaaacca 1200
tgtctccgga gaggagacca gttgagatta ggccagctac agcagctgat atggccgcgg 1260
tttgtgatat cgttaaccat tacattgaga cgtctacagt gaactttagg acagagccac 1320
aaacaccaca agagtggatt gatgatctag agaggttgca agatagatac ccttggttgg 1380
ttgctgaggt tgagggtgtt gtggctggta ttgcttacgc tgggccctgg aaggctagga 1440
acgcttacga ttggacagtt gagagtactg tttacgtgtc acataggcat caaaggttgg 1500
gcctaggatc tacattgtac acacatttgc ttaagtctat ggaggcgcaa ggttttaagt 1560
ctgtggttgc tgttataggc cttccaaacg atccatctgt taggttgcat gaggctttgg 1620
gatacacagc ccggggtaca ttgcgcgcag ctggatacaa gcatggtgga tggcatgatg 1680
ttggtttttg gcaaagggat tttgagttgc cagctcctcc aaggccagtt aggccagtta 1740
cccaaatctg agtagttagc ttaatcacct agagctcgat cggcggcaat agcttcttag 1800
cgccatcccg ggttgatcct atctgtgttg aaatagttgc ggtgggcaag gctctctttc 1860
agaaagacag gcggccaaag gaacccaagg tgaggtgggc tatggctctc agttccttgt 1920

ggaagcgctt ggtctaaggt gcagaggtgt tagcgggatg aagcaaaagt gtccgattgt 1980
aacaagatat gttgatccta cgtaaggata ttaaagtatg tattcatcac taatataatc 2040
agtgtattcc aatatgtact acgatttcca atgtctttat tgtcgccgta tgtaatcggc 2100
gtcacaaaat aatccccggt gactttcttt taatccagga tgaaataata tgttattata 2160
atttttgcga tttggtccgt tataggaatt gaagtgtgct tgaggtcggt cgccaccact 2220
cccatttcat aattttacat gtatttgaaa aataaaaatt tatggtattc aatttaaaca 2280
cgtatacttg taaagaatga tatcttgaaa gaaatatagt ttaaatattt attgataaaa 2340
taacaagtca ggtattatag tccaagcaaa aacataaatt tattgatgca agtttaaatt 2400
cagaaatatt tcaataactg attatatcag ctggtacatt gccgtagatg aaagactgag 2460
tgcgatatta tggtgtaata cataggaatt cgtttaaacg atctgcgtct aattttcggt 2520
ccaacttgca caggaaagac gtcgaccgcg gtagctcttg cccagcagac tgggcttcca 2580
gtcctttcgc tcgatcgggt ccaatgttgt cctcagctgt gaaccggaag cggacgacca 2640
acagtggaag aactgaaagg aacgagccgt ctataccttg atgatcggcc tctggtgaag 2700
ggtatcatcg cagccaagca agctcatgaa aggctgatgg gggaggtgta taattatgag 2760
gcccacggcg ggcttattct ttagggagga tctatctcgt tgctcaagtg catggcgcaa 2820
agcagttatt ggagtgcgga ttttcgttgg catattattc gccacgagtt agcagacgaa 2880
gagaccttca tgaacgtggc caaggccaga gttaagcaga tgttacgccc tgctgcaggc 2940
ctttctatta tccaatagtt ggttgatctt tggaaagagc ctcggctgag gcccatactg 3000
aaagagatcg atggatatcg atatgccatg ttgtttgcta gccagaacca gatcacatcc 3060
gatatgctat tgcagcttga cgcagatatg gaggataagt tgattcatgg gatcgctcag 3120
gagtagctca tccatgcacg ccgacaagaa cagaaattcc gtcgagttaa cgcagccgct 3180
tacgacggat tcgaaggtca tccattcgga atgtattagt ttgcaccagc tccgcgtcac 3240
acctgtcttc atttgaataa gatgttagca attgttttta gctttgtctt gttgtggcag 3300
ggcggcaagt gcttcagaca tcattctgtt ttcaaatttt atgctggaga acagcttctt 3360
aattcctttg gaaataatag actgcgtctt aaaattcaga tgtctggata tagatatgat 3420
tgtaaaataa cctatttaag tgtcatttag aacataagtt ttatgaatgt tcttccattt 3480
tcgtcatcga acgaataaga gtaaatacac ctttttttaac attacaaata agttcttata 3540
cgttgtttat acaccgggaa tcatttccat tattttcgcg caaaagtcac ggatattcgt 3600
gaaagcgaca taaactgcga aatttgcggg gagtgtcttg agtttgcctc gaggctagcg 3660
catgcacata gacacacaca tcatctcatt gatgcttggt aataattgtc attagattgt 3720
ttttatgcat agatgcactc gaaatcagcc aattttagac aagtatcaaa cggatgtgac 3780
ttcagtacat taaaaacgtc cgcaatgtgt tattaagttg tctaagcgtc aatttgattt 3840
acaattgaat atatcctgcc ccagccagcc aacagctcga tttacaattg aatatatcct 3900
gccggccggc ccacgcgtgt cgaggaattc tgatctggcc cccatttgga cgtgaatgta 3960

gacacgtcga aataaagatt tccgaattag aataatttgt ttattgcttt cgcctataaa 4020
tacgacggat cgtaatttgt cgttttatca aaatgtactt tcattttata ataacgctgc 4080
ggacatctac atttttgaat tgaaaaaaaa ttggtaatta ctctttcttt ttctccatat 4140
tgaccatcat actcattgct gatccatgta gatttcccgg acatgaagcc atttacaatt 4200
gaatatatcc tgccgccgct gccgctttgc acccggtgga gcttgcatgt tggtttctac 4260
gcagaactga gccggttagg cagataattt ccattgagaa ctgagccatg tgcaccttcc 4320
ccccaacacg gtgagcgacg gggcaacgga gtgatccaca tgggactttt aaacatcatc 4380
cgtcggatgg cgttgcgaga gaagcagtcg atccgtgaga tcagccgacg caccgggcag 4440
gcgcgcaaca cgatcgcaaa gtatttgaac gcaggtacaa tcgagccgac gttcacgcgg 4500
aacgaccaag caagcttggc tgccattttt ggggtgaggc cgttcgcggc cgaggggcgc 4560
agcccctggg gggatgggag gcccgcgtta gcgggccggg agggttcgag aagggggggc 4620
acccccttc ggcgtgcgcg gtcacgcgca cagggcgcag ccctggttaa aaacaaggtt 4680
tataaatatt ggtttaaaag caggttaaaa gacaggttag cggtggccga aaaacgggcg 4740
gaaacccttg caaatgctgg attttctgcc tgtggacagc ccctcaaatg tcaataggtg 4800
cgcccctcat ctgtcagcac tctgcccctc aagtgtcaag gatcgcgccc ctcatctgtc 4860
agtagtcgcg cccctcaagt gtcaataccg cagggcactt atccccaggc ttgtccacat 4920
catctgtggg aaactcgcgt aaaatcaggc gttttcgccg atttgcgagg ctggccagct 4980
ccacgtcgcc ggccgaaatc gagcctgccc ctcatctgtc aacgccgcgc cgggtgagtc 5040
ggcccctcaa gtgtcaacgt ccgcccctca tctgtcagtg agggccaagt tttccgcgag 5100
gtatccacaa cgccggcggc cgcggtgtct cgcacacggc ttcgacggcg tttctggcgc 5160
gtttgcaggg ccatagacgg ccgccagccc agcggcgagg gcaaccagcc cggtgagcgt 5220
cggaaagggt cgacggatct tttccgctgc ataaccctgc ttcggggtca ttatagcgat 5280
tttttcggta tatccatcct ttttcgcacg atatacagga ttttgccaaa gggttcgtgt 5340
agactttcct tggtgtatcc aacggcgtca gccgggcagg ataggtgaag taggcccacc 5400
cgcgagcggg tgttccttct tcactgtccc ttattcgcac ctggcggtgc tcaacgggaa 5460
tcctgctctg cgaggctggc cggctaccgc cggcgtaaca gatgagggca agcggatggc 5520
tgatgaaacc aagccaacca ggaagggcag cccacctatc aaggtgtact gccttccaga 5580
cgaacgaaga gcgattgagg aaaaggcggc ggcggccggc atgagcctgt cggcctacct 5640
gctggccgtc ggccagggct acaaaatcac gggcgtcgtg gactatgagc acgtccgcga 5700
gctggcccgc atcaatggcg acctgggccg cctgggcggc ctgctgaaac tctggctcac 5760
cgacgacccg cgcacggcgc ggttcggtga tgccacgatc ctcgccctgc tggcgaagat 5820
cgaagagaag caggacgagc ttggcaaggt catgatgggc gtggtccgcc cgagggcaga 5880
gccatgactt ttttagccgc taaaacggcc ggggggtgcg cgtgattgcc aagcacgtcc 5940
ccatgcgctc catcaagaag agcgacttcg cggagctggt attcgtgcag ggcaagattc 6000

```
ggaataccaa gtacgagaag gacggccaga cggtctacgg gaccgacttc attgccgata    6060
aggtggatta tctggacacc aaggcaccag gcgggtcaaa tcaggaataa gggcacattg    6120
ccccggcgtg agtcggggca atcccgcaag gagggtgaat gaatcggacg tttgaccgga    6180
aggcatacag gcaagaactg atcgacgcgg ggttttccgc cgaggatgcc gaaaccatcg    6240
caagccgcac cgtcatgcgt gcgccccgcg aaaccttcca gtccgtcggc tcgatggtcc    6300
agcaagctac ggccaagatc gagcgcgaca gcgtgcaact ggctccccct gccctgcccg    6360
cgccatcggc cgccgtggag cgttcgcgtc gtctcgaaca ggaggcggca ggtttggcga    6420
agtcgatgac catcgacacg cgaggaacta tgacgaccaa gaagcgaaaa accgccggcg    6480
aggacctggc aaaacaggtc agcgaggcca agcaggccgc gttgctgaaa cacacgaagc    6540
agcagatcaa ggaaatgcag ctttccttgt tcgatattgc gccgtggccg gacacgatgc    6600
gagcgatgcc aaacgacacg gcccgctctg ccctgttcac cacgcgcaac aagaaaatcc    6660
cgcgcgaggc gctgcaaaac aaggtcattt tccacgtcaa caaggacgtg aagatcacct    6720
acaccggcgt cgagctgcgg gccgacgatg acgaactggt gtggcagcag gtgttggagt    6780
acgcgaagcg cacccctatc ggcgagccga tcaccttcac gttctacgag ctttgccagg    6840
acctgggctg gtcgatcaat ggccggtatt acacgaaggc cgaggaatgc ctgtcgcgcc    6900
tacaggcgac ggcgatgggc ttcacgtccg accgcgttgg gcacctggaa tcggtgtcgc    6960
tgctgcaccg cttccgcgtc ctggaccgtg gcaagaaaac gtcccgttgc caggtcctga    7020
tcgacgagga aatcgtcgtg ctgtttgctg gcgaccacta cacgaaattc atatgggaga    7080
agtaccgcaa gctgtcgccg acggcccgac ggatgttcga ctatttcagc tcgcaccggg    7140
agccgtaccc gctcaagctg gaaaccttcc gcctcatgtg cggatcggat tccacccgcg    7200
tgaagaagtg gcgcgagcag gtcggcgaag cctgcgaaga gttgcgaggc agcggcctgg    7260
tggaacacgc ctgggtcaat gatgacctgg tgcattgcaa acgctagggc cttgtggggt    7320
cagttccggc tgggggttca gcagccagcg ctttactggc atttcaggaa caagcgggca    7380
ctgctcgacg cacttgcttc gctcagtatc gctcgggacg cacggcgcgc tctacgaact    7440
gccgataaac agaggattaa aattgacaat tgtgattaag gctcagattc gacggcttgg    7500
agcggccgac gtgcaggatt tccgcgagat ccgattgtcg gccctgaaga aagctccaga    7560
gatgttcggg tccgtttacg agcacgagga gaaaaagccc atggaggcgt tcgctgaacg    7620
gttgcgagat gccgtggcat tcggcgccta catcgacggc gagatcattg ggctgtcggt    7680
cttcaaacag gaggacggcc ccaaggacgc tcacaaggcg catctgtccg gcgttttcgt    7740
ggagcccgaa cagcgaggcc gaggggtcgc cggtatgctg ctgcgggcgt tgccggcggg    7800
tttattgctc gtgatgatcg tccgacagat tccaacggga atctggtgga tgcgcatctt    7860
catcctcggc gcacttaata tttcgctatt ctggagcttg ttgtttattt cggtctaccg    7920
cctgccgggc ggggtcgcgg cgacggtagg cgctgtgcag ccgctgatgg tcgtgttcat    7980
ctctgccgct ctgctaggta gcccgatacg attgatggcg gtcctggggg ctatttgcgg    8040
```

aactgcgggc gtggcgctgt tggtgttgac accaaacgca gcgctagatc ctgtcggcgt 8100
cgcagcgggc ctggcggggg cggtttccat ggcgttcgga accgtgctga cccgcaagtg 8160
gcaacctccc gtgcctctgc tcacctttac cgcctggcaa ctggcggccg gaggacttct 8220
gctcgttcca gtagctttag tgtttgatcc gccaatcccg atgcctacag gaaccaatgt 8280
tctcggcctg gcgtggctcg gcctgatcgg agcgggttta acctacttcc tttggttccg 8340
ggggatctcg cgactcgaac ctacagttgt ttccttactg ggctttctca gcccccgagc 8400
gcttagtggg aatttgtacc ccttatcgaa ccgggagcac aggatgacgc ctaacaattc 8460
attcaagccg acaccgcttc gcggcgcggc ttaattcagg agttaaacat catgagggaa 8520
gcggtgatcg ccgaagtatc gactcaacta tcagaggtag ttggcgtcat cgagcgccat 8580
ctcgaaccga cgttgctggc cgtacatttg tacggctccg cagtggatgg cggcctgaag 8640
ccacacagtg atattgattt gctggttacg gtgaccgtaa ggcttgatga aacaacgcgg 8700
cgagctttga tcaacgacct tttggaaact tcggcttccc ctggagagag cgagattctc 8760
cgcgctgtag aagtcaccat tgttgtgcac gacgacatca ttccgtggcg ttatccagct 8820
aagcgcgaac tgcaatttgg agaatggcag cgcaatgaca ttcttgcagg tatcttcgag 8880
ccagccacga tcgacattga tctggctatc ttgctgacaa aagcaagaga acatagcgtt 8940
gccttggtag gtccagcggc ggaggaactc tttgatccgg ttcctgaaca ggatctattt 9000
gaggcgctaa atgaaacctt aacgctatgg aactcgccgc ccgactgggc tggcgatgag 9060
cgaaatgtag tgcttacgtt gtcccgcatt tggtacagcg cagtaaccgg caaaatcgcg 9120
ccgaaggatg tcgctgccga ctgggcaatg gagcgcctgc cggcccagta tcagcccgtc 9180
atacttgaag ctaggcaggc ttatcttgga caagaagatc gcttggcctc gcgcgcagat 9240
cagttggaag aatttgttca ctacgtgaaa ggcgagatca ccaaggtagt cggcaaataa 9300
tgtctaacaa ttcgttcaag ccgacgccgc ttcgcggcgc ggcttaactc aagcgttaga 9360
gagctgggga agactatgcg cgatctgttg aaggtggttc taagcctcgt cttgcgatgg 9420
catttcgatc cattcccatt ccgcgctcaa gatggcttcc cctcggcagt tcatcagggc 9480
taaatcaatc tagccgactt gtccggtgaa atgggctgca ctccaacaga aacaatcaaa 9540
caaacataca cagcgactta ttcacacgag ctcaaattac aacggtatat atcctgccag 9600
tcagcatcat cacaccaaaa gttaggcccg aatagtttga aattagaaag ctcgcaattg 9660
aggtctacag gccaaattcg ctcttagccg tacaatatta ctcaccggat cctaaccggt 9720
gtgatcatgg gccgcgatta aaaatctcaa ttatatttgg tctaatttag tttggtattg 9780
agtaaaacaa attcgaacca aaccaaaata taaatatata gtttttatat atatgccttt 9840
aagacttttt atagaatttt ctttaaaaaa tatctagaaa tatttgcgac tcttctggca 9900
tgtaatattt cgttaaatat gaagtgctcc atttttatta actttaaata attggttgta 9960
cgatcacttt cttatcaagt gttactaaaa tgcgtcaatc tctttgttct tccatattca 10020
tatgtcaaaa cctatcaaaa ttcttatata tctttttcga atttgaagtg aaatttcgat 10080

aatttaaaat taaatagaac atatcattat ttaggtatca tattgatttt tatacttaat 10140
tactaaattt ggttaacttt gaaagtgtac atcaacgaaa aattagtcaa acgactaaaa 10200
taaataaata tcatgtgtta ttaagaaaat tctcctataa gaatatttta atagatcata 10260
tgtttgtaaa aaaaattaat ttttactaac acatatattt acttatcaaa aatttgacaa 10320
agtaagatta aaataatatt catctaacaa aaaaaaaacc agaaaatgct gaaaacccgg 10380
caaaaccgaa ccaatccaaa ccgatatagt tggtttggtt tgattttgat ataaaccgaa 10440
ccaactcggt ccatttgcac ccctaatcat aatagcttta atatttcaag atattattaa 10500
gttaacgttg tcaatatcct ggaaattttg caaaatgaat caagcctata tggctgtaat 10560
atgaatttaa aagcagctcg atgtggtggt aatatgtaat ttacttgatt ctaaaaaaat 10620
atcccaagta ttaataattt ctgctaggaa gaaggttagc tacgatttac agcaaagcca 10680
gaatacaatg aaccataaag tgattgaagc tcgaaatata cgaaggaaca aatattttta 10740
aaaaaatacg caatgacttg gaacaaaaga aagtgatata tttttgttc ttaaacaagc 10800
atcccctcta aagaatggca gttttccttt gcatgtaact attatgctcc cttcgttaca 10860
aaaattttgg actactattg ggaacttctt ctgaaaatag tggccaccgc ttaat 10915

<210> 67
<211> 1448
<212> DNA
<213> Artificial

<220>
<223> PvPhas v2 promoter

<400> 67

ctcccagtat cattatagtg aaagttttgg ctctctcgcc ggtggttttt tacctctatt 60
taaaggggtt ttccacctaa aaattctggt atcattctca ctttacttgt tactttaatt 120
tctcataatc tttggttgaa attatcacgc ttccgcacac gatatcccta caaatttatt 180
atttgttaaa cattttcaaa ccgcataaaa ttttatgaag tcccgtctat ctttaatgta 240
gtctaacatt ttcatattga aatatataat ttacttaatt ttagcgttgg tagaaagcat 300
aatgatttat tcttattctt cttcatataa atgtttaata tacaatataa acaaattctt 360
taccttaaga aggatttccc attttatatt ttaaaaatat atttatcaaa tatttttcaa 420
ccacgtaaat ctcataataa taagttgttt caaaagtaat aaaatttaac tccataattt 480
ttttattcga ctgatcttaa agcaacaccc agtgacacaa ctagccattt ttttctttga 540
ataaaaaaat ccaattatca ttgtattttt tttatacaat gaaaatttca ccaaacaatg 600
atttgtggta tttctgaagc aagtcatgtt atgcaaaatt ctataattcc catttgacac 660
tacggaagta actgaagatc tgcttttaca tgcgagacac atcttctaaa gtaattttaa 720
taatagttac tatattcaag atttcatata tcaaatactc aatattactt ctaaaaaatt 780
aattagatat aattaaaata ttactttttt aattttaagt ttaattgttg aatttgtgac 840
tattgattta ttattctact atgtttaaat tgttttatag atagtttaaa gtaaatataa 900

gtaatgtagt agagtgttag agtgttaccc taaaccataa actataagat ttatggtgga 960
ctaattttca tatatttctt attgctttta ccttttcttg gtatgtaagt ccgtaactgg 1020
aattactgtg ggttgccatg acactctgtg gtcttttggt tcatgcatgg atcttgcgca 1080
agaaaaagac aaagaacaaa gaaaaaagac aaaacagaga gacaaaacgc aatcacacaa 1140
ccaactcaaa ttagtcactg gctgatcaag atcgccgcgt ccatgtatgt ctaaatgcca 1200
tgcaaagcaa cacgtgctta acatgcactt taaatggctc acccatctca acccacacac 1260
aaacacattg cctttttctt catcatcacc acaaccacct gtatatattc attctcttcc 1320
gccacctcaa tttcttcact tcaacacacg tcaacctgca tatgcgtgtc atcccatgcc 1380
caaatctcca tgcatgttcc aaccaccttc tctcttatat aatacctata aatacctcta 1440
atatcact 1448

<210> 68
<211> 85
<212> DNA
<213> Artificial

<220>
<223> PvPhas 5' UTR

<400> 68

cacttctttc atcatccatc catccagagt actactactc tactactata ataccccaac 60
ccaactcata ttcaatacta ctcta 85

<210> 69
<211> 129
<212> DNA
<213> Artificial

<220>
<223> PvPhas 3' UTR v1

<400> 69

```
agtatgaact aaaatgcatg taggtgtaag agctcatgga gagcatggaa tattgtatcc     60
gaccatgtaa cagtataata actgagctcc atctcacttc ttctatgaat aaacaaagga    120
tgttatgat                                                            129
```

<210> 70
<211> 1088
<212> DNA
<213> Artificial

<220>
<223> PvPhas 3' MAR v2

<400> 70

```
atattaacac tctatctatg caccttattg ttctatgata aatttcctct tattattata     60
aatcatctga atcgtgacgg cttatggaat gcttcaaata gtacaaaaac aaatgtgtac    120
tataagactt tctaaacaat tctaacttta gcattgtgaa cgagacataa gtgttaagaa    180
gacataacaa ttataatgga agaagtttgt ctccatttat atattatata ttacccactt    240
```

```
atgtattata ttaggatgtt aaggagacat aacaattata aagagagaag tttgtatcca     300
tttatatatt atatactacc catttatata ttatacttat ccacttattt aatgtcttta     360
taaggtttga tccatgatat ttctaatatt ttagttgata tgtatatgaa aaggtactat     420
ttgaactctc ttactctgta taaaggttgg atcatcctta aagtgggtct atttaatttt     480
attgcttctt acagataaaa aaaaaattat gagttggttt gataaaatat tgaaggattt     540
aaaataataa taaataataa ataacatata atatatgtat ataaatttat tataatataa     600
catttatcta taaaaaagta aatattgtca taaatctata caatcgttta gccttgctgg     660
aacgaatctc aattatttaa acgagagtaa acatatttga ctttttggtt atttaacaaa     720
ttattattta acactatatg aaattttttt tttttatcag caaagaataa aattaaatta     780
agaaggacaa tggtgtccca atccttatac aaccaacttc cacaagaaag tcaagtcaga     840
gacaacaaaa aaacaagcaa aggaaatttt ttaatttgag ttgtcttgtt tgctgcataa     900
tttatgcagt aaaacactac acataaccct tttagcagta gagcaatggt tgaccgtgtg     960
cttagcttct tttattttat ttttttatca gcaaagaata aataaaataa aatgagacac    1020
ttcagggatg tttcaaccct tatacaaaac cccaaaaaca agtttcctag caccctacca    1080
acgaattc                                                             1088
```

<210> 71
<211> 457
<212> DNA
<213> Artificial

<220>
<223> AtuORF 3' UTR v1

<400> 71

```
tatcaaaatc tatttagaaa tacacaatat tttgttgcag gcttgctgga gaatcgatct      60
gctatcataa aaattacaaa aaaattttat ttgcctcaat tattttagga ttggtattaa     120
ggacgcttaa attatttgtc gggtcactac gcatcattgt gattgagaag atcagcgata     180
cgaaatattc gtagtactat cgataattta tttgaaaatt cataagaaaa gcaaacgtta     240
catgaattga tgaaacaata caaagacaga taaagccacg cacatttagg atattggccg     300
agattactga atattgagta agatcacgga atttctgaca ggagcatgtc ttcaattcag     360
cccaaatggc agttgaaata ctcaaaccgc cccatatgca ggagcggatc attcattgtt     420
tgtttggttg cctttgccaa catgggagtc caaggtt                              457
```

<210> 72
<211> 68
<212> PRT
<213> Artificial

<220>
<223> N-terminal residues 1-68 of AnD9DS

<400> 72

```
Met Ser Ala Pro Thr Ala Asp Ile Arg Ala Arg Ala Pro Glu Ala Lys
1               5                   10                  15

Lys Val His Ile Ala Asp Thr Ala Ile Asn Arg His Asn Trp Tyr Lys
            20                  25                  30

His Val Asn Trp Leu Asn Val Phe Leu Ile Ile Gly Ile Pro Leu Tyr
        35                  40                  45

Gly Cys Ile Gln Ala Phe Trp Val Pro Leu Gln Leu Lys Thr Ala Ile
    50                  55                  60

Trp Ala Val Ile
65
```

<210> 73
<211> 175
<212> PRT
<213> Artificial

<220>
<223> C-terminal residues 281-455 of AnD9DS

<400> 73

```
Ser Ile Trp Ala Trp Lys Gln Leu Gly Leu Ala Tyr Asp Leu Lys Lys
1               5                   10                  15

Phe Arg Ala Asn Glu Ile Glu Lys Gly Arg Val Gln Gln Leu Gln Lys
            20                  25                  30

Lys Leu Asp Arg Lys Arg Ala Thr Leu Asp Trp Gly Thr Pro Leu Asp
        35                  40                  45

Gln Leu Pro Val Met Glu Trp Asp Asp Tyr Val Glu Gln Ala Lys Asn
    50                  55                  60

Gly Arg Gly Leu Val Ala Ile Ala Gly Val Val His Asp Val Thr Asp
65                  70                  75                  80

Phe Ile Lys Asp His Pro Gly Gly Lys Ala Met Ile Ser Ser Gly Ile
                85                  90                  95

Gly Lys Asp Ala Thr Ala Met Phe Asn Gly Gly Val Tyr Tyr His Ser
            100                 105                 110

Asn Ala Ala His Asn Leu Leu Ser Thr Met Arg Val Gly Val Ile Arg
        115                 120                 125

Gly Gly Cys Glu Val Glu Ile Trp Lys Arg Ala Gln Lys Glu Asn Val
    130                 135                 140

Glu Tyr Val Arg Asp Gly Ser Gly Gln Arg Val Ile Arg Ala Gly Glu
145                 150                 155                 160

Gln Pro Thr Lys Ile Pro Glu Pro Ile Pro Thr Ala Asp Ala Ala
                165                 170                 175
```

<210> 74
<211> 14103
<212> DNA
<213> Artificial

<220>
<223> Plasmid pDAB110110

<400> 74

```
gtacaaaaaa gcaggcttct agacctaggt ggagtcatca cgcagactat ctcagcatgt      60
gcgtagcacg cggccgcctc ccagtatcat tatagtgaaa gttttggctc tctcgccggt     120
ggtttttac ctctatttaa aggggttttc cacctaaaaa ttctggtatc attctcactt     180
tacttgttac tttaatttct cataatcttt ggttgaaatt atcacgcttc cgcacacgat     240
atccctacaa atttattatt tgttaaacat tttcaaaccg cataaaattt tatgaagtcc     300
cgtctatctt taatgtagtc taacattttc atattgaaat atataattta cttaatttta     360
gcgttggtag aaagcataat gatttattct tattcttctt catataaatg tttaatatac     420
aatataaaca aattctttac cttaagaagg atttcccatt ttatatttta aaaatatatt     480
tatcaaatat ttttcaacca cgtaaatctc ataataataa gttgtttcaa aagtaataaa     540
atttaactcc ataatttttt tattcgactg atcttaaagc aacacccagt gacacaacta     600
gccatttttt tctttgaata aaaaaatcca attatcattg tatttttttt atacaatgaa     660
aatttcacca aacaatgatt tgtggtattt ctgaagcaag tcatgttatg caaaattcta     720
taattcccat ttgacactac ggaagtaact gaagatctgc ttttacatgc gagacacatc     780
ttctaaagta attttaataa tagttactat attcaagatt tcatatatca aatactcaat     840
attacttcta aaaaattaat tagatataat taaaatatta ctttttaat tttaagttta      900
attgttgaat ttgtgactat tgatttatta ttctactatg tttaaattgt tttatagata     960
gtttaaagta aatataagta atgtagtaga gtgttagagt gttaccctaa accataaact    1020
ataagattta tggtggacta attttcatat atttcttatt gcttttacct tttcttggta    1080
tgtaagtccg taactggaat tactgtgggt tgccatgaca ctctgtggtc ttttggttca    1140
tgcatggatc ttgcgcaaga aaaagacaaa gaacaaagaa aaaagacaaa acagagagac    1200
aaaacgcaat cacacaacca actcaaatta gtcactggct gatcaagatc gccgcgtcca    1260
tgtatgtcta aatgccatgc aaagcaacac gtgcttaaca tgcactttaa atggctcacc    1320
catctcaacc cacacacaaa cacattgcct ttttcttcat catcaccaca accacctgta    1380
tatattcatt ctcttccgcc acctcaattt cttcacttca acacacgtca acctgcatat    1440
gcgtgtcatc ccatgcccaa atctccatgc atgttccaac caccttctct cttatataat    1500
acctataaat acctctaata tcactcactt ctttcatcat ccatccatcc agagtactac    1560
tactctacta ctataatacc ccaacccaac tcatattcaa tactactcta ggatccaaca    1620
```

atggctgcac ttgatagcat ccctgaggac aaagcaacta gctccaagtc aacccacata 1680
cagtaccaag aggtcacgtt taggaactgg tacaagaaaa tcaactggct caacacgacc 1740
cttgttgtcc tcattcctgc tcttgggttg tacttgacga gaaccacacc tctcaccaga 1800
cctaccctca tttggtctgt tctctactat ttctgtacag cgtttggcat cactggtggc 1860
taccacagac tttggtccca taggtcttac agtgcgaggt tgccattgag actcttcctg 1920
gctttcactg gagctggtgc gatccaaggt tctgcaagat ggtggtcagc caatcatagg 1980
gcacatcacc gttggacgga caccatgaag gacccctact ctgtgatgag aggactgctg 2040
ttctcccaca taggttggat ggttctcaac tctgatccaa aggtcaaagg cagaacagat 2100
gtttctgatc ttgactctga tcccgtcgtt gtgtggcaac acaaacacta tggcaagtgt 2160
ttgctctttg ccgcttggat ctttccgatg atagtggctg ggctgggttg gggagattgg 2220
tggggtggac ttgtctatgc tggcatcata cgtgcctgct ttgttcagca agccactttc 2280
tgtgtcaact cattggcaca ttggataggt gaacaaccgt ttgatgacag acgtactcca 2340
agggatcatg ttctgactgc gttggtcaca atgggagaag gataccacaa cttccaccat 2400
gagtttccga gtgactacag aaatgccatc atttggtatc agtatgaccc tacaaagtgg 2460
ctcatctatc tcttcagctt gggtcccttc ccattggcct actctctcaa gaccttccgt 2520
tccaatgaga ttgagaaagg aaggcttcag caacagcaaa aggctcttga caagaaaaga 2580
agtggtcttg attggggact tcctctcttc cagcttccag tgatctcatg ggatgacttt 2640
caagctcgtt gcaaagaaag tggagagatg cttgttgctg ttgctggagt gatccatgat 2700
gtctcccagt tcattgaaga tcatcctggt gggaggagcc tcattagaag tgctgttggg 2760
aaagatggga ctggcatgtt caatggtgga gtgtatgaac attcaaacgc cgcacacaac 2820
ttgctgagca caatgagagt tggagtcttg agaggtggac aagaagtgga ggtttggaag 2880
aaacagaggg tggatgttct tgggaagtca gacattcttc gtcaagtgac aagggtggag 2940
cgtctggtgg aaggagctgt tgcagcgtga tgagtagtta gcttaatcac ctagagctcg 3000
gtcaccagta tgaactaaaa tgcatgtagg tgtaagagct catggagagc atggaatatt 3060
gtatccgacc atgtaacagt ataataactg agctccatct cacttcttct atgaataaac 3120
aaaggatgtt atgatatatt aacactctat ctatgcacct tattgttcta tgataaattt 3180
cctcttatta ttataaatca tctgaatcgt gacggcttat ggaatgcttc aaatagtaca 3240
aaaacaaatg tgtactataa gactttctaa acaattctaa ctttagcatt gtgaacgaga 3300
cataagtgtt aagaagacat aacaattata atggaagaag tttgtctcca tttatatatt 3360
atatattacc cacttatgta ttatattagg atgttaagga gacataacaa ttataaagag 3420
agaagtttgt atccatttat atattatata ctacccattt atatattata cttatccact 3480
tatttaatgt ctttataagg tttgatccat gatatttcta atattttagt tgatatgtat 3540
atgaaaaggt actatttgaa ctctcttact ctgtataaag gttggatcat ccttaaagtg 3600
ggtctattta attttattgc ttcttacaga taaaaaaaaa attatgagtt ggtttgataa 3660

aatattgaag gatttaaaat aataataaat aataaataac atataatata tgtatataaa 3720
tttattataa tataacattt atctataaaa aagtaaatat tgtcataaat ctatacaatc 3780
gtttagcctt gctggaacga atctcaatta tttaaacgag agtaaacata tttgactttt 3840
tggttattta acaaattatt atttaacact atatgaaatt tttttttttt atcagcaaag 3900
aataaaatta aattaagaag gacaatggtg tcccaatcct tatacaacca acttccacaa 3960
gaaagtcaag tcagagacaa caaaaaaaca agcaaaggaa attttttaat ttgagttgtc 4020
ttgtttgctg cataatttat gcagtaaaac actacacata accctttag cagtagagca 4080
atggttgacc gtgtgcttag cttcttttat tttatttttt tatcagcaaa gaataaataa 4140
aataaaatga gacacttcag ggatgtttca acccttatac aaaaccccaa aaacaagttt 4200
cctagcaccc taccaacgaa ttcgcggccg cttaattaag atgagtgata ctcaggactc 4260
aggactcact ctgctgatca ctagtgctag cctcgaggtc gaccagcttt cttgtacaaa 4320
gtggttgcgg ccgcttaatt aaatttaaat tcaattaatg caatcttgat tttcaacaac 4380
gaaggtaatg gcgtaaaaga aaaaatgtat gttattgtat tgatctttca tgatgttgaa 4440
gcgtgccata atatgatgat gtataattaa aatattaact gtcgcatttt attgaaatgg 4500
cactgttatt tcaaccatat ctttgattct gttacatgac acgactgcaa gaagtaaata 4560
atagacgccg ttgttaaaga attgctatca tatgtgccta actagaggga atttgagcgt 4620
cagacctaat caaatattac aaaatatctc actctgtcgc cagcaatggt gtaatcagcg 4680
cagacaaatg gcgtaaagat cgcggaaaaa cctccccgag tggcatgata gctgcctctg 4740
tattgctgat ttagtcagcc ttatttgact taagggtgcc ctcgttagtg acaaattgct 4800
ttcaaggaga cagccatgcc ccacactttg ttgaaaaaca aattgccttt ggggagacgg 4860
taaagccagt tgctcttcaa taaggaatgt cgaggaggca atgtaaccgc ctctggtagt 4920
acacttctct aatccaaaaa tcaatttgta ttcaagatac cgcaaaaaac ttatggttta 4980
aaccctgcag gactagtcca gaaggtaatt atccaagatg tagcatcaag aatccaatgt 5040
ttacgggaaa aactatggaa gtattatgta agctcagcaa gaagcagatc aatatgcggc 5100
acatatgcaa cctatgttca aaaatgaaga atgtacagat acaagatcct atactgccag 5160
aatacgaaga agaatacgta gaaattgaaa aagaagaacc aggcgaagaa aagaatcttg 5220
aagacgtaag cactgacgac aacaatgaaa agaagaagat aaggtcggtg attgtgaaag 5280
agacatagag gacacatgta aggtggaaaa tgtaagggcg gaaagtaacc ttatcacaaa 5340
ggaatcttat cccccactac ttatcctttt atatttttcc gtgtcatttt tgcccttgag 5400
ttttcctata taaggaacca agttcggcat ttgtgaaaac aagaaaaaat ttggtgtaag 5460
ctattttctt tgaagtactg aggatacaac ttcagagaaa tttgtaagtt tgtaggtacc 5520
agatctggat cccaaaccat gtctccggag aggagaccag ttgagattag gccagctaca 5580
gcagctgata tggccgcggt ttgtgatatc gttaaccatt acattgagac gtctacagtg 5640
aactttagga cagagccaca aacaccacaa gagtggattg atgatctaga gaggttgcaa 5700

```
gatagatacc cttggttggt tgctgaggtt gagggtgttg tggctggtat tgcttacgct   5760
gggccctgga aggctaggaa cgcttacgat tggacagttg agagtactgt ttacgtgtca   5820
cataggcatc aaaggttggg cctaggatct acattgtaca cacatttgct taagtctatg   5880
gaggcgcaag gttttaagtc tgtggttgct gttataggcc ttccaaacga tccatctgtt   5940
aggttgcatg aggctttggg atacacagcc cggggtacat tgcgcgcagc tggatacaag   6000
catggtggat ggcatgatgt tggtttttgg caaagggatt ttgagttgcc agctcctcca   6060
aggccagtta ggccagttac ccaaatctga gtagttagct taatcaccta gagctcgatc   6120
ggcggcaata gcttcttagc gccatcccgg gttgatccta tctgtgttga aatagttgcg   6180
gtgggcaagg ctctctttca gaaagacagg cggccaaagg aacccaaggt gaggtgggct   6240
atggctctca gttccttgtg gaagcgcttg gtctaaggtg cagaggtgtt agcgggatga   6300
agcaaaagtg tccgattgta acaagatatg ttgatcctac gtaaggatat taaagtatgt   6360
attcatcact aatataatca gtgtattcca atatgtacta cgatttccaa tgtctttatt   6420
gtcgccgtat gtaatcggcg tcacaaaata atccccggtg actttctttt aatccaggat   6480
gaaataatat gttattataa tttttgcgat ttggtccgtt ataggaattg aagtgtgctt   6540
gaggtcggtc gccaccactc ccatttcata attttacatg tatttgaaaa ataaaaattt   6600
atggtattca atttaaacac gtatacttgt aaagaatgat atcttgaaag aaatatagtt   6660
taaatattta ttgataaaat aacaagtcag gtattatagt ccaagcaaaa acataaattt   6720
attgatgcaa gtttaaattc agaaatattt caataactga ttatatcagc tggtacattg   6780
ccgtagatga aagactgagt gcgatattat ggtgtaatac ataggaattc gtttaaacga   6840
tctgcgtcta attttcggtc caacttgcac aggaaagacg tcgaccgcgg tagctcttgc   6900
ccagcagact gggcttccag tcctttcgct cgatcgggtc caatgttgtc ctcagctgtg   6960
aaccggaagc ggacgaccaa cagtggaaga actgaaagga acgagccgtc tataccttga   7020
tgatcggcct ctggtgaagg gtatcatcgc agccaagcaa gctcatgaaa ggctgatggg   7080
ggaggtgtat aattatgagg cccacggcgg gcttattctt tagggaggat ctatctcgtt   7140
gctcaagtgc atggcgcaaa gcagttattg gagtgcggat tttcgttggc atattattcg   7200
ccacgagtta gcagacgaag agaccttcat gaacgtggcc aaggccagag ttaagcagat   7260
gttacgccct gctgcaggcc tttctattat ccaatagttg gttgatcttt ggaaagagcc   7320
tcggctgagg cccatactga aagagatcga tggatatcga tatgccatgt tgtttgctag   7380
ccagaaccag atcacatccg atatgctatt gcagcttgac gcagatatgg aggataagtt   7440
gattcatggg atcgctcagg agtagctcat ccatgcacgc cgacaagaac agaaattccg   7500
tcgagttaac gcagccgctt acgacggatt cgaaggtcat ccattcggaa tgtattagtt   7560
tgcaccagct ccgcgtcaca cctgtcttca tttgaataag atgttagcaa ttgtttttag   7620
ctttgtcttg ttgtggcagg gcggcaagtg cttcagacat cattctgttt tcaaatttta   7680
tgctggagaa cagcttctta attcctttgg aaataataga ctgcgtctta aaattcagat   7740
```

gtctggatat agatatgatt gtaaaataac ctatttaagt gtcatttaga acataagttt 7800
tatgaatgtt cttccatttt cgtcatcgaa cgaataagag taaatacacc ttttttaaca 7860
ttacaaataa gttcttatac gttgtttata caccgggaat catttccatt attttcgcgc 7920
aaaagtcacg gatattcgtg aaagcgacat aaactgcgaa atttgcgggg agtgtcttga 7980
gtttgcctcg aggctagcgc atgcacatag acacacacat catctcattg atgcttggta 8040
ataattgtca ttagattgtt tttatgcata gatgcactcg aaatcagcca attttagaca 8100
agtatcaaac ggatgtgact tcagtacatt aaaaacgtcc gcaatgtgtt attaagttgt 8160
ctaagcgtca atttgattta caattgaata tatcctgccc cagccagcca acagctcgat 8220
ttacaattga atatatcctg ccggccggcc cacgcgtgtc gaggaattct gatctggccc 8280
ccatttggac gtgaatgtag acacgtcgaa ataaagattt ccgaattaga ataatttgtt 8340
tattgctttc gcctataaat acgacggatc gtaatttgtc gttttatcaa aatgtacttt 8400
cattttataa taacgctgcg gacatctaca tttttgaatt gaaaaaaaat tggtaattac 8460
tctttctttt tctccatatt gaccatcata ctcattgctg atccatgtag atttcccgga 8520
catgaagcca tttacaattg aatatatcct gccgccgctg ccgctttgca cccggtggag 8580
cttgcatgtt ggtttctacg cagaactgag ccggttaggc agataatttc cattgagaac 8640
tgagccatgt gcaccttccc cccaacacgg tgagcgacgg ggcaacggag tgatccacat 8700
gggactttta aacatcatcc gtcggatggc gttgcgagag aagcagtcga tccgtgagat 8760
cagccgacgc accgggcagg cgcgcaacac gatcgcaaag tatttgaacg caggtacaat 8820
cgagccgacg ttcacgcgga acgaccaagc aagcttggct gccatttttg gggtgaggcc 8880
gttcgcggcc gaggggcgca gcccctgggg ggatgggagg cccgcgttag cgggccggga 8940
gggttcgaga agggggggca cccccccttcg gcgtgcgcgg tcacgcgcac agggcgcagc 9000
cctggttaaa aacaaggttt ataaatattg gtttaaaagc aggttaaaag acaggttagc 9060
ggtggccgaa aaacgggcgg aaacccttgc aaatgctgga ttttctgcct gtggacagcc 9120
cctcaaatgt caataggtgc gcccctcatc tgtcagcact ctgcccctca agtgtcaagg 9180
atcgcgcccc tcatctgtca gtagtcgcgc ccctcaagtg tcaataccgc agggcactta 9240
tccccaggct tgtccacatc atctgtggga aactcgcgta aaatcaggcg ttttcgccga 9300
tttgcgaggc tggccagctc cacgtcgccg gccgaaatcg agcctgcccc tcatctgtca 9360
acgccgcgcc gggtgagtcg gcccctcaag tgtcaacgtc cgcccctcat ctgtcagtga 9420
gggccaagtt ttccgcgagg tatccacaac gccggcggcc gcggtgtctc gcacacggct 9480
tcgacggcgt ttctggcgcg tttgcagggc catagacggc cgccagccca gcggcgaggg 9540
caaccagccc ggtgagcgtc ggaaagggtc gacggatctt ttccgctgca taaccctgct 9600
tcggggtcat tatagcgatt ttttcggtat atccatcctt tttcgcacga tatacaggat 9660
tttgccaaag ggttcgtgta gactttcctt ggtgtatcca acggcgtcag ccgggcagga 9720
taggtgaagt aggcccaccc gcgagcgggt gttccttctt cactgtccct tattcgcacc 9780

tggcggtgct caacgggaat cctgctctgc gaggctggcc ggctaccgcc ggcgtaacag 9840
atgagggcaa gcggatggct gatgaaacca agccaaccag gaagggcagc ccacctatca 9900
aggtgtactg ccttccagac gaacgaagag cgattgagga aaaggcggcg gcggccggca 9960
tgagcctgtc ggcctacctg ctggccgtcg gccagggcta caaaatcacg ggcgtcgtgg 10020
actatgagca cgtccgcgag ctggcccgca tcaatggcga cctgggccgc ctgggcggcc 10080
tgctgaaact ctggctcacc gacgacccgc gcacggcgcg gttcggtgat gccacgatcc 10140
tcgccctgct ggcgaagatc gaagagaagc aggacgagct tggcaaggtc atgatgggcg 10200
tggtccgccc gagggcagag ccatgacttt tttagccgct aaaacggccg gggggtgcgc 10260
gtgattgcca agcacgtccc catgcgctcc atcaagaaga gcgacttcgc ggagctggta 10320
ttcgtgcagg gcaagattcg gaataccaag tacgagaagg acggccagac ggtctacggg 10380
accgacttca ttgccgataa ggtggattat ctggacacca aggcaccagg cgggtcaaat 10440
caggaataag ggcacattgc cccggcgtga gtcggggcaa tcccgcaagg agggtgaatg 10500
aatcggacgt ttgaccggaa ggcatacagg caagaactga tcgacgcggg gttttccgcc 10560
gaggatgccg aaaccatcgc aagccgcacc gtcatgcgtg cgccccgcga aaccttccag 10620
tccgtcggct cgatggtcca gcaagctacg gccaagatcg agcgcgacag cgtgcaactg 10680
gctccccctg ccctgcccgc gccatcggcc gccgtggagc gttcgcgtcg tctcgaacag 10740
gaggcggcag gtttggcgaa gtcgatgacc atcgacacgc gaggaactat gacgaccaag 10800
aagcgaaaaa ccgccggcga ggacctggca aaacaggtca gcgaggccaa gcaggccgcg 10860
ttgctgaaac acacgaagca gcagatcaag gaaatgcagc tttccttgtt cgatattgcg 10920
ccgtggccgg acacgatgcg agcgatgcca aacgacacgg cccgctctgc cctgttcacc 10980
acgcgcaaca agaaaatccc gcgcgaggcg ctgcaaaaca aggtcatttt ccacgtcaac 11040
aaggacgtga agatcaccta caccggcgtc gagctgcggg ccgacgatga cgaactggtg 11100
tggcagcagg tgttggagta cgcgaagcgc acccctatcg gcgagccgat caccttcacg 11160
ttctacgagc tttgccagga cctgggctgg tcgatcaatg gccggtatta cacgaaggcc 11220
gaggaatgcc tgtcgcgcct acaggcgacg gcgatgggct tcacgtccga ccgcgttggg 11280
cacctggaat cggtgtcgct gctgcaccgc ttccgcgtcc tggaccgtgg caagaaaacg 11340
tcccgttgcc aggtcctgat cgacgaggaa atcgtcgtgc tgtttgctgg cgaccactac 11400
acgaaattca tatgggagaa gtaccgcaag ctgtcgccga cggcccgacg gatgttcgac 11460
tatttcagct cgcaccggga gccgtacccg ctcaagctgg aaaccttccg cctcatgtgc 11520
ggatcggatt ccacccgcgt gaagaagtgg cgcgagcagg tcggcgaagc ctgcgaagag 11580
ttgcgaggca gcggcctggt ggaacacgcc tgggtcaatg atgacctggt gcattgcaaa 11640
cgctagggcc ttgtggggtc agttccggct gggggttcag cagccagcgc tttactggca 11700
tttcaggaac aagcgggcac tgctcgacgc acttgcttcg ctcagtatcg ctcgggacgc 11760
acggcgcgct ctacgaactg ccgataaaca gaggattaaa attgacaatt gtgattaagg 11820

ctcagattcg acggcttgga gcggccgacg tgcaggattt ccgcgagatc cgattgtcgg 11880
ccctgaagaa agctccagag atgttcgggt ccgtttacga gcacgaggag aaaaagccca 11940
tggaggcgtt cgctgaacgg ttgcgagatg ccgtggcatt cggcgcctac atcgacggcg 12000
agatcattgg gctgtcggtc ttcaaacagg aggacggccc caaggacgct cacaaggcgc 12060
atctgtccgg cgttttcgtg gagcccgaac agcgaggccg aggggtcgcc ggtatgctgc 12120
tgcgggcgtt gccggcgggt ttattgctcg tgatgatcgt ccgacagatt ccaacgggaa 12180
tctggtggat gcgcatcttc atcctcggcg cacttaatat ttcgctattc tggagcttgt 12240
tgtttatttc ggtctaccgc ctgccgggcg gggtcgcggc gacggtaggc gctgtgcagc 12300
cgctgatggt cgtgttcatc tctgccgctc tgctaggtag cccgatacga ttgatggcgg 12360
tcctgggggc tatttgcgga actgcgggcg tggcgctgtt ggtgttgaca ccaaacgcag 12420
cgctagatcc tgtcggcgtc gcagcgggcc tggcgggggc ggtttccatg gcgttcggaa 12480
ccgtgctgac ccgcaagtgg caacctcccg tgcctctgct cacctttacc gcctggcaac 12540
tggcggccgg aggacttctg ctcgttccag tagctttagt gtttgatccg ccaatcccga 12600
tgcctacagg aaccaatgtt ctcggcctgg cgtggctcgg cctgatcgga gcgggtttaa 12660
cctacttcct ttggttccgg gggatctcgc gactcgaacc tacagttgtt tccttactgg 12720
gctttctcag cccccgagcg cttagtggga atttgtaccc cttatcgaac cgggagcaca 12780
ggatgacgcc taacaattca ttcaagccga caccgcttcg cggcgcggct taattcagga 12840
gttaaacatc atgagggaag cggtgatcgc cgaagtatcg actcaactat cagaggtagt 12900
tggcgtcatc gagcgccatc tcgaaccgac gttgctggcc gtacatttgt acggctccgc 12960
agtggatggc ggcctgaagc cacacagtga tattgatttg ctggttacgg tgaccgtaag 13020
gcttgatgaa acaacgcggc gagctttgat caacgacctt ttggaaactt cggcttcccc 13080
tggagagagc gagattctcc gcgctgtaga agtcaccatt gttgtgcacg acgacatcat 13140
tccgtggcgt tatccagcta agcgcgaact gcaatttgga gaatggcagc gcaatgacat 13200
tcttgcaggt atcttcgagc cagccacgat cgacattgat ctggctatct tgctgacaaa 13260
agcaagagaa catagcgttg ccttggtagg tccagcggcg gaggaactct ttgatccggt 13320
tcctgaacag gatctatttg aggcgctaaa tgaaacctta acgctatgga actcgccgcc 13380
cgactgggct ggcgatgagc gaaatgtagt gcttacgttg tcccgcattt ggtacagcgc 13440
agtaaccggc aaaatcgcgc cgaaggatgt cgctgccgac tgggcaatgg agcgcctgcc 13500
ggcccagtat cagcccgtca tacttgaagc taggcaggct tatcttggac aagaagatcg 13560
cttggcctcg cgcgcagatc agttggaaga atttgttcac tacgtgaaag gcgagatcac 13620
caaggtagtc ggcaaataat gtctaacaat tcgttcaagc cgacgccgct tcgcggcgcg 13680
gcttaactca agcgttagag agctggggaa gactatgcgc gatctgttga aggtggttct 13740
aagcctcgtc ttgcgatggc atttcgatcc attcccattc cgcgctcaag atggcttccc 13800
ctcggcagtt catcagggct aaatcaatct agccgacttg tccggtgaaa tgggctgcac 13860

tccaacagaa acaatcaaac aaacatacac agcgacttat tcacacgagc tcaaattaca 13920

acggtatata tcctgccagt cagcatcatc acaccaaaag ttaggcccga atagtttgaa 13980

attagaaagc tcgcaattga ggtctacagg ccaaattcgc tcttagccgt acaatattac 14040

tcaccggatc ctaaccggtt taattaaggc gcgccatgcc cgggcaagcg gccgcacaag 14100

ttt 14103

<210> 75
<211> 13812
<212> DNA
<213> Artificial

<220>
<223> Plasmid pDAB110112

<400> 75

```
cttgtacaaa gtggttgcgg ccgcttaatt aaatttaaat tcaattaatg caatcttgat      60
tttcaacaac gaaggtaatg gcgtaaaaga aaaaatgtat gttattgtat tgatctttca     120
tgatgttgaa gcgtgccata atatgatgat gtataattaa aatattaact gtcgcatttt     180
attgaaatgg cactgttatt tcaaccatat ctttgattct gttacatgac acgactgcaa     240
gaagtaaata atagacgccg ttgttaaaga attgctatca tatgtgccta actagaggga     300
atttgagcgt cagacctaat caaatattac aaaatatctc actctgtcgc cagcaatggt     360
gtaatcagcg cagacaaatg gcgtaaagat cgcggaaaaa cctccccgag tggcatgata     420
gctgcctctg tattgctgat ttagtcagcc ttatttgact taagggtgcc ctcgttagtg     480
acaaattgct ttcaaggaga cagccatgcc ccacactttg ttgaaaaaca aattgccttt     540
ggggagacgg taaagccagt tgctcttcaa taaggaatgt cgaggaggca atgtaaccgc     600
ctctggtagt acacttctct aatccaaaaa tcaatttgta ttcaagatac cgcaaaaaac     660
ttatggttta aaccctgcag gactagtcca gaaggtaatt atccaagatg tagcatcaag     720
aatccaatgt ttacgggaaa aactatggaa gtattatgta agctcagcaa gaagcagatc     780
aatatgcggc acatatgcaa cctatgttca aaaatgaaga atgtacagat acaagatcct     840
atactgccag aatacgaaga agaatacgta gaaattgaaa aagaagaacc aggcgaagaa     900
aagaatcttg aagacgtaag cactgacgac aacaatgaaa agaagaagat aaggtcggtg     960
attgtgaaag agacatagag gacacatgta aggtggaaaa tgtaagggcg gaaagtaacc    1020
ttatcacaaa ggaatcttat cccccactac ttatcctttt atatttttcc gtgtcatttt    1080
tgcccttgag ttttcctata taaggaacca agttcggcat ttgtgaaaac aagaaaaaat    1140
ttggtgtaag ctattttctt tgaagtactg aggatacaac ttcagagaaa tttgtaagtt    1200
tgtaggtacc agatctggat cccaaaccat gtctccggag aggagaccag ttgagattag    1260
gccagctaca gcagctgata tggccgcggt ttgtgatatc gttaaccatt acattgagac    1320
gtctacagtg aactttagga cagagccaca aacaccacaa gagtggattg atgatctaga    1380
gaggttgcaa gatagatacc cttggttggt tgctgaggtt gagggtgttg tggctggtat    1440
```

tgcttacgct gggccctgga aggctaggaa cgcttacgat tggacagttg agagtactgt 1500
ttacgtgtca cataggcatc aaaggttggg cctaggatct acattgtaca cacatttgct 1560
taagtctatg gaggcgcaag gttttaagtc tgtggttgct gttataggcc ttccaaacga 1620
tccatctgtt aggttgcatg aggctttggg atacacagcc cggggtacat tgcgcgcagc 1680
tggatacaag catggtggat ggcatgatgt tggtttttgg caaagggatt ttgagttgcc 1740
agctcctcca aggccagtta ggccagttac ccaaatctga gtagttagct taatcaccta 1800
gagctcgatc ggcggcaata gcttcttagc gccatcccgg gttgatccta tctgtgttga 1860
aatagttgcg gtgggcaagg ctctctttca gaaagacagg cggccaaagg aacccaaggt 1920
gaggtgggct atggctctca gttccttgtg gaagcgcttg gtctaaggtg cagaggtgtt 1980
agcgggatga agcaaaagtg tccgattgta acaagatatg ttgatcctac gtaaggatat 2040
taaagtatgt attcatcact aatataatca gtgtattcca atatgtacta cgatttccaa 2100
tgtctttatt gtcgccgtat gtaatcggcg tcacaaaata atccccggtg actttctttt 2160
aatccaggat gaaataatat gttattataa tttttgcgat ttggtccgtt ataggaattg 2220
aagtgtgctt gaggtcggtc gccaccactc ccatttcata attttacatg tatttgaaaa 2280
ataaaaattt atggtattca atttaaacac gtatacttgt aaagaatgat atcttgaaag 2340
aaatatagtt taaatattta ttgataaaat aacaagtcag gtattatagt ccaagcaaaa 2400
acataaattt attgatgcaa gtttaaattc agaaatattt caataactga ttatatcagc 2460
tggtacattg ccgtagatga aagactgagt gcgatattat ggtgtaatac ataggaattc 2520
gtttaaacga tctgcgtcta attttcggtc caacttgcac aggaaagacg tcgaccgcgg 2580
tagctcttgc ccagcagact gggcttccag tcctttcgct cgatcgggtc caatgttgtc 2640
ctcagctgtg aaccggaagc ggacgaccaa cagtggaaga actgaaagga acgagccgtc 2700
tataccttga tgatcggcct ctggtgaagg gtatcatcgc agccaagcaa gctcatgaaa 2760
ggctgatggg ggaggtgtat aattatgagg cccacggcgg gcttattctt tagggaggat 2820
ctatctcgtt gctcaagtgc atggcgcaaa gcagttattg gagtgcggat tttcgttggc 2880
atattattcg ccacgagtta gcagacgaag agaccttcat gaacgtggcc aaggccagag 2940
ttaagcagat gttacgccct gctgcaggcc tttctattat ccaatagttg gttgatcttt 3000
ggaaagagcc tcggctgagg cccatactga aagagatcga tggatatcga tatgccatgt 3060
tgtttgctag ccagaaccag atcacatccg atatgctatt gcagcttgac gcagatatgg 3120
aggataagtt gattcatggg atcgctcagg agtagctcat ccatgcacgc cgacaagaac 3180
agaaattccg tcgagttaac gcagccgctt acgacggatt cgaaggtcat ccattcggaa 3240
tgtattagtt tgcaccagct ccgcgtcaca cctgtcttca tttgaataag atgttagcaa 3300
ttgtttttag ctttgtcttg ttgtggcagg gcggcaagtg cttcagacat cattctgttt 3360
tcaaatttta tgctggagaa cagcttctta attcctttgg aaataataga ctgcgtctta 3420
aaattcagat gtctggatat agatatgatt gtaaaataac ctatttaagt gtcatttaga 3480

acataagttt tatgaatgtt cttccatttt cgtcatcgaa cgaataagag taaatacacc 3540
ttttttaaca ttacaaataa gttcttatac gttgtttata caccgggaat catttccatt 3600
attttcgcgc aaaagtcacg gatattcgtg aaagcgacat aaactgcgaa atttgcgggg 3660
agtgtcttga gtttgcctcg aggctagcgc atgcacatag acacacacat catctcattg 3720
atgcttggta ataattgtca ttagattgtt tttatgcata gatgcactcg aaatcagcca 3780
attttagaca agtatcaaac ggatgtgact tcagtacatt aaaaacgtcc gcaatgtgtt 3840
attaagttgt ctaagcgtca atttgattta caattgaata tatcctgccc cagccagcca 3900
acagctcgat ttacaattga atatatcctg ccggccggcc cacgcgtgtc gaggaattct 3960
gatctggccc ccatttggac gtgaatgtag acacgtcgaa ataaagattt ccgaattaga 4020
ataatttgtt tattgctttc gcctataaat acgacggatc gtaatttgtc gttttatcaa 4080
aatgtacttt cattttataa taacgctgcg gacatctaca tttttgaatt gaaaaaaaat 4140
tggtaattac tctttctttt tctccatatt gaccatcata ctcattgctg atccatgtag 4200
atttcccgga catgaagcca tttacaattg aatatatcct gccgccgctg ccgctttgca 4260
cccggtggag cttgcatgtt ggtttctacg cagaactgag ccggttaggc agataatttc 4320
cattgagaac tgagccatgt gcaccttccc cccaacacgg tgagcgacgg ggcaacggag 4380
tgatccacat gggactttta aacatcatcc gtcggatggc gttgcgagag aagcagtcga 4440
tccgtgagat cagccgacgc accgggcagg cgcgcaacac gatcgcaaag tatttgaacg 4500
caggtacaat cgagccgacg ttcacgcgga acgaccaagc aagcttggct gccatttttg 4560
gggtgaggcc gttcgcggcc gaggggcgca gcccctgggg ggatgggagg cccgcgttag 4620
cgggccggga gggttcgaga agggggggca ccccccttcg gcgtgcgcgg tcacgcgcac 4680
agggcgcagc cctggttaaa aacaaggttt ataaatattg gtttaaaagc aggttaaaag 4740
acaggttagc ggtggccgaa aaacgggcgg aaacccttgc aaatgctgga ttttctgcct 4800
gtggacagcc cctcaaatgt caataggtgc gcccctcatc tgtcagcact ctgcccctca 4860
agtgtcaagg atcgcgcccc tcatctgtca gtagtcgcgc ccctcaagtg tcaataccgc 4920
agggcactta tccccaggct tgtccacatc atctgtggga aactcgcgta aaatcaggcg 4980
ttttcgccga tttgcgaggc tggccagctc cacgtcgccg gccgaaatcg agcctgcccc 5040
tcatctgtca acgccgcgcc gggtgagtcg gcccctcaag tgtcaacgtc cgcccctcat 5100
ctgtcagtga gggccaagtt ttccgcgagg tatccacaac gccggcggcc gcggtgtctc 5160
gcacacggct tcgacggcgt ttctggcgcg tttgcagggc catagacggc cgccagccca 5220
gcggcgaggg caaccagccc ggtgagcgtc ggaaagggtc gacggatctt ttccgctgca 5280
taaccctgct tcggggtcat tatagcgatt ttttcggtat atccatcctt tttcgcacga 5340
tatacaggat tttgccaaag ggttcgtgta gactttcctt ggtgtatcca acggcgtcag 5400
ccgggcagga taggtgaagt aggcccaccc gcgagcgggt gttccttctt cactgtccct 5460
tattcgcacc tggcggtgct caacgggaat cctgctctgc gaggctggcc ggctaccgcc 5520

ggcgtaacag atgagggcaa gcggatggct gatgaaacca agccaaccag gaagggcagc 5580
ccacctatca aggtgtactg ccttccagac gaacgaagag cgattgagga aaaggcggcg 5640
gcggccggca tgagcctgtc ggcctacctg ctggccgtcg gccagggcta caaaatcacg 5700
ggcgtcgtgg actatgagca cgtccgcgag ctggcccgca tcaatggcga cctgggccgc 5760
ctgggcggcc tgctgaaact ctggctcacc gacgacccgc gcacggcgcg gttcggtgat 5820
gccacgatcc tcgccctgct ggcgaagatc gaagagaagc aggacgagct tggcaaggtc 5880
atgatgggcg tggtccgccc gagggcagag ccatgacttt tttagccgct aaaacggccg 5940
gggggtgcgc gtgattgcca agcacgtccc catgcgctcc atcaagaaga gcgacttcgc 6000
ggagctggta ttcgtgcagg gcaagattcg gaataccaag tacgagaagg acggccagac 6060
ggtctacggg accgacttca ttgccgataa ggtggattat ctggacacca aggcaccagg 6120
cgggtcaaat caggaataag ggcacattgc cccggcgtga gtcggggcaa tcccgcaagg 6180
agggtgaatg aatcggacgt ttgaccggaa ggcatacagg caagaactga tcgacgcggg 6240
gttttccgcc gaggatgccg aaaccatcgc aagccgcacc gtcatgcgtg cgccccgcga 6300
aaccttccag tccgtcggct cgatggtcca gcaagctacg gccaagatcg agcgcgacag 6360
cgtgcaactg gctccccctg ccctgcccgc gccatcggcc gccgtggagc gttcgcgtcg 6420
tctcgaacag gaggcggcag gtttggcgaa gtcgatgacc atcgacacgc gaggaactat 6480
gacgaccaag aagcgaaaaa ccgccggcga ggacctggca aaacaggtca gcgaggccaa 6540
gcaggccgcg ttgctgaaac acacgaagca gcagatcaag gaaatgcagc tttccttgtt 6600
cgatattgcg ccgtggccgg acacgatgcg agcgatgcca aacgacacgg cccgctctgc 6660
cctgttcacc acgcgcaaca agaaaatccc gcgcgaggcg ctgcaaaaca aggtcatttt 6720
ccacgtcaac aaggacgtga agatcaccta caccggcgtc gagctgcggg ccgacgatga 6780
cgaactggtg tggcagcagg tgttggagta cgcgaagcgc acccctatcg gcgagccgat 6840
caccttcacg ttctacgagc tttgccagga cctgggctgg tcgatcaatg gccggtatta 6900
cacgaaggcc gaggaatgcc tgtcgcgcct acaggcgacg gcgatgggct tcacgtccga 6960
ccgcgttggg cacctggaat cggtgtcgct gctgcaccgc ttccgcgtcc tggaccgtgg 7020
caagaaaacg tcccgttgcc aggtcctgat cgacgaggaa atcgtcgtgc tgtttgctgg 7080
cgaccactac acgaaattca tatgggagaa gtaccgcaag ctgtcgccga cggcccgacg 7140
gatgttcgac tatttcagct cgcaccggga gccgtacccg ctcaagctgg aaaccttccg 7200
cctcatgtgc ggatcggatt ccacccgcgt gaagaagtgg cgcgagcagg tcggcgaagc 7260
ctgcgaagag ttgcgaggca gcggcctggt ggaacacgcc tgggtcaatg atgacctggt 7320
gcattgcaaa cgctagggcc ttgtggggtc agttccggct gggggttcag cagccagcgc 7380
tttactggca tttcaggaac aagcgggcac tgctcgacgc acttgcttcg ctcagtatcg 7440
ctcgggacgc acggcgcgct ctacgaactg ccgataaaca gaggattaaa attgacaatt 7500
gtgattaagg ctcagattcg acggcttgga gcggccgacg tgcaggattt ccgcgagatc 7560

```
cgattgtcgg ccctgaagaa agctccagag atgttcgggt ccgtttacga gcacgaggag    7620
aaaaagccca tggaggcgtt cgctgaacgg ttgcgagatg ccgtggcatt cggcgcctac    7680
atcgacggcg agatcattgg gctgtcggtc ttcaaacagg aggacggccc caaggacgct    7740
cacaaggcgc atctgtccgg cgttttcgtg gagcccgaac agcgaggccg aggggtcgcc    7800
ggtatgctgc tgcgggcgtt gccggcgggt ttattgctcg tgatgatcgt ccgacagatt    7860
ccaacgggaa tctggtggat gcgcatcttc atcctcggcg cacttaatat ttcgctattc    7920
tggagcttgt tgtttatttc ggtctaccgc ctgccgggcg gggtcgcggc gacggtaggc    7980
gctgtgcagc cgctgatggt cgtgttcatc tctgccgctc tgctaggtag cccgatacga    8040
ttgatggcgg tcctgggggc tatttgcgga actgcgggcg tggcgctgtt ggtgttgaca    8100
ccaaacgcag cgctagatcc tgtcggcgtc gcagcgggcc tggcgggggc ggtttccatg    8160
gcgttcggaa ccgtgctgac ccgcaagtgg caacctcccg tgcctctgct cacctttacc    8220
gcctggcaac tggcggccgg aggacttctg ctcgttccag tagctttagt gtttgatccg    8280
ccaatcccga tgcctacagg aaccaatgtt ctcggcctgg cgtggctcgg cctgatcgga    8340
gcgggtttaa cctacttcct ttggttccgg gggatctcgc gactcgaacc tacagttgtt    8400
tccttactgg ctttctcag cccccgagcg cttagtggga atttgtaccc cttatcgaac    8460
cgggagcaca ggatgacgcc taacaattca ttcaagccga caccgcttcg cggcgcggct    8520
taattcagga gttaaacatc atgagggaag cggtgatcgc cgaagtatcg actcaactat    8580
cagaggtagt tggcgtcatc gagcgccatc tcgaaccgac gttgctggcc gtacatttgt    8640
acggctccgc agtggatggc ggcctgaagc cacacagtga tattgatttg ctggttacgg    8700
tgaccgtaag gcttgatgaa acaacgcggc gagctttgat caacgacctt ttggaaactt    8760
cggcttcccc tggagagagc gagattctcc gcgctgtaga agtcaccatt gttgtgcacg    8820
acgacatcat tccgtggcgt tatccagcta agcgcgaact gcaatttgga gaatggcagc    8880
gcaatgacat tcttgcaggt atcttcgagc cagccacgat cgacattgat ctggctatct    8940
tgctgacaaa agcaagagaa catagcgttg ccttggtagg tccagcggcg gaggaactct    9000
ttgatccggt tcctgaacag gatctatttg aggcgctaaa tgaaacctta acgctatgga    9060
actcgccgcc cgactgggct ggcgatgagc gaaatgtagt gcttacgttg tcccgcattt    9120
ggtacagcgc agtaaccggc aaaatcgcgc cgaaggatgt cgctgccgac tgggcaatgg    9180
agcgcctgcc ggcccagtat cagcccgtca tacttgaagc taggcaggct tatcttggac    9240
aagaagatcg cttggcctcg cgcgcagatc agttggaaga atttgttcac tacgtgaaag    9300
gcgagatcac caaggtagtc ggcaaataat gtctaacaat tcgttcaagc cgacgccgct    9360
tcgcggcgcg gcttaactca agcgttagag agctggggaa gactatgcgc gatctgttga    9420
aggtggttct aagcctcgtc ttgcgatggc atttcgatcc attcccattc cgcgctcaag    9480
atggcttccc ctcggcagtt catcagggct aaatcaatct agccgacttg tccggtgaaa    9540
tgggctgcac tccaacagaa acaatcaaac aaacatacac agcgacttat tcacacgagc    9600
```

tcaaattaca acggtatata tcctgccagt cagcatcatc acaccaaaag ttaggcccga 9660
atagtttgaa attagaaagc tcgcaattga ggtctacagg ccaaattcgc tcttagccgt 9720
acaatattac tcaccggatc ctaaccggtt taattaaggc gcgccatgcc cgggcaagcg 9780
gccgcacaag tttgtacaaa aaagcaggct tctagaccta ggtggagtca tcacgcagac 9840
tatctcagca tgtgcgtagc acgcggccgc ctcccagtat cattatagtg aaagttttgg 9900
ctctctcgcc ggtggttttt tacctctatt taaaggggtt ttccacctaa aaattctggt 9960
atcattctca ctttacttgt tactttaatt tctcataatc tttggttgaa attatcacgc 10020
ttccgcacac gatatcccta caaatttatt atttgttaaa cattttcaaa ccgcataaaa 10080
ttttatgaag tcccgtctat ctttaatgta gtctaacatt ttcatattga aatatataat 10140
ttacttaatt ttagcgttgg tagaaagcat aatgatttat tcttattctt cttcatataa 10200
atgtttaata tacaatataa acaaattctt taccttaaga aggatttccc attttatatt 10260
ttaaaaatat atttatcaaa tatttttcaa ccacgtaaat ctcataataa taagttgttt 10320
caaaagtaat aaaatttaac tccataattt ttttattcga ctgatcttaa agcaacaccc 10380
agtgacacaa ctagccattt ttttctttga ataaaaaaat ccaattatca ttgtattttt 10440
tttatacaat gaaaatttca ccaaacaatg atttgtggta tttctgaagc aagtcatgtt 10500
atgcaaaatt ctataattcc catttgacac tacggaagta actgaagatc tgcttttaca 10560
tgcgagacac atcttctaaa gtaattttaa taatagttac tatattcaag atttcatata 10620
tcaaatactc aatattactt ctaaaaaatt aattagatat aattaaaata ttactttttt 10680
aattttaagt ttaattgttg aatttgtgac tattgattta ttattctact atgtttaaat 10740
tgttttatag atagtttaaa gtaaatataa gtaatgtagt agagtgttag agtgttaccc 10800
taaaccataa actataagat ttatggtgga ctaattttca tatatttctt attgctttta 10860
ccttttcttg gtatgtaagt ccgtaactgg aattactgtg ggttgccatg acactctgtg 10920
gtcttttggt tcatgcatgg atcttgcgca agaaaaagac aaagaacaaa gaaaaaagac 10980
aaaacagaga gacaaaacgc aatcacacaa ccaactcaaa ttagtcactg gctgatcaag 11040
atcgccgcgt ccatgtatgt ctaaatgcca tgcaaagcaa cacgtgctta acatgcactt 11100
taaatggctc acccatctca acccacacac aaacacattg ccttttcttt catcatcacc 11160
acaaccacct gtatatattc attctcttcc gccacctcaa tttcttcact tcaacacacg 11220
tcaacctgca tatgcgtgtc atcccatgcc caaatctcca tgcatgttcc aaccaccttc 11280
tctcttatat aatacctata aatacctcta atatcactca cttctttcat catccatcca 11340
tccagagtac tactactcta ctactataat accccaaccc aactcatatt caatactact 11400
ctaggatcca acaatggctc ccaacatttc tgaggatgtc aatggtgttc tttttgagtc 11460
agatgcggca acccctgatt tggctctttc cacaccacct gtgcaaaaag ctgacaacag 11520
acccaagcaa cttgtgtgga ggaacatttt gcttttcgct tacttgcacc tcgcagctct 11580
ctacggaggc tatttgtttc tcttcagtgc aaaatggcag accgacattt tcgcttacat 11640

tctttatgtc atctctggac tggggataac tgctggggca catagactct gggctcacaa 11700
gtcatacaaa gccaagtggc cactcagagt tatactggtc atcttcaaca cggttgcctt 11760
tcaagacgct gctatggatt gggctcgtga ccatagaatg catcacaagt acagcgagac 11820
cgacgcggac ccacacaatg caacgagagg tttcttcttc tctcacattg gctggcttct 11880
tgttaggaaa catcctgatc tgaaagaaaa agggaaggga ctcgacatga gtgatctcct 11940
tgctgatcca atactccgtt ttcagaagaa gtactatctg atcctcatgc ctctggcctg 12000
ttttgtgatg ccaaccgtta tcccggttta cttttgggga gaaacttgga caaatgcttt 12060
cttcgtggca gccatgttcc gttatgcttt catcctgaat gttacctggt tggtgaactc 12120
tgccgcacac aagtggggag acaaacccta tgacaagtcc atcaagcctt ccgaaaacct 12180
ttcagttgcg atgtttgctt tgggagaagg atttcacaat taccatcaca cttttccgtg 12240
ggactacaag acagcagagc ttggaaacaa caagttgaac ttcacaacaa cgttcatcaa 12300
tttctttgcg aaaatcggtt gggcctatga tttgaagact gtgagtgatg acattgtcaa 12360
gaacagggtc aagagaactg gcgatggaag ccatcatctc tggggctggg gtgatgagaa 12420
tcagagcaaa gaagagatag atgcagccat taggatcaac cctaaagacg attgagtagt 12480
tagcttaatc acctagagct cggtcaccag tatgaactaa aatgcatgta ggtgtaagag 12540
ctcatggaga gcatggaata ttgtatccga ccatgtaaca gtataataac tgagctccat 12600
ctcacttctt ctatgaataa acaaaggatg ttatgatata ttaacactct atctatgcac 12660
cttattgttc tatgataaat ttcctcttat tattataaat catctgaatc gtgacggctt 12720
atggaatgct tcaaatagta caaaaacaaa tgtgtactat aagactttct aaacaattct 12780
aactttagca ttgtgaacga gacataagtg ttaagaagac ataacaatta taatggaaga 12840
agtttgtctc catttatata ttatatatta cccacttatg tattatatta ggatgttaag 12900
gagacataac aattataaag agagaagttt gtatccattt atatattata tactacccat 12960
ttatatatta tacttatcca cttatttaat gtctttataa ggtttgatcc atgatatttc 13020
taatatttta gttgatatgt atatgaaaag gtactatttg aactctctta ctctgtataa 13080
aggttggatc atccttaaag tgggtctatt taattttatt gcttcttaca gataaaaaaa 13140
aaattatgag ttggtttgat aaaatattga aggatttaaa ataataataa ataataaata 13200
acatataata tatgtatata aatttattat aatataacat ttatctataa aaaagtaaat 13260
attgtcataa atctatacaa tcgtttagcc ttgctggaac gaatctcaat tatttaaacg 13320
agagtaaaca tatttgactt tttggttatt taacaaatta ttatttaaca ctatatgaaa 13380
tttttttttt ttatcagcaa agaataaaat taaattaaga aggacaatgg tgtcccaatc 13440
cttatacaac caacttccac aagaaagtca agtcagagac aacaaaaaaa caagcaaagg 13500
aaattttta atttgagttg tcttgtttgc tgcataattt atgcagtaaa acactacaca 13560
taacccttt agcagtagag caatggttga ccgtgtgctt agcttcttt attttatttt 13620
tttatcagca aagaataaat aaaataaaat gagacacttc agggatgttt caacccttat 13680

acaaaacccc aaaaacaagt ttcctagcac cctaccaacg aattcgcggc cgcttaatta 13740
agatgagtga tactcaggac tcaggactca ctctgctgat cactagtgct agcctcgagg 13800
tcgaccagct tt 13812

<210> 76
<211> 1434
<212> DNA
<213> Magnaporthe grisea

<400> 76

gaattcatgg cttcgtcatc ttcctccgtg ccggagttgg ctgccgcctt ccctgatggc 60
actaccgact tcaagcccat gaggaacacc aagggctacg acgtcagcaa gccgcacatt 120
tccgagacac ctatgacact caagaactgg cataagcacg tcaactggct caacaccacc 180
ttcatcttgt ttgtgcccct ggctggtctc atatccactt actgggtccc tctgcagtgg 240
aagacggctg tatgggctgt cgtctactac ttcaacaccg gcctgggaat tactgccggt 300
taccaccgac tttgggctca cagctcgtac aaggcctcgc ttccgctcaa aatctacctt 360
gccgccgttg gcgctggtgc cgtcgagggc tccatcagat ggtggtccaa cggtcaccgc 420
gcacaccacc gatacaccga taccgagaag gacccctact cagtccgcaa gggtctcctg 480
tactcacaca tgggatggat gcttctgaag cagaacccca agaagcaggg ccgcaccgac 540
atcaccgacc tgaacgagga ccccgttgtc gtttggcagc accgcaactt cctcaagtgt 600
gttatcttca tggccctcgt cttccccaca cttgtggctg gccttggctg gggtgactac 660
tggggaggtt tcatctacgg aggtattctg cgtgtcttct tcgtccagca ggccaccttc 720
tgcgtcaact cgcttgccca ctggctcggt gaccagcctt tcgacgatcg caactcgccg 780
cgtgatcacg tcatcacagc cctggtcacc cttggagagg gataccacaa cttccaccac 840
gagttccctt cggactaccg caacgctatt gagtggtacc agtatgaccc caccaagtgg 900
tcaatctgga tctggaagca gcttggtctt gcccacaacc tgaagcagtt ccgccaaaac 960
gagattgaga agggacgcgt ccagcagctg cagaagaagc tcgaccagaa gcgcgccaag 1020
cttgattggg gtattccctt ggagcagctt cccgttgtta gctgggatga ctttgttgag 1080
cagtccaaga acggaaaggc ttggattgca gttgccggtg tcatccacga tgttggtgac 1140
ttcatcaagg accaccctgg tggcagagct ctcatcaact cggccattgg caaggacgca 1200
accgcaatct tcaacggcgg tgtttacaac cactccaacg ccgctcacaa cctgctctcg 1260
actatgcgtg tgggtgtttt gcgtggcggc tgcgaggttg agatctggaa gcgcgcccag 1320
tccgaaaaca aggacgtctc aaccgtcgtt gattcttcgg gtaaccgcat cgtccgcgcg 1380
ggtgggcaag cgaccaaggt cgtccagcct gttccgggtg ctcaggccgc gtga 1434

<210> 77
<211> 212
<212> PRT

<213> Leptosphaeria nodorum

<400> 77

```
Tyr Tyr Phe Cys Thr Ala Phe Gly Ile Thr Gly Gly Tyr His Arg Leu
1               5                   10                  15

Trp Ser His Arg Ser Tyr Ser Ala Arg Leu Pro Leu Arg Leu Phe Leu
            20                  25                  30

Ala Phe Thr Gly Ala Gly Ala Ile Gln Gly Ser Ala Arg Trp Trp Ser
        35                  40                  45

Ala Asn His Arg Ala His His Arg Trp Thr Asp Thr Met Lys Asp Pro
    50                  55                  60

Tyr Ser Val Met Arg Gly Leu Leu Phe Ser His Ile Gly Trp Met Val
65                  70                  75                  80

Leu Asn Ser Asp Pro Lys Val Lys Gly Arg Thr Asp Val Ser Asp Leu
                85                  90                  95

Asp Ser Asp Pro Val Val Val Trp Gln His Lys His Tyr Gly Lys Cys
            100                 105                 110

Leu Leu Phe Ala Ala Trp Ile Phe Pro Met Ile Val Ala Gly Leu Gly
        115                 120                 125

Trp Gly Asp Trp Trp Gly Gly Leu Val Tyr Ala Gly Ile Ile Arg Ala
    130                 135                 140

Cys Phe Val Gln Gln Ala Thr Phe Cys Val Asn Ser Leu Ala His Trp
145                 150                 155                 160

Ile Gly Glu Gln Pro Phe Asp Asp Arg Arg Thr Pro Arg Asp His Val
                165                 170                 175

Leu Thr Ala Leu Val Thr Met Gly Glu Gly Tyr His Asn Phe His His
            180                 185                 190

Glu Phe Pro Ser Asp Tyr Arg Asn Ala Ile Ile Trp Tyr Gln Tyr Asp
        195                 200                 205

Pro Thr Lys Trp
    210
```

<210> 78
<211> 216
<212> PRT
<213> Helicoverpa zea

<400> 78

```
Leu Tyr Val Ile Ser Gly Leu Gly Ile Thr Ala Gly Ala His Arg Leu
1               5                   10                  15

Trp Ala His Lys Ser Tyr Lys Ala Lys Trp Pro Leu Arg Val Ile Leu
            20                  25                  30

Val Ile Phe Asn Thr Val Ala Phe Gln Asp Ala Ala Met Asp Trp Ala
        35                  40                  45

Arg Asp His Arg Met His His Lys Tyr Ser Glu Thr Asp Ala Asp Pro
    50                  55                  60

His Asn Ala Thr Arg Gly Phe Phe Phe Ser His Ile Gly Trp Leu Leu
65                  70                  75                  80

Val Arg Lys His Pro Asp Leu Lys Glu Lys Gly Lys Gly Leu Asp Met
                85                  90                  95

Ser Asp Leu Leu Ala Asp Pro Ile Leu Arg Phe Gln Lys Lys Tyr Tyr
            100                 105                 110

Leu Ile Leu Met Pro Leu Ala Cys Phe Val Met Pro Thr Val Ile Pro
        115                 120                 125

Val Tyr Phe Trp Gly Glu Thr Trp Thr Asn Ala Phe Phe Val Ala Ala
    130                 135                 140

Met Phe Arg Tyr Ala Phe Ile Leu Asn Val Thr Trp Leu Val Asn Ser
145                 150                 155                 160

Ala Ala His Lys Trp Gly Asp Lys Pro Tyr Asp Lys Ser Ile Lys Pro
                165                 170                 175

Ser Glu Asn Leu Ser Val Ala Met Phe Ala Leu Gly Glu Gly Phe His
            180                 185                 190

Asn Tyr His His Thr Phe Pro Trp Asp Tyr Lys Thr Ala Glu Leu Gly
        195                 200                 205

Asn Asn Lys Leu Asn Phe Thr Thr
    210                 215
```